(19) Europäisches Patentamt
European Patent Office
Office européen des brevets

(11) EP 4 442 828 A1

(12) EUROPEAN PATENT APPLICATION
published in accordance with Art. 153(4) EPC

(43) Date of publication:
**09.10.2024 Bulletin 2024/41**

(21) Application number: **22901261.2**

(22) Date of filing: **29.11.2022**

(51) International Patent Classification (IPC):
***C12N 15/62*** (2006.01) ***A61K 31/4745*** (2006.01)
***A61K 39/395*** (2006.01) ***A61K 45/00*** (2006.01)
***A61K 47/68*** (2017.01) ***A61P 35/00*** (2006.01)
***A61P 43/00*** (2006.01) ***C07K 7/08*** (2006.01)
***C07K 16/18*** (2006.01) ***C07K 19/00*** (2006.01)
***C12N 9/64*** (2006.01) ***C12N 15/13*** (2006.01)
***C12P 21/02*** (2006.01) ***C40B 40/10*** (2006.01)

(52) Cooperative Patent Classification (CPC):
**A61K 31/4745; A61K 39/395; A61K 45/00; A61K 47/68; A61P 35/00; A61P 43/00; C07K 7/08; C07K 16/00; C07K 16/18; C07K 19/00; C12N 9/64; C12N 15/62; C12P 21/02; C40B 40/10**

(86) International application number:
**PCT/JP2022/043846**

(87) International publication number:
**WO 2023/100829 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2021 JP 2021194701**

(71) Applicant: **Daiichi Sankyo Company, Limited**
**Tokyo 103-8426 (JP)**

(72) Inventors:
- **KUDO Shota**
**Tokyo 103-8426 (JP)**
- **ISHIZUKA Mikihiro**
**Tokyo 103-8426 (JP)**
- **KAMEI Reiko**
**Tokyo 103-8426 (JP)**
- **TERAUCHI Tomoko**
**Tokyo 103-8426 (JP)**
- **SAEKI Kazunori**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) PROTEASE-CLEAVABLE MASKED ANTIBODIES

(57) This invention provides a novel masked antibody. The masked antibody is a molecule that binds to a target antigen, which comprises a moiety binding to a target antigen, a first peptide recognizing a target antigen-binding site comprised in such moiety, and a second peptide comprising an amino acid sequence cleaved by a protease, wherein, after the second peptide is cleaved by a protease, the molecule has higher binding intensity to the target antigen, compared with that before it is cleaved.

EP 4 442 828 A1

Fig. 1

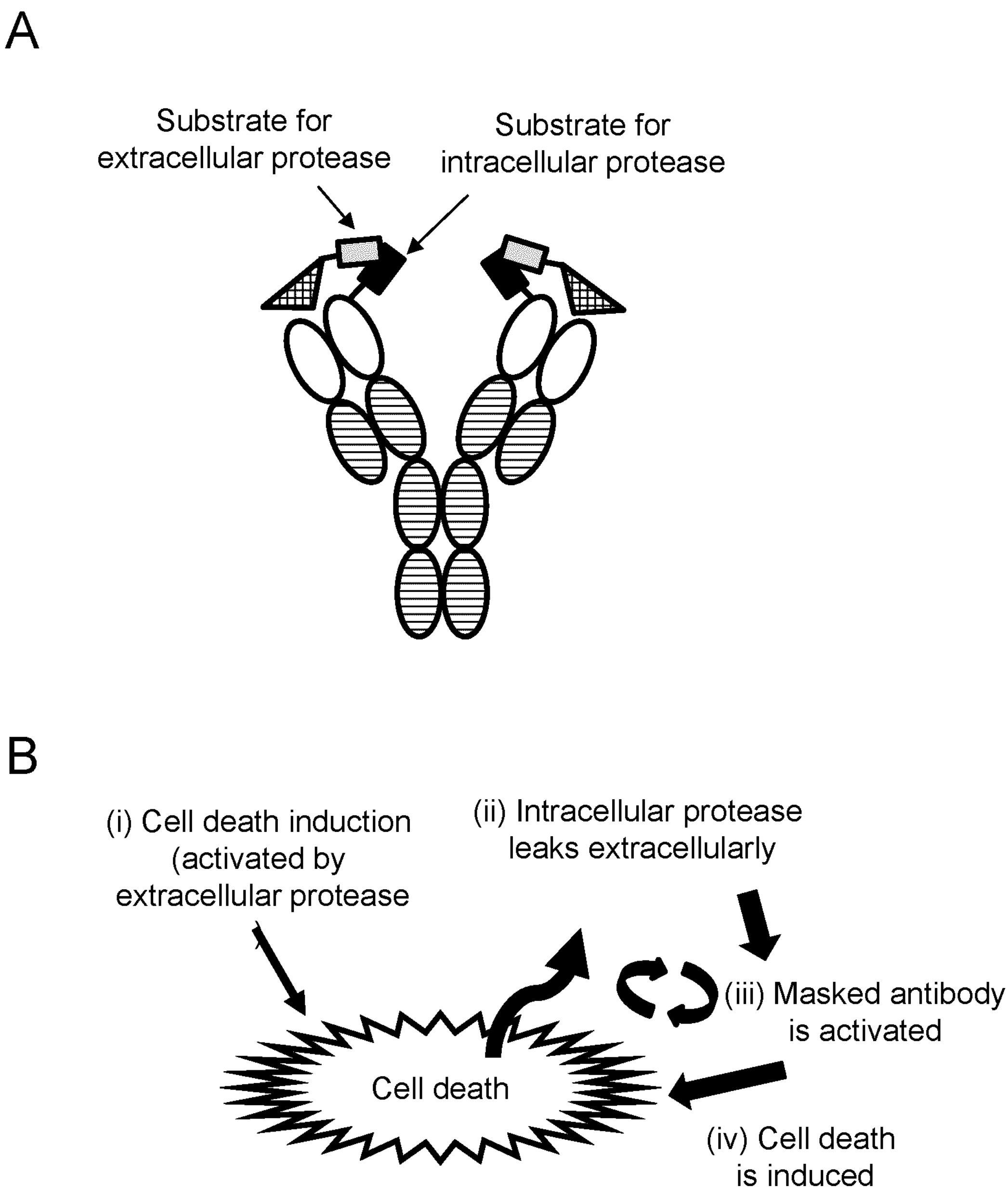
A
Substrate for
extracellular protease
Substrate for
intracellular protease
B
(i) Cell death induction
(activated by
extracellular protease
(ii) Intracellular protease
leaks extracellularly
(iii) Masked antibody
is activated
Cell death
(iv) Cell death
is induced

## Description

Technical Field

The present invention relates to protease-cleavable masked antibodies activated by the action of proteases.

Background Art

Antibodies have very high recognition specificity or binding intensity to antigens; i.e., target molecules, and a wide variety of molecules such as low-molecular-weight compounds, peptides, and proteins, can be antigens. In addition, antibody drugs can exert high productivity and stability *in vivo,* and, therefore, development thereof targeting a wide variety of diseases, such as cancer, immunity disorders, and infections, has been in progress. In recent years, research and development of antibody drugs with higher efficacy, such as antibody drug conjugates (ADC) comprising cytotoxic agents bound to antibodies and bispecific antibodies capable of crosslinking target cells to cytotoxic T cells to attack the target cells (T-cell engaging (TCE)), have been in progress. ADC and TCE exert potent antitumor activity. When target molecules are expressed in healthy tissue, however, ADC and TCE become toxic on the healthy tissue through the target molecules (Non-Patent Literature 1).

Modified antibodies that specifically act on tumor tissue with the utilization of the properties of the tumor microenvironment (e.g., enhanced protease activity in tumor tissue); that is, masked antibodies, have been known. Since binding intensity of masked antibodies is suppressed in healthy tissue, masked antibodies are expected as antibody drugs with high safety because of the low toxicity on healthy tissue through the target molecule (Non-Patent Literature 2). A masked antibody is composed of an antibody, a masking domain for inhibiting antibody binding, and a cleavable linker linking the antibody to the masking domain, which can be cleaved by a protease (Patent Literature 1). As masking domains, for example, a coiled-coil domain that does not directly interact with an antibody has been known in addition to a mimotope peptide that binds to a complementarity determining region (CDR) of an antibody (Non-Patent Literature 3). A cleavable linker comprises substrates for extracellular proteases exhibiting enhanced activity in tumor tissue, such as matrix metalloproteinase (MMP) and urokinase type plasminogen activator (uPA). This enables activation of tumor-tissue-selective antibodies (Patent Literature 2). In the preceding study on masking of the anti-EGFR antibody (Cetuximab), for example, activation of masked antibodies in tumor tissue, lowering in the exposure to the skin where EGFR is expressed, the prolonged blood half life, and improved safety have been reported (Non-Patent Literature 4). In addition, masking techniques have been applied to a wide variety of biological molecules, such as bispecific antibodies or cytokines (Patent Literatures 3 and 4).

Masked antibodies are activated by proteases localized extracellularly (membrane type or secretory type). In addition to matrix metalloproteinase (MMP) and urokinase type plasminogen activator (uPA), a wide variety of extracellular proteases including matriptase (MT-SP1) and cathepsin (CTS) are known to show enhanced activity in tumor tissue (Non-Patent Literature 5). Legumain (LGMN) that is known to be localized in the lysosome in the cell is reported to be present outside the cell in the tumor environment (Non-Patent Literature 6). Such proteases are known to be secreted extracellularly from cancer cells or stromal cells existing in the vicinity of cancer cells and involved in a wide variety of processes including survival, proliferation, infiltration, and metastasis of cancer cells. Masked antibodies are activated by the proteases existing outside the cell and act in a tumor-tissue-selective manner.

Protein-cleaving proteases are also present in the cell (in the cytoplasm) and they are involved in a wide variety of biological processes, such as amino acid metabolism, signal transmission, immunity, and apoptosis, as with the ubiquitin proteasome or autophagy lysosome system. In addition, intracytoplasmic proteases are reported to leak to the outside of cells upon cell death or a damage imposed on a cell membrane (Non-Patent Literature 7).

Citation List

Patent Literature

Patent Literature 1: WO 2009/025846
Patent Literature 2: WO 2010/081173
Patent Literature 3: WO 2016/014974
Patent Literature 4: WO 2020/069398

Non Patent Literature

Non Patent Literature 1: Nature Review Drug Discovery; 17: 197-223, 2018
Non Patent Literature 2: Expert Opin. Biol. Ther.; 14 (8): 1049-53, 2014
Non Patent Literature 3: ACS Cent. Sci.; 7: 724-38, 2021
Non Patent Literature 4: Sci. Transl. Med.; 5, 207: 1-10, 2013
Non Patent Literature 5: Annual Review of Cancer Biology ;2: 353-76, 2018
Non Patent Literature 6: Scientific Reports; 5 (16599): 1-9, 2015
Non Patent Literature 7: Anal. Biochem.; 366 (2): 197-206, 2007

Summary of Invention

Technical Problem

The present invention provides masked antibodies with performance superior to that of conventional masked antibodies.

Solution to Problem

The present inventors have conducted concentrated studies in order to dissolve the problems described above. As a result, they succeeded in improving masked antibodies with the use of intracytoplasmic proteases comprising protein-cleaving proteases in the cytoplasm and leaking from tumor cells upon cell death or a damage imposed on a cell membrane. This has led to the completion of the present invention.

Specifically, the present invention includes the following.

[1] A molecule comprising a moiety [a], a moiety [b], and a moiety [c] indicated below and binding to a target antigen:

a moiety [a]: a moiety binding to the target antigen;
a moiety [b]: a first peptide recognizing a target antigen-binding site included in the moiety [a]; and
a moiety [c]: a second peptide comprising an amino acid sequence cleaved by a protease localized in the cytoplasm.

[2] The molecule according to [1], wherein the second peptide has higher binding affinity to the target antigen after it is cleaved by a protease localized in the cytoplasm than that before it is cleaved.
[3] The molecule according to [1] or [2], wherein the second peptide in the moiety [c] further comprises an amino acid sequence cleaved by an extracellular protease.
[4] The molecule according to any of [1] to [3], wherein the first peptide, the second peptide, and the moiety binding to the target antigen are connected in that order.
[5] The molecule according to any of [1] to [4], wherein the moiety binding to the target antigen is a polypeptide consisting of an amino acid sequence that is not comprised in the first peptide or the second peptide.
[6] The molecule according to any of [1] to [5], which consists of a polypeptide.
[7] The molecule according to [6], which comprises the first peptide, the second peptide, and the moiety binding to the target antigen or the moiety binding to the target antigen, the second peptide, and the first peptide are connected in that order from the amino terminus toward the carboxyl terminus.
[8] The molecule according to any of [1] to [7], wherein a moiety selected from the group consisting of the first peptide, the second peptide, and the moiety binding to a target antigen is ligated to either or both of the other two moieties via linker (or linkers).
[9] The molecule according to any of [1] to [8], wherein the protease localized in the cytoplasm leaks extracellularly upon cell death or a damage imposed on a cell membrane.
[10] The molecule according to any one of [1] to [9], wherein the protease localized in the cytoplasm is selected from among calpain, caspase, and tripeptidyl peptidase and it is preferable that calpain be calpain 1 or calpain 2, caspase be caspase 1, caspase 8, caspase 3, or caspase 7, and tripeptidyl peptidase be tripeptidyl peptidase 1 or tripeptidyl peptidase 2.
[11] The molecule according to any one of [1] to [10], wherein the protease localized in the cytoplasm is a calcium-dependent or calcium-requiring protease.
[12] The molecule according to any one of [1] to [11], wherein the protease localized in the cytoplasm is calpain 1

or calpain 2.
[13] The molecule according to any of [3] to [12], wherein the extracellular protease is expressed at a higher level, exists in a larger amount, or has higher catalytic activity in diseased tissue than in healthy tissue.
[14] The molecule according to [13], wherein the diseased tissue is tumor tissue and/or stromal tissue.
[15] The molecule according to any one of [3] to [14], wherein the extracellular protease is selected from the group consisting of matrix metalloproteinase, urokinase type plasminogen activator, matriptase, legumain, and cathepsin and it is preferable that the matrix metalloproteinase be at least one substance selected from among MMP1, MMP2, MMP3, MMP7, MMP9, MMP12, and MMP14 and that the cathepsin be at least one substance selected from among cathepsin B, cathepsin D, cathepsin S, and cathepsin L.
[16] The molecule according to any one of [1] to [15], wherein the second peptide comprises an amino acid sequence cleaved by the extracellular protease and the amino acid sequence cleaved by the protease localized in the cytoplasm or an amino acid sequence cleaved by a protease localized in the cytoplasm and an amino acid sequence cleaved by the extracellular protease located in that order from the amino terminus toward the carboxyl terminus, preferably, an amino acid sequence cleaved by the extracellular protease and an amino acid sequence cleaved by the protease localized in the cytoplasm located in that order from the amino terminus toward the carboxyl terminus.
[17] The molecule according to any one of [1] to [16], wherein the amino acid sequence cleaved by the protease localized in the cytoplasm comprises at least one sequence selected from among SEQ ID NOs: 12 and 44 to 53.
[18] The molecule according to any one of [1] to [17], wherein the target antigen is a tumor antigen.
[19] The molecule according to any one of [1] to [18], which comprises another moiety [d] that binds to a moiety binding to the target antigen and the moiety [d] does not comprise the first peptide or the second peptide.
[20] The molecule according to [19], wherein the moiety [d] is at least one substance selected from the group consisting of an antibody that is not a moiety binding to the target antigen or an antigen-binding fragment thereof, a peptide comprising an amino acid sequence that is not comprised in the first peptide or the second peptide, a cytokine, a toxin, a radioactive isotope, a label molecule, a photosensitive substance, an immunostimulant, an antitumor compound, a drug, a payload, and a polymer.
[21] The molecule according to [20], wherein the antitumor compound is a camptothecin derivative or a pyrrolobenzodiazepine derivative, preferably, the camptothecin derivative be N-[(1 S,9S)-9-ethyl-5-fluoro-9-hydroxy-4-methyl-10, 13-dioxo-2,3,9, 10, 13, 15-hexahydro-1H,12H-benzo[de]pyrano[3',4':6,7]indolizino[1,2-b]quinolin-1-yl]-2-hydroxyacetamide.
[22] The molecule according to [20], wherein the immunostimulant is a cyclic dinucleotide derivative.
[23] The molecule according to any one of [19] to [22], which consists of a polypeptide or the moiety [d] and a polypeptide.
[24] A method for identifying a peptide binding to a complementarity determining region (CDR) comprised in an antibody or an antigen-binding fragment of an antibody comprising a step (i) and a step (ii):

(i) a step of bringing a peptide library comprising a repeat sequence of aromatic amino acid and Pro into contact with the CDR; and
(ii) a step of collecting a peptide which binds to the CDR.

[25] The method according to [24], wherein the repeat sequence of aromatic amino acid and Pro is a ZPZP motif (wherein Z represents any aromatic amino acid selected from among histidine (His), phenylalanine (Phe), tyrosine (Tyr), and tryptophan (Trp); and P represents proline).
[26] The method according to [24] or [25], which further comprises a step of preparing the peptide by recombination, *in vitro* translation, chemical synthesis, or peptide synthesis.
[27] A molecule binding to a target antigen, wherein the CDR is comprised in the moiety that binds to the target antigen as defined in [1] and the first peptide is obtained by the method according to [26].
[28] A method for producing the molecule according to any one of [1] to [23], which comprises a step of preparing a peptide comprising an amino acid sequence comprised in the first peptide and/or an amino acid sequence comprised in the second peptide by recombination, *in vitro* translation, chemical synthesis, or peptide synthesis.
[29] A molecule binding to a target antigen, which is obtained by the method according to [28].
[30] A method for producing the molecule according to [6], [7], or [23], which comprises a step (iii):
(iii) a step of introducing a polynucleotide comprising a nucleotide sequence encoding an amino acid sequence comprised in a moiety that binds to the target antigen and optionally an amino acid sequence comprised in the first peptide, and/or an amino acid sequence comprised in the second peptide into a cell, culturing the cell, and collecting a polypeptide binding to the target antigen from the culture product.
[31] A molecule binding to the target antigen, which is obtained by the method according to [30].
[32] A pharmaceutical composition comprising the molecule according to any one of [1] to [23], [27], [29], and [31], a salt thereof, or a hydrate of the molecule or salt.

[33] The pharmaceutical composition according to [32], which is an anti-cancer agent.

[34] A peptide library comprising a repeat sequence of aromatic amino acid and Pro.

[35] The peptide library according to [34], wherein the repeat sequence of aromatic amino acid and Pro is a ZPZP motif (wherein Z represents any aromatic amino acid selected from among histidine (His), phenylalanine (Phe), tyrosine (Tyr), and tryptophan (Trp); and P represents proline).

The description incorporates the contents disclosed by JP Patent Application No. 2021-194701, based on which the priority of the present application claims.

Advantageous Effects of Invention

According to the present invention, masked antibodies are activated by the action of proteases in the tumor environment, tumor specificity of the masked antibodies are improved, and the masked antibodies exert excellent effects as antitumor agents.

Brief Description of Drawings

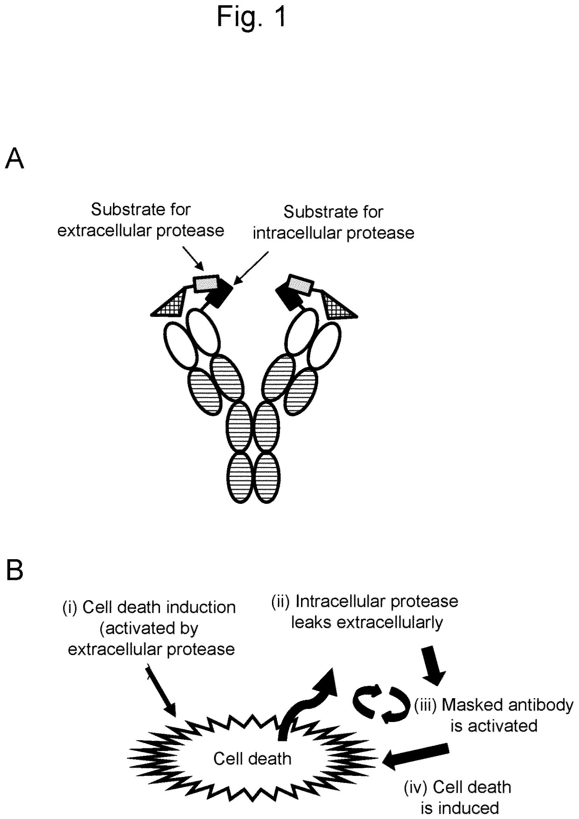

[Figure 1] Figure 1 shows a concept of the present invention. (A) shows an example of a format of a masked antibody that can be activated by an extracellular protease and an intracellular protease. A masking domain (lattice) binding to a variable region (white) of an antibody is fused to a variable region of an antibody heavy chain through a cleavable linker. A linker comprises a sequence (gray) of a substrate for an extracellular protease and a sequence (black) of a substrate for an intracellular protease. A variable region of an antibody is fused to a constant region (horizontal line). (B) shows activation of a masked antibody with the use of an intracellular protease. (1) to (4) each show that cell death is induced by the action of the masked antibody activated by the extracellular protease, the intracellular protease leaks extracellularly from the dead cell, the masked antibody is activated by the intracellular protease, and the masked antibody activated by the intracellular protease exerts cytotoxic activity.

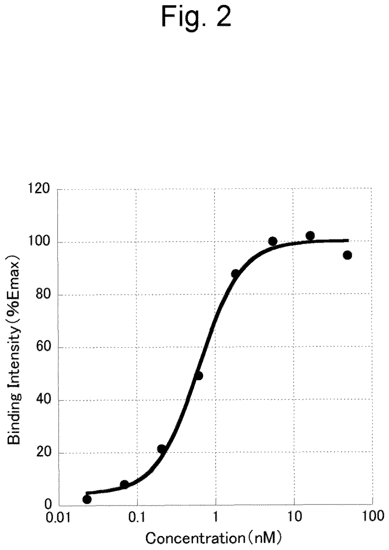

[Figure 2] Figure 2 shows the results of ELISA evaluation of the interaction between the conventional anti-TROP2 antibody HT1-11 described in WO 2015/098099 and the human TROP2 antigen. HT1-11 was found to have bound to the antigen in a concentration-dependent manner.

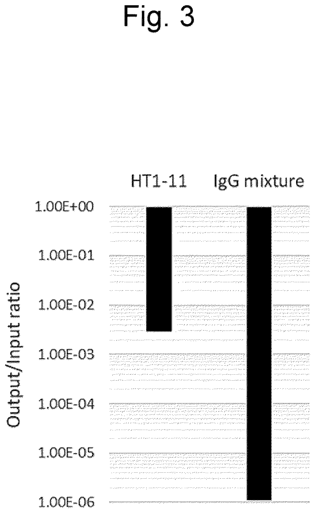

[Figure 3] Figure 3 shows a degree of concentration of peptide binders using the output/input ratio after panning of the anti-TROP2 antibody HT 1-11 evaluated by a pull-down experiment. A higher value in HT1-11 than in human serum-derived IgG (IgG mixture) indicates concentration of the peptides binding specifically to HT1-11.

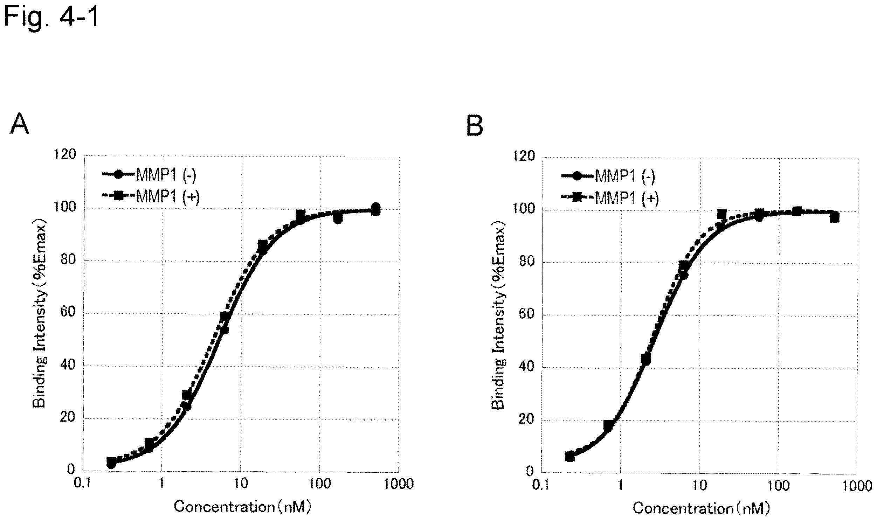

[Figure 4-1] Figure 4-1 shows the results of ELISA evaluation of the interaction between the scFv-type anti-TROP2 antibody and the human TROP2 antigen. Binding intensity was evaluated under the condition without the addition of MMP (solid line) and under the condition with the addition of M1VVIP1 (dotted line). (A) HT1-11-scFv-HL exhibited equivalent binding intensity regardless of the addition of MMP. (B) HT1-11-scFv-LH exhibited equivalent binding intensity regardless of the addition of MMP.

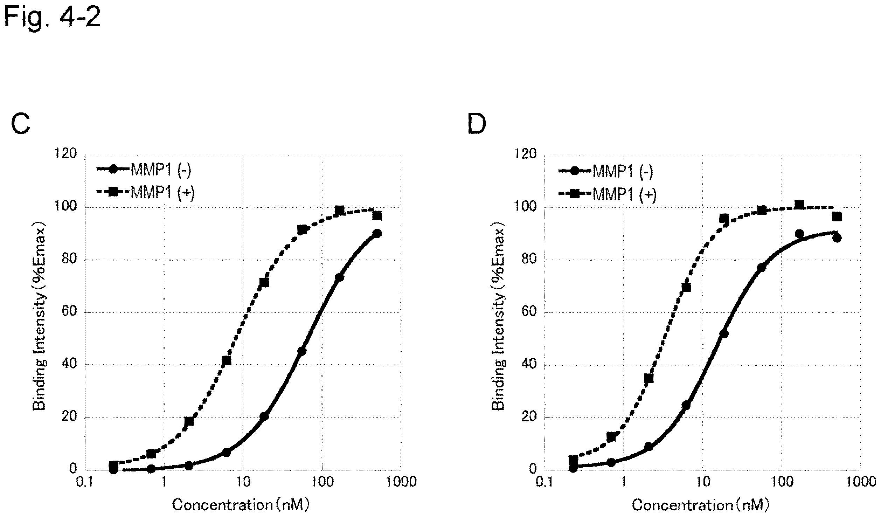

[Figure 4-2] Figure 4-2 shows the results of ELISA evaluation of the interaction between the scFv-type anti-TROP2 antibody and the human TROP2 antigen. Binding intensity was evaluated under the condition without the addition of MMP (solid line) and under the condition with the addition of MMP1 (dotted line). (C) MHT1001 exhibited higher binding intensity under the condition with the addition of MMP1 than under the condition without the addition of MMP1. (D) MHT1002 exhibited higher binding intensity under the condition with the addition of MMP1 than under the condition without the addition of MMP 1.

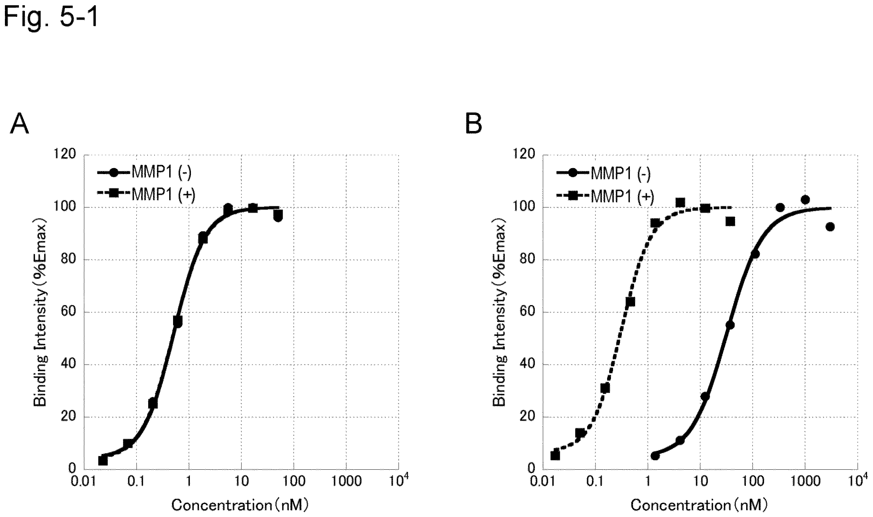

[Figure 5-1] Figure 5-1 shows the results of ELISA evaluation of the interaction between the IgG-type anti-TROP2 masked antibody and the human TROP2 antigen. Binding intensity was evaluated under the condition without the addition of a protease (solid line) and under the condition with the addition of a protease (dotted line). (A) HT1-11 exhibited equivalent binding intensity regardless of the addition of MMP. (B) MHT1007 exhibited higher binding intensity under the condition with the addition of MMP1 than under the condition without the addition of MMP1.

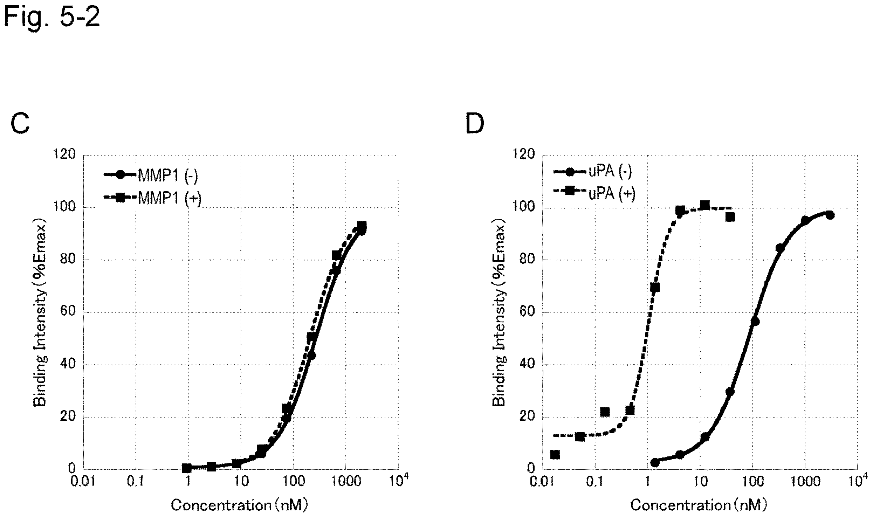

[Figure 5-2] Figure 5-2 shows the results of ELISA evaluation of the interaction between the IgG-type anti-TROP2 masked antibody and the human TROP2 antigen. Binding intensity was evaluated under the condition without the addition of a protease (solid line) and under the condition with the addition of a protease (dotted line). (C) MHT1008 exhibited equivalent binding intensity regardless of the addition of MMP. (D) MHT1009 exhibited higher binding intensity under the condition with the addition of uPA than under the condition without the addition of uPA.

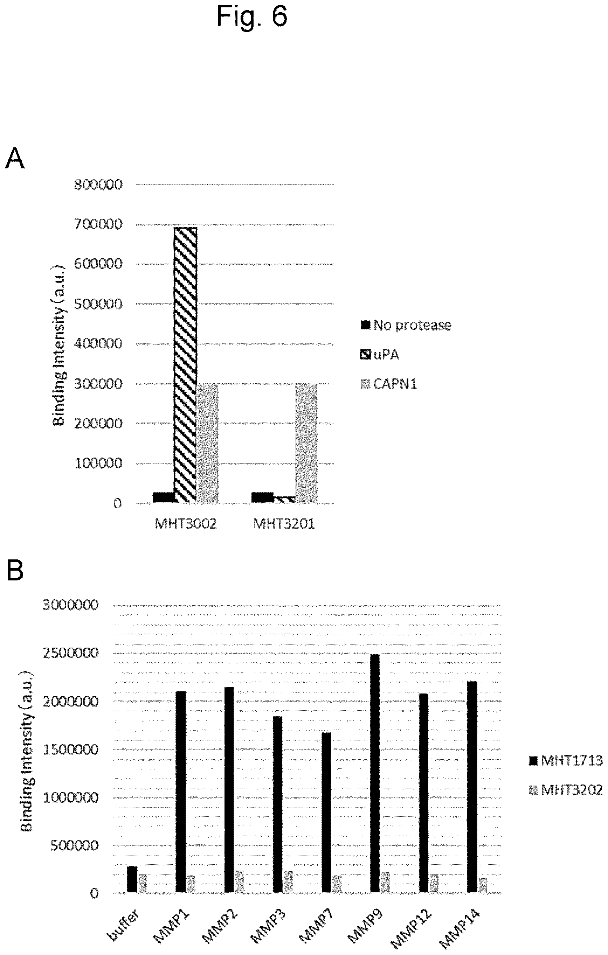

[Figure 6] Figure 6 shows the results of ELISA evaluation of the interaction between the anti-TROP2 masked antibody comprising the CAPN substrate and the human TROP2 antigen. (A) MHT3002 exhibited higher binding intensity under the condition with the addition of uPA or CAPN1 than under the condition without the addition of a protease.

MHT3201 exhibited higher binding intensity selectively under the condition with the addition of CAPN1 than under the condition without the addition of a protease. (B) MHT1713 exhibited higher binding intensity under the condition with the addition of various types of MMPs than under the condition without the addition of MMP. MHT3202 exhibited equivalent binding intensity under any conditions as with the condition without the addition of MMP.

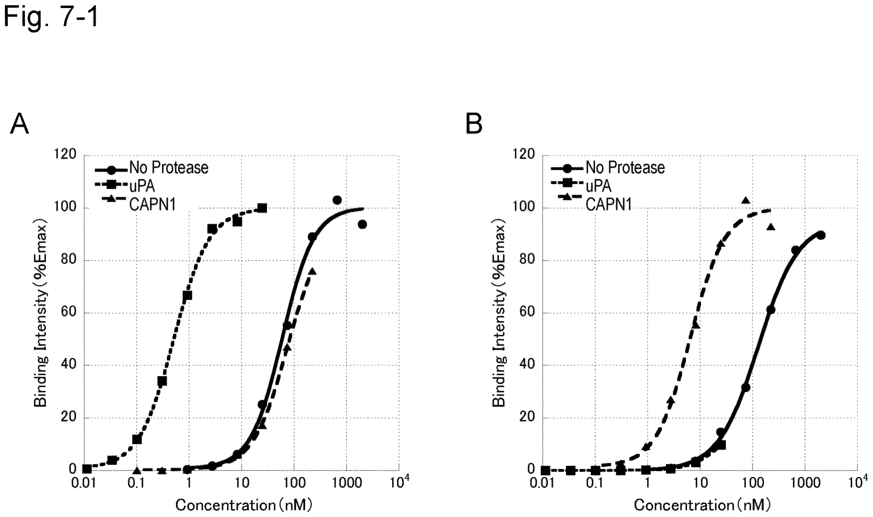

[Figure 7-1] Figure 7-1 shows the results of ELISA evaluation of the interaction between the anti-TROP2 masked antibody comprising the uPA substrate or the CAPN substrate, and the human TROP2 antigen. Binding intensity was evaluated under the condition without the addition of a protease (solid line), under the condition with the addition of uPA (dotted line), and under the condition with the addition of CAPN1 (broken line). (A) MHT3423 exhibited higher binding intensity selectively under the condition with the addition of uPA than under the condition without the addition of a protease. (B) MHT3201 exhibited higher binding intensity selectively under the condition with the addition of CAPN1 than under the condition without the addition of a protease.

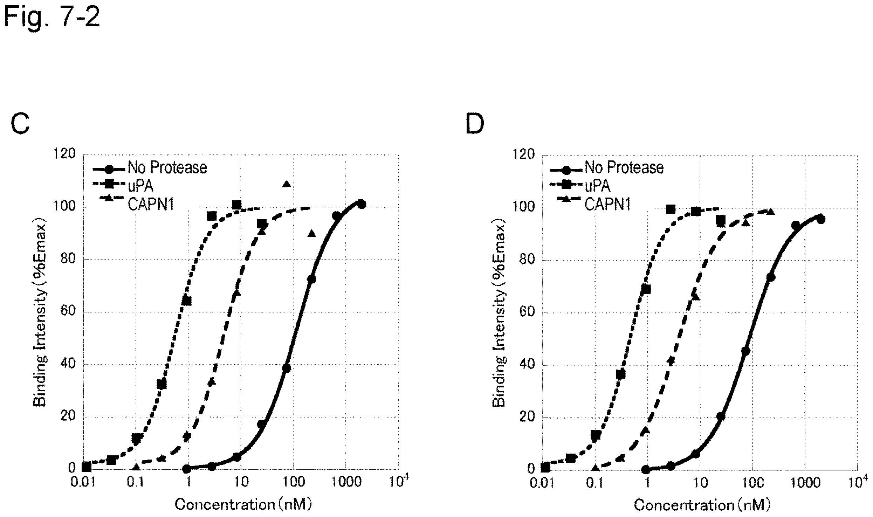

[Figure 7-2] Figure 7-2 shows the results of ELISA evaluation of the interaction between the anti-TROP2 masked antibody comprising the uPA substrate or the CAPN substrate, and the human TROP2 antigen. Binding intensity was evaluated under the condition without the addition of a protease (solid line), under the condition with the addition of uPA (dotted line), and under the condition with the addition of CAPN1 (broken line). (C) MHT3202 exhibited higher binding intensity under the condition with the addition of uPA and CAPN 1 than under the condition without the addition of a protease. (D) MHT3203 exhibited higher binding intensity under the condition with the addition of uPA and CAPN 1 than under the condition without the addition of a protease.

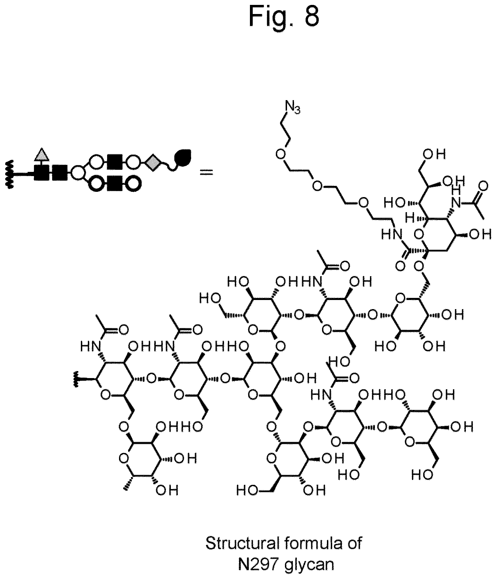

[Figure 8] Figure 8 shows the N297 glycan of a glycan-modified antibody (the MSG1-type glycan comprising an azide group introduced into a sialic acid at the non-reducing terminus (WO 2019/065964)).

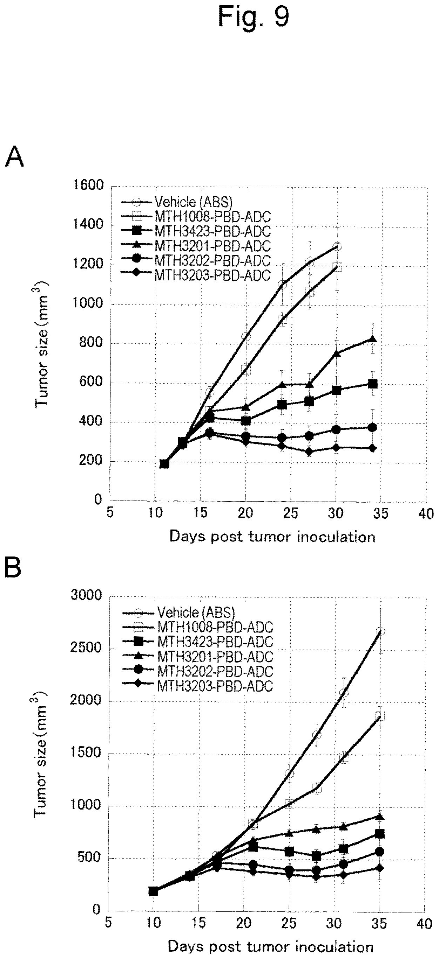

[Figure 9] Figure 9 shows antitumor activity of MHT1008-PBD-ADC (open square), MHT3423-PBD-ADC (closed square), MHT3201-PBD-ADC (up-pointing closed triangle), MHT3202-PBD-ADC (closed circle), MHT3203-PBD-ADC (closed rhomboid), and Vehicle (ABS) (circle) in mouse models transplanted with (A) the TROP2-positive human lung mucoepidermoid carcinoma cell line NCI-H292 or (B) the TROP2-positive human pharyngeal carcinoma cell line FaDu. An error bar in the figure indicates a standard error (n = 6).

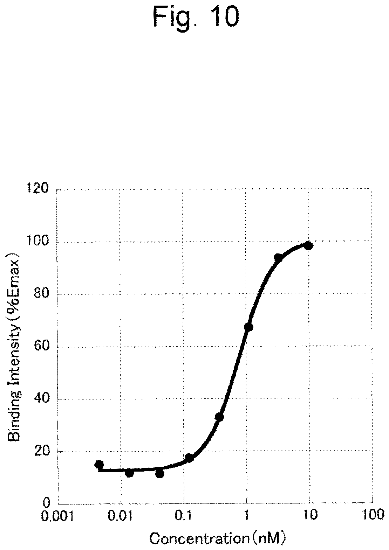

[Figure 10] Figure 10 shows the results of ELISA evaluation of the interaction between the conventional anti-CD98 antibody hM23H1L1 described in WO 2015/146132 and the human CD98 antigen. hM23H1L1 was found to have bound to the antigen in a concentration-dependent manner.

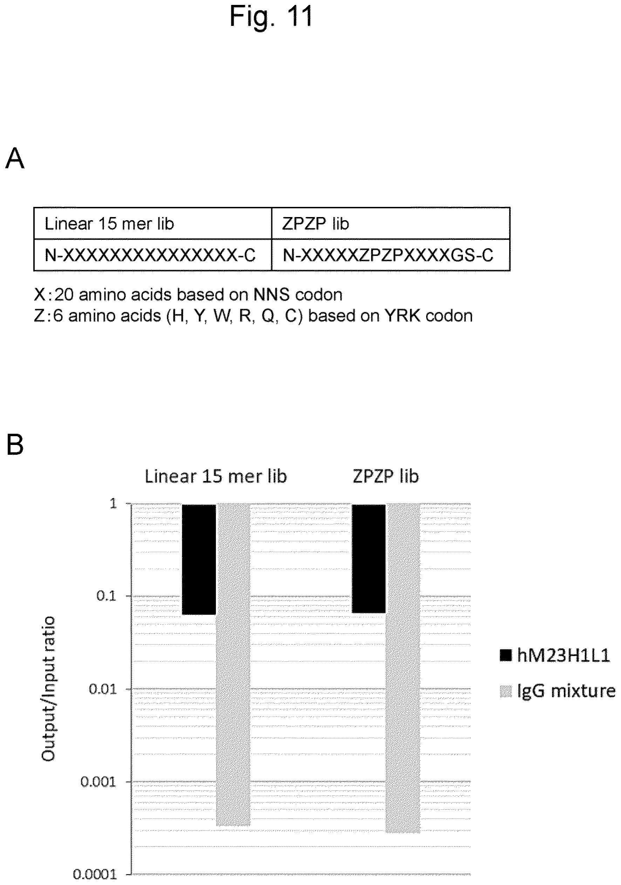

[Figure 11] Figure 11 shows (A) types of peptide libraries and (B) a degree of concentration of peptide binders using the output/input ratio after panning of the anti-CD98 antibody hM23H1L1 evaluated by a pull-down experiment. A higher value in hM23H1L1 than in human serum-derived IgG (IgG mixture) indicates concentration of the peptide bindings specifically to hM23H1L1.

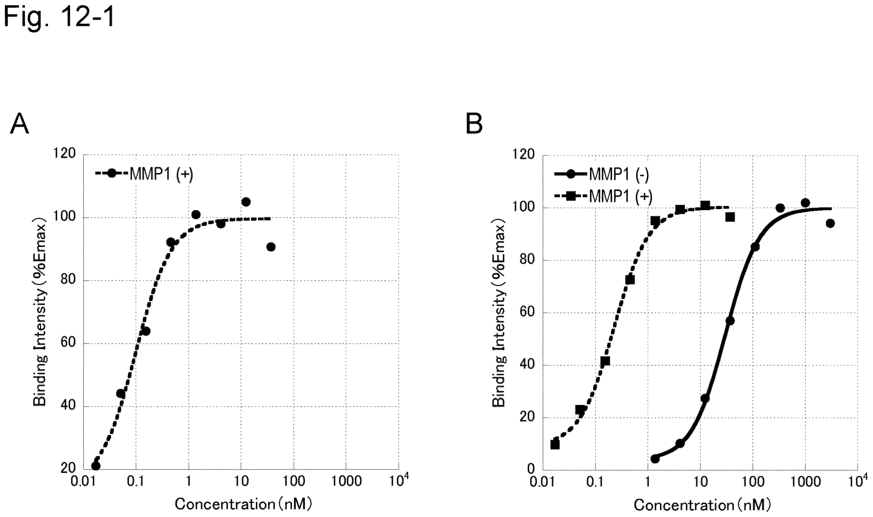

[Figure 12-1] Figure 12-1 shows the results of ELISA evaluation of the interaction between the IgG-type anti-CD98 masked antibody and the human CD98 antigen. Binding intensity was evaluated under the condition without the addition of a protease (solid line) and under the condition with the addition of a protease (dotted line). (A) hM23H1L1 exhibited concentration-dependent binding intensity under the condition with the addition of MMP1. (B) MhM1008 exhibited higher binding intensity under the condition with the addition of MMP1 than under the condition without the addition of MMP1.

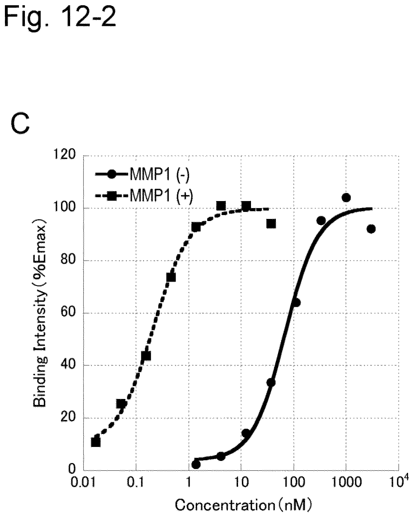

[Figure 12-2] Figure 12-2 shows the results of ELISA evaluation of the interaction between the IgG-type anti-CD98 masked antibody and the human CD98 antigen. Binding intensity was evaluated under the condition without the addition of a protease (solid line) and under the condition with the addition of a protease (dotted line). (C) MhM1013 exhibited higher binding intensity under the condition with the addition of MMP1 than under the condition without the addition of MMP1.

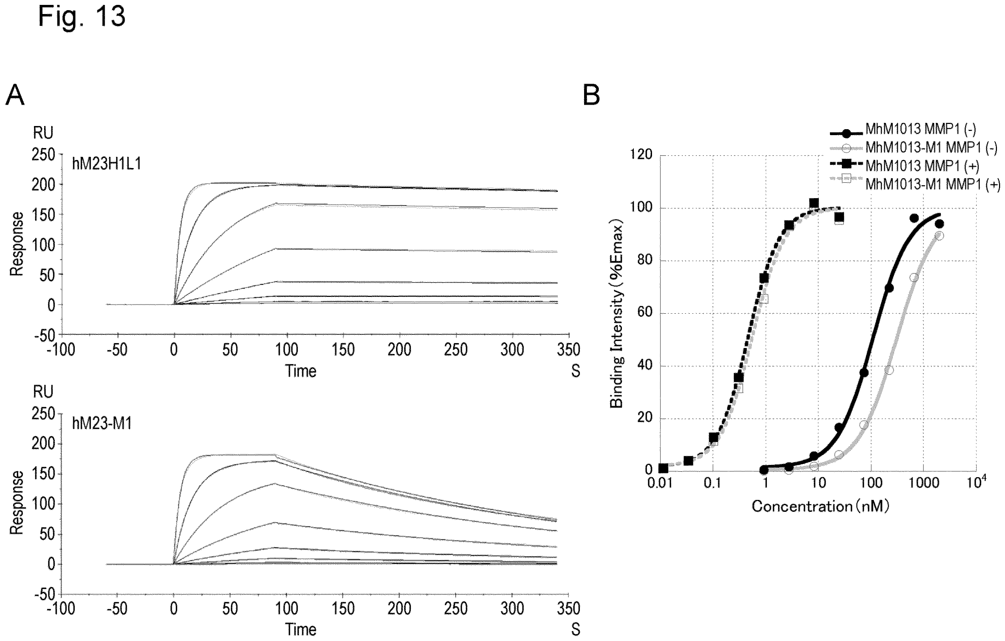

[Figure 13] Figure 13 shows (A) the results of evaluation by SPR (surface plasmon resonance) of binding intensity of hM23-M1 comprising a point mutation introduced into CDR1 of the hM23H1L1 heavy chain variable region to the human CD98 antigen and (B) the results of ELISA evaluation of binding intensity of MhM1013-M1 comprising the aforementioned mutation introduced into MhM1013 to the human CD98 antigen. MhM1013-M1 (gray) exhibited masking effects equivalent to or higher than those of MhM1013 (black).

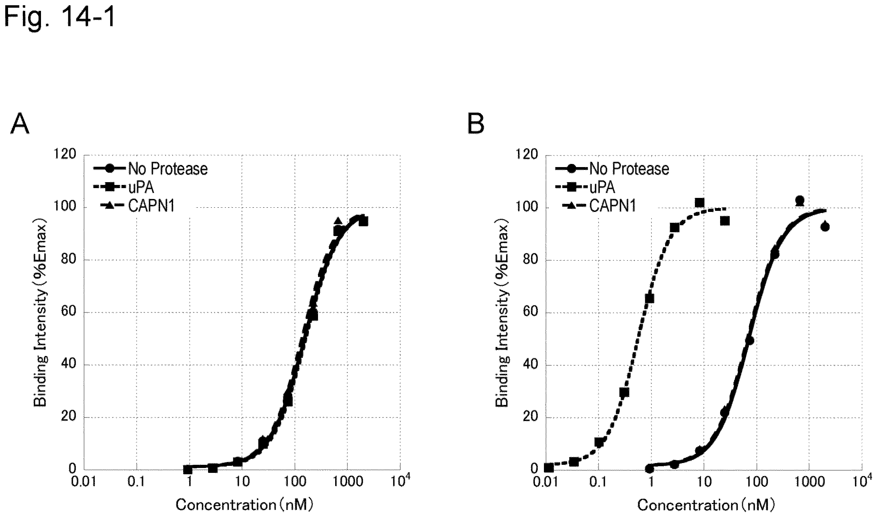

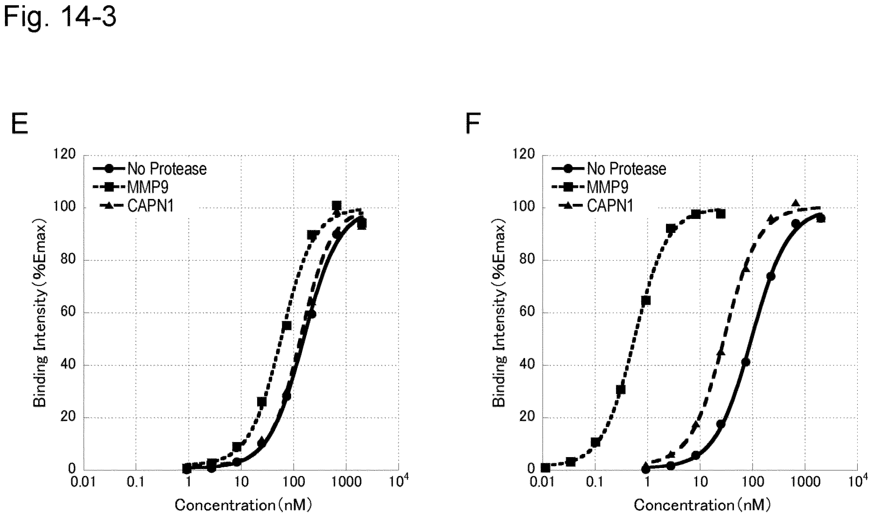

[Figure 14-1] Figure 14-1 shows the results of ELISA evaluation of the interaction between the anti-CD98 masked antibody comprising a cleavable linker with a different protease substrate and the human CD98 antigen. Binding intensity was evaluated under the condition without the addition of a protease (solid line), under the condition with the addition of uPA or MMP9 (dotted line), and under the condition with the addition of CAPN1 (broken line). (A) MhM1018-M1 exhibited equivalent binding intensity under any conditions. (B) MhM1019-M1 exhibited higher binding intensity selectively under the condition with the addition of uPA than under the condition without the addition of a protease.

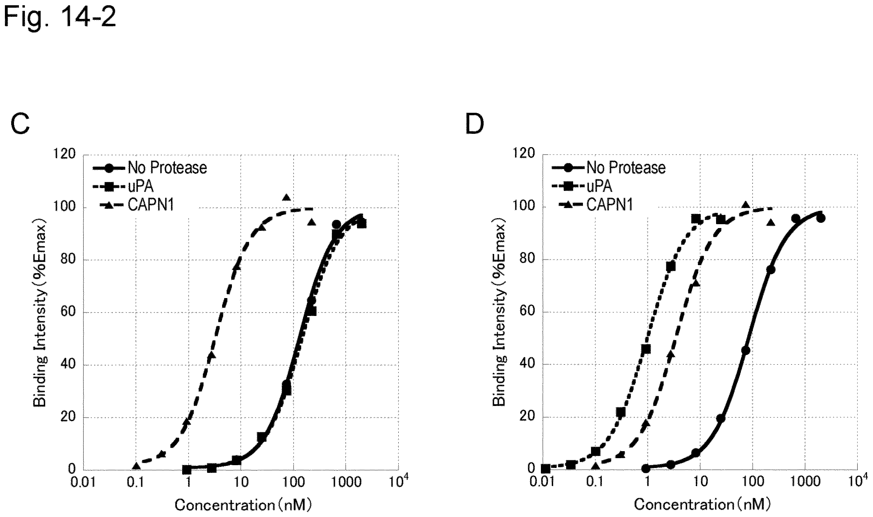

[Figure 14-2] Figure 14-2 shows the results of ELISA evaluation of the interaction between the anti-CD98 masked antibody comprising a cleavable linker with a different protease substrate and the human CD98 antigen. Binding intensity was evaluated under the condition without the addition of a protease (solid line), under the condition with

the addition of uPA or MMP9 (dotted line), and under the condition with the addition of CAPN1 (broken line). (C) MhM1020-M1 exhibited higher binding intensity selectively under the condition with the addition of CAPN1 than under the condition without the addition of a protease. (D) MhM1021-M1 exhibited higher binding intensity under the condition with the addition of uPA and CAPN1 than under the condition without the addition of a protease.

[Figure 14-3] Figure 14-3 shows the results of ELISA evaluation of the interaction between the anti-CD98 masked antibody comprising a cleavable linker with a different protease substrate and the human CD98 antigen. Binding intensity was evaluated under the condition without the addition of a protease (solid line), under the condition with the addition of uPA or MMP9 (dotted line), and under the condition with the addition of CAPN1 (broken line). (E) MhM1018-M1 exhibited slightly improved binding intensity under the condition with the addition of MMP9, but it exhibited equivalent binding intensity under other conditions. (F) MhM1022-M1 exhibited higher binding intensity under the condition with the addition of MMP9 than under the condition without the addition of a protease. MhM1022-M1 exhibited slightly improved binding intensity under the condition with the addition of CAPN1.

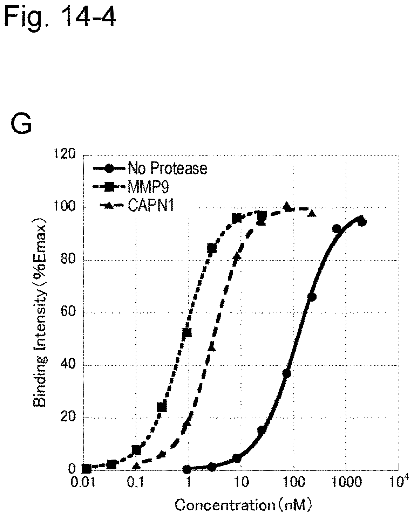

[Figure 14-4] Figure 14-4 shows the results of ELISA evaluation of the interaction between the anti-CD98 masked antibody comprising a cleavable linker with a different protease substrate and the human CD98 antigen. Binding intensity was evaluated under the condition without the addition of a protease (solid line), under the condition with the addition of uPA or MMP9 (dotted line), and under the condition with the addition of CAPN1 (broken line). (G) MhM1023-M1 exhibited higher binding intensity under the condition with the addition of MMP9 and CAPN1 than under the condition without the addition of a protease.

[Figure 15] Figure 15 shows antitumor activity of MhM1018-M1-DXd-ADC (open square), MhM1022-M1-DXd-ADC (closed square), MhM1020-M1-DXd-ADC (up-pointing closed triangle), MhM1023-M1-DXd-ADC (closed circle), and Vehicle (ABS) (circle) in mouse models transplanted with the CD98-positive human pharyngeal carcinoma cell line FaDu. An error bar in the figure indicates a standard error (n = 6).

[Figure 16] Figure 16 shows (A) the results of ELISA evaluation of the interaction between the conventional anti-EGFR antibody Cetuximab and human EGFR and (B) the results of ELISA evaluation of the interaction between the conventional anti-GPRC5D antibody C3022 described in WO 2018/147245 and the human GPRC5D antigen. The antibody was found to have bound to the antigen in a concentration-dependent manner.

[Figure 17] Figure 17 shows a degree of concentration of peptide binders using the output/input ratio after panning of (A) the anti-EGFR antibody Cetuximab or (B) the anti-GPRC5D antibody C3022. A higher value in the target antibody than in human serum-derived IgG (IgG mixture) indicates concentration of the target-antibody-specific peptides.

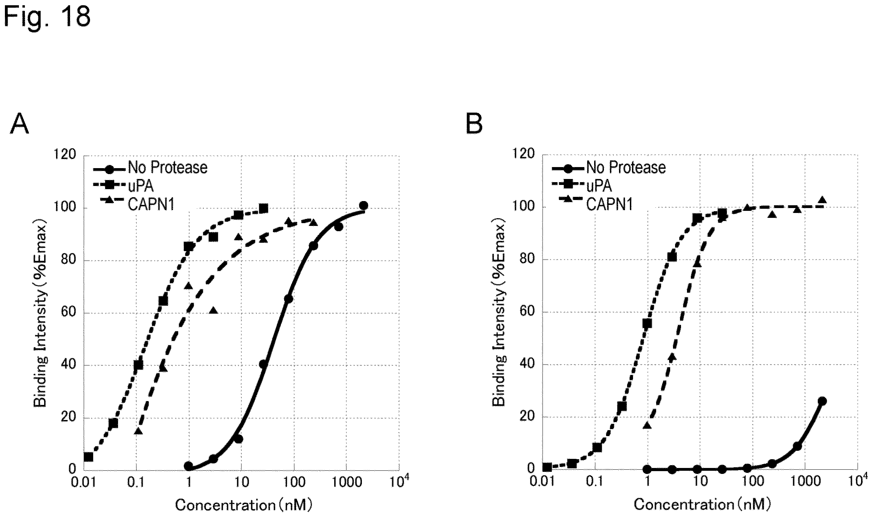

[Figure 18] Figure 18 shows (A) the results of ELISA evaluation of the interaction between the anti-EGFR masked antibody comprising both the uPA substrate and the CAPN substrate, and the human EGFR antigen or (B) the results of ELISA evaluation of the interaction between the anti-GPRC5D masked antibody comprising both the uPA substrate and the CAPN substrate, and the human GPRC5D antigen. Binding intensity was evaluated under the condition without the addition of a protease (solid line), under the condition with the addition of uPA (dotted line), and under the condition with the addition of CAPN1 (broken line). The masked antibody exhibited higher binding intensity under the condition with the addition of uPA and CAPN1 than under the condition without the addition of a protease.

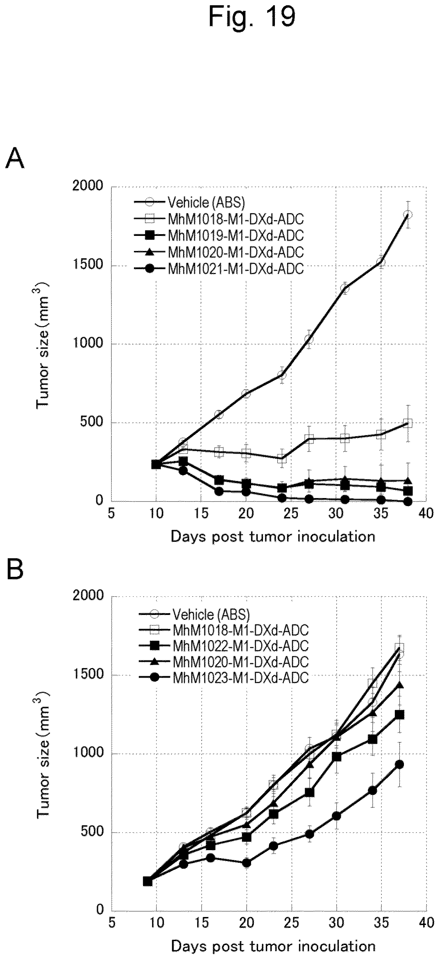

[Figure 19] Figure 19 shows antitumor activity of (A) MhM1018-M1-DXd-ADC (open square), MhM1019-M1-DXd-ADC (closed square), MhM1020-M1-DXd-ADC (up-pointing closed triangle), MhM1021-M1-DXd-ADC (closed circle), and Vehicle (ABS) (circle) and (B) MhM1018-M1-DXd-ADC (open square), MhM1022-M1-DXd-ADC (closed square), MhM1020-M1-DXd-ADC (up-pointing closed triangle), MhM1023-M1-DXd-ADC (closed circle), and Vehicle (ABS) (circle) in mouse models transplanted with the CD98-positive human lung squamous cell carcinoma cell line EBC-1 ((A) dose: 3 mg/kg; (B) dose: 1 mg/kg). An error bar in the figure indicates a standard error (n = 6).

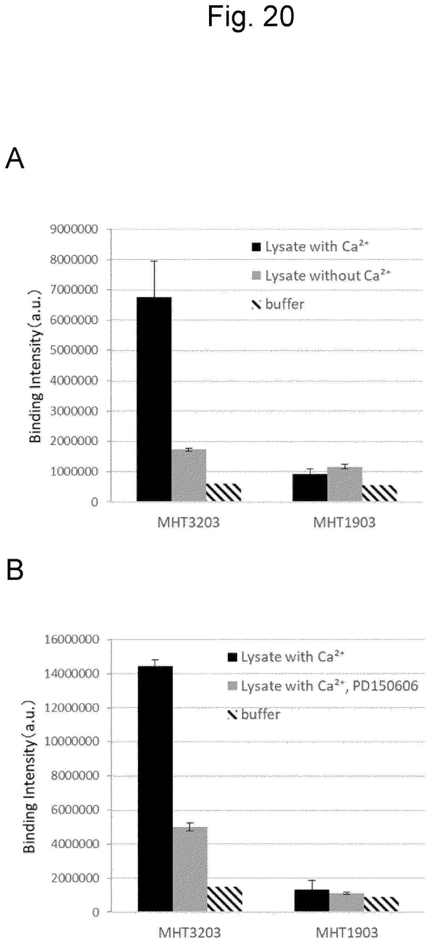

[Figure 20] Figure 20 shows binding intensity of the anti-TROP2 masked antibody MHT3203 or MHT1903 when it is allowed to react with (A) a cell lysate containing calcium chloride (black), a cell lysate not containing calcium chloride (gray), and a buffer not containing a lysate (diagonal line) and (B) a cell lysate containing calcium chloride (black), a cell lysate containing calcium chloride and a CAPN inhibitor PD150606 (gray), and a buffer not containing a lysate (diagonal line). An error bar in the figure indicates a standard error (n = 3). The buffer conditions are represented as a mean (n = 2).

[Figure 21] Figure 21 shows binding intensity of the anti-TROP2 masked antibody MHT3202 when it is allowed to react with a protease or two types of proteases. An error bar in the figure indicates a standard error (n = 3); and ** indicates a significant difference (significance level: 1%).

[Figure 22] Figure 22 shows the results of ELISA evaluation of the interaction between the anti-TROP2 masked antibody comprising the uPA substrate and the CAPN substrate, and the human TROP2 antigen. Binding intensity was evaluated under the condition without the addition of a protease (solid line), under the condition with the addition of uPA (dotted line), and under the condition with the addition of CAPN1 (broken line). (A) MHT3203 exhibited higher binding intensity under the condition with the addition of uPA and CAPN1 than under the condition without the

addition of a protease. (B) MHT3219 exhibited higher binding intensity under the condition with the addition of uPA and CAPN1 than under the condition without the addition of a protease.

[Figure 23] Figure 23 shows antitumor activity of MHT3203-PBD-AD C (open square), MHT3219-PBD-ADC (closed square), and Vehicle (ABS) (circle) in mouse models transplanted with (A) the TROP2-positive human lung mucoepidermoid carcinoma cell line NCI-H292 or (B) the TROP2-positive human pharyngeal carcinoma cell line FaDu. An error bar in the figure indicates a standard error (n = 6).

[Figure 24] Figure 24 shows binding intensity of the anti-TROP2 masked antibody comprising a cleavable linker with a different protease substrate when it is allowed to react with a buffer (diagonal line), CAPN1 (black), CAPN2 (gray), and uPA (white). Binding intensity is indicated relative to the value obtained upon reaction with uPA, which is designated as 100%.

[Figure 25] Figure 25 shows antitumor activity of MHT3203-PBD-ADC (open square), MHT5082-PBD-ADC (closed square), MHT5085-PBD-ADC (up-pointing closed triangle), MHT5086-PBD-ADC (closed circle), MHT5093-PBD-ADC (closed rhomboid), MHT5094-PBD-ADC (down-pointed triangle), MHT5095-PBD-ADC (rhomboid), and Vehicle (ABS) (circle) in mouse models transplanted with (A) the TROP2-positive human lung mucoepidermoid carcinoma cell line NCI-H292 or (B) the TROP2-positive human pharyngeal carcinoma cell line FaDu. An error bar in the figure indicates a standard error (n = 5 or 6).

[Figure 26] Figure 26 shows the full-length amino acid sequence of the HT1-11 H chain (SEQ ID NO: 1).

[Figure 27] Figure 27 shows the full-length amino acid sequence of the HT1-11 L chain (SEQ ID NO: 2).

[Figure 28] Figure 28 shows the full-length amino acid sequence of MHT1001 (SEQ ID NO: 3).

[Figure 29] Figure 29 shows the full-length amino acid sequence of MHT1002 (SEQ ID NO: 4).

[Figure 30] Figure 30 shows the full-length amino acid sequence of HT1-11-scFv-HL (SEQ ID NO: 5).

[Figure 31] Figure 31 shows the full-length amino acid sequence of HT1-11-scFv-LH (SEQ ID NO: 6).

[Figure 32] Figure 32 shows the full-length amino acid sequence of the MHT1007 H chain (SEQ ID NO: 7).

[Figure 33] Figure 33 shows the full-length amino acid sequence of the MHT1008 H chain (SEQ ID NO: 8).

[Figure 34] Figure 34 shows the full-length amino acid sequence of the MHT1009 H chain (SEQ ID NO: 9).

[Figure 35] Figure 35 shows the full-length amino acid sequence of the MHT3002 H chain (SEQ ID NO: 10).

[Figure 36] Figure 36 shows the full-length amino acid sequence of human CAPN1 (SEQ ID NO: 11).

[Figure 37] Figure 37 shows the amino acid sequence of a novel CAPN substrate (SEQ ID NO: 12).

[Figure 38] Figure 38 shows the full-length amino acid sequence of the MHT3201 H chain (SEQ ID NO: 13).

[Figure 39] Figure 39 shows the full-length amino acid sequence of the MHT3202 H chain (SEQ ID NO: 14).

[Figure 40] Figure 40 shows the full-length amino acid sequence of the MHT1713 H chain (SEQ ID NO: 15).

[Figure 41] Figure 41 shows the full-length amino acid sequence of the MHT3423 H chain (SEQ ID NO: 16).

[Figure 42] Figure 42 shows the full-length amino acid sequence of the MHT3203 H chain (SEQ ID NO: 17).

[Figure 43] Figure 43 shows the full-length amino acid sequence of the hM23H1L1 H chain (SEQ ID NO: 18).

[Figure 44] Figure 44 shows the full-length amino acid sequence of the hM23H1L1 L chain (SEQ ID NO: 19).

[Figure 45] Figure 45 shows the full-length amino acid sequence of the MhM1008 L chain (SEQ ID NO: 20).

[Figure 46] Figure 46 shows the full-length amino acid sequence of the MhM1013 L chain (SEQ ID NO: 21).

[Figure 47] Figure 47 shows the full-length amino acid sequence of the hM23-M1 H chain (SEQ ID NO: 22).

[Figure 48] Figure 48 shows the full-length amino acid sequence of the hM23-M1 L chain (SEQ ID NO: 23).

[Figure 49] Figure 49 shows the full-length amino acid sequence of the MhM1018-M1 L chain (SEQ ID NO: 24).

[Figure 50] Figure 50 shows the full-length amino acid sequence of the MhM1019-M1 L chain (SEQ ID NO: 25).

[Figure 51] Figure 51 shows the full-length amino acid sequence of the MhM1020-M1 L chain (SEQ ID NO: 26).

[Figure 52] Figure 52 shows the full-length amino acid sequence of the MhM1021-M1 L chain (SEQ ID NO: 27).

[Figure 53] Figure 53 shows the full-length amino acid sequence of the MhM1022-M1 L chain (SEQ ID NO: 28).

[Figure 54] Figure 54 shows the full-length amino acid sequence of the MhM1023-M1 L chain (SEQ ID NO: 29).

[Figure 55] Figure 55 shows the full-length amino acid sequence of the Cetuximab H chain (SEQ ID NO: 30).

[Figure 56] Figure 56 shows the full-length amino acid sequence of the Cetuximab L chain (SEQ ID NO: 31).

[Figure 57] Figure 57 shows the full-length amino acid sequence of the C3022 H chain (SEQ ID NO: 32).

[Figure 58] Figure 58 shows the full-length amino acid sequence of the C3022 L chain (SEQ ID NO: 33).

[Figure 59] Figure 59 shows the full-length amino acid sequence of the MCE-2105 L chain (SEQ ID NO: 34).

[Figure 60] Figure 60 shows the full-length amino acid sequence of the MC3-9003 H chain (SEQ ID NO: 35).

[Figure 61] Figure 61 shows the amino acid sequences recognized by human uPA and serving as substrates therefor and the amino acid sequences recognized by human MMP1 or human MMP9 and serving as substrates therefor (SEQ ID NOs: 36 to 41).

[Figure 62] Figure 62 shows the full-length amino acid sequence of the MHT1903 H chain (SEQ ID NO: 42).

[Figure 63] Figure 63 shows the full-length amino acid sequence of the MHT3219 H chain (SEQ ID NO: 43).

[Figure 64] Figure 64 shows the amino acid sequences of the novel CAPN substrates (SEQ ID NOs: 44 to 53).

[Figure 65] Figure 65 shows the full-length amino acid sequence of the MHT5082 H chain (SEQ ID NO: 54).

[Figure 66] Figure 66 shows the full-length amino acid sequence of the MHT5084 H chain (SEQ ID NO: 55).
[Figure 67] Figure 67 shows the full-length amino acid sequence of the MHT5085 H chain (SEQ ID NO: 56).
[Figure 68] Figure 68 shows the full-length amino acid sequence of the MHT5086 H chain (SEQ ID NO: 57).
[Figure 69] Figure 69 shows the full-length amino acid sequence of the MHT5090 H chain (SEQ ID NO: 58).
[Figure 70] Figure 70 shows the full-length amino acid sequence of the MHT5091 H chain (SEQ ID NO: 59).
[Figure 71] Figure 71 shows the full-length amino acid sequence of the MHT5092 H chain (SEQ ID NO: 60).
[Figure 72] Figure 72 shows the full-length amino acid sequence of the MHT5093 H chain (SEQ ID NO: 61).
[Figure 73] Figure 73 shows the full-length amino acid sequence of the MHT5094 H chain (SEQ ID NO: 62).
[Figure 74] Figure 74 shows the full-length amino acid sequence of the MHT5095 H chain (SEQ ID NO: 63).
[Figure 75] Figure 75 shows the amino acid sequence of the MMP linker (SEQ ID NO: 64). Description of Embodiments

Hereafter, the present invention is described in detail.

1. Definition

The term "DXd" used herein refers to "N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide."
The term "PBD" used herein refers to "pyrrolobenzodiazepine."
The term "mimotope" used herein refers to a "peptide binding to a complementarity determining region (CDR) of an antibody." The amino acid sequence of the mimotope is not necessarily consistent with the amino acid sequence (epitope) of the antigen that is recognized by an antibody (Molecular Immunology; 23 (7): 709-715, 1986).
The term "antibody" used herein refers to immunoglobulin comprising a constant region and a variable region. An antibody is not particularly limited, and it may be a naturally occurring or partially or completely synthesized immunoglobulin.
A basic four-chain antibody structure is composed of two identical light chains (L chains) and two identical heavy chains (H chains). A light chain binds to a heavy chain by a single covalent disulfide bond. Two heavy chains are bound to each other by one or more disulfide bonds in accordance with heavy chain isotypes. A light chain and a heavy chain each have an intra-chain disulfide bond with regular intervals. In a light chain and a heavy chain, there are a constant region exhibiting very high amino acid sequence similarity and a variable region exhibiting low amino acid sequence similarity. A light chain comprises, at its amino terminus, a variable region (VL) adjacent to a constant region (CL). A heavy chain comprises, at its amino terminus, a variable region (VH) adjacent to 3 constant regions (CH1/CH2/CH3). VL is paired with VH, and CL is aligned with a first constant region of a heavy chain (CH1). VL is paired with VH to form a single antigen-binding site.
Constant regions of the antibody of the present invention are not particularly limited. The antibody of the present invention to be used for treatment or prevention of human diseases preferably comprises constant regions of a human antibody. Examples of heavy chain constant regions of a human antibody include $C\gamma 1$, $C\gamma 2$, $C\gamma 3$, $C\gamma 4$, $C\mu$, $C\delta$, $C\alpha 1$, $C\alpha 2$, and $C\varepsilon$. Examples of light chain constant regions of a human antibody include $C\kappa$ and $C\lambda$.
Fab comprises a heavy chain VH, CH1 adjacent thereto, a light chain VL, and CL adjacent thereto. VH and VL each comprise a complementarity determining region (CDR). A linker or joint may be present between VH and CH1 and between VL and CL.
Fc (also referred to as an "Fc region") is a carboxyl terminal region of a heavy chain constant region, it comprises CH2 and CH3, and it is a dimer. Fc of the present invention may comprise a naturally occurring (wild type) sequence or it may comprise a sequence derived from the naturally occurring sequence by mutation (referred to as "mutant Fc"). In the polyspecific molecule and the bispecific molecule of the resent invention, a Fc region is preferably mutant Fc, and more preferably a combination of Fc regions capable of forming a heterodimer. An example of a combination of Fc regions is a combination of Fc (i) in the first polypeptide and Fc (ii) in the second polypeptide described below. A combination is not limited thereto, provided that such combination of Fc regions is capable of aggregation (formation of a heterodimer).
Examples of mutant Fc include, but are not limited to, a modified Fc region comprised in a heteropolymer with improved stability (including a heterodimer Fc region) disclosed in WO 2013/063702, Fc including an immunoglobulin CD3 region induced from the IgG antibody with a "knob" and a "hole" comprised in a heteropolymer disclosed in WO 1996/27011, Fc including a CH3 domain comprised in a heterodimer that becomes electrostatically advantageous by substitution of one or more amino acids with charged amino acids disclosed in WO 2009/089004, a heterodimer Fc region comprised in a heterodimer involving steric mutation and/or pI (isoelectric point) mutation disclosed in WO 2014/110601, and a heterodimer Fc including a CH3 domain with a modification to eliminate or reduce the binding to protein A disclosed in WO 2010/151792.
A variable region is composed of a region with an extreme variability referred to as a hypervariable region (HVR) and relatively invariable regions referred to as framework regions (FRs) divided by the HVR. Naturally occurring

heavy chain and light chain variable regions comprise 4 FRs connected by 3 hypervariable regions, a hypervariable region of each chain and a hypervariable region of other chains are maintained very close thereto, and such regions contribute to formation of an antigen-binding site of an antibody.

A heavy chain and a light chain of an antibody molecule are known to comprise 3 complementarity determining regions (CDRs). A complementarity determining region is also referred to as a hypervariable region, it is present within variable regions of a heavy chain and a light chain of the antibody where variability of a primary structure is particularly high, and, in general, it is separated in 3 positions in a primary structure of a polypeptide chain of a heavy chain and a light chain. In the present invention, complementarity determining regions of a heavy chain of an antibody are denoted as CDRH1, CDRH2, and CDRH3 from the amino terminus of the heavy chain amino acid sequence, and complementarity determining regions of a light chain are denoted as CDRL1, CDRL2, and CDRL3 from the amino terminus of the light chain amino acid sequence. These regions are adjacent to each other sterically and determine specificity to the antigens to which they bind.

In the present invention, the position and the length of CDR were determined in accordance with the definition of IMGT (Developmental and Comparative Immunology 27, 2003, 55-77).

FR is a variable region other than CDR. In general, a variable region comprises 4 FRs; i.e., FR1, FR2, FR3, and FR4.

CDRs and FRs comprised in the heavy chain and in the light chain are provided in the orders of FRH1-CDRH1-FRH2-CDRH2-FRH3-CDRH3-FRH4 and FRL1-CDRL1-FRL2-CDRL2-FRL3-CDRL3-FRL4, respectively, from the amino terminus toward the carboxyl terminus.

CDR and FR positions can be determined in accordance with various definitions well known in the art, such as the definitions of Kabat, Chothia, AbM, contact, in addition to IMGT.

In the present invention, a "site" to which an antibody binds; i.e., a "site" that is recognized by an antibody, is a partial peptide or a partial higher-order structure of an antigen to which an antibody binds or which is recognized by the antibody.

In the present invention, such site is referred to as an epitope or an antibody binding site. In the present invention, a "mutant antibody" refers to a polypeptide having an amino acid sequence derived from the amino acid sequence of the original antibody by substitution, deletion, or addition ("addition" encompasses "insertion") (hereafter, collectively referred to as "mutation") of amino acids and binding to the target antigen of the present invention. The number of mutant amino acids in such mutant antibody is 1, 2, 3, 4, 5, 6, 7, 8, 9, 10, 12, 15, 20, 25, 30, 40, or 50. Such mutant antibody is within the scope of the "antibody" of the present invention.

In the present invention, the term "several" in "one or several" indicates 2 to 10.

The term "molecule" used herein indicates a molecule comprising the antibody or the antigen-binding fragment of the antibody described above. In addition, the term "molecule" encompasses a polyspecific molecule formed of an antibody or a plurality of antigen-binding fragments derived therefrom.

In the present invention, the phrase "A has B" indicates that "A comprises B" or "B is bound, added, or fused to A." For example, "an antibody having a substrate" can be understood as "an antibody comprising a substrate," "an antibody to which a substrate is bound," "an antibody to which a substrate is added," or "an antibody to which a substrate is fused."

## 2. Molecule that binds to target antigen

The present invention relates to a molecule that binds to a target antigen, which binds specifically to the target antigen in a particular environment.

The term "particular environment" refers to an environment in particular tissue, and an example thereof is a cancer microenvironment. The term "cancer microenvironment" refers to an environment in cancer tissue, and an example thereof is the environment in which a protease is present.

The molecule that binds to the target antigen of the present invention comprises a moiety binding to a target antigen, a first peptide recognizing a target antigen-binding site included in the moiety, and a second peptide comprising an amino acid sequence cleaved by a protease localized in the cytoplasm, and it comprises the first peptide, the second peptide, and the moiety binding to a target antigen ligated in that order. The term "a moiety binding to a target antigen" is also referred to as "a moiety that binds to a target antigen." In the present invention, a moiety that binds to a target antigen may be referred to as "a moiety [a]," the first peptide may be referred to as "a moiety [b]," and the second peptide may be referred to as "a moiety [c]."

A moiety binding to a target antigen is preferably a polypeptide. Such moiety binds to a target antigen by the antibody-antigen reaction or the protein (e.g., receptor)-ligand binding. A moiety binding to a target antigen is more preferably an antibody binding to a target antigen by the antibody-antigen reaction or an antigen-binding fragment of an antibody.

Antibody

Examples of the antibodies of the present invention include an antibody derived from a non-human animal (a non-human animal antibody), a human antibody, a chimerized antibody (also referred to as a "chimera antibody"), and a humanized antibody, with the human antibody or the humanized antibody being preferable. The antibody of the present invention encompasses a mutant of an antibody (the "mutant antibody" described below). For example, the human antibody encompasses a human mutant antibody and the humanized antibody encompasses a humanized mutant antibody.

Examples of non-human animal antibodies include antibodies derived from vertebrates, such as mammalians and birds. Examples of mammalian-derived antibodies include antibodies derived from rodents, such as mouse antibody and rat antibody, and antibodies derived from camels. An example of a bird-derived antibody is a chicken antibody.

Examples of chimerized antibodies include, but are not limited to, antibodies comprising a variable region derived from a non-human animal antibody bound to a constant region derived from a human antibody (human immunoglobulin).

Examples of humanized antibodies include, but are not limited to, a humanized antibody prepared by transplanting CDR in a variable region of a non-human animal antibody into a human antibody (a variable region of human immunoglobulin), a humanized antibody prepared by transplanting, in addition to CDR, a part of a sequence of a framework region of a non-human animal antibody into a human antibody, and a humanized antibody prepared by substitution of 1 or more amino acids derived from a non-human animal antibody with amino acids derived from a human antibody.

An antibody can be prepared by a variety of known techniques. For example, an antibody can be prepared by a method involving the use of a hybridoma, cell-mediated immunity, or genetic recombination. Also, a phage-display-derived human antibody selected from a human antibody library can be obtained. In a phage display method, for example, a human antibody variable region may be expressed as scFv on a phage surface, and an antigen-binding phage may then be selected. The gene of the phage selected upon its binding to the antigen may be analyzed, so that a DNA sequence encoding a human antibody variable region binding to the antigen can be determined. If a DNA sequence of the antigen-binding scFv is elucidated, an expression vector comprising such sequence may be prepared, introduced into an adequate host cell, and expressed therein. Thus, a human antibody can be obtained (WO 1992/01047, WO 1992/20791, WO 1993/06213, WO 1993/11236, WO 1993/19172, WO 1995/01438, WO 1995/15388, Annu. Rev. Immunol., 1994, 12, 433-455). A human antibody can be obtained by the method involving the use of a human antibody-producing mouse having a human genome DNA fragment comprising human antibody heavy chain and light chain genes (see, for example, Tomizuka, K. et al., Nature Genetics, 1997, 16, pp. 133-143; Kuroiwa, Y. et. al., Nuc. Acids Res., 1998, 26, pp. 3447-3448; Yoshida, H. et.al., Animal Cell Technology: Basic and Applied Aspects vol. 10, pp. 69-73 (Kitagawa, Y., Matuda, T. and Iijima, S. eds.), Kluwer Academic Publishers, 1999; Tomizuka, K. et. al., Proc. Natl. Acad. Sci., U.S.A., 2000, 97, pp. 722-727), although the method is not limited thereto. As constant regions of an antibody used for the treatment or prevention of human diseases, constant regions of a human antibody are preferably used. Examples of heavy chain constant regions of a human antibody include $C\gamma1$, $C\gamma2$, $C\gamma3$, $C\gamma4$, $C\mu$, $C\delta$, $C\alpha1$, $C\alpha2$, and $C\varepsilon$. Examples of light chain constant regions of a human antibody include $C\kappa$ and $C\lambda$.

The term "an antigen-binding fragment of an antibody" refers to a partial fragment of an antibody having activity of binding to an antigen, which is composed of a heavy chain variable region and a light chain variable region. Examples of "an antigen-binding fragment of an antibody" include, but are not limited to, antigen-binding fragments, such as Fab, $F(ab')_2$, scFv, Fab', Fv, and single-domain antibody (sdAb). Such antigen-binding fragment of the antibody may be obtained by treating a full-length molecule of an antibody protein with an enzyme such as papain or pepsin, or it may be a recombinant protein produced in an adequate host cell with the use of a recombinant gene.

Mutant of antibody or binding fragment thereof

A mutant of the antibody according to the present invention or an antigen binding fragment thereof can be preferably provided with, for example, lowered susceptibility to protein degradation or oxidation, maintained or improved biological activity or functions, suppression of lowering or change in such activity or functions, improved or regulated antigen-binding ability, physicochemical properties, or functional properties. A protein is known to change its functions or activity upon alternation of a particular amino acid side chain on its surface, and examples include deamidation of an asparagine side chain and isomerization of an aspartic acid side chain. An antibody resulting from substitution of a particular amino acid with another amino acid so as to prevent the amino acid side chain from changing is within the scope of the mutant antibody of the present invention.

An example of the mutant antibody of the present invention is an antibody comprising an amino acid sequence derived from the amino acid sequence of the original antibody or its antigen binding fragment by conservative amino acid substitution. Conservative amino acid substitution occurs within an amino acid group associated with the amino acid side chain.

Preferable amino acid groups are as follows: the acidic group: aspartic acid and glutamic acid; the basic group: lysine, arginine, and histidine; the non-polar group: alanine, valine, leucine, isoleucine, proline, phenylalanine, methionine, and tryptophan; and the uncharged polar family: glycine, asparagine, glutamine, cysteine, serine, threonine, and tyrosine. Other preferable amino acid groups are as follows: the aliphatic hydroxy group: serine and threonine; the amide-containing group: asparagine and glutamine; the aliphatic group: alanine, valine, leucine, and isoleucine; and the aromatic group: phenylalanine, tryptophan, and tyrosine. In such mutant antibody, amino acid substitution is preferably carried out by refraining from lowering the antigen-binding intensity of the original antibody.

Modified antibody, modified binding fragment thereof, or complex thereof

The present invention provides a modified antibody or a modified binding fragment thereof. The modified antibody according to the present invention or the modified binding fragment thereof has been subjected to chemical or biological modification. Examples of chemical modification include a bond of a chemical portion to the amino acid skeleton and chemical modification of N-bound or O-bound carbohydrate chains. Examples of biological modification include post-translational modification (e.g., glycan addition to an N-bond or O-bond, remodeling of glycans, processing of the amino terminal or carboxyl terminal region, deamidation, aspartic acid isomerization, and methionine oxidation), and methionine addition to the amino terminus by expression in a prokaryotic host cell. Also, labels that enable detection or isolation of the antibody or antigen according to the present invention, such as an enzyme label, a fluorescence label, and an affinity label, are within the scope of the modified antibody or antigen as described above. The modified antibody according to the present invention or the binding fragment thereof as described above is useful for improvement of stability and retentivity in blood of the original antibody according to the present invention or the binding fragment thereof, reduction of the antigenicity, detection or isolation of the antibody or antigen, and other purposes.

Examples of chemical portions comprised in the chemically modified antibody or binding fragment thereof include water-soluble polymers, such as polyethylene glycol (PEG), ethylene glycol/propylene glycol polymer, carboxymethyl cellulose, dextran, and polyvinyl alcohol.

Examples of biologically modified antibody or binding fragment thereof include a modified antibody or binding fragment thereof prepared by enzyme treatment or cell treatment, a fused antibody or binding fragment thereof comprising a tag or other peptide added thereto by gene recombination, and an antibody or binding fragment thereof prepared with the use of a host cell expressing an endogenous or exogenous glycan modifying enzyme.

Such modification may be provided at any desired position in the antibody or the binding fragment thereof, and the same or two or more different types of modification may be provided at one or more positions.

However, deletion of such heavy chain sequence or modification of a heavy chain or light chain sequence has a little effect on the antigen-binding ability and effector functions of the antibody (e.g., complement activation or antibody-dependent cytotoxicity), and such effect is preferably insignificant.

Accordingly, the present invention encompasses the antibody subjected to such deletion or modification. Examples include a deletion mutant lacking 1 or 2 amino acids from the heavy chain carboxyl terminus (Journal of Chromatography A; 705; 129-134, 1995), a deletion mutant lacking 2 amino acids (glycine and lysine) from the heavy chain carboxyl terminus and additionally subjected to amidation of proline at the carboxyl terminus (Analytical Biochemistry, 360: 75-83, 2007), and an antibody resulting from pyroglutamilation of an amino-terminal glutamine or glutamic acid of the antibody heavy chain or light chain (WO 2013/147153) (they are collectively referred to as "deletion mutants"). As long as the antigen-binding ability and effector functions are retained, the antibody of the present invention lacking the heavy chain and light chain carboxyl termini is not limited to the deletion mutants described above. When the antibody of the present invention comprises 2 or more chains (e.g., heavy chains), such 2 or more chains (e.g., heavy chains) may be either or both of the full-length heavy chain or a heavy chain selected from the group consisting of the deletion mutants described above. While the quantitative ratio or the number ratio of molecules of the deletion mutant would be influenced by the type and culture conditions of cultured cells of mammalian animals producing the antibody of the present invention, main components of the antibody of the present invention can be either one or both of the 2 heavy chains lacking 1, 2, or several amino acids from the carboxyl terminus.

In addition, the antibody of the present invention or an antigen-binding fragment thereof (e.g., those comprised in the molecule, the polyspecific molecule, and the bispecific molecule of the present invention) comprising one to several amino acids derived from the expression vector and/or signal sequence added to the amino terminus and/or carboxy terminus (and partially or entirely modified as described above) are within the scope of the modified antibody of the present invention or the modified antigen-binding fragment thereof, as long as the antigen-binding intensity of interest is maintained. A molecule comprising such modified antibody or modified antigen-binding fragment thereof is within the scope of the molecule of the present invention.

In the present invention, the "the antibody or the binding fragment thereof" encompasses "the modified antibody or the modified antigen-binding fragment thereof." In addition, the "the antibody or antigen-binding fragment thereof" comprised in the molecule, the polyspecific molecule, and the bispecific molecule of the present invention encompasses

"the modified antibody or the modified antigen-binding fragment thereof."

Antibody dependent cellular cytotoxicity can be potentiated by regulation (glycosylation, fucose removal, and the like) of modification of a glycan bound to the antibody of the present invention. Known techniques for regulation of antibody glycan modification are disclosed in, for example, WO 1999/54342, WO 2000/61739, and WO 2002/31140, although techniques are not limited thereto.

Moiety binding to target antigen: moiety [a]

A target antigen is a molecule that is associated with a particular disease. When a subject is afflicted with a disease related to an antigen or molecule causing a particular disease, a molecule that is associated with the particular disease is expressed or shows enhanced expression in an abnormal cell that develops in the case of such disease. When the moiety binding to a target antigen binds to the molecule, the molecule can attack the abnormal cell, relieve the disease symptom, or treat the disease. The abnormal cell is preferably a tumor cell or a stromal cell. In the tumor cell, a target antigen is a tumor antigen. In the stromal cell, a target antigen is a molecule that is expressed in the stromal cell. The stromal cell interacts with the tumor cell and plays a key role in cancer growth and progression. The tumor antigen is expressed in the tumor cell or a tumor cell resulting from canceration of the normal cell. When the moiety binding to a target antigen binds to the tumor antigen, it inhibits tumor cell growth, damages the tumor cell, or kills the tumor cell (apoptosis or necrosis).

Examples of tumor antigens include CD98, TROP2, EGFR, GPRC5D, CD33, CD37, DR5, EPHA2, FGFR2, FGFR4, FOLR1, VEGF, CD20, CD22, CD70, PD-L1, CTLA-4, CD166, CD71, CD47, CDH6, CD147, Mesothelin, A33, CanAg, G250, MUC1, GPNMB, Integrin, Tenascin-C, CLDN6, DLL-3, and SLC44A4.

Examples of antibodies reacting with the tumor antigens include the anti-CD98 antibody (described in, for example, JP 2017-114763 A, WO 2007/114496, WO 2008/017828, WO 2009/043922, WO 2009/090553, JP 2012-092068 A, WO 2011/118804, and WO 2013/078377), the anti-TROP2 antibody (described in, for example, Linnenbach A. J. et al., Proc. Natl. Acad. Sci., vol. 86 (No. 1), pp. 27-31, 1989, WO 2008/144891, WO 2011/145744, WO 2011/155579, WO 2013/077458, WO 2003/074566, WO 2011/068845, WO 2013/068946, US 7999083, and WO 2015/098099), the anti-EGFR antibodies, such as panitumumab, nimotuzumab, cetuximab, ametumumab (SY-101), SYN-004, SCT-200, tomuzotuximab, GC-1118, GR-1401, depatuxizumab (ABT-806), Serclutamab, AMG595, and matuzumab, and the anti-GPRC5D antibodies (described in, for example, WO 2018/147245 and WO 2016/090329). The moiety binding to a target antigen according to the present invention may be derived from or comprise such antibodies.

An example of a molecule that is expressed in a stromal cell is a fibroblast activation protein (FAP).

A moiety binding to a target antigen preferably comprises an amino acid sequence that is not comprised in the first peptide or the second peptide, and it is more preferably a polypeptide consisting of such amino acid sequence.

A target antigen and a moiety binding to the target antigen may be an antigen and an antibody that binds to the antigen or an antigen-binding fragment of an antibody. The target antigen and the moiety binding to the target antigen are not limited to the combination indicated above, and examples thereof include a ligand and a receptor that binds to the ligand (or vice versa) and a cytokine and a receptor to which the cytokine binds (or vice versa). Examples of molecules other than antibodies include a non-immunoglobulin protein that binds to a target antigen, a nucleic acid molecule such as a nucleic acid aptamer, and a low-molecular-weight compound.

First peptide: moiety [b]

A first peptide that recognizes the target antigen-binding site comprised in a moiety binding to a target antigen (a moiety [a]) recognizes the target antigen-binding site comprised in the moiety binding to the target antigen and masks the site. Thus, the first peptide disables, makes it difficult, or inhibits or impedes the moiety binding to a target antigen to bind to the target antigen. When a moiety binding to a target antigen is an antibody or an antigen-binding fragment of an antibody (hereafter referred to as "antibody or the like"), the first peptide binds to an antibody or the like at its antigen-binding region. An antigen-binding region of an antibody or the like is present in a variable region of an antibody or the like, and, in particular, it is present in a complementarity determining region (CDR). Since an antibody or the like binds to an epitope (antigen determining group) of an antigen, the first peptide is a peptide that mimics an epitope. A peptide that mimics an epitope of an antigen is referred to as a "mimotope." In the present invention, the first peptide is preferably a mimotope that binds to CDR. A mimotope is a peptide consisting of 6 to 30, preferably 10 to 20, more preferably 13 to 17, and particularly preferably 15 amino acids. A mimotope can be prepared by, for example, preparing various types of display (e.g., phage display, ribosome display, nucleic acid display, or bacteria display) libraries consisting of the number of amino acids indicated above, screening the libraries by panning, selecting phage particles displaying peptides that recognize the target antigen-binding site comprised in a moiety binding to a target antigen, and obtaining DNA encoding the mimotope and a nucleotide sequence thereof from the phage particles. Whether or not a peptide of interest is a mimotope (i.e., a peptide binding to an antibody CDR) can be determined by, for example, crystallizing a

masked antibody or an antibody-peptide composite and subjecting the composite to X-ray crystal structure analysis. If the peptide binding to an antibody (competitively) with the antigen is confirmed by SPR or other means, the results of observation strongly suggest that the peptide is a mimotope that binds to the antibody CDR (see Example 4).

As described above, a moiety binding to a target antigen may be a molecule other than an antibody or an antigen-binding fragment of an antibody (hereafter referred to as "antibody or the like"). When a target antigen is a ligand, an example of the first peptide other than the mimotope is a peptide that recognizes the ligand-binding site in a receptor to which the ligand binds. When a target antigen is a cytokine, an example of the first peptide is a peptide that recognizes the cytokine-binding site in a receptor to which the cytokine binds. When a moiety binding to a target antigen is a non-immunoglobulin protein, the first peptide is a peptide that recognizes the target antigen site in the protein. When a moiety binding to a target antigen is a nucleic acid molecule, the first peptide is a peptide that recognizes the target antigen site in the nucleic acid molecule. When a moiety binding to a target antigen is a low-molecular-weight compound, the first peptide is a peptide that recognizes the target antigen site in the low-molecular-weight compound.

Second peptide: moiety [c]

A second peptide comprising an amino acid sequence cleaved by a protease localized in the cytoplasm serves as a substrate for a protease localized in the cytoplasm and comprises an amino acid sequence cleaved by a protease. The protease recognizes and cleaves an amino acid sequence comprised in the second peptide. In the present invention, a "protease localized in the cytoplasm" is also referred to as an "intracellular protease," and these two terms are interchangeable. The second peptide is also referred to as a "cleavable linker."

In the present invention, the second peptide preferably comprises an amino acid sequence served as a substrate for an extracellular protease and cleaved by a protease (it is simply referred to as a "substrate;" a substrate cleaved by a given protease is also referred to as "the protease substrate"). In such a case, an amino acid sequence serving as a substrate for an intracellular protease can be referred to as a first cleavable amino acid sequence, and an amino acid sequence serving as a substrate for an extracellular protease can be referred to as a second cleavable amino acid sequence. In the present invention, the inculsion of the first cleavable amino acid sequence and the second cleavable amino acid sequence in the second peptide is also referred to as combining the first cleavable amino acid sequence and the second celavavle amino acid sequence as protease cleavable sequences in the second peptide.

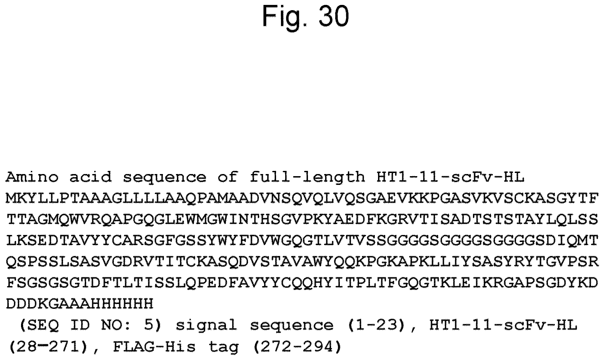

The term "intracellular protease" is also referred to as an "intracellularly active protease." After it is expressed in a cell, it acts in the cell without being secreted extracellularly, and it is associated with cell apoptosis. Examples of intracellular proteases include cytoplasmic cysteine proteases, such as caspase, calpain (also referred to as "CAPN"), and tripeptidyl peptidase. Examples of calpain isoforms include CAPN1 ($\mu$-calpain), CAPN2 (m-calpain), CAPN3, CAPN4, CAPN5, CAPN6, CAPN7, CAPN8, CAPN9, CAPN10, CAPN11, CAPN12, CAPN13, CAPN14, CAPN15, CAPN16, and CAPN17. In the present invention, any of such isoforms can be used, and CAPN1 (calpain 1) or CAPN2 (calpain 2) is preferably used. Examples of tripeptidyl peptidase isoforms include tripeptidyl peptidase 1 and tripeptidyl peptidase 2. In the present invention, any of such isoforms can be used, and tripeptidyl peptidase 2 is preferably used. Accordingly, the first cleavable amino acid sequence in the second peptide is not particularly limited, provided that it is an amino acid sequence serving as a substrate for the intracellular protease; that is, an amino acid sequence that is recognized and cleaved by a protease. A preferable example thereof is an amino acid sequence that is recognized by human CAPN1 and serves as a substrate therefor (it may be referred to as a "CAPN1 substrate" or "CAPN substrate"), and a preferable example of the CAPN substrate is PLFAAP (Figure 30, SEQ ID NO: 12).

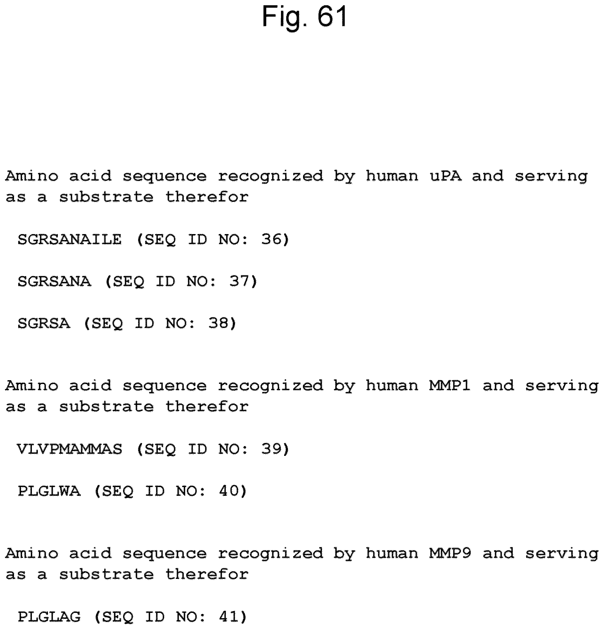

An extracellular protease is also referred to as an "extracellularly active protease," it is expressed in a form comprising a signal sequence, it is secreted to the outside of a cell, and it acts outside the cell. Examples of extracellular proteases include the urokinase type plasminogen activator (u-PA), matrix metalloproteinase (MMP), plasmin, cathepsin, matriptase, and legumain. Examples of MMP isoforms include MMP1, MMP2, MMP3, MMP7, MMP8, MMP9, MMP10, MMP11, MMP12, MMP13, MMP14, MMP15, MMP16, MMP17, MMP18, MMP19, MMP20, MMP21, MMP23A, MMP23B, MMP24, MMP25, MMP26, MMP27, and MMP28. In the present invention, any of such isoforms can be used. Examples of cathepsin isoforms include cathepsin A, cathepsin B, cathepsin C, cathepsin D, cathepsin E, cathepsin F, cathepsin G, cathepsin H, cathepsin K, cathepsin L1, cathepsin L2, cathepsin O, cathepsin S, cathepsin W, and cathepsin X/Z. In the present invention, any of such isoforms can be used. Accordingly, the second cleavable amino acid sequence in the second peptide is not particularly limited, provided that it is an amino acid sequence serving as a substrate for the extracellular protease; that is, an amino acid sequence that is recognized and cleaved by a protease. A preferable example of the second cleavable amino acid sequence is an amino acid sequence that is recognized by human uPA and serves as a substrate therefor (it may be referred to as a "uPA substrate"), and the uPA substrates are preferably SGRSANAILE (SEQ ID NO: 36, Figure 61), SGRSANA (SEQ ID NO: 37, Figure 61), and SGRSA (SEQ ID NO: 38, Figure 61). A preferable example is an amino acid sequence that is recognized by human MMP1 and serves as a substrate therefor (it may be referred to as a "MMP1 substrate" or "MMP substrate"), and the MMP1 substrates are preferably VLVPMAMMAS (SEQ ID NO: 39, Figure 61) and PLGLWA (SEQ ID NO: 40, Figure 61). A preferable example

is an amino acid sequence that is recognized by human MMP9 and serves as a substrate therefor (it may be referred to as a "MMP9 substrate"), and the MMP9 substrate is preferably PLGLAG (SEQ ID NO: 41, Figure 61).

When the second peptide comprises both the first cleavable amino acid sequence, which is a substrate for an intracellular protease, and the second cleavable amino acid sequence, which is substrate for an extracellular protease, the order thereof is not limited. For example, a molecule binding to a target antigen comprising the first peptide, the second peptide, and the moiety binding to a target antigen ligated in that order may comprise the second cleavable amino acid sequence, which is a substrate for an extracellular protease, in a region closer to the first peptide and the first cleavable amino acid sequence, which is a substrate for an intracellular protease, in a region closer to the target-binding site. A molecule binding to a target antigen comprising the first peptide, the second peptide, and the moiety binding to a target antigen ligated in that order may comprise the first cleavable amino acid sequence, which is a substrate for an intracellular protease, in a region closer to the first peptide and the second cleavable amino acid sequence, which is a substrate for an extracellular protease, in a region closer to the target-binding site. It is preferable that the second cleavable amino acid sequence and the first cleavable amino acid sequence be ligated in that order from the amino terminus toward the carboxyl terminus. It is more preferable that an amino acid sequence cleaved by a preferable extracellular protease uPA (the uPA substrate) or an amino acid sequence cleaved by a preferable extracellular protease MMP (the MMP substrate) be provided in a region closer to the amino terminus than an amino acid sequence cleaved by a preferable intracellular protease CAPN (the CAPN substrate). Accordingly, a molecule binding to a target antigen preferably comprises the first peptide, the second cleavable amino acid sequence, the first cleavable amino acid sequence, and a moiety that binds to a target antigen ligated in that order or the first peptide, the first cleavable amino acid sequence, the second cleavable amino acid sequence, and a moiety that binds to a target antigen ligated in that order. A molecule binding to a target antigen more preferably comprises the first peptide, the second cleavable amino acid sequence, the first cleavable amino acid sequence, and a moiety that binds to a target antigen ligated in that order, and it further preferably comprises the first peptide, the uPA substrate or the MMP substrate, the CAPN substrate, and the moiety that binds to a target antigen ligated in that order.

While the preceding several paragraphs mainly describe the case in which the second cleavable amino acid sequence is comprised in the second peptide, the second cleavable amino acid sequence may be comprised in a peptide other than the second peptide, provided that it is comprised in a molecule that binds to the target antigen according to the present invention. For example, the second cleavable amino acid sequence may be comprised in the terminus of the first peptide or a third peptide inserted into a region between the second peptide and the moiety that binds to a target antigen.

The first peptide, the second peptide, and the moiety that binds to a target antigen may each bind to a linker (a portion that connects two regions, which is preferably an amino acid sequence or a peptide consisting of the amino acid sequence). Specifically, any of the first peptide, the second peptide, and the moiety binding to a target antigen may bind to either or both of the other two moieties by a linker (or linkers). The linker is a peptide consisting of 1 to 30, preferably 2 to 20, and more preferably 2 to 10 amino acids. When the moiety that binds to a target antigen is an antibody or an antigen-binding fragment thereof, the antibody heavy or light chain or an antigen-binding fragment thereof may bind to the first peptide or the second peptide. Alternatively, the amino terminus, the carboxyl terminus, a region other than the termini, or a moiety that modifies such regions (e.g., a glycan or polymer) may bind to the first peptide or the second peptide.

Molecule binding to target antigen

The molecule binding to the target antigen according to the present invention comprises a moiety binding to a target antigen (a moiety [a]), a first peptide recognizing the target antigen-binding site in the moiety [a] (a moiety [b]), and a second peptide comprising an amino acid sequence cleaved by a protease localized in the cytoplasm (a moiety [c]). It is preferable that these moieties be directly or indirectly connected to each other. The first peptide binds to a target antigen-binding site in the moiety binding to the target antigen and masks the target antigen-binding site. As a result, it is impossible or difficult that the target antigen-binding site binds to the target antigen. In Figure 1 A, a moiety binding to a target antigen is a divalent antibody (IgG), the second peptide comprises a substrate for an extracellular protease (the second cleavable amino acid sequence, a gray portion in Figure 1 A) ligated to a substrate for an intracellular protease (the first cleavable amino acid sequence, a black portion in Figure 1 A), and the first peptide indicated by a lattice binds to and masks the antigen-binding site of the antibody. The first peptide can recognize and bind to a target antigen-binding site. When the second peptide is cleaved and the first peptide is present by itself, however, the first peptide and the target antigen-binding site bind to each other or dissociate from each other depending on physicochemical conditions. Accordingly, they cannot bind to each other permanently. In the molecule binding to the target antigen according to the present invention, for example, the first peptide binds to the moiety binding to a target antigen through the second peptide. In the molecule binding to the target antigen, accordingly, the first peptide is positioned in the vicinity of the target antigen-binding site in the moiety binding to a target antigen, and a majority of the target antigen-binding

site may be masked under the equilibrium conditions, unless the molecular conformation is changed. It should be noted that the action mechanism is not limited thereto.

When an amino acid sequence serving as a substrate for a protease comprised in the second peptide is cleaved by a protease, the first peptide is dissociated from a molecule binding to a target antigen, and the first peptide cannot keep binding to a target antigen-binding site. As a result, the target antigen-binding site comprised in a moiety binding to a target antigen can bind to the target antigen, and the binding affinity to the target antigen can be higher than that before it is cleaved.

Specifically, a molecule binding to the target antigen according to the present invention can bind to a target antigen with higher affinity in the presence of a protease than that in the absence of a protease.

It is preferable that intracellular proteases used in the present invention be expressed at higher levels in abnoramal cells than in normal cells, exist in larger quantities, or have higher catalytic activity. In the case of such preferable intracellular proteases, the total activity of the intracellular proteases is enhanced in the presence of abnormal cells, and (the first cleavable amino acid sequence) of the second peptide is easily cleaved.

Under ordinary circumstances, intracellular proteases are not secreted to the outside of cells. If cells are broken due to cell death or cell membrane damage, intracellular proteases leak to the outside of cells, and the intracellular proteases can act outside the cells. A molecule binding to the target antigen according to the present invention is deduced to act in the manner described below. When intracellular proteases leak extracellularly, an amino acid sequence serving as a substrate for an intracellular protease comprised in the second peptide is cleaved by the action of the intracellular proteases, the first peptide recognizing and masking the target antibody-binding site in a moiety that binds to a target antigen is dissociated, and a moiety binding to a target antigen can thus bind to a target antigen with higher affinity.

As described above, the target antigen according to the present invention is not particularly limited, provided that such antigen is associated with a particular disease. The target antigen according to the present invention is preferably present in an abnormal cell, such as a tumor cell and/or a stromal cell and causes disorders.

When an amino acid sequence serving as a substrate for an intracellular protease in the second peptide in a molecule binding to the target antigen according to the present invention is cleaved by the action of an intracellular protease leaked from an abnormal cell, a moiety binding to a target antigen is considered to bind to a target antigen in an abnormal cell with higher affinity, accelerate induction of cell death to an abnormal cell, and eliminate the cause of a disease caused by an abnormal cell more satisfactorily.

When an amino acid sequence serving as a substrate for an extracellular protease is present in the second peptide in addition to an intracellular protease, an amino acid sequence serving as a substrate for an extracellular protease in the second peptide is cleaved by the action of the extracellular protease secreted extracellularly from an abnormal cell or a cell in the vicinity thereof, the first peptide recognizing and masking the target antibody binding site of a moiety binding to a target antigen is dissociated, and the moiety that binds to a target antigen can bind to the target antigen with higher affinity. If an amino acid sequence serving as a substrate for an extracellular protease in the second peptide in a molecule binding to the target antigen according to the present invention is cleaved by the action of the extracellular protease leaked from an abnormal cell, a moiety binding to a target antigen binds to a target antigen of an abnormal cell with higher affinity, and induction of cell death (apoptosis) of a diseased cell is further accelerated, it is deduced that a cell is broken and an intracellular protease leaks extracellularly from the cell. In addition, an intracellular protease leaks extracellularly from a cell with a cell membrane damage, such as a cell with a broken or dead (necrosis) cell membrane in tumor tissue. As a result, an amino acid sequence serving as a substrate for an intracellular protease in the second peptide in a molecule binding to the target antigen according to the present invention is cleaved by the action of an intracellular protease leaked from an abnormal cell, a moiety binding to a target antigen binds to a target antigen in an abonormal cell and kills the abonormal cell. Thus, the cause of a disease caused by an abnormal cell may be eliminated more satisfactorily. When an amino acid sequence (the second cleavable amino acid sequence) serving as a substrate for an extracellular protease is present in the molecule binding to the target antigen according to the present invention, such as the second peptide, specifically, the amino acid sequence serving as a substrate for the extracellular protease and the amino acid sequence serving as a substrate for the intracellular protease are successively cleaved by the extracellular protease and the intracellular protease, and the first peptide binding to and masking the target antibody binding site of the moiety that binds to a target antigen is dissociated. Thus, the moiety that binds to a target antigen can easily bind to the target antigen, and the binding affinity of a molecule binding to the target antigen according to the present invention to the target antigen may be enhanced.

When a tumor cell is a target and a molecule binding to the target antigen according to the present invention reaches tumor tissue, an amino acid sequence serving as a substrate for an extracellular protease in the second peptide is cleaved by the action of extracellular protease that is present in a tumor environment in the vicinity of tumor tissue, the first peptide masking the target antigen-binding site is dissociated, a moiety binding to a target antigen in the molecule binds to the target antigen in the tumor cell with higher affinity, and induction of cell death to the tumor cell is accelerated. As a result, the tumor cell is broken, the intracellular protease leaks extracellularly from the cell, and the intracellular protease is supplied to the tumor environment. Subsequently, an amino acid sequence serving as a substrate for an

intracellular protease in the second peptide is cleaved by the action of the intracellular protease leaked from the tumor cell. The first peptide masking the target antigen-binding site remaining uncleaved by the action of the extracellular protease by itself is then dissociated. As a result, binding affinity of a molecule binding to a target antigen to the target antigen is further enhanced in comparison with that before it is cleaved, and the moiety binding to a target antigen binds to the target antigen in the tumor cell with higher affinity. Thereafter, the tumor cell is induced to undergo cell death, so as to treat the tumor. Specifically, the second peptide is cleaved by the action of both the extracellular protease and the intracellular protease and the first peptide that has recognized, bound to, and masked the target antigen-binding site in the moiety binding to a target antigen is dissociated from the target antigen-binding site. Thus, the molecule binding to the target antigen can strongly bind to the target antigen. The second peptide can be partially cleaved selectively by the extracellular protease alone or the intracellular protease alone; however, more of the second peptide are cleaved by the action of the both proteases and the first peptide can be dissociated from the target antigen-binding site. The molecule binding to the target antigen according to the present invention is deduced to exert its effects primarily based on the mechanism as described above. It should be noted that the mechanism of the molecule is not limited thereto and that the molecule may exert its effects by another mechanism or in combination with other mechanisms.

Another moiety

The molecule binding to the target antigen according to the present invention may further comprise another moiety. In the present invention, such another moiety is referred to as "a moiety [d]." The moiety [d] binds to a moiety that binds to a target antigen of the molecule. The moiety [d] consists of one or more compounds selected from the group consisting of an antibody, which is not the moiety that binds to a target antigen, or an antigen binding fragment thereof, a peptide comprising an amino acid sequence that is not comprised in the first peptide or the second peptide, a cytokine, a toxin, a radioactive isotope, a label molecule, a photosensitive substance (it may be referred to as a "photosensitizer"), an immune potentiator, an antitumor compound, a drug, a payload, and a polymer.

Examples of a peptide comprising an amino acid sequence that is not comprised in the first peptide or the second peptide include, but are not limited to, an antibody, an antigen-binding fragment of an antibody, a non-immunoglobulin protein existing in nature, an artificial protein, a receptor protein or a ligand binding fragment thereof, a ligand protein, and a protein that regulates the blood kinetics (e.g., antibody Fc or albumin). Examples of an antibody include, but are not limited to, an antibody, which is not a molecule binding to a target antigen, an antibody binding to a site other than the target-binding site in a molecule binding to a target antigen, and an antibody or the like binding to a molecule binding to a target antigen to serve as a multispecific molecule (e.g., bispecific antibody). Examples of cytokine include, but are not limited to, interleukin, interferon, chemokine, colony-stimulating factor, tumor necrosis factor, and growth factor. Examples of toxin include, but are not limited to, biotoxins, such as cyanotoxin, hemotoxin, necrotoxin, neurotoxin, and cytotoxin, and environmental toxin. Examples of a radioactive isotope include, but are not limited to, $^{131}I$, $^{211}AT$, and $^{89}Sr$. Examples of a label molecule include, but are not limited to, fluorescent substances, such as FITC and PE, enzymes, such as HRP and AP, and biotin. Examples of a photosensitive substance include, but are not limited to, phthalocyanine derivative, chlorin derivative, and bacteriochlorin derivative. An example of an immune potentiator is, but is not limited to, an adjuvant. Examples of a polymer include, but are not limited to, a natural or artificial glycan, synthetic resin, and polyethylene glycol. Examples of an antitumor compound include, but are not limited to, a topoisomerase inhibitor, a mitotic inhibitor, a cell division inhibitor, a microtubule polymerization/depolymerization inhibitor, a modulator of a glucocorticoid receptor, a DNA binder, an alkylating agent, a radioactive isotope, siRNA, and an antibody or an antigen binding fragment thereof. Examples of drugs include, but are not limited to, an antitumor agent, an immune potentiator, a cytokine, an antitumor compound, a drug, and a payload comprised in ADC described below.

When a moiety [d] is a polypeptide, the molecule binding to the target antigen according to the present invention may consist of a polypeptide. When a moiety [d] is a compound other than a polypeptide, the molecule binding to the target antigen according to the present invention may consist of the moiety [d] and a polypeptide.

The moiety [d] may be ligated to the molecule binding to the target antigen according to the present invention by a linker.

When the moiety [d] is an antitumor compound, drug, or payload and the molecule binding to a target antigen is an antibody or an antigen binding site thereof (an antibody or the like), the molecule binding to a target antigen comprising the moiety [d] can be referred to as an "antibody-drug conjugate (ADC)." ADC is described in, for example, Methods Mol. Biol., 2013, 1045: 1-27; Nature Biotechnology, 2005, 23, pp.1137-1146. An antitumor compound is not particularly limited, provided that such substance can exert pharmacological effects when an antibody or the like binds thereto. Examples of antitumor compounds that can be used as antitumor agents include emtansine (a 4-({3-[(3-{[(1S)-2-{[(1S,2R,3S,5S,6S,16E,18E,20R,21S)-11-chloro-21-hydroxy-12,20-dimethoxy-2,5,9,16-tetramethyl-8,23-dioxo-4,24-dioxa-9,22-diazatetracyclo[19.3.1.110,14.03,5]hexacosa-10,12,14(26),16,18-pentaen-6-yl]oxy}-1-methyl-2-oxoethyl]methylamino}-3-oxopropyl)sulfanyl]-2,5-dioxopyrrolidin-1-yl}methyl)cyclohexylcarbonyl group) (e.g., WO 2001/000244 and WO 2001/000245) and a topoisomerase inhibitor (e.g., Liang, X. et al., Eur. J. Med. Chem., vol. 171,

2019, pp. 129-168), with topoisomerase type I inhibitors, such as a camptothecin derivative, an active metabolite of irinotecan SN-38 (e.g., EP 137145 A1 and US 4604463 A), exatecan (e.g., EP 495432 A1 and US 5637770 A), and an exatecan derivative (e.g., WO 2014/057687), being preferable. A preferable example of an exatecan derivative is N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide (e.g., WO 2014/057687, WO 2014/061277, WO 2015/146132, WO 2020/100954, and WO 2015/098099). Examples of other antitumor compounds include a pyrrolobenzodiazepine derivative (e.g., WO 2019/065964, WO 2013/173496, WO 2014/130879, WO 2017/004330, WO 2017/004025, WO 2017/020972, WO 2016/036804, WO 2015/095124, WO 2015/052322, WO 2015/052534, WO 2016/115191, WO 2015/052321, WO 2015/031693, and WO 2011/130613). Preferable examples of pyrrolobenzodiazepine derivatives include (11a'S)-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-1',11a'-dihydro-5'H-spiro[cyclopropane-1,2'-pyrrolo[2,1-c][1,4]benzodiazepin]-5'-one, (11a'S)-7'-methoxy-8'-[(5-{ [(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-1',10',11',11a'-tetrahydro-5'H-spiro[cyclopropane-1,2'-pyrrolo[2,1-c][1,4]benzodiazepin]-5'-one, (11a'S,11a""S)-8',8"-[1,5-pentanediylbis(oxy)]bis(7'-methoxy-1',11a'-dihydro-5'H-spiro[cyclopropane-1,2'-pyrrolo[2,1-c][1,4]benzodiazepin]-5'-one), and (11a'S)-7'-methoxy-8'-(3-{[(11aS)-7-methoxy-2-(4-methoxyphenyl)-5-oxo-5,10,11,11a-tetrahydro-1H-pyrrolo[2,1-c] [1,4]benzodiazepin-8-yl]oxy(propoxy)-1',11a'-dihydro-5'H-spiro[cyclopropane-1,2'-pyrrolo[2,1-c][1,4]benzodiazepin]-5'-one (WO 2019/065964). Drugs may be immune potentiators, such as the STING agonist (e.g., WO 2021/202984, WO 2020/229982, WO 2020/050406, and WO 2021/177438), the TLR7/8 agonist, or the TLR8 agonist (e.g., WO 2018/009916 and WO 2019/084060). A preferable example of the STING agonist is a cyclic dinucleotide derivative. Examples of cyclic dinucleotide derivatives include (5R,7R,8R,12aR,14R,15R,15aS,16R)-15,16-dihydroxy-7-[1-(2-hydroxyethyl)-6-oxo-1,6-dihydro-9H-purin-9-yl]-2,10-bis(sulfanyl)-14-(6,7,8,9-tetrahydro-2H-2,3,5,6-tetraazabenzo[cd]azulen-2-yl)octahydro-2H,10H,12H-5,8-methano-2$\lambda^5$,10$\lambda^5$-flo[3,2-1][1,3,6,9,11,2,10]pentaoxadiphosphacyclotetradecine-2,10-dione, (5R,7R,8R,12aR,14R,15R,15aR,16R)-15-fluoro-16-hydroxy-7-[1-(2-hydroxyethyl)-6-oxo-1,6-dihydro-9H-purin-9-yl]-2,10-bis(sulfanyl)-14-(6,7,8,9-tetrahydro-2H-2,3,5,6-tetraazabenzo[cd]azulen-2-yl)octahydro-2H,10H,12H-5,8-methano-2$\lambda$,10$\lambda$-flo[3,2-1][1,3,6,9,11,2,10]pentaoxadiphosphacyclotetradecine-2,10-dione, (5R,7R,8R,12aR,14R,15R,15aR,16R)-7-[1-(2-aminoethyl)-6-oxo-1,6-dihydro-9H-purin-9-yl-14-(8,9-dihydro-6-thia-2,3,5-triazabenzo[cd]azulen-2(7H)-yl)-15-fluoro-16-hydroxy-2,10-bis(sulfanyl)octahydro-2H,10H,12H-5,8-methano-2$\lambda^5$,10$\lambda^5$-flo[3,2-1][1,3,6,9,11,2,10]pentaoxadiphosphacyclotetradecine-2,10-dione, and N -(2-{9-[(5R,7R,8R,12aR,14R,15R,15aR,16R)-14-(8,9-dihydro-6-thia-2,3,5-triazabenzo[cd]azulen-2(7H)-yl)-15-fluoro-16-hydroxy-2,10-dioxo-2,10-bis(sulfanyl)octahydro-2H,10H,12H-5,8-methano-2$\lambda^5$,10$\lambda^5$-flo[3,2-1][1,3,6,9,11,2,10]pentaoxadiphosphacyclotetradecin-7-yl]-6-oxo-6,9-dihydro-1H-purin-1-yl}ethyl)-2-hydroxyacetamide (WO 2021/177438). In the present invention, the moiety [d] is bound to a moiety that binds to a target antigen. When the moiety that binds to a target antigen is an antibody or an antigen binding fragment thereof, the moiety [d] can be bound to the antibody or an antigen binding fragment thereof in accordance with a conventional technique. For producing a glycoprotein molecule comprising a therapeutic or preventive antibody or an Fc region thereof, technologies which modify glycans thereon to be homogeneous have been known. As a method of making glycans attached to a glycoprotein homogeneous, a transglycosylation reaction using an enzyme has been known. This reaction is a multi-step procedure comprising *in vitro* cleavage (hydrolysis) of a glycan and *in vitro* condensation of another glycan (transglycosylation). When conversion of the N-glycan is intended, in particular, a group of enzymes referred to as endo-β-N-acetylglucosaminidase (ENGase) is used. Such enzymes are required to have 1) an ability to hydrolyze a complex-type glycan in a substrate-specific manner and 2) an ability to perform a transglycosylation reaction to a predetermined structure. As transglycosylation reactions, an oxazoline method comprising transferring a glycan with an activated reducing end, such as a glycan with an oxazolylated reducing end, to a GlcNAc (N-acetylglucosamine) acceptor with the use of a single ENGase and a one-pot method comprising directly transferring a glycan having a reducing end that is not activated to a GlcNAc acceptor with the use of two types of ENGases are known (WO 2022/050300 and WO 2018/003983). In the present invention, a moiety [d], such as an antitumor compound, drug, or payload, can bind to an antibody or an antigen binding fragment thereof directly or by a linker by, for example, a transglycosylation reaction in accordance with the methods described in the literatures indicated above. When the moiety [d] is a photosensitive substance and a moiety binding to a target antigen is or comprises an antibody or an antigen binding fragment thereof (an antibody or the like), a molecule binding to a target antigen comprising the moiety [d] can be used for photodynamic therapy (PDT), and such molecule can be referred to as an "antibody-directed phototherapy (ADP) molecule." The ADP molecule is described in, for example, Antibodies, 2013, 2, pp. 270-305. A photosensitive substance is not particularly limited, provided that it can exert pharmacological effects by applying light to a site to which an antibody or the like has bound. Examples of photosensitive substances include (2S)-2-[[2-[(2S,3S)-7-carboxy-3-(2-carboxyethyl)-17-ethenyl-12-ethyl-2,8,13,18-tetramethyl-2,3,23,24-tetrahydroporphyrin-5-yl]acetyl]amino]butanedioic acid (e.g., US Patent No. 5633275 and US Patent No. RE37180), IR700 (IRDye® 700DX) (e.g., WO 2013/009475, WO 2004/038378, WO 2015/187677, WO 2017/031363, WO 2017/031367, WO 2018/156815, WO 2019/232478, and WO 202020/5623), and 2,4-difluoro-N-methyl-3-[10,15,20-tris[2,6-difluoro-3-(methylsulfamoyl)phenyl]-2,3,12,13,22,24-hexahydroporphyrin-5-yl]benzenesulfonamide (e.g., WO 2016/151458).

In tissue comprising a cell having a target antigen or a region in the vicinity thereof, a cleavable linker comprised in a molecule binding to a target antigen (i.e., a second peptide) is cleaved, and an active ingredient comprised in the molecule binding to a target antigen (e.g., a drug) exerts its effects.

3. Method for producing molecule binding to target antigen

(1) Method for identifying mimotope comprised in first peptide

A peptide binding to a complementarity determining region (CDR) in an antibody of interest or an antigen binding fragment thereof can be identified using a peptide library. A peptide library may be constructed in accordance with a conventional technique. For example, a peptide library by various display systems, such as a ribosome composed of completely random amino acids may be constructed, and a peptide exhibiting high affinity to the CDR may be selected. Also, a peptide library by various display systems having a repeat motif of aromatic amino acid and Pro (a ZPZP motif) near the center (ZPZP lib) may be constructed, and a peptide exhibiting high affinity to the CDR may be selected. Z represents an aromatic amino acid selected from among histidine (His), phenylalanine (Phe), tyrosine (Tyr), and tryptophan (Trp), and P represents proline.

A mimotope comprised in the first peptide can be identified by the method described above.

An antibody CDR loop comprises a large quantity of aromatic amino acids. Since aromatic amino acids easily interact with each other, aromatic amino acids are preferably present near the center of a peptide.

The present invention includes a peptide library by various display systems used to identify the first peptide obtained by the method described above, mimotopes, and peptides binding to molecules other than antibodies (e.g., cytokines).

(2) Method for producing molecule binding to target antigen

A peptide, which is a molecule binding to the target antigen according to the present invention and comprises an amino acid sequence comprised in the first peptide and/or an amino acid sequence comprised in the second peptide, can be prepared by, for example, recombination, *in vitro* translation, chemical synthesis, or peptide synthesis.

For example, a polynucleotide comprising a nucleotide sequence encoding an amino acid sequence comprised in a moiety that binds to a target antigen and, according to need, an amino acid sequence comprised in the first peptide and/or an amino acid sequence comprised in the second peptide is introduced into a cell, the cell is cultured, and a polypeptide binding to the target antigen is collected from the culture product. Thus, the molecule binding to the target antigen according to the present invention can be produced.

To a polynucleotide comprising a nucleotide sequence encoding an amino acid sequence comprised in a moiety that binds to a target antigen and, according to need, an amino acid sequence comprised in the first peptide and/or an amino acid sequence comprised in the second peptide, DNAs each encoding a relevant peptide may be ligated, and an element, such as a promoter, an enhancer, or a polyadenylation signal, may further be operably ligated thereto. When DNA is "operably ligated" herein, DNA is ligated to an element, so that the element can exert their functions. DNA may be inserted into an expression vector, a host cell may be transformed with the aid of the vector, and the host cell may then be cultured, produced, and collected. The vector may comprise DNA encoding a signal peptide that accelerates secretion of a molecule binding to a target antigen from a host cell. In such a case, DNA encoding a signal peptide is ligated in-frame to DNA encoding a molecule binding to a target antigen. After the molecule binding to a target antigen is produced, the signal peptide may be removed, so as to obtain a molecule binding to a target antigen as a mature protein.

An expression vector is not particularly limited, as long as it can replicate DNA of interest in an animal cell, a bacterial cell, a yeast cell, or other host, and examples thereof include known plasmids and phages. Examples of a vector used to construct an expression vector include pcDNA™ (Thermo Fisher Scientific), Flexi® vector (Promega), pUC19, pUEX2 (Amersham), pGEX-4T, pKK233-2 (Pharmacia), and pMAMneo (Clontech). As host cells, prokaryotic cells such as *Escherichia coli* and *Bacillus subtilis* and eukaryotic cells such as yeasts and animal cells can be used, with the use of eukaryotic cells being preferable. Examples of animal cells include the human embryonic kidney cell line HEK293 and the Chinese hamster ovary (CHO) cell. It is sufficient to introduce an expression vector into a host cell by a known method to transform the host cell. Examples of methods include an electroporation method, a calcium phosphate precipitation method, and a DEAE-dextran transfection method. The produced antibody can be purified by usual protein isolation or purification methods. For example, affinity chromatography or other chromatography techniques, filtration, ultrafiltration, salting out, dialysis, and the like can be suitably selected and combined.

4. Use of molecule binding to target antigen according to the present invention

The molecule binding to the target antigen according to the present invention, a salt thereof, or a hydrate of the molecule or salt (hereafter, they are referred to as "the molecule or the like binding to the target antigen according to the present invention") can be used as an agent for prevention or treatment of a disease caused by an abnormal cell.

When an abonormal cell is a tumor cell, the molecule binding to the target antigen according to the present invention can be used as an anticancer agent.

The anti-cancer agent can be used for one type or two or more types of cancer species selected from among carcinoma, sarcoma, lymphoma, leukemia, myeloma, germinoma, brain tumor, carcinoid, neuroblastoma, retinoblastoma, and nephroblastoma. Specific examples of carcinoma include kidney cancer, melanoma, squamous cell cancer, basal cell cancer, conjunctival cancer, oral cavity cancer, laryngeal cancer, pharyngeal cancer, thyroid gland cancer, lung cancer, breast cancer, esophageal cancer, gastric cancer, duodenal cancer, small bowel cancer, large bowel cancer, rectal cancer, appendiceal cancer, anal cancer, liver cancer, gallbladder cancer, bile duct cancer, pancreatic cancer, adrenal cancer, bladder cancer, prostate cancer, uterine cancer, and vaginal cancer. Specific examples of sarcoma include liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral neurilemmoma, retroperitoneal sarcoma, synoviosarcoma, uterine sarcoma, gastrointestinal stromal tumor, leiomyosarcoma, and epithelioid sarcoma. Specific examples of lymphoma include B-cell lymphoma, T/NK-cell lymphoma, and Hodgkin's lymphoma. Specific examples of leukemia include myelogenic leukemia, lymphatic leukemia, myeloproliferative disorder, and myelodysplastic syndrome. A specific example of myeloma is multiple myeloma. Specific examples of germinoma include testicular cancer and ovarian cancer. Specific examples of brain tumor include neuroglioma and meningioma.

The antitumor agent of the present invention can contain a molecule that binds to the target antigen of the present invention in an amount effective for treatment, as well as pharmaceutically acceptable carriers, diluents, solubilizers, emulsifiers, preservatives, aids, and the like. The "pharmaceutically acceptable carriers" and the like can be suitably selected from a broad range according to the type of a target disease and the dosage form of a drug. An administration method for the antitumor agent of the present invention can be suitably selected. For example, the antitumor agent can be injected, and local injection, intraperitoneal injection, selective intravenous infusion, intravenous injection, subcutaneous injection, organ perfusate infusion, and the like can be employed. Further, an injection solution can be formulated using a carrier comprising a salt solution, a glucose solution, or a mixture of salt water and a glucose solution, various types of buffer solutions, or the like. Further, a powder may be formulated and mixed with a liquid carrier to prepare an injection solution before use.

Other administration methods can be suitably selected along with development of a formulation. For example, oral solutions, powders, pills, capsules, tablets, and the like can be applied for oral administration. For oral solutions, oral liquid preparations such as suspensions and syrups can be produced using water, saccharides such as sucrose, sorbitol, and fructose, glycols such as polyethylene glycol, oils such as sesame oil and soybean oil, preservatives such as alkyl parahydroxybenzoates, flavors such as strawberry flavor and peppermint, and the like. Powders, pills, capsules, and tablets can be formulated using excipients such as lactose, glucose, sucrose, and mannitol, disintegrating agents such as starch and alginate soda, lubricants such as magnesium stearate and talc, binders such as polyvinyl alcohol, hydroxypropyl cellulose, and gelatin, surfactants such as fatty acid esters, plasticizers such as glycerin, and the like. Tablets and capsules are preferred unit dosage forms for the composition of the present invention in that they are easily administered. Solid production carriers are used to produce tablets and capsules.

The effective dose of the molecule or the like that binds to the target antigen of the present invention used for treatment may be changed according to characteristics of symptoms to be treated and the patient's age and condition and may be finally determined by a physician. For example, one dose is 0.0001 mg to 100 mg per kg of body weight. The predetermined dose may be administered once every one to 180 days, or the dose may be divided into two doses, three doses, four doses, or more doses per day and administered at appropriate intervals.

The molecule or the like that binds to the target antigen of the present invention can be used in combination with another drug as an agent for prevention or treatment of a disease caused by an abonormal cell. The molecule or the like that binds to the target antigen of the present invention can be administered to a person who has or is at a risk of a disease caused by an abonormal cell before, simultaneously with, or after the administration of the other drug. When the molecule or the like that binds to the target antigen of the present invention is administered simultaneously with the other drug, they may be administered in the form of a combination drug thereof (i.e., a preparation comprising both of the molecule and the drug) or a single agent (i.e., a preparation comprising either thereof).

Examples

Hereafter, the present invention is described in greater detail with reference to the examples, although the present invention is not limited to these examples.

In the following examples, genetic engineering procedures were performed in accordance with the method described in Molecular Cloning (Sambrook, J., Fritsch, E. F. and Maniatis, T., Cold Spring Harbor Laboratory Press, 1989) and methods described in other experimental protocols employed by a person skilled in the art, unless otherwise specified. When a commercially available reagent or kit was to be used, the procedures in accordance with the instructions of such commercial product were employed. Synthesis of primers required for gene synthesis or vector construction was outsourced, according to need (Fasmac Co., Ltd. and Thermo Fisher Scientific).

(Example 1) Preparation of anti-TROP2 antibody HT1-11

1)-1 Expression and purification of anti-TROP2 antibody HT1-11

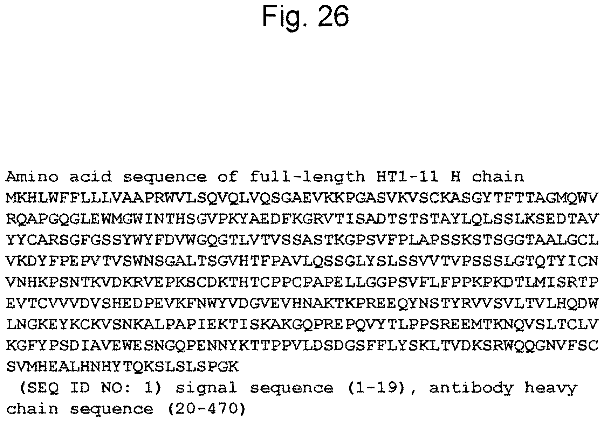

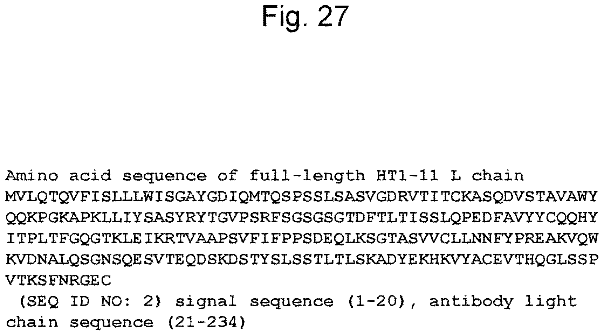

A mammalian cell expression vector comprising, as the backbone, pcDNA 3.3 (Thermo Fisher Scientific) into which DNA encoding the heavy chain (Figure 26, SEQ ID NO: 1) or light chain (Figure 27, SEQ ID NO: 2) of the known anti-TROP2 antibody described in WO 2015/098099 has been cloned was introduced into Expi293F cells, and an antibody molecule was purified from the transient expression culture supernatant. Expi293F cells (Thermo Fisher Scientific) were subcultured in accordance with the instructions. A culture solution of the Expi293F cells in a logarithmic growth phase was diluted to $2.5 \times 10^6$ cells/ml in the Expi293 Expression medium (Thermo Fisher Scientific), heavy chain and light chain expression vectors and polyethyleneimine (Polyscience) were added to Opti-Pro SFM medium (Thermo Fisher Scientific), the reaction was allowed to proceed, and the reaction product was then added to the Expi293F cells. Agitation culture was performed in an incubator at 37°C in the presence of 8% $CO_2$ at 135 rpm for 5 days. MabSelectSuRe (Cytiva) equilibrated with PBS (pH 7.4) was added to the culture supernatant after centrifugation to allow the target antibody molecules to adsorb thereto. After the non-adsorbed components were removed with the aid of PBS, the adsorbed components were eluted using acetate buffer (pH 3.5). The elution fraction was neutralized with the aid of Tris buffer (pH 9.0), and the buffer was exchanged with 25 mM histidine, 5(w/v)% sorbitol (pH 6.0) using an ultrafiltration membrane. The resultant was concentrated, according to need, and applied to a gel filtration column Superdex 200 Increase (Cytiva) equilibrated with 25 mM histidine, 300 mM NaCl, 5(w/v)% sorbitol (pH 5.5) in advance to collect a monomer fraction. The purified sample was subjected to SDS-polyacrylamide electrophoresis (SDS-PAGE) and size exclusion chromatography (SEC) to evaluate the degree of monomer purification. The concentration of the purified antibody was determined by a method known to a person skilled in the art based on the absorbance at 280 nm obtained with the use of a spectrophotometer and the absorbance coefficient determined by the PACE method (Protein Science; 4 (11): 2411-2423, 1995).

1)-2 Evaluation of binging intensity between anti-TROP2 antibody HT1-11 and anti-TROP2 antigen by ELISA

NeutrAvidin (Thermo Fisher Scientific) diluted to 1 μg/ml with PBS was added at 50 μl/well to a 96-well Maxisorp plate (Black, Nunc) and immobilized at 4°C overnight. The plate was washed with PBS containing 0.05% (w/v) Tween-20 (BioRad) (ELISA buffer) and then blocked with Blocker Casein (Thermo Fisher Scientific). The plate was washed with ELISA buffer, the biotinylated human TROP2 antigen (Accession Number: P09758; the extracellular domain was purified by the method known to a person skilled in the art and the C-terminal Avi tag sequence was then biotinylated) diluted to 1 μg/ml with PBS was added at 50 μl/well, and the resultant was then agitated for 30 minutes at room temperature. The plate was washed with ELISA buffer, the antibody of Example 1)-1 with the concentration thereof being adjusted with ELISA buffer was added at 50 μl/well, and the resultant was then agitated for 30 minutes at room temperature. The plate was washed with ELISA buffer, 50 μl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 2, HT1-11 was found to have bound to the human TROP2 antigen in a concentration-dependent manner.

(Example 2) Concentration of mimotope peptide binding to anti-TROP2 antibody HT1-11

A peptide library for ribosome display composed of completely random 15 amino acids (Linear 15mer lib) was constructed, and a peptide capable of binding to HT1-11 was concentrated by a method known to a person skilled in the art. At the outset, human serum-derived IgG (Sigma Aldrich) biotinylated with EZ-Link NHS-PEG4-Bioin (Thermo Fisher Scientific) or HT1-11 was immobilized on Dynabeads Streptavidin M-280 (Thermo Fisher Scientific), and the ribosome displaying a peptide (hereafter referred to as "RD") was allowed to react with human serum-derived IgG-bound beads. RDs that did not bind to the beads were collected using a magnet stand (DynaMag-2, Thermo Fisher Scientific) and then allowed to react with the HT1-11-bound beads. RDs that did not bind to HT1-11 were removed by washing

with the use of a magnet stand, and mRNAs were purified from RDs that had bound to HT1-11. Thereafter, RDs were prepared again by RT-PCR and *in vitro* translation. This process of panning was performed 3 times.

mRNAs after the third round of panning were subjected to *in vitro* translation to prepare RDs, and the prepared RDs were then allowed to react with Dynabeads Streptavidin M-280 comprising biotinylated HT1-11 or human serum-derived IgG immobilized thereon (amount of input: $6 \times 10^{11}$). RDs that did not bind were removed by washing with the use of a magnet stand, mRNAs were collected from the RDs that had bound, and the amount collected (output) was quantified and evaluated by RT-qPCR. As shown in Figure 3, the number of mRNAs collected under the HT1-11-immobilized conditions was 2,624 times greater than that collected under the human serum IgG-immobilized conditions. The results indicate concentration of peptides binding specifically to HT1-11.

(Example 3) Screening of mimotope peptide binding to anti-TROP2 antibody 3)-1 Preparation of HT1-11 scFv to which peptide obtained by panning was fused

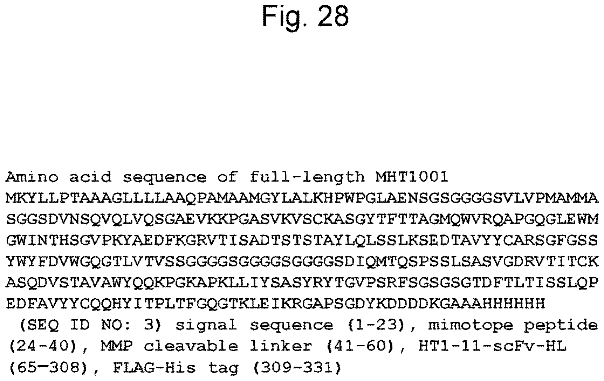

A restriction enzyme was added to the DNA fragment after the third round of panning, and a DNA fragment encoding a random peptide fragment was purified by a method known to a person skilled in the art. A DNA fragment was ligated to an *E. coli* expression vector by a method known to a person skilled in the art, so that the pelB signal sequence, the DNA fragment, the MMP cleavable linker (comprising a conventional MMP substrate (Journal of Controlled Release; 161: 804-812, 2012); amino acids 41 to 60 in the amino acid sequence as shown in SEQ ID NO: 3 and Figure 28), HT1-11 scFv (in the order of VH-VL or VL-VH), the FLAG tag, and the His tag would be translated in that order, so as to transform *E. coli* XL-1 Blue (Agilent Technologies). *E. coli* cells were cultured in the presence of isopropyl-(β-D-thiogalactopyranoside (IPTG) (Sigma-Aldrich), and a culture supernatant comprising peptide-fused scFv was collected.

3)-2 Acquisition of mimotope peptide

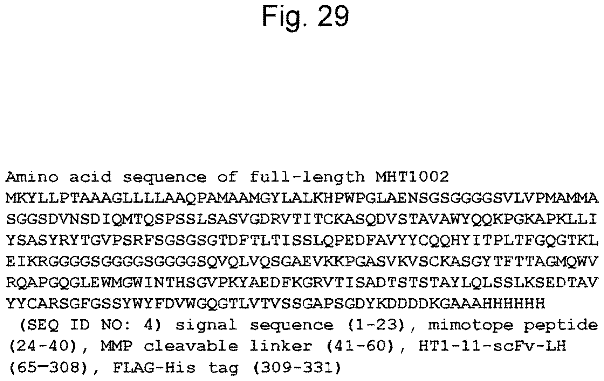

Binding intensity to the human TROP2 antigen was evaluated by ELISA in the same manner as in Example 1)-2. A culture supernatant comprising the peptide-fused scFv was diluted to 10-fold using TBS comprising 2 mM calcium chloride (MMP buffer) and allowed to react with 100 nM active human MMP1 (Accession Number: P03956) at 37°C for 15 minutes to cleave the MMP cleavable linker. The resultant was then added to the plate comprising the human TROP2 antigen immobilized thereon at 50 μl/well. The bound scFv was detected using the HRP-labeled anti-FLAG antibody (Sigma-Aldrich) diluted to 5,000-fold with ELISA buffer. The same procedure was implemented without the addition of MMP1, and clones exhibiting the high binding intensity ratio (under the condition with the addition of MMP1/under the condition without the addition of MMP1) were selected as positive clones. After an expression vector of positive clones was purified, the sequence of the translated region was analyzed by a method known to a person skilled in the art, and unique clones MHT1001 (Figure 28, SEQ ID NO: 3) and MHT1002 (Figure 29, SEQ ID NO: 4) were obtained.

3)-3 Evaluation of binding intensity of positive clone

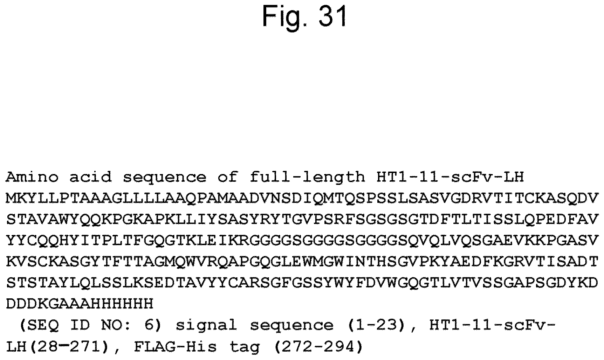

HT1-11-scFv-HL (Figure 30, SEQ ID NO: 5), HT1-11-scFv-LH (Figure 31, SEQ ID NO: 6), and the positive clones MHT1001 and MHT1002 identified in Example 3)-2 were expressed in *E. coli* XL-1 Blue, purified from the culture supernatant with the use of Ni Sepharose excel (Cytiva), and buffer-exchanged to TBS. The concentration of the purified antibody was determined by a method known to a person skilled in the art based on the absorbance at 280 nm obtained with the use of a spectrophotometer and the absorbance coefficient determined by the PACE method (Protein Science; 4 (11): 2411-2423, 1995). With the use of the purified samples, binding intensity to the human TROP2 antigen was evaluated by ELISA under the condition with the addition of MMP1 and under the condition without the addition of MMP1 in the same manner as in Example 3)-2. The antibody was adjusted to 1 μM with the use of MMP buffer, MMP 1 was added to the final concentration of 1 μM or 0 μM, and the reaction was allowed to proceed at 37°C for 15 minutes. Thereafter, the antibody with the concentration thereof being adjusted with ELISA buffer was added to the wells.

As shown in Figure 4-1 (A) and Figure 4-1 (B), HT1-11-scFv-HL and HT1-11-scFv-LH to which the mimotope peptide had not fused exhibited equivalent binding intensity with or without the addition of MMP1. Also, MHT1001 and MHT1002 to which the mimotope peptide had fused was found to exhibit higher binding intensity under the condition with the addition of MMP1 than under the condition without the addition of MMP1, and the $EC_{50}$ ratios of binding (under the condition without the addition of MMP1/under the condition with the addition of MMP1) were 7.9 and 4.3 (Figure 4-2 (C) and Figure 4-2 (D)), respectively.

(Example 4) Preparation of anti-TROP2 masked antibody and evaluation of binding intensity thereof

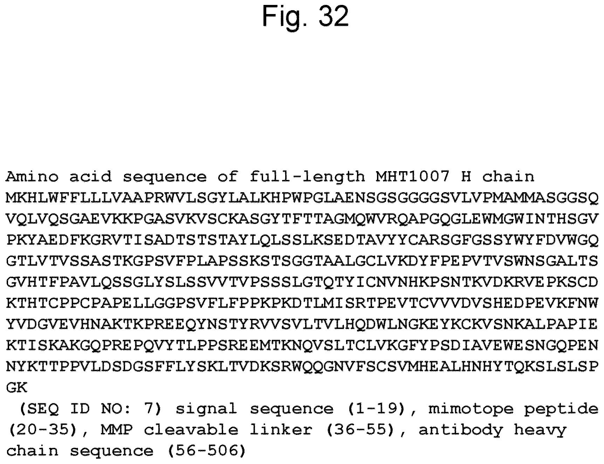

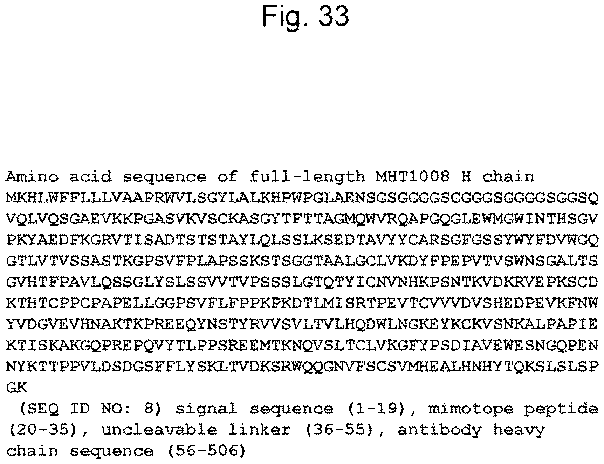

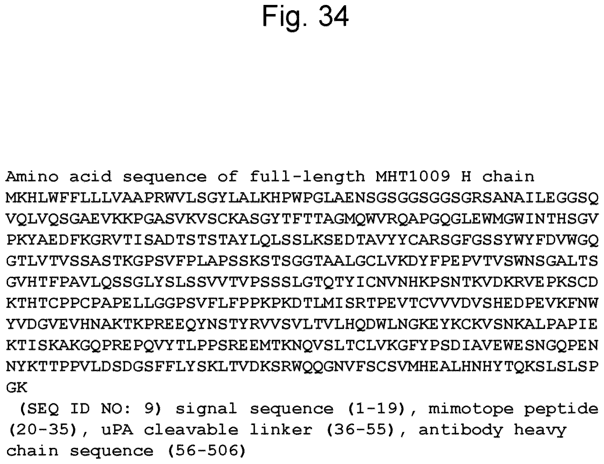

An expression vector for a heavy chain of the anti-TROP2 masked antibody MHT1007 comprising the MHT1001 mimotope peptide and the MMP cleavable linker (see Example 3)-1) (Table 1, Figure 32, SEQ ID NO: 7) fused thereto

was constructed by a method known to a person skilled in the art. Also, an expression vector for a heavy chain of MHT1008 in which the MMP cleavable linker portion had been modified into an uncleavable linker that would not be cleaved by a protease (Table 1, Figure 33, SEQ ID NO: 8) and an expression vector for a heavy chain of MHT1009 in which the MMP cleavable linker portion had been modified into an uPA cleavable linker (comprising a conventional uPA substrate (Journal of Biological Chemistry; 272 (33): 20456-20462, 1997): amino acids 36 to 55 in the amino acid sequence shown in Figure 34 and SEQ ID NO: 9) were constructed. An expression vector for the antibody heavy chain was used in combination with the expression vector for the HT1-11 light chain, masked antibodies were purified from the culture supernatant of the Expi293F cells in the same manner as in Example 1)-1, and the protein concentration was then determined. The masked antibodies prepared were designated as MHT1007, MHT1008, and MHT1009 corresponding to the names of heavy chain expression vectors (Table 1).

[Table 1]

| Anti-TROP2 masked antibody and amino acid sequence information | | |
|---|---|---|
| Masked antibody | Heavy chain sequence | Light chain sequence |
| MHT1007 | MHT1007 (SEQ ID NO: 7) | HT1-11 (SEQ ID NO: 2) |
| MHT1008 | MHT1008 (SEQ ID NO: 8) | HT1-11 (SEQ ID NO: 2) |
| MHT1009 | MHT1009 (SEQ ID NO: 9) | HT1-11 (SEQ ID NO: 2) |
| MHT3002 | MHT3002 (SEQ ID NO: 10) | HT1-11 (SEQ ID NO: 2) |
| MHT3201 | MHT3201 (SEQ ID NO: 13) | HT1-11 (SEQ ID NO: 2) |
| MHT3202 | MHT3202 (SEQ ID NO: 14) | HT1-11 (SEQ ID NO: 2) |
| MHT1713 | MHT1713 (SEQ ID NO: 15) | HT1-11 (SEQ ID NO: 2) |
| MHT3423 | MHT3423 (SEQ ID NO: 16) | HT1-11 (SEQ ID NO: 2) |
| MHT3203 | MHT3203 (SEQ ID NO: 17) | HT1-11 (SEQ ID NO: 2) |

Except for the ways described below, binding intensity to the human TROP2 antigen was evaluated by ELISA under the condition with the addition of a protease and under the condition without the addition in the same manner as in Example 1)-2 and Example 3)-2. Under the condition with the addition of a protease, MMP1 or active human uPA (Accession Number: P00749) (final concentration: 300 nM) was added to the 3 μM antibody, the reaction was allowed to proceed at 37°C, and the antibody with the concentration thereof being adjusted with ELISA buffer was added to wells comprising the human TROP2 antigen immobilized thereon.

As shown in Figure 5-1 (A), HT1-11 to which the mimotope peptide had not fused exhibited equivalent binding intensity with or without the addition of MMP1. Under the condition without the addition of a protease, binding intensity of MHT1007 comprising the mimotope peptide fused thereto was lower than that of HT1-11, and masking effects were also observed as with the case of scFv. Also, binding intensity was higher under the condition with the addition of MMP1 than under the condition without the addition of MMP1, and the $EC_{50}$ ratio of binding (under the condition without the addition of M1VVIP1/under the condition with the addition of MMP1) was 104 (Figure 5-1(B)). MHT1008 comprising an uncleavable linker exhibited lowered binding intensity with or without the addition of MMP1 (Figure 5-2(C)). MHT1009 comprising the uPA cleavable linker exhibited higher binding intensity under the condition with the addition of uPA than under the condition without the addition of uPA, the $EC_{50}$ ratio of binding (under the condition without the addition of uPA/under the condition with the addition of uPA) was 83 (Figure 5-2(D)). The results demonstrate that the mimotope obtained in Example 3 would function as a masking domain not only in the scFv form but also in the IgG form and that a cleavable linker sequence could be modified while maintaining the masking effects.

In order to verify that the peptide obtained was the mimotope, the Fab region of MHT1007 comprising the masking peptide and the MMP cleavable linker (see Example 3)-1) was prepared by a method known to a person skilled in the art, and the Fab region was crystallized and subjected to X-ray crystallography. The results demonstrate that the peptide had bound to the CDR of HT1-11 (the data are not shown). In addition, a chemically synthesized masking peptide was found to bind to HT1-11 competitively with the TROP2 antigen by SPR (the data are not shown).

(Example 5) Selection of calpain (CAPN) cleavable sequence using anti-TROP2 masked antibody

5)-1 Modification of CAPN cleavable sequence

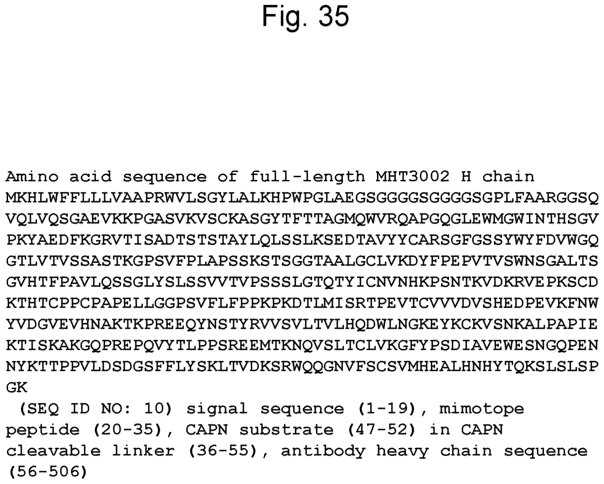

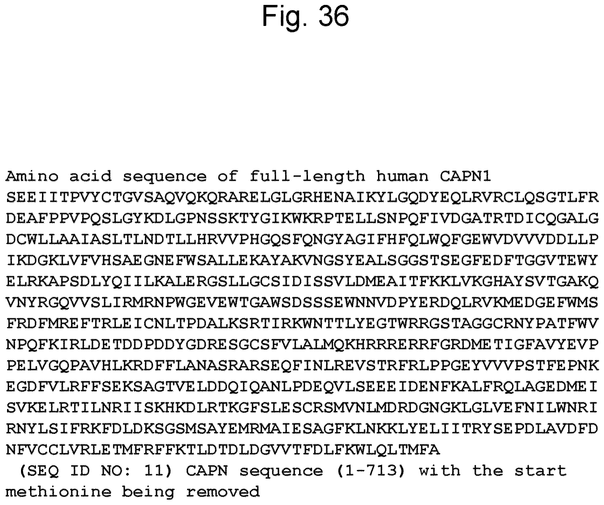

CAPN localized in the cytoplasm is indicated to leak extracellularly upon cell death (Non-Patent Literature 7). In order to examine that binding intensity of the masked antibody would be improved with the use of an intracellular protease; i.e., the CAPN substrate sequence, in combination with an extracellular protease; i.e., the uPA substrate sequence, compared with the binding intensity achieved with the use of the uPA substrate by itself, a CAPN substrate sequence was appropriately designed. The anti-TROP2 masked antibody MHT3002 (Table 1, Figure 35, SEQ ID NO: 10) comprising the known CAPN substrate composed of the amino acid sequence PLFAAR (SEQ ID NO: 10, amino acids 47 to 52 in the amino acid sequence shown in Figure 35) (Biochimica et Biophysica Acta.; 1794 (10): 1505-1509, 2009) comprising the cleavable linker was prepared. Except for the ways described below, binding intensity to the human TROP2 antigen was evaluated by ELISA under the condition with the addition of a protease and under the condition without the addition thereof in the same manner as in Example 4. After the masked antibodies were diluted to 10 nM with MMP buffer, 200 nM uPA or CAPN1 (Novus Biologicals; Figure 36, SEQ ID NO: 11) was added, the reaction was allowed to proceed at 37°C, and the reaction product was added to wells comprising the human TROP2 antigen immobilized thereon.

As shown in Figure 6 (A), binding intensity of MHT3002 was improved under the condition with the addition of uPA and CAPN1. This indicates that MHT3002 would be activated by both proteases.

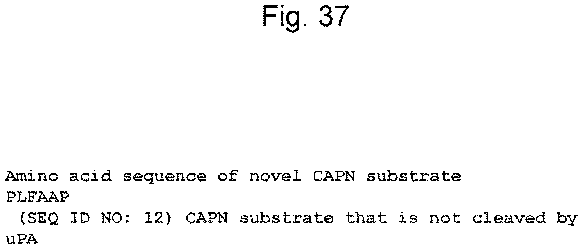

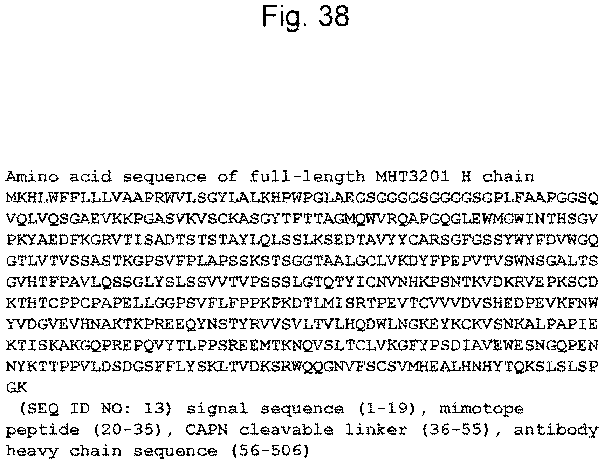

uPA is known to cleave just after Arg. In order to obtain a CAPN substrate that is not cleaved by uPA, a novel CAPN substrate (PLFAAP; Figure 37, SEQ ID NO: 12) was designed by substituting R in the CAPN substrate sequence (PLFAAR) with P (PLFAAP; Figure 37, SEQ ID NO: 12). The anti-TROP2 masked antibody MHT3201 comprising a cleavable linker comprising the obtained sequence was prepared (Table 1, Figure 38, SEQ ID NO: 13), and sensitivity thereof to uPA and CAPN1 was evaluated by ELISA in the manner described above.

As shown in Figure 6 (A), binding intensity of the anti-TROP2 masked antibody MHT3201 was improved selectively under the condition with the addition of CAPN1, and MHT3201 was not activated by uPA.

5)-2 Reactivity of novel CAPN substrate (PLFAAP) to MMP

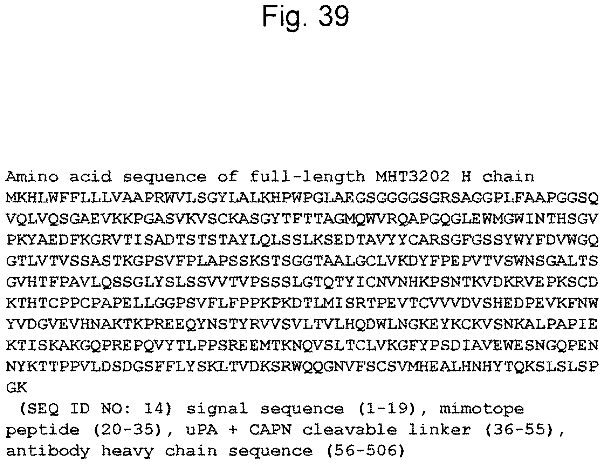

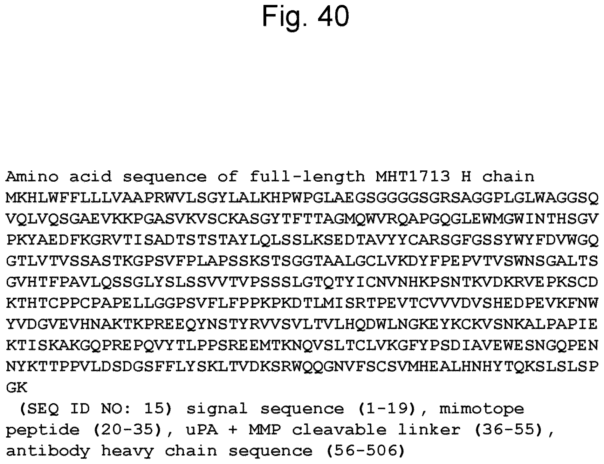

MHT3202 comprising both a conventional uPA substrate (Journal of Biological Chemistry; 272 (33): 20456-20462, 1997) and a novel CAPN substrate (PLFAAP) in the cleavable linker (Table 1, Figure 39, SEQ ID NO: 14) and MHT1713 comprising the uPA substrate and a conventional MMP substrate composed of PLGLWA (Biopolymers; 40 (4): 399-416, 1996) (Table 1, Figure 40, SEQ ID NO: 15) were prepared. Whether or not MHT3202 and MHT1713 would be activated by human MMP1 (Accession Number: P03956), human MMP2 (Accession Number: P08253), human MMP3 (Accession Number: P08254), human MMP7 (Accession Number: P09237), human MMP9 (Accession Number: P14780), human MMP12 (Accession Number: P39900), or human MMP14 (Accession Number: P50281) was evaluated by ELISA in the same manner as in Example 5)-1, except for the ways described below. The antibodies were diluted to 20 nM with MMP buffer, the proteases were added to 50 nM, the reaction was allowed to proceed at 37°C, and the reaction product was then added to the wells comprising the human TROP2 antigen immobilized thereon.

As shown in Figure 6 (B), binding intensity of MHT1713 was improved under the condition with the addition of MMP, but binding intensity of MHT3202 was not improved under the condition with the addition of MMP. The results demonstrate that a novel CAPN substrate (PLFAAP) would not be cleaved by the MMP.

(Example 6) Preparation of anti-TROP2 masked antibody subjected to ADC preparation and evaluation of binding intensity thereof

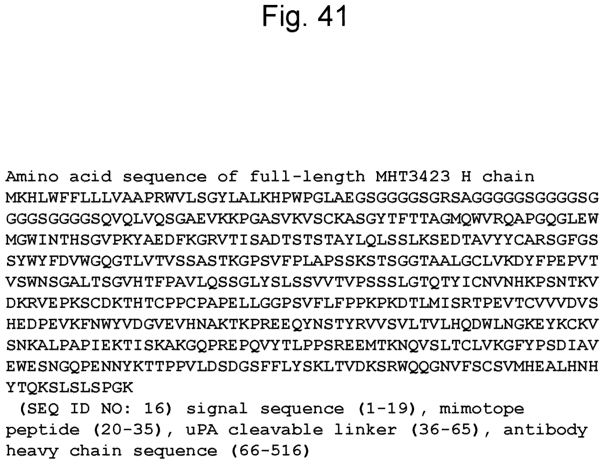

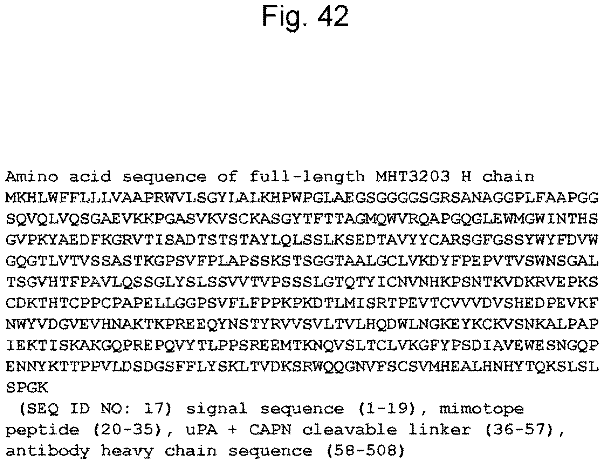

In order to examine that binding intensity of the masked antibody would be improved with the use of an intracellular protease; i.e., the CAPN substrate sequence, in combination with an extracellular protease; i.e., the uPA substrate sequence, compared with the binding intensity achieved with the use of the uPA substrate by itself, MHT3423 selectively comprising the uPA substrate (Table 1, Figure 41, SEQ ID NO: 16), MHT3201 comprising selectively the CAPN substrate, MHT3202 comprising both the uPA substrate and the CAPN substrate, and MHT3203 comprising both a partial sequence of the conventional uPA substrate (Journal of Biological Chemistry; 272 (33): 20456-20462, 1997) and the CAPN substrate (Table 1, Figure 42, SEQ ID NO: 17) were designed. The antibodies were prepared in the same manner as in Example 1)-1, and binding intensity of the antibodies to the human TROP2 antigen was evaluated by ELISA under the condition with the addition of a protease and under the condition without the addition thereof in the same manner as in Example 4, except for the ways described below. Under the condition with the addition of a protease, the antibody was diluted to 2,000 nM with MMP buffer, 200 nM uPA or CAPN1 was added thereto, the reaction was allowed to proceed at 37°C, and the antibody with the concentration thereof being adjusted with MMP buffer was added to the wells comprising the

human TROP2 antigen immobilized thereon. Under the condition without the addition of a protease, the MMP-buffered antibody was also added to the wells in the same manner.

As shown in Figure 7-1 (A), binding intensity of MHT3423 was improved under the condition with the addition of uPA, compared with the binding intensity achieved under the condition without the addition of an enzyme, and binding intensity was not improved under the condition with the addition of CAPN1. Also, binding intensity of MHT3201 was improved selectively under the condition with the addition of CAPN1 (Figure 7-1 (B)). Binding intensity of MHT3202 and MHT3203 comprising both the uPA substrate and the CAPN substrate was improved both under the condition with the addition of uPA and under the condition with the addition of CAPN1 (Figure 7-2 (C) and Figure 7-2 (D)). The results demonstrate that the designed masked antibodies have protease sensitivity of interest.

(Example 7) Production of antibody-drug conjugate

As drug linkers to produce antibody-drug conjugates, N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide (Example 58, Step 8, WO 2014/057687, hereafter, referred to as "Linker 1") and N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepine]-10'(5'H)-yl]carbonyl}oxy)methyl]phenyl}-L-alanineamide (Example 2-1, WO 2020/196474, hereafter, referred to as "Linker 2") were used.

Common operation for production and identification of antibody-drug conjugate

Common operation A: Concentration of aqueous solution of antibody and aqueous solution of antibody-drug conjugate

Aqueous solutions were concentrated using Amicon Ultra (50,000 MWCO, Millipore Corporation) and Allegra X-15R Centrifuge (Beckman Coulter) (SX4750A) (4000 g).

Common operation B: Measurement of antibody concentration

Antibody concentration was measured with the use of the UV photometer (Nanodrop 1000, Thermo Fisher Scientific Inc.) in accordance with the manufacturer's instructions.

Common operation C: Antibody buffer exchange

C-1: Buffer exchange with phosphate buffer comprising NaCl (50 mM) and EDTA (5 mM) (50 mM, pH 6.0) (hereafter, referred to as "PBS 6.0/EDTA")

The NAP-25 columns (Cat. No. 17085202, Cytiva, NAP-25 Columns Sephadex, hereafter referred to as "NAP-25") were equilibrated with phosphate buffer comprising NaCl (50 mM) and EDTA (5 mM) (50 mM, pH 6.0) (hereafter, referred to as "PBS 6.0/EDTA") with the use of NAP-25 columns in accordance with the manufacturer's instructions. To a NAP-25 column, 2.5 ml of an aqueous solution of an antibody was applied, and a fraction (3.5 ml) was eluted with the use of 3.5 ml of PBS 6.0/EDTA. In accordance with the common operation A and the common operation B, this fraction was concentrated, and the concentration thereof was measured. Thereafter, the concentration was adjusted to 10 mg/ml with the use of PBS 6.0/EDTA.

C-2: Buffer exchange with phosphate buffer (50 mM, pH 6.0) (hereafter, referred to as "PB 6.0")

PB 6.0 was added to the aqueous solution of the antibody, and the aqueous solution was concentrated in accordance with the common operation A. This operation was repeated several times, the concentration was measured (the common operation B), and the concentration was adjusted to 10 mg/ ml with the use of PB 6.0.

Common operation D: Purification of antibody-drug conjugate

An antibody fraction was eluted with the use of NAP-25 in accordance with the manufacturer's instructions. For column equilibration and elution, 5 (w/v)% sorbitol-10 mM acetate buffer (hereafter referred to as "ABS") was used.

Common operation E: Measurement of antibody concentration in antibody-drug conjugate

The concentration C' (mg/ml) of the antibody-drug conjugate was determined in accordance with Equation (I): Absorbance A280 = molar absorption coefficient $\varepsilon_{280}$ ($L \cdot mol^{-1} \cdot cm^{-1}$) × molar concentration C ($mol \cdot L^{-1}$) × cellular optical path length l (cm) based on the Lambert-Beer law.

$$C'(mg \cdot mL^{-1}) = MW(g \cdot mol^{-1}) \cdot C(mol \cdot L^{-1}) = \frac{A_{280} \cdot MW(g \cdot mol^{-1})}{\varepsilon_{280}(L \cdot mol^{-1} \cdot cm^{-1}) \cdot l(cm)} \quad \text{Equation (I)}$$

Values used to calculate C' (mg/ ml) were determined as described below.

- Absorbance A280: Actually measured UV absorbance of the aqueous solution of the antibody-drug conjugate at 280 nm
  Measurement was performed with the use of Lamba25 (PerkinElmer)
- Molar weight MW ($g \cdot mol^{-1}$): Estimated antibody molecular weight determined based on the amino acid sequence of the antibody (used as the approximate value of the molar mass of the antibody-drug conjugate).
- Optical path length l (cm): 1 cm
- Molar absorption coefficient $\varepsilon_{280}$ of the antibody drug conjugate: determined in accordance with Equation (II) below

$\varepsilon_{280}$ = molar absorption coefficient of the antibody $\varepsilon_{Ab,280}$ + molar absorption coefficient of the drug $\varepsilon_{DL,280}$ × number of grugs bound    Equation (II)

$\varepsilon_{Ab,280}$: the molar absorption coefficient of the antibody at 280 nm
$\varepsilon_{DL,280}$: the molar absorption coefficient of the drug linker at 280 nm
Number of drugs bound: determined in accordance with the common operation F (see the description below)
$\varepsilon_{Ab,280}$ determined based on the amino acid sequence of the antibody in accordance with a known calculation method (Protein Science, 1995, vol. 4, 2411-2423) was used (see Table 2 below).

$\varepsilon_{DL,280}$ of the Linker 1 was determined by allowing the linker 1 to react with mercaptoethanol or N-acetylcysteine, converting a maleimide group into succinimidethioether to obtain a compound, and measuring a molar absorption coefficient (280 nm) of the compound. As $\varepsilon_{DL,280}$ of the Linker 2, the actually measured molar absorption coefficient (280 nm) was used.

Common operation F: Measurement of average number of drugs bound to an antibody molecule in an antibody-drug conjugate

The average number of drugs bound to an antibody molecule in an antibody-drug conjugate was measured by reversed-phase chromatography (RPC).

F-1. Sample preparation (reduction of disulfide between antibody molecules)

After an aqueous solution of dithiothreitol (DTT) (100 mM, 15 μl) was added to the solution of the antibody-drug conjugate (approximately 1 mg/ml, 60 μl), the mixture was incubated at 37°C for 30 minutes, and the antibody'disulfide bonds between its light chains and heavy chains were cleaved.

F-2. HPLC analysis

HPLC analysis was performed under the following conditions.

- HPLC system: Agilent1290 HPLC system (Agilent Technologies)
- Detector: ultraviolet photometer (measurement wavelength: 280 nm)
- Column: ACQUITY UPLC BEH Phenyl (2.1 × 50 mm, 1.7 μm, 130 Å; Waters, PIN186002884)
- Column temperature: 75°C
- Mobile phase A: 0.10 (v/v)% trifluoroacetic acid (TFA), 15 (v/v)% 2-propanol solution
- Mobile phase B: 0.075 (v/v)% TFA, 15 (v/v)% 2-propanol, acetonitrile solution
- Gradient program: (min, B%): (0, 14)-(15, 36)-(17, 80)-(17.01, 14)-(25, 14)

- Amount of sample injection: 10 μl

F-3. Data analysis

F-3-1

Compared to the antibody light chain (L0) and heavy chain (H0) to which no drug has bound, the drug-bound light chain (a light chain comprising "i" number of drugs bound thereto: Li) and heavy chain (a heavy chain comprising "i" number of drugs bound thereto: Hi) exhibit enhanced hydrophobicity in proportion of the number of drugs bound thereto, and the retention time is prolonged. In comparison with the retention time of L0 and H0, accordingly, the detection peaks can be assigned to any of L0, L1, H0, H1, H2, and H3.

F-3-2

Since a drug linker has UV absorption, the peak area values are corrected in accordance with the following formula with the use of the molar absorption coefficients of the light chain, the heavy chain, and the drug linker according to the number of binding of the drug linker.

$$\text{Corrected peak area value of light chain comprising "i" number of drugs bound thereto } (A_{Li})$$

$$= \text{peak area} \times \frac{\text{molar absorption coefficient of light chain}}{\text{molar absorption coefficient of light chain} + \text{number of drugs bound (i)} \times \text{molar absorption coefficient of drug linker}}$$

$$\text{Corrected peak area value of heavy chain comprising "i" number of drugs bound thereto } (A_{Hi})$$

$$= \text{peak area} \times \frac{\text{molar absorption coefficient of heavy chain}}{\text{molar absorption coefficient of heavy chain} + \text{number of drugs bound (i)} \times \text{molar absorption coefficient of drug linker}}$$

The molar absorption coefficients (280 nm) of antibody light chain and heavy chain deduced based on the amino acid sequences of the antibody light chain and heavy chain in accordance with the conventional method of calculation (Protein Science, 1995, vol. 4, 2411-2423) were used (see Table 2 below).

F-3-3

An each chain peak area ratio (%) relative to the corrected total peak area value was determined in accordance with the equations below.

$$\text{Peak area ratio of light chain comprising "i" number of drugs bound thereto } = \frac{A_{Li}}{A_{L0} + A_{L1}} \times 100$$

$$\text{Peak area ratio of heavy chain comprising "i" number of drugs bound thereto } = \frac{A_{Hi}}{A_{H0} + A_{H1} + A_{H2} + A_{H3}} \times 100$$

Corrected peak area of each of $A_{Li}$, $A_{Hi}$: $L_i$, $H_i$

F-3-4

The average number of drugs bound to an antibody molecule in an antibody-drug conjugate was determined in accordance with the equation below.

Average number of drugs bound = (L0 peak area ratio × 0 + L1 peak area ratio × 1 + H0 peak area ratio × 0 + H1 peak area ratio × 1 + H2 peak area ratio × 2 + H3 peak area ratio × 3)/100 × 2

[Table 2]

| Molar absorption coefficient, absorption coefficient, and molecular weight of antibody | | | | | |
|---|---|---|---|---|---|
| Antibody | Molar absorption coefficient at 280 nm ($L \cdot mol^{-1} \cdot cm^{-1}$) | | | Absorption coefficient ($L \cdot g^{-1} \cdot cm^{-1}$) | Molecular weight of antibody |
| | Whole | Heavy chain | Light chain | | |
| MhM1018-M1 | 254340 | 77008 | 50162 | 1.67 | 152093 |
| MhM1019-M1 | 254340 | 77008 | 50162 | 1.67 | 152689 |
| MhM1020-M1 | 254340 | 77008 | 50162 | 1.67 | 152710 |
| MhM1021-M1 | 254340 | 77008 | 50162 | 1.66 | 153078 |
| MhM1022-M1 | 254340 | 77008 | 50162 | 1.67 | 152594 |
| MhM1023-M1 | 254340 | 77008 | 50162 | 1.66 | 152982 |
| MHT3423 | 237380 | 90988 | 27702 | 1.56 | 152399 |
| MHT3201 | 237380 | 90988 | 27702 | 1.57 | 151360 |
| MHT3202 | 237380 | 90988 | 27702 | 1.57 | 151587 |
| MHT3203 | 237380 | 90988 | 27702 | 1.56 | 151957 |
| MHT1008 | 237380 | 90988 | 27702 | 1.57 | 151086 |

| Drug linker | Molar absorption coefficient at 280 run ($L \cdot mol^{-1} \cdot cm^{-1}$) |
|---|---|
| Linker 1 | 5440 |
| Linker 2 | 23155 |

7)-1 Synthesis of MhM1018-M1-DXd-ADC

[Chemical formula 1]

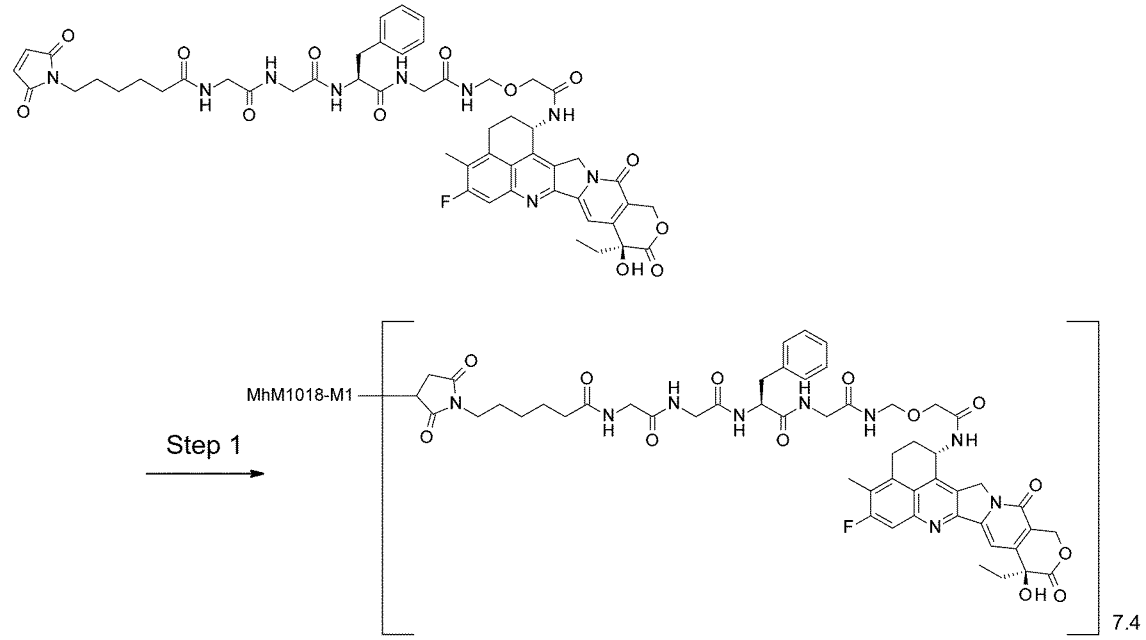


Step 1: Antibody-drug conjugate (1)

Antibody reduction:

A PBS6.0/EDTA solution of MhM1018-M1 produced in Example 13 was prepared in accordance with the common operation B and the common operation C-1 to be 10 mg/ml. To 1.45 ml of the resulting solution, an aqueous solution of 1 M potassium dihydrogen phosphate (Nacalai Tesque, Inc.; 0.0218 ml) and an aqueous solution of 10 mM TCEP (Tokyo Chemical Industry Co., Ltd.) (0.0581 ml; 6.0 molar equivalents per one molecule of the antibody) were added. The resultant was incubated at 37°C for 2 hours to reduce disulfide bonds between antibody chains.

Conjugation of antibody to drug linker:

The solution was incubated at 15°C for 10 minutes. Subsequently, a solution of the Linker 1 in 10 mM dimethylsulfoxide (0.0969 ml; 10 molar equivalents per one molecule of the antibody) was added, and the mixture was incubated at 15°C for 1 hour to bind a drug linker to an antibody. Subsequently, an aqueous solution of 100 mM NAC (Sigma-Aldrich Co. LLC) (0.0097 ml; 10 molar equivalents per one molecule of the antibody) was added, the mixture was stirred, and the resultant was allowed to stand at room temperature for 20 minutes to terminate the reaction of the drug linker.

Purification:

The solution was purified in accordance with the common operation D to obtain 7 ml of a solution comprising the title antibody-drug conjugate "MhM1018-M1-DXd-ADC."

Property evaluation:

The property values indicated below were obtained in accordance with the common operation E and the common operation F.
Antibody concentration: 1.75 mg/ml; antibody yield: 12.27 mg (83%); average number of drugs bound to an antibody molecule (n): 7.4

7)-2 Synthesis of MhM1019-M1-DXd-ADC

[Chemical Formula 2]

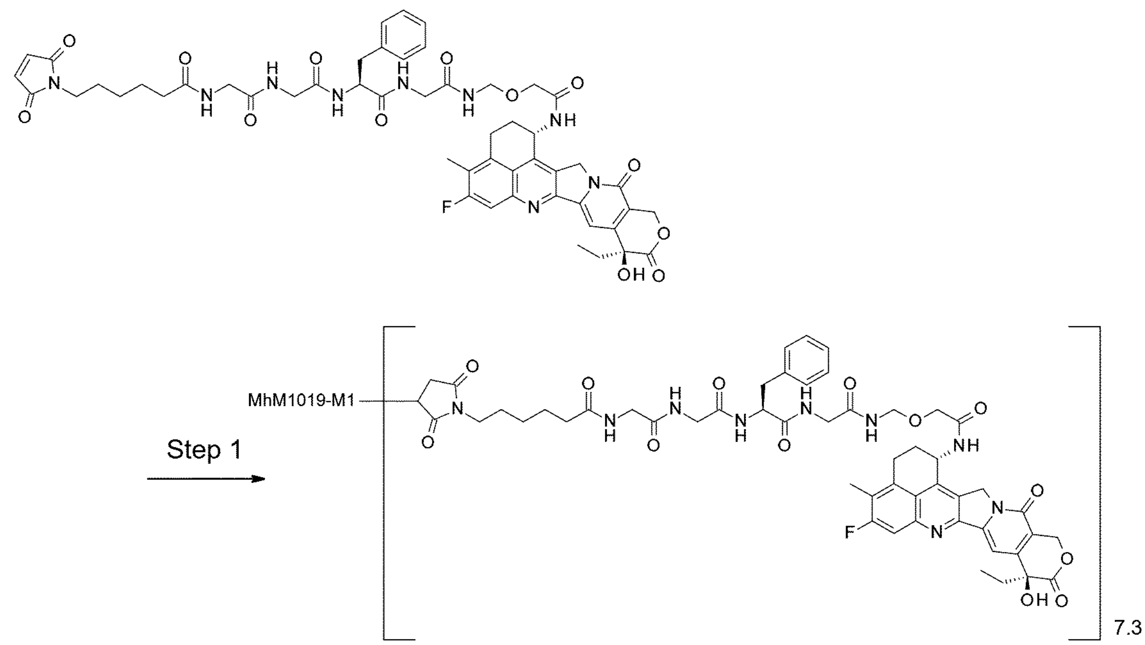


Step 1: Antibody-drug conjugate (2)

With the use of 1.50 ml of MhM1019-M1 prepared in Example 13 (a 280 nm absorption coefficient (1.66 ml $mg^{-1}$ $cm^{-1}$) was used), the title antibody-drug conjugate "MhM1019-M1-DXd-ADC" was obtained in the same manner as in Step 1 of Example 7)-1.

Property evaluation:

The property values indicated below were obtained in accordance with the common operation E and the common operation F.
Antibody concentration: 1.85 mg/ml; antibody yield: 12.94 mg (87%); average number of drugs bound to an antibody molecule (n): 7.3

7)-3 Synthesis of MhM1020-M1-DXd-ADC

[Chemical Formula 3]

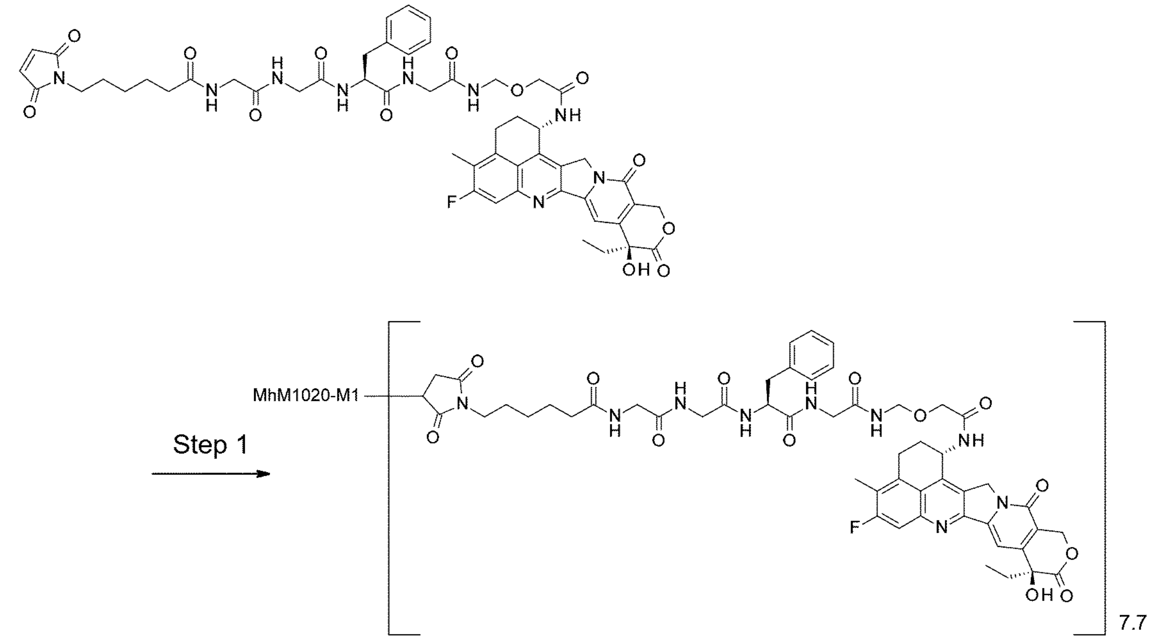


Step 1: Antibody-drug conjugate (3)

With the use of 1.44 ml of MhM1020-M1 prepared in Example 13 (a 280 nm absorption coefficient (1.66 ml $mg^{-1}$ $cm^{-1}$) was used), the title antibody-drug conjugate "MhM1020-M1-DXd-ADC" was obtained in the same manner as in Step 1 of Example 7)-1.

Property evaluation:

The property values indicated below were obtained in accordance with the common operation E and the common operation F.
Antibody concentration: 1.40 mg/ml; antibody yield: 9.79 mg (67%); average number of drugs bound to an antibody molecule (n): 7.7

7)-4 Synthesis of MhM1021-M1-DXd-ADC

[Chemical Formula 4]

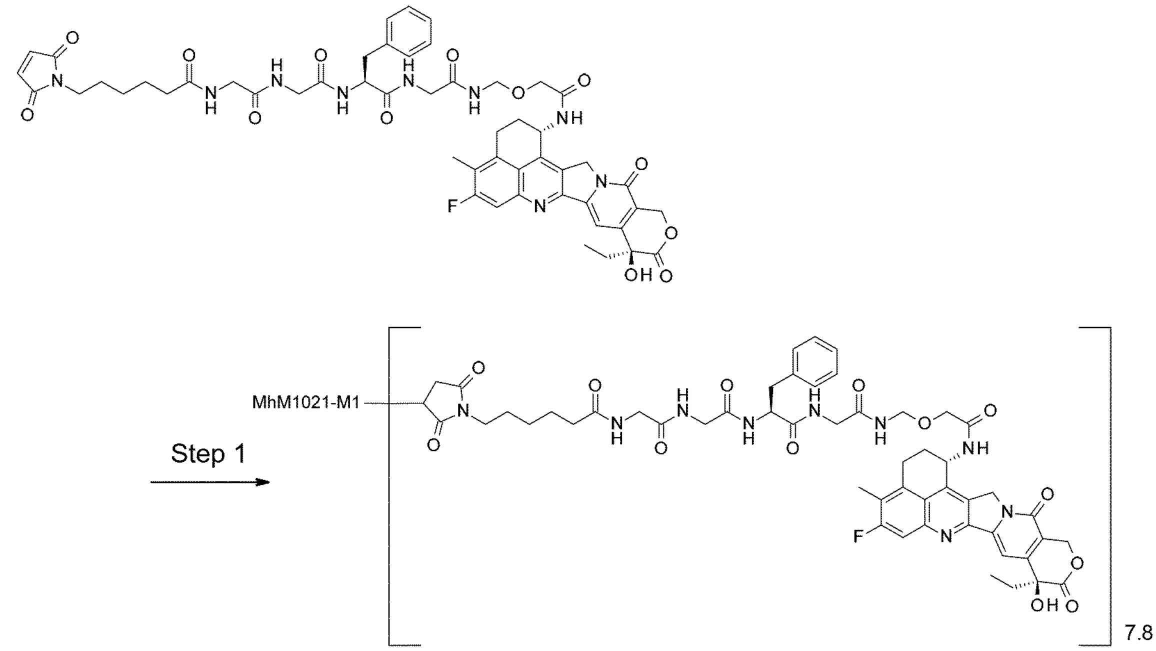


Step 1: Antibody-drug conjugate (4)

With the use of 1.43 ml of MhM1021-M1 prepared in Example 13 (a 280 nm absorption coefficient (1.66 ml $mg^{-1}$ $cm^{-1}$) was used), the title antibody-drug conjugate "MhM1021-M1-DXd-ADC" was obtained in the same manner as in Step 1 of Example 7)-1.

Property evaluation:

The property values indicated below were obtained in accordance with the common operation E and the common operation F.
Antibody concentration: 1.44 mg/ml; antibody yield: 10.10 mg (69%); average number of drugs bound to an antibody molecule (n): 7.8

7)-5 Synthesis of MhM1022-M1-DXd-ADC

[Chemical Formula 5]

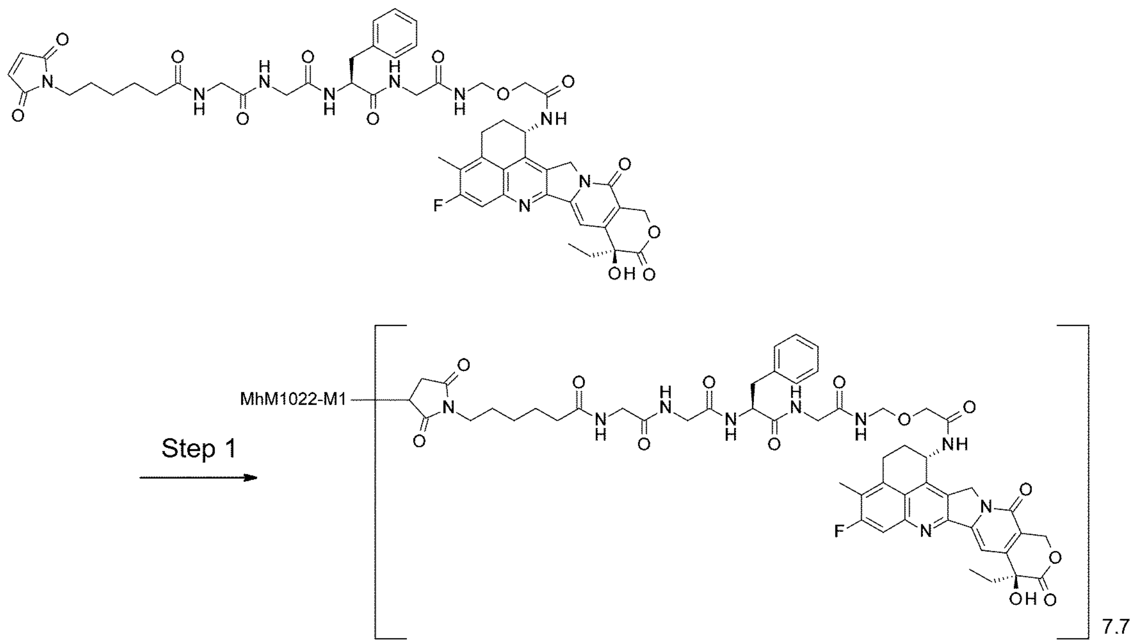


Step 1: Antibody-drug conjugate (5)

With the use of 1.51 ml of MhM1022-M1 prepared in Example 13 (a 280 nm absorption coefficient (1.66 ml $mg^{-1}$ $cm^{-1}$) was used), the title antibody-drug conjugate "MhM1022-M1-DXd-ADC" was obtained in the same manner as in Step 1 of Example 7)-1.

Property evaluation:

The property values indicated below were obtained in accordance with the common operation E and the common operation F.
Antibody concentration: 1.52 mg/ml; antibody yield: 10.62mg (69%); average number of drugs bound to an antibody molecule (n): 7.7

7)-6 Synthesis of MhM1023-M1-DXd-ADC

[Chemical Formula 6]

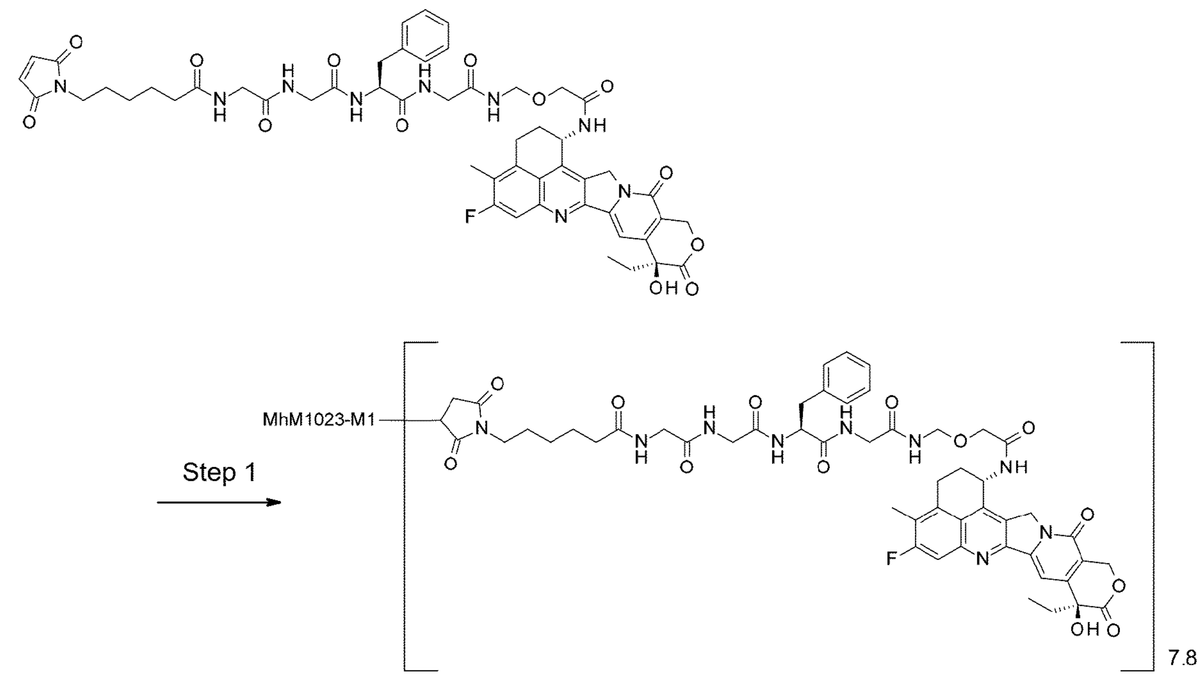


Step 1: Antibody-drug conjugate (6)

With the use of 1.36 ml of MhM1023-M1 prepared in Example 13 (a 280 nm absorption coefficient (1.66 ml $mg^{-1}$ $cm^{-1}$) was used), the title antibody-drug conjugate "MhM1023-M1-DXd-ADC" was obtained in the same manner as in Step 1 of Example 7)-1.

Property evaluation:

The property values indicated below were obtained in accordance with the common operation E and the common operation F.
Antibody concentration: 1.76 mg/ml; antibody yield: 12.32 mg (89%); average number of drugs bound to an antibody molecule (n): 7.8

7)-7 Synthesis of MHT3423-PBD-ADC

[Chemical Formula 7]

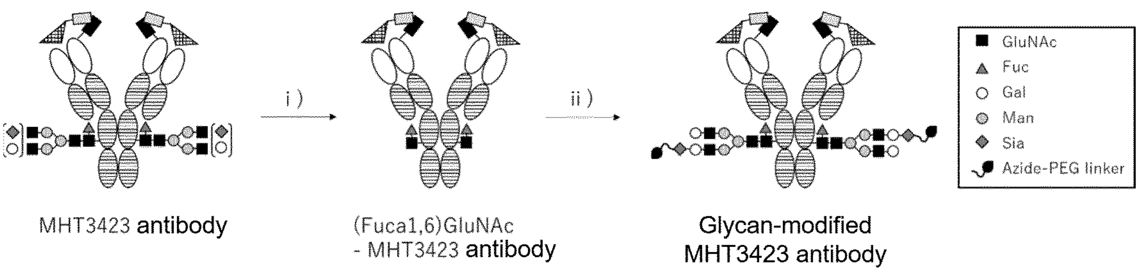

i ) Endo S enzyme

ii ) Endo S (D233Q/Q303L) enzyme, glycan oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fuca1,6)GlucNAc-MHT3423 antibody

The MHT3423 antibody solution prepared in Example 6 was buffer-exchanged to phosphate buffer (50 mM, pH 6.0) (hereafter referred to as "PB6.0") in accordance with the common operation C-2 to obtain 1.15 ml of a 19.4mg/ml antibody solution. To the resulting solution, 0.0148 ml of the Endo S solution (PBS, 7.52 mg/ml) was added, and the resultant was incubated at 37°C for 2 hours. After cleavage of the glycan was confirmed with the use of the Agilent 2100 bioanalyzer, the solution was purified with GST and a protein A column (AKTA). After the target fraction was substituted with PB6.0, the solution of (Fuca1,6)GlucNAc-MHT3423 antibody (19.3 mg/ml, 0.995 ml) was obtained.

Step 2: Preparation of glycan-modified MHT3423 antibody

To the (Fuca1,6)GlucNAc-MHT3423 antibody (PB6.0) (19.3 mg/ml, 0.995 ml) obtained in Step 1 above, a solution of glycan oxazoline (3aR,5R,6S,7R,7aR)-3a,6,7,7a-tetrahydro-7-hydroxy-5-(hydroxymethyl)-2-methyl-5H-pyrano[3,2-d]oxazol-6-yl O-[N5-acetyl-N1-[2-[2-[2-(2-azidoethoxy)ethoxy]ethoxy]ethyl]-α-neuraminamidosyl]-(2→6)-O-β-D-galactopyranosyl-(1→4)-O-2-(acetylamino)-2-deoxy-β-D-glucopyranosyl-(1→2)-O-α-D-mannopyranosyl-(1→3)-O-[O-β-D-galactopyranosyl-(1→4)-O-2-(acetylamino)-2-deoxy-β-D-glucopyranosyl-(1→2)-α-D-mannopyranosyl-(1-6)]-β-D-mannopyranoside (Example 56, WO 2019/065964, hereafter referred to as the "[N3-PEG(3)]-MSG1-Ox)" (Figure 8) (PB6.0) (50.0 mg/ml, 0.0578 ml) and the GST-Endo S D233Q/Q303L solution (WO 2017/010559) (PBS) (4.80 mg/ml, 0.0800 ml) were added, and the mixture was incubated at 30°C for 3 hours. After glycan transfer was confirmed with the use of the Agilent 2100 bioanalyzer, the solution was purified with GST and a CHT column (AKTA). A fraction comprising a target product was substituted with 10 mM acetate buffer solution, 5 (w/v/)% sorbitol (pH 5.5) (hereafter referred to as "ABS") to obtain the glycan-modified MHT3423 antibody (9.99 mg/ml, 1.56 ml).

[Chemical Formula 8]

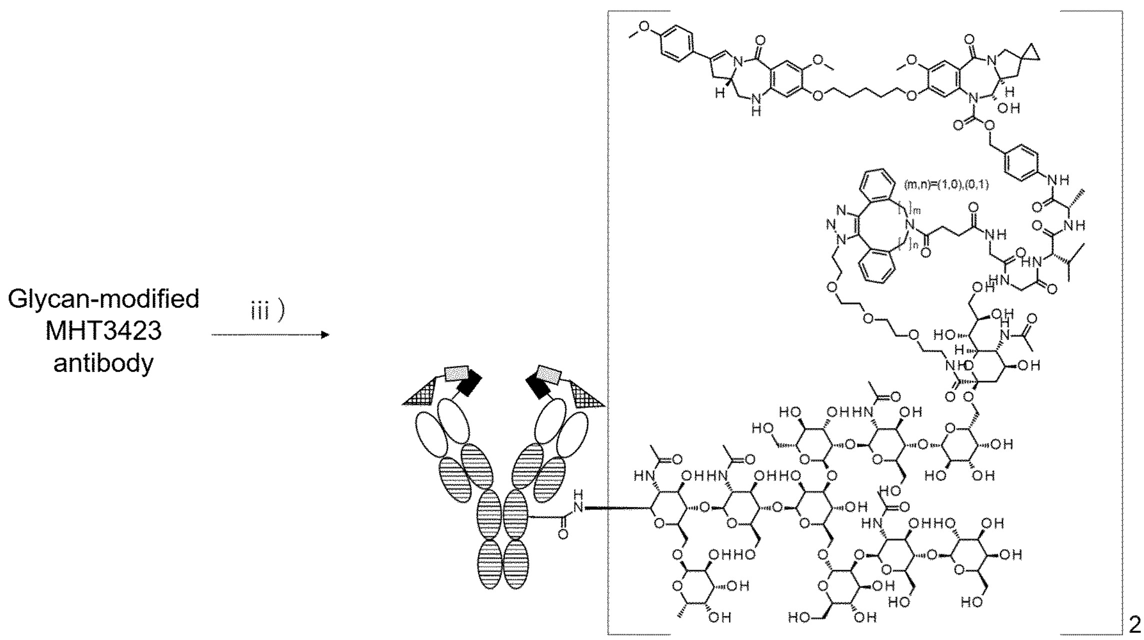


MHT3423 -PBD-ADC

iii) Linker 2

Step 3: Conjugation of antibody to drug linker

To the glycan-modified MHT3423 (ABS) (9.99 mg/ml, 0.500 ml) obtained in Step 2 above, 1,2-propanediol (0.480 ml) and a solution of the Linker 2 in 10 mM dimethyl sulfoxide (0.0197 ml; 6 molar equivalents per one molecule of the antibody) were added at room temperature, and the reaction was allowed to proceed with the use of the tube rotator (MTR-103, AS ONE Corporation) at room temperature for 48 hours.

Purification operation:

The solution was purified two times in accordance with the common operation D to obtain 2.50 ml of the MHT3423-PBD-ADC solution.

Property evaluation:

The property values indicated below were obtained in accordance with the common operation E and the common operation F.
Antibody concentration: 1.59 mg/ml; antibody yield: 3.97 mg (79%); average number of drugs bound to an antibody molecule (n): 1.8

7)-8 Synthesis of MHT3201-PBD-ADC

[Chemical Formula 9]

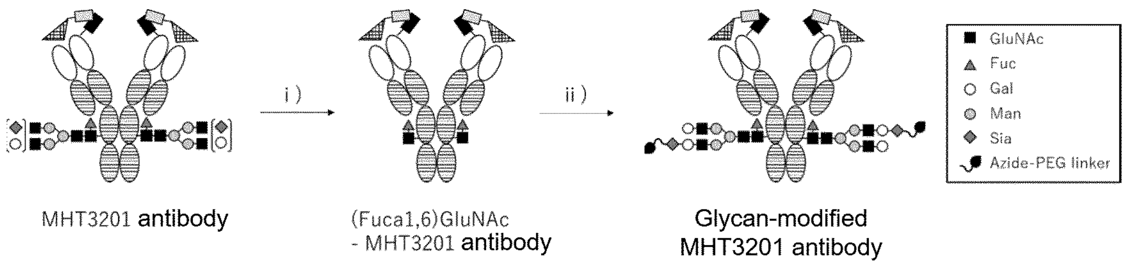


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT3201 antibody

The MHT3201 antibody solution prepared in Example 6 was buffer-exchanged to PB6.0 and adjusted to ca. 20.2 mg/ml (1.43 ml). In the same manner as in Step 1 of Example 7)-7, the (Fucα1,6)GlcNAc-MHT3201 antibody solution (PB6.0) (19.4 mg/ml, 1.27 ml) was obtained.

Step 2: Preparation of glycan-modified MHT3201

With the use of the (Fucα1,6)GlcNAc-MHT3201 antibody solution (PB6.0) (19.4 mg/ml, 1.27 ml) obtained in Step 1 above, the glycan-modified MHT3201 antibody (ABS) (9.96 mg/ml, 1.72 ml) was obtained in the same manner as in Step 2 of Example 7)-7.

[Chemical Formula 10]

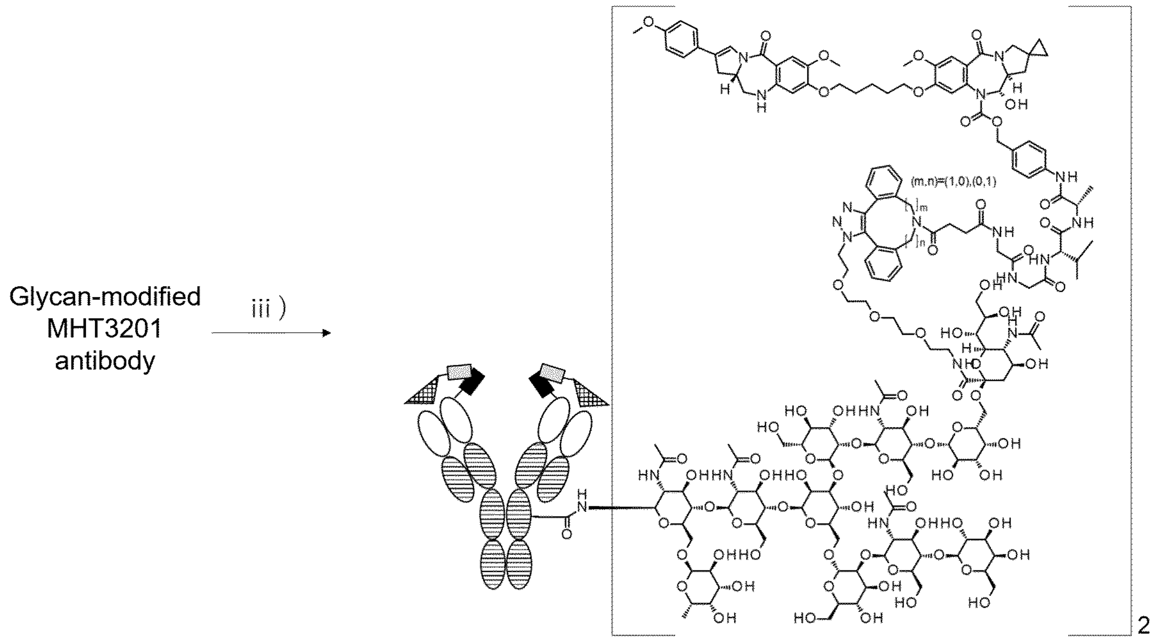


MHT3201 -PBD-ADC

iii) Linker 2

Step 3: Conjugation of antibody to drug linker

With the use of the glycan-modified MHT3201 (ABS) (9.96 mg/ml, 0.500 ml) obtained in Step 2 above, 2.50 ml of the MHT3201-PBD-ADC antibody solution (ABS) was obtained in the same manner as in Step 3 of Example 7)-7.

Property evaluation:

The property values indicated below were obtained in accordance with the common operation E and the common operation F.

Antibody concentration: 1.41 mg/ml; antibody yield: 3.52 mg (71%); average number of drugs bound to an antibody molecule (n): 1.9

7)-9 Synthesis of MHT3202-PBD-ADC

[Chemical Formula 11]

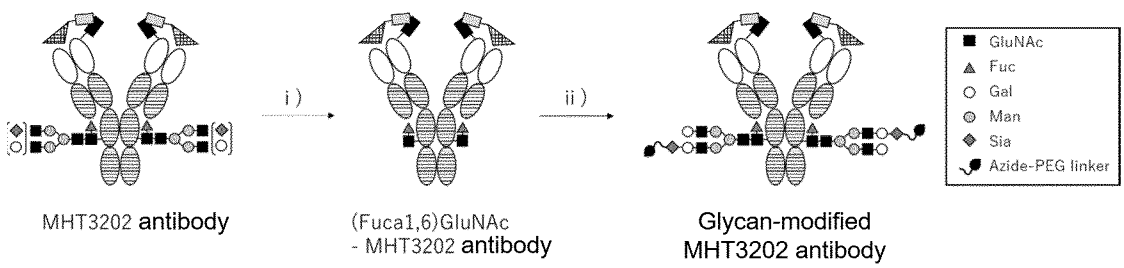

i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT3202 antibody

The MHT3202 antibody solution prepared in Example 6 was buffer-exchanged to PB6.0 and adjusted to ca. 19.8 mg/ml (1.12 ml). In the same manner as in Step 1 of Example 7)-7, the (Fucα1,6)GlcNAc-MHT3202 antibody solution (PB6.0) (20.5 mg/ml, 0.959 ml) was obtained.

Step 2: Preparation of glycan-modified MHT3202 antibody

With the use of the (Fucα1,6)GlcNAc-MHT3202 antibody solution (PB6.0) (20.5 mg/ml, 0.959 ml) obtained in Step 1 above, the glycan-modified MHT3202 antibody (ABS) (9.93 mg/ml, 1.74 ml) was obtained in the same manner as in Step 2 of Example 7)-7.

[Chemical Formula 12]

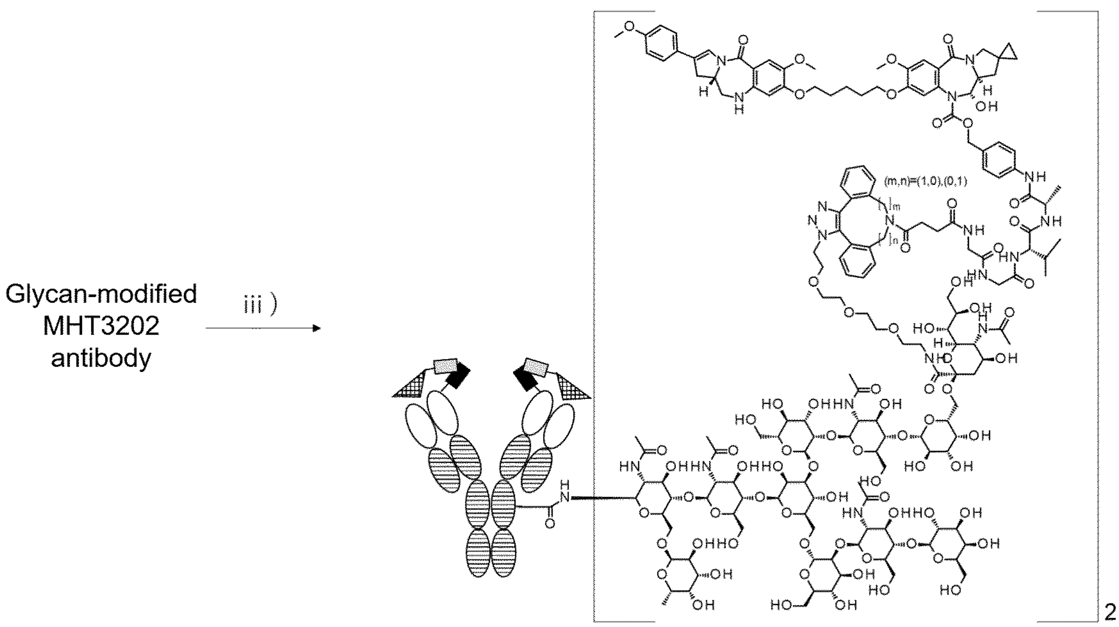


MHT3202 -PBD-ADC

iii) Linker 2

Step 3: Conjugation of antibody to drug linker

With the use of the glycan-modified MHT3202 (ABS) (9.93 mg/ml, 0.500 ml) obtained in Step 2 above, 2.50 ml of the MHT3202-PBD-ADC solution (ABS) was obtained in the same manner as in Step 3 of Example 7)-7.

Property evaluation:

The property values indicated below were obtained in accordance with the common operation E and the common operation F.

Antibody concentration: 1.51 mg/ml; antibody yield: 3.77 mg (76%); average number of drugs bound to an antibody molecule (n): 1.8

7)-10 Synthesis of MHT3203-PBD-ADC

[Chemical Formula 13]

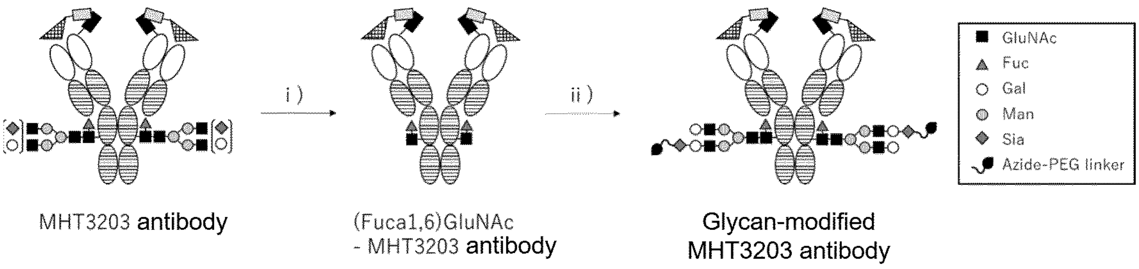


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT3203 antibody

The MHT3203 antibody solution prepared in Example 6 was buffer-exchanged to PB6.0 and adjusted to ca. 19.1 mg/ml (1.29 ml). In the same manner as in Step 1 of Example 7)-7, the (Fucα1,6)GlcNAc-MHT3203 antibody solution (PB6.0) (19.8 mg/ml, 0.969 ml) was obtained.

Step 2: Preparation of glycan-modified MHT3203 antibody

With the use of the (Fucα1,6)GlcNAc-MHT3203 antibody solution (PB6.0) (19.8 mg/ml, 0.969 ml) obtained in Step 1 above, the glycan-modified MHT3203 antibody (PB6.0) (9.77 mg/ml, 1.77 ml) was obtained in the same manner as in Step 2 of Example 7)-7.

[Chemical Formula 14]

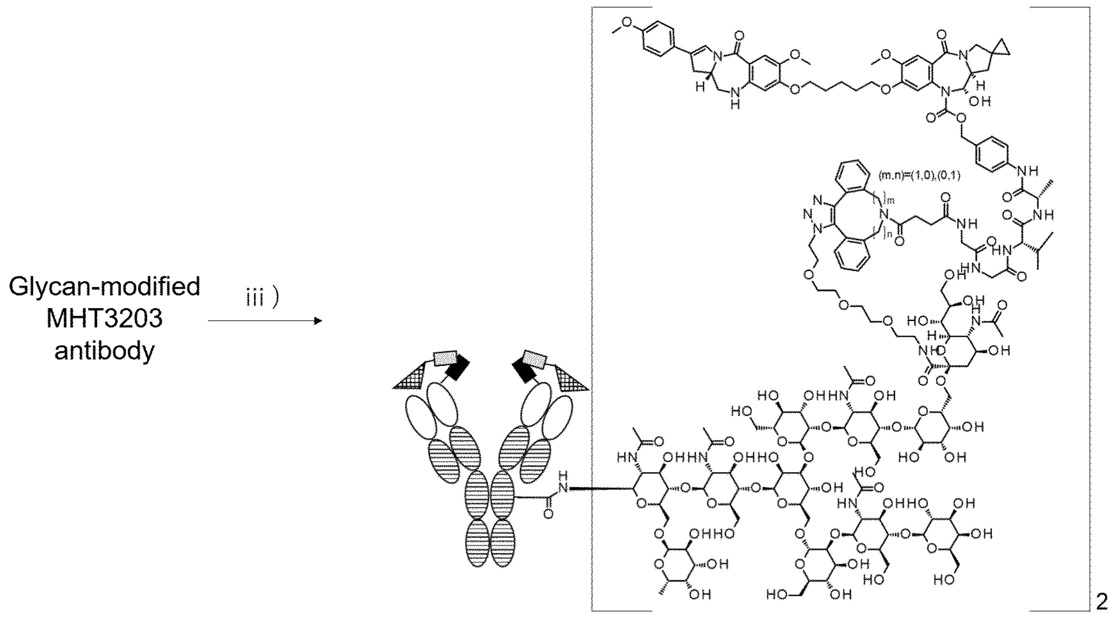


MHT3203 -PBD-ADC

iii) Linker 2

Step 3: Conjugation of antibody to drug linker

With the use of the glycan-modified MHT3203 (ABS) (9.77 mg/ml, 0.500 ml) obtained in Step 2 above, 2.50 ml of the MHT3203-PBD-ADC solution (ABS) was obtained in the same manner as in Step 3 of Example 7)-7.

Property evaluation:

The property values indicated below were obtained in accordance with the common operation E and the common operation F.

Antibody concentration: 1.49 mg/ml; antibody yield: 3.73 mg (76%); average number of drugs bound to an antibody molecule (n): 1.8

7)-11 Synthesis of MHT1008-PBD-ADC

[Chemical Formula 15]

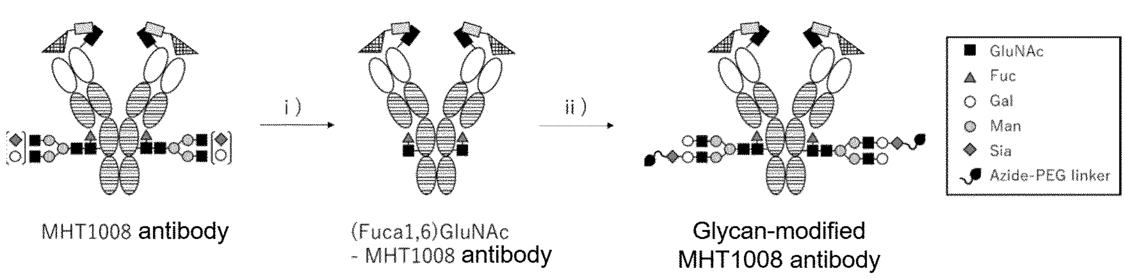


i) Endo S enzyme

ii) Endo S (D233Q/Q303L) enzyme, glycan-oxazoline

The N297 glycan of the glycan-modified antibody is the MSG1-type glycan comprising an azide group introduced

into a sialic acid at the non-reducing terminus (WO 2019/065964) (Figure 8).

Step 1: Preparation of (Fucα1,6)GlcNAc-MHT1008 antibody

The MHT1008 antibody solution prepared in Example 4 was buffer-exchanged to PB6.0 and adjusted to ca. 16.6 mg/ml (0.880 ml). In the same manner as in Step 1 of Example 7)-7, the (Fucα1,6)GlcNAc-MHT1008 antibody solution (PB6.0) (16.8 mg/ml, 0.800 ml) was obtained.

Step 2: Preparation of glycan-modified MHT1008 antibody

With the use of the 16.8 mg/ml (Fucα1,6)GlcNAc-MHT1008 antibody solution (PB6.0) (0.800 ml) obtained in Step 1 above, the glycan-modified MHT1008 antibody (ABS) (11.4 mg/ml, 1.00 ml) was obtained in the same manner as in Step 2 of Example 7)-7.

[Chemical Formula 16]

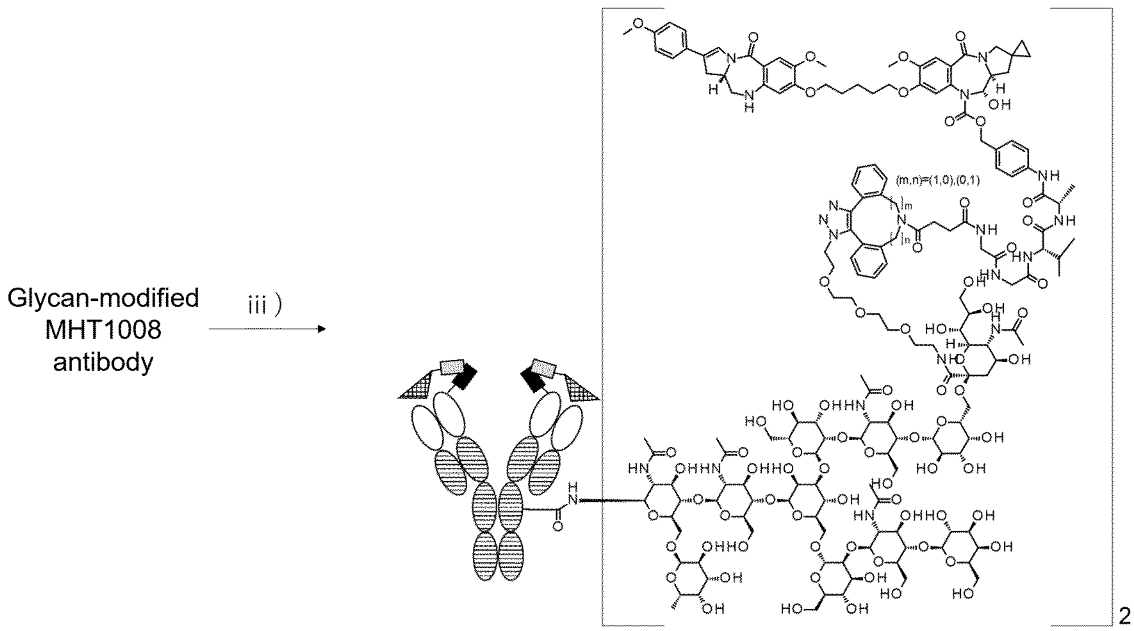


MHT1008 -PBD-ADC

iii) Linker 2

Step 3: Conjugation of antibody to drug linker

With the use of the glycan-modified MHT1008 antibody (ABS) (11.4 mg/ml, 0.450 ml) obtained in Step 2 above, 2.50 ml of the MHT1008-PBD-ADC solution (ABS) was obtained in the same manner as in Step 3 of Example 7)-7.

Property evaluation:

The property values indicated below were obtained in accordance with the common operation E and the common operation F.
Antibody concentration: 1.55 mg/ml; antibody yield: 3.87 mg (77%); average number of drugs bound to an antibody molecule (n): 1.9

7)-12 Synthesis of MHT3219-PBD-ADC

[Chemical Formula 17]

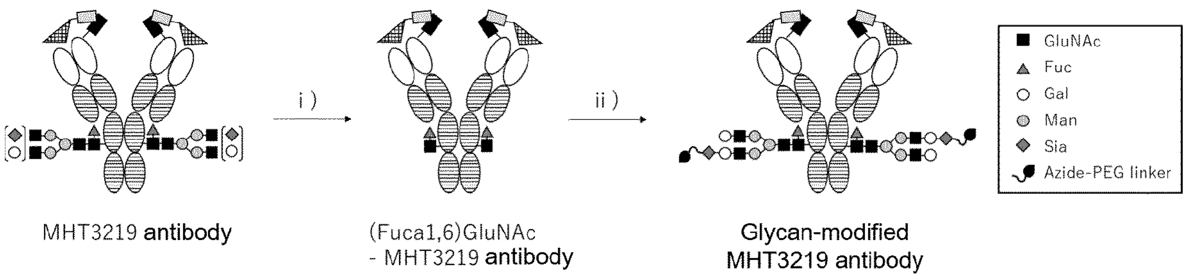


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT3219 antibody

The MHT3219 antibody solution prepared in Example 21 was buffer-exchanged to PB6.0 and adjusted to ca. 15.0 mg/ml (1.70 ml). In the same manner as in Step 1 of Example 7)-7, the (Fucα1,6)GlcNAc-MHT3219 antibody solution (PB6.0) (17.9 mg/ml, 1.30 ml) was obtained.

Step 2: Preparation of glycan-modified MHT3219 antibody

With the use of the 17.9 mg/ml (Fucα1,6)GlcNAc-MHT3219 antibody solution (PB6.0) (1.30 ml) obtained in Step 1 above, the glycan-modified MHT3219 antibody (ABS) (9.90 mg/ml, 2.10 ml) was obtained in the same manner as in Step 2 of Example 7)-7.

[Chemical Formula 18]

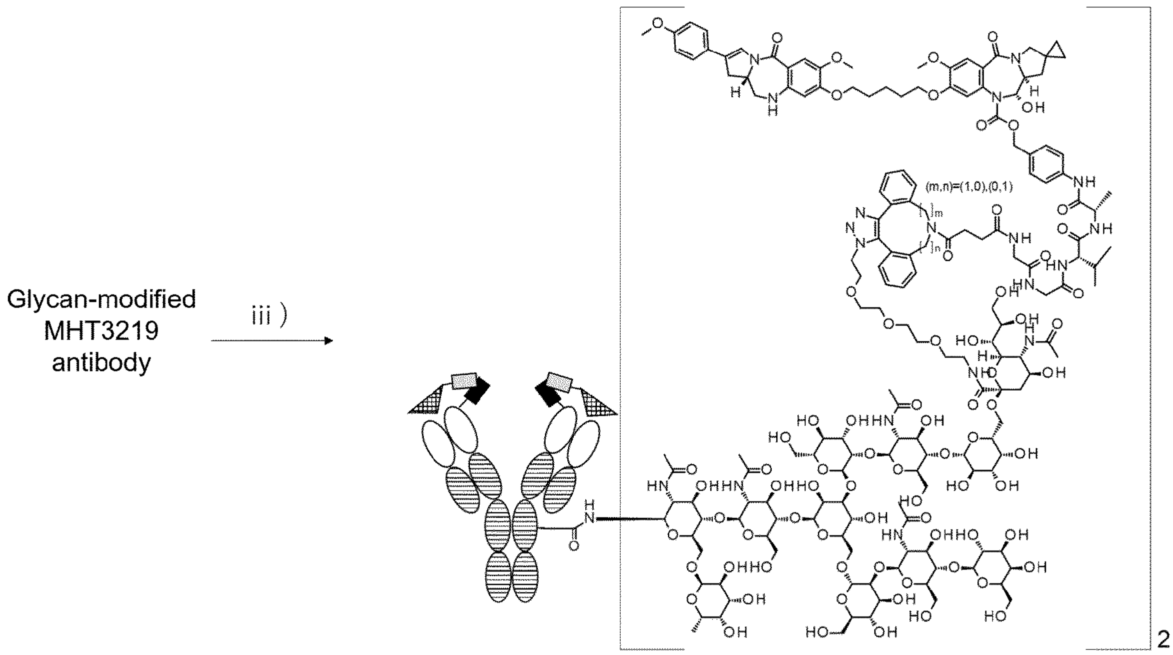


MHT3219-PBD-ADC

iii) Linker 2

Step 3: Conjugation of antibody to drug linker

With the use of the glycan-modified MHT3219 antibody (ABS) (9.90 mg/ml, 0.50 ml) obtained in Step 2 above, 3.50 ml of the MHT3219-PBD-ADC solution (ABS) was obtained in the same manner as in Step 3 of Example 7)-7.

Property evaluation:

The property values indicated below were obtained in accordance with the common operation E and the common operation F.
Antibody concentration: 0.90 mg/ml; antibody yield: 3.13 mg (63%); average number of drugs bound to an antibody molecule (n): 1.9

7)-13 Synthesis of MHT5082-PBD-ADC

[Chemical Formula 19]

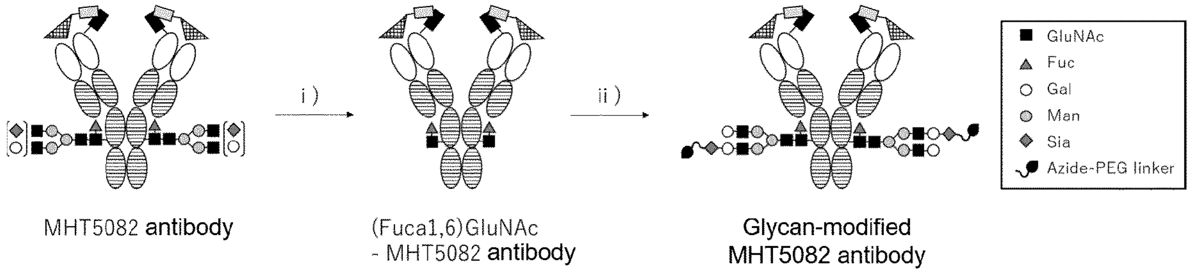


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5082 antibody

The MHT5082 antibody solution prepared in Example 22 was buffer-exchanged to PB6.0 and adjusted to ca. 15.8 mg/ml (1.50 ml). In the same manner as in Step 1 of Example 7)-7, the (Fucα1,6)GlcNAc-MHT5082 antibody solution (PB6.0) (12.5 mg/ml, 1.4 ml) was obtained.

Step 2: Preparation of glycan-modified MHT5082 antibody

With the use of the 12.5 mg/ml (Fucα1,6)GlcNAc-MHT5082 antibody solution (PB6.0) (1.40 ml) obtained in Step 1 above, the glycan-modified MHT5082 antibody (ABS) (9.10 mg/ml, 1.20 ml) was obtained in the same manner as in Step 2 of Example 7)-7.

[Chemical Formula 20]

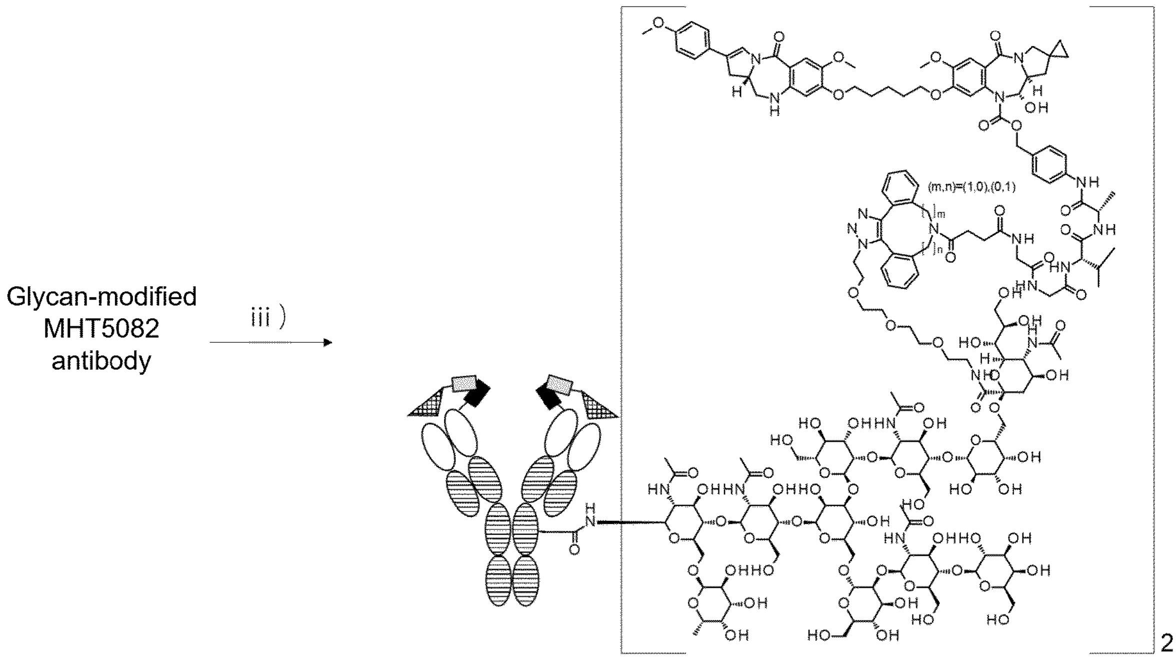


MHT5082-PBD-ADC

iii) Linker 2

Step 3: Conjugation of antibody to drug linker

With the use of the glycan-modified MHT5082 antibody (ABS) (9.10 mg/ml, 0.60 ml) obtained in Step 2 above, 3.0 ml of the MHT5082-PBD-ADC solution (ABS) was obtained in the same manner as in Step 3 of Example 7)-7.

Property evaluation:

The property values indicated below were obtained in accordance with the common operation E and the common operation F.
Antibody concentration: 0.57 mg/ml; antibody yield: 2.00 mg (37%); average number of drugs bound to an antibody molecule (n): 1.8

7)-14 Synthesis of MHT5085-PBD-ADC

[Chemical Formula 21]

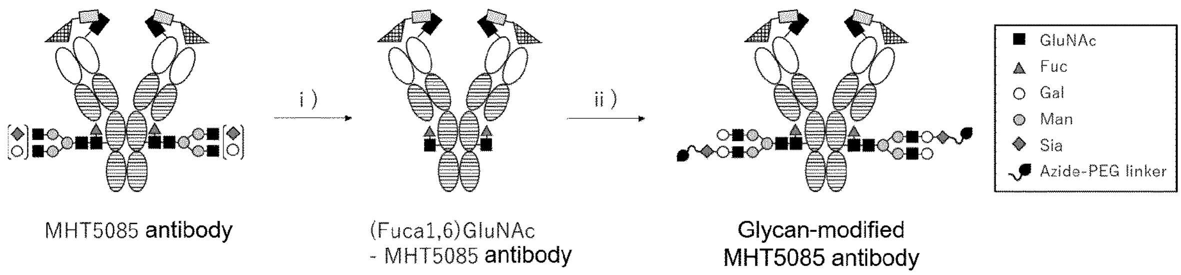


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5085 antibody

The MHT5085 antibody solution prepared in Example 22 was buffer-exchanged to PB6.0 and adjusted to ca. 15.3 mg/ml (1.50 ml). In the same manner as in Step 1 of Example 7)-7, the (Fucα1,6)GlcNAc-MHT5085 antibody solution (PB6.0) (11.0 mg/ml, 1.40 ml) was obtained.

Step 2: Preparation of glycan-modified MHT5085 antibody

With the use of the 11.0 mg/ml (Fucα1,6)GlcNAc-MHT5085 antibody solution (PB6.0) (1.40 ml) obtained in Step 1 above, the glycan-modified MHT5085 antibody (ABS) (9.20 mg/ml, 1.20 ml) was obtained in the same manner as in Step 2 of Example 7)-7.

[Chemical Formula 22]

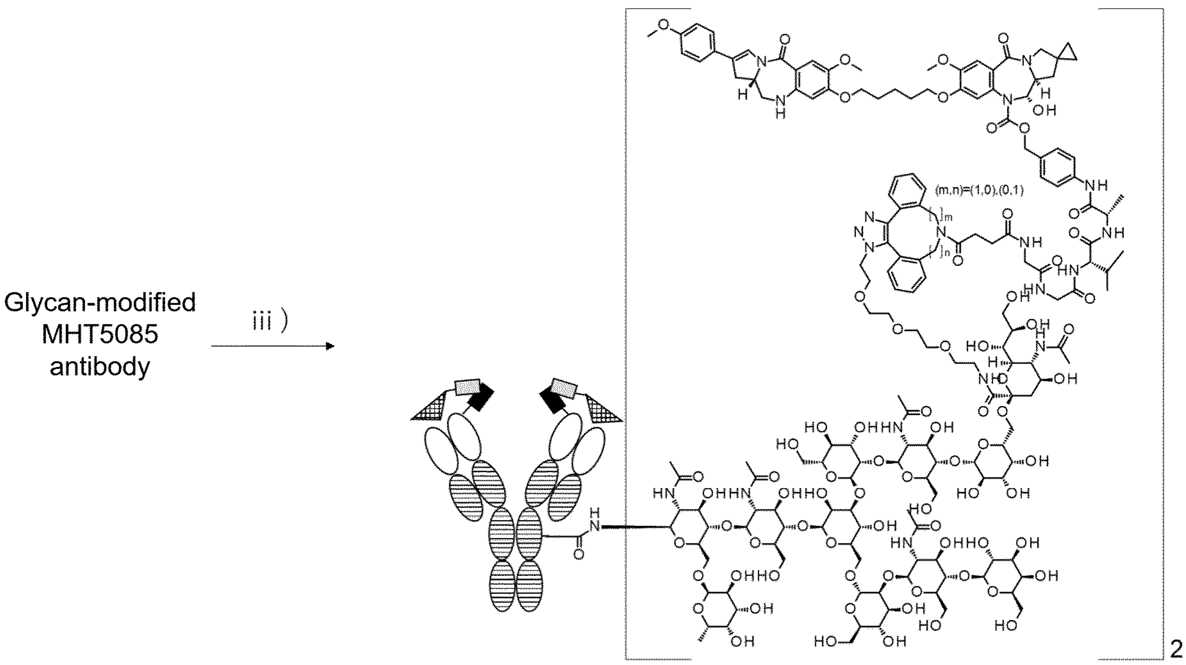


MHT5085-PBD-ADC

iii) Linker 2

Step 3: Conjugation of antibody to drug linker

With the use of the glycan-modified MHT5085 antibody (ABS) (9.20 mg/ml, 0.60 ml) obtained in Step 2 above, 3.50 ml of the MHT5085-PBD-ADC solution (ABS) was obtained in the same manner as in Step 3 of Example 7)-7.

Property evaluation:

The property values indicated below were obtained in accordance with the common operation E and the common operation F.

Antibody concentration: 1.38 mg/ml; antibody yield: 4.90 mg (89%); average number of drugs bound to an antibody molecule (n): 1.8

7)-15 Synthesis of MHT5086-PBD-ADC

[Chemical Formula 23]

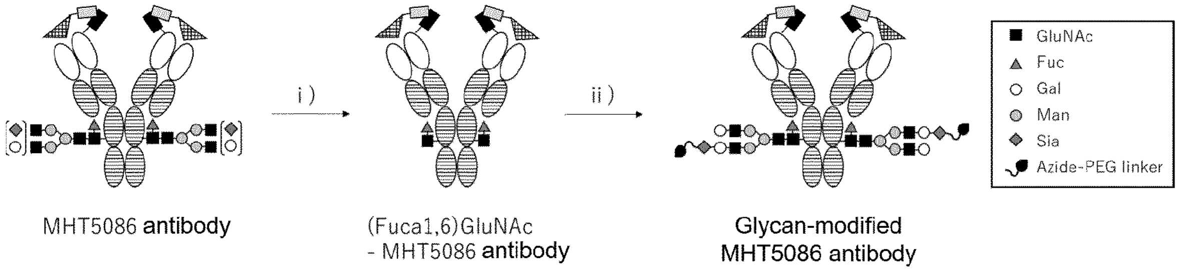


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5086 antibody

The MHT5086 antibody solution prepared in Example 22 was buffer-exchanged to PB6.0 and adjusted to ca. 15.7 mg/ml (1.50 ml). In the same manner as in Step 1 of Example 7)-7, the (Fucα1,6)GlcNAc-MHT5086 antibody solution (PB6.0) (15.0 mg/ml, 1.20 ml) was obtained.

Step 2: Preparation of glycan-modified MHT5086 antibody

With the use of the 15.0 mg/ml (Fucα1,6)GlcNAc-MHT5086 antibody solution (PB6.0) (1.20 ml) obtained in Step 1 above, the glycan-modified MHT5086 antibody (ABS) (10.5 mg/ml, 1.20 ml) was obtained in the same manner as in Step 2 of Example 7)-7.

[Chemical Formula 24]

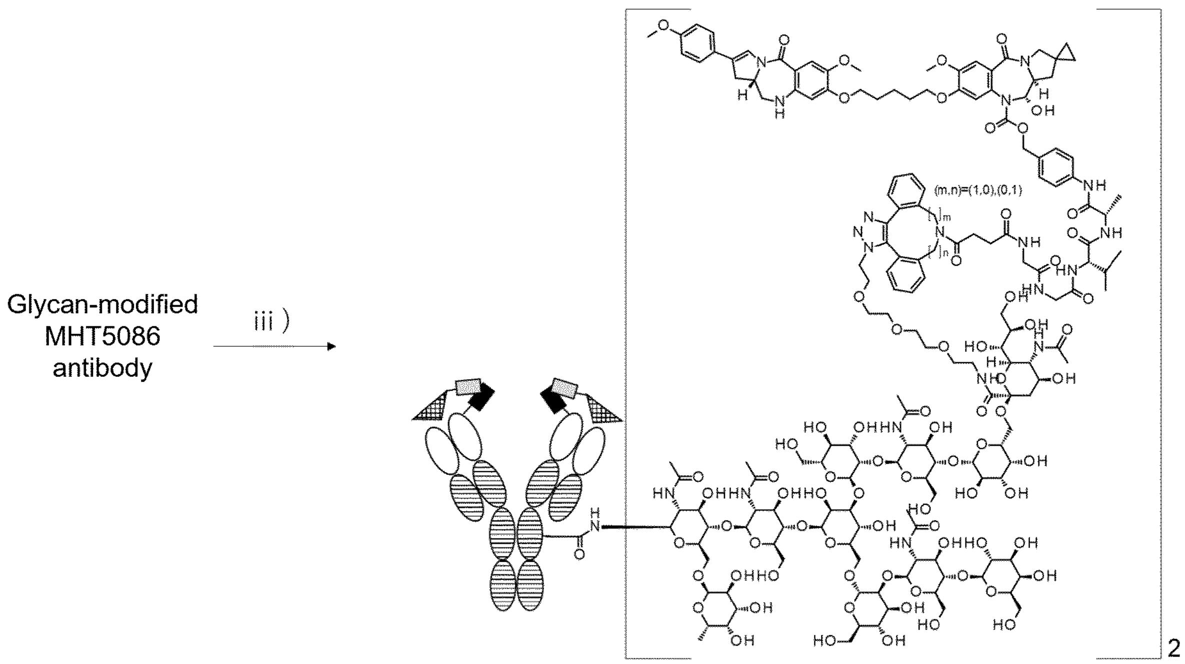


MHT5086-PBD-ADC

iii) Linker 2

Step 3: Conjugation of antibody to drug linker

With the use of the glycan-modified MHT5086 antibody (ABS) (10.5 mg/ml, 0.60 ml) obtained in Step 2 above, 3.50 ml of the MHT5086-PBD-ADC solution (ABS) was obtained in the same manner as in Step 3 of Example 7)-7.

Property evaluation:

The property values indicated below were obtained in accordance with the common operation E and the common operation F.
Antibody concentration: 1.55 mg/ml; antibody yield: 5.11 mg (81%); average number of drugs bound to an antibody molecule (n): 1.8

7)-16 Synthesis of MHT5093-PBD-ADC

[Chemical Formula 25]

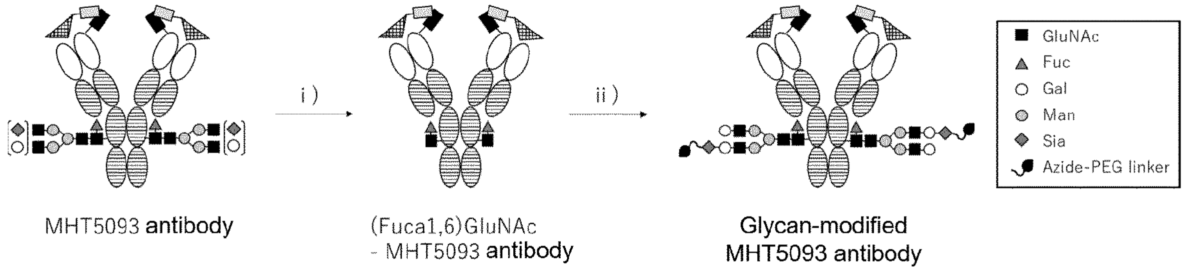


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5093 antibody

The MHT5093 antibody solution prepared in Example 22 was buffer-exchanged to PB6.0 and adjusted to ca. 13.4 mg/ml (1.50 ml). In the same manner as in Step 1 of Example 7)-7, the (Fucα1,6)GlcNAc-MHT5093 antibody solution (PB6.0) (15.3 mg/ml, 1.10 ml) was obtained.

Step 2: Preparation of glycan-modified MHT5093 antibody

With the use of the 15.3 mg/ml (Fucα1,6)GlcNAc-MHT5093 antibody solution (PB6.0) (1.10 ml) obtained in Step 1 above, the glycan-modified MHT5093 antibody (ABS) (10.9 mg/ml, 1.20 ml) was obtained in the same manner as in Step 2 of Example 7)-7.

[Chemical Formula 26]

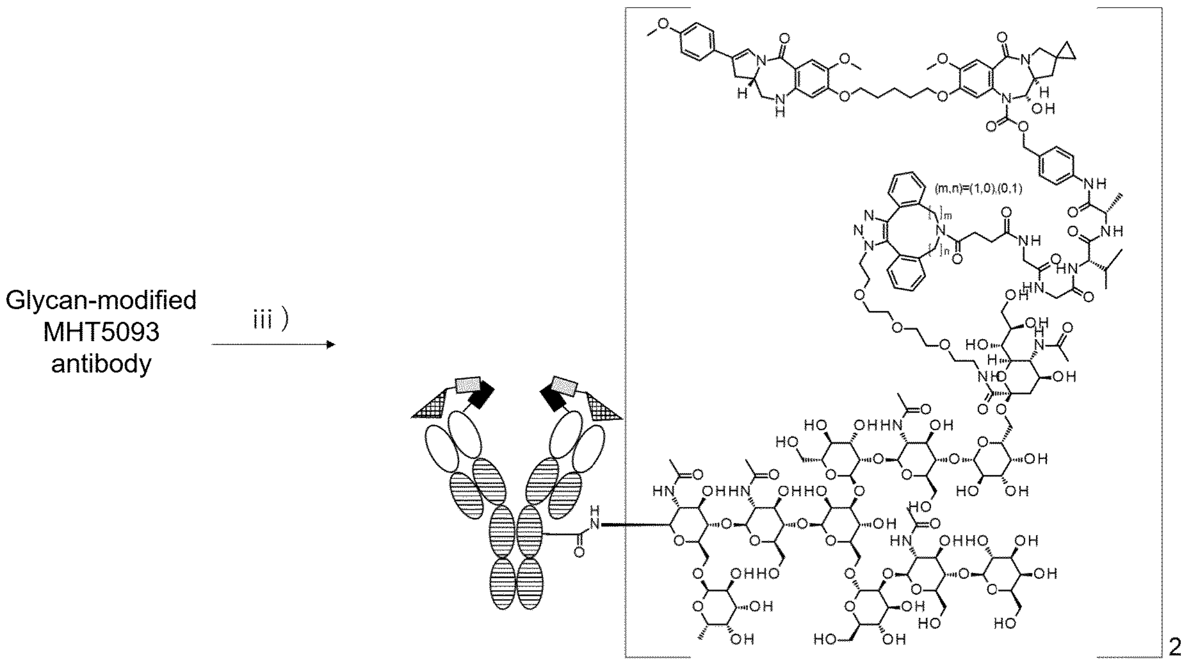


MHT5093-PBD-ADC

iii) Linker 2

Step 3: Conjugation of antibody to drug linker

With the use of the glycan-modified MHT5093 antibody (ABS) (10.9 mg/ml, 0.60 ml) obtained in Step 2 above, 3.50 ml of the MHT5093-PBD-ADC solution (ABS) was obtained in the same manner as in Step 3 of Example 7)-7.

Property evaluation:

The property values indicated below were obtained in accordance with the common operation E and the common operation F.
Antibody concentration: 1.56 mg/ml; antibody yield: 5.32 mg (81%); average number of drugs bound to an antibody molecule (n): 1.9

7)-17 Synthesis of MHT5094-PBD-ADC

[Chemical Formula 27]

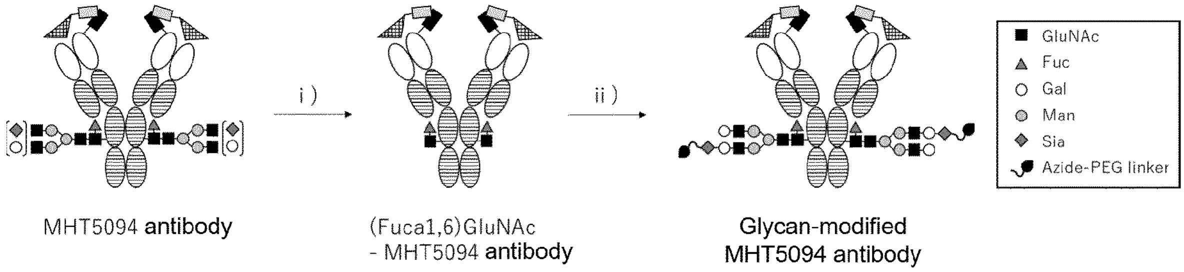


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5094 antibody

The MHT5094 antibody solution prepared in Example 22 was buffer-exchanged to PB6.0 and adjusted to ca. 13.9 mg/ml (1.50 ml). In the same manner as in Step 1 of Example 7)-7, the (Fucα1,6)GlcNAc-MHT5094 antibody solution (PB6.0) (15.8 mg/ml, 1.10 ml) was obtained.

Step 2: Preparation of glycan-modified MHT5094 antibody

With the use of the 15.8 mg/ml (Fucα1,6)GlcNAc-MHT5094 antibody solution (PB6.0) (1.10 ml) obtained in Step 1 above, the glycan-modified MHT5094 antibody (ABS) (9.10 mg/ml, 1.20 ml) was obtained in the same manner as in Step 2 of Example 7)-7.

[Chemical Formula 28]

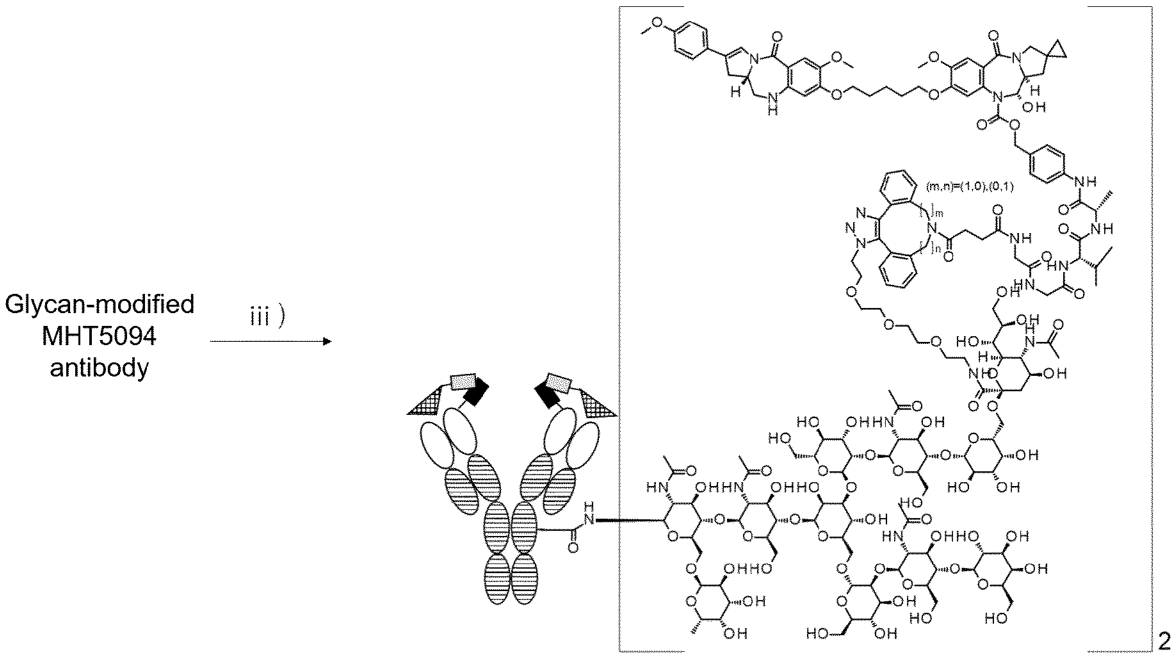


MHT5094-PBD-ADC

iii) Linker 2

Step 3: Conjugation of antibody to drug linker

With the use of the glycan-modified MHT5094 antibody (ABS) (9.10 mg/ml, 0.60 ml) obtained in Step 2 above, 3.50 ml of the MHT5094-PBD-ADC solution (ABS) was obtained in the same manner as in Step 3 of Example 7)-7.

Property evaluation:

The property values indicated below were obtained in accordance with the common operation E and the common operation F.
Antibody concentration: 1.37 mg/ml; antibody yield: 4.41 mg (81%); average number of drugs bound to an antibody molecule (n): 1.9

7)-18 Synthesis of MHT5095-PBD-ADC

[Chemical Formula 29]

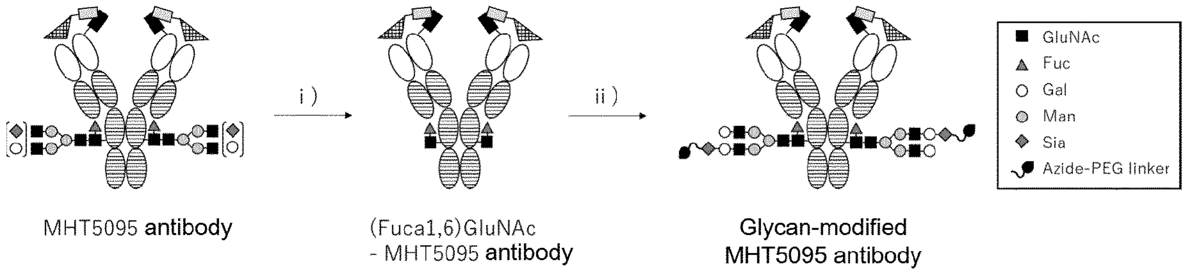


i) Endo S enzyme

ii) Endo S (D233Q/Q303L) enzyme, glycan-oxazoline

The N297 glycan of the glycan-modified antibody is the MSG1-type glycan comprising an azide group introduced into a sialic acid at the non-reducing terminus (WO 2019/065964) (Figure 8).

Step 1: Preparation of (Fucα1,6)GlcNAc-MHT5095 antibody

The MHT5095 antibody solution prepared in Example 22 was buffer-exchanged to PB6.0 and adjusted to ca. 12.4 mg/ml (1.50 ml). In the same manner as in Step 1 of Example 7)-7, the (Fucα1,6)GlcNAc-MHT5095 antibody solution (PB6.0) (15.6 mg/ml, 1.10 ml) was obtained.

Step 2: Preparation of glycan-modified MHT5095 antibody

With the use of the 15.6 mg/ml (Fucα1,6)GlcNAc-MHT5095 antibody solution (PB6.0) (1.10 ml) obtained in Step 1 above, the glycan-modified MHT5095 antibody (ABS) (10.5 mg/ml, 1.20 ml) was obtained in the same manner as in Step 2 of Example 7)-7.

[Chemical Formula 30]

Glycan-modified MHT5095 antibody

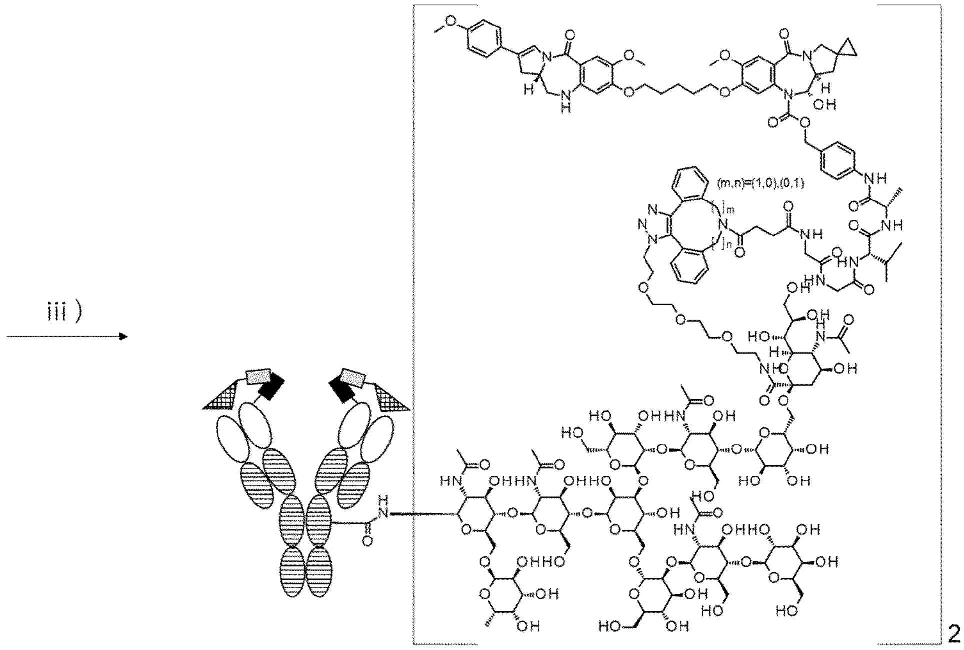


MHT5095-PBD-ADC

iii) Linker 2

Step 3: Conjugation of antibody to drug linker

With the use of the glycan-modified MHT5095 antibody (ABS) (10.5 mg/ml, 0.60 ml) obtained in Step 2 above, 3.50 ml of the MHT5095-PBD-ADC solution (ABS) was obtained in the same manner as in Step 3 of Example 7)-7.

Property evaluation:

The property values indicated below were obtained in accordance with the common operation E and the common operation F.
Antibody concentration: 1.52 mg/ml; antibody yield: 5.18 mg (82%); average number of drugs bound to an antibody molecule (n): 1.9

(Example 8) *In vivo* pharmaceutical efficacy test of anti-TROP2 masked antibody-drug conjugate

The antitumor effects of the antibody-drug conjugate were evaluated using animal models prepared by transplanting the TROP2-positive human tumor cells into immunodeficient mice. Before the experiment, 4- to 5-week-old BALB/c nude mice (CAnN.Cg-Foxn1[nu]/CrlCrlj[Foxn1nu/Foxn1nu], Charles River Laboratories Japan, Inc.) were conditioned under SPF conditions for 3 or more days. The mice were fed with sterilized solid feeds (FR-2, Funabashi Farms Co., Ltd) and sterilized tap water (prepared with the addition of 5 to 15 ppm sodium hypochlorite solutions). The major diameter and the minor diameter of the transplanted tumor were measured two times a week with the use of an electronic digital caliper (CD-15CX, Mitutoyo Corp.), and the tumor volume was determined in accordance with the equation indicated below.

$$\text{Tumor volume (mm}^3) = 1/2 \times \text{major diameter (mm)} \times [\text{minor diameter (mm)}]^2$$

All the antibody-drug conjugates were diluted with ABS buffer (10 mM acetate buffer, 5 (w/v/)% sorbitol, pH 5.5) (NACALAI), and the antibody solution was administered intravenously into the caudal vein at 10 ml/kg. ABS buffer was administered to a control group (a vehicle group) in the same manner. Groups each consisting of 6 mice were subjected to the experiment.

8)-1 Antitumor effects (1)

The cells of the TROP2-positive human lung mucoepidermoid carcinoma cell line NCI-H292 (ATCC) were suspended in saline, 5 × $10^6$ cells were transplanted subcutaneously into the right abdominal region of female nude mice (Day 0), and the mice were assigned randomly into groups on Day 11. On the day of grouping, 5 types of the antibody-drug conjugates prepared in Example 7 (clone names: MHT1008-PBD-ADC, MHT3423-PBD-ADC, MHT3201-PBD-ADC, MHT3202-PBD-ADC, and MHT3203-PBD-ADC) were administered intravenously into the caudal vein at a dose of 0.4 mg/kg. The results are shown in Figure 9 (A). The horizontal axis indicates the number of days, the vertical axis indicates the tumor volume, and the error range is the standard error (SE).

MHT3202-PBD-ADC and MHT3203-PBD-ADC comprising both the uPA substrate and the CAPN substrate exerted higher antitumor effects than MHT3423-PBD-ADC selectively comprising the uPA substrate or MHT3201-PBD-ADC selectively comprising the CAPN substrate.

8)-2 Antitumor effects (2)

The cells of the TROP2-positive human pharyngeal carcinoma cell line FaDu (ATCC) were suspended in saline, 3 × $10^6$ cells were transplanted subcutaneously into the right abdominal region of female nude mice (Day 0), and the mice were assigned randomly into groups on Day 10. On the day of grouping, 5 types of the antibody-drug conjugates prepared in Example 7 (clone names: MHT1008-PBD-ADC, MHT3423-PBD-ADC, MHT3201-PBD-ADC, MHT3202-PBD-ADC, and MHT3203-PBD-ADC) were administered intravenously into the caudal vein at a dose of 0.4 mg/kg. The results are shown in Figure 9 (B). The horizontal axis indicates the number of days, the vertical axis indicates the tumor volume, and the error range is the standard error (SE).

MHT3202-PBD-ADC and MHT3203-PBD-ADC comprising both the uPA substrate and the CAPN substrate exerted higher antitumor effects than MHT3423-PBD-ADC selectively comprising the uPA substrate or MHT3201-PBD-ADC selectively comprising the CAPN substrate.

(Example 9) Evaluation of binding intensity of anti-CD98 antibody hM23H1L1

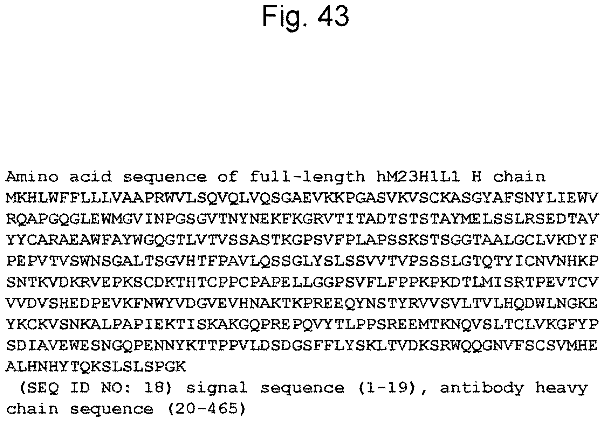

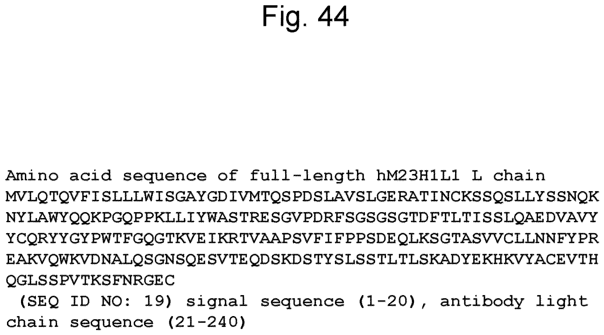

The conventional anti-CD98 antibody hM23H1L1 described in WO 2015/146132 (the heavy chain sequence (Figure 43, SEQ ID NO: 18); the light chain sequence (Figure 44, SEQ ID NO: 19)) was prepared in the same manner as in Example 1)-1, and binding intensity thereof to the human CD98 antigen was evaluated by ELISA.

NeutrAvidin (Thermo Fisher Scientific) diluted to 1 μg/ml with PBS was added at 50 μl/well to a 96-well Maxisorp plate (Black, Nunc) and immobilized at 4°C overnight. The plate was washed three times with PBS (ELISA buffer) containing 0.05(w/v)% Tween-20 (BioRad) and then blocked with Blocker Casein (Thermo Fisher Scientific). The plate was washed with ELISA buffer, the biotinylated human CD98 antigen (Accession Number: P08195; the extracellular domain was purified by the method known to a person skilled in the art and then biotinylated with EZ-Link NHS-PEG4-Bioin (Thermo Fisher Scientific)) diluted to 1 μg/ml with PBS was added at 50 μl/well, and the resultant was agitated for 30 minutes at room temperature. The plate was washed with ELISA buffer, the anti-CD98 antibody hM23H1L1 solution prepared with ELISA buffer was added at 50 μl/well, and the resultant was then agitated for 30 minutes at room temperature. The plate was washed with ELISA buffer, 50 μl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 10, hM23H1L1 was found to have bound to the human CD98 antigen in a concentration-dependent manner.

(Example 10) Concentration of mimotope peptide binding to anti-CD98 antibody hM23H1L1

A peptide library composed of completely random 15 amino acids (Linear 15mer lib) or a peptide library by ribosome display having a repeat motif of aromatic amino acid and Pro near the center (ZPZP lib) were constructed, and a peptide capable of binding to hM23H1L1 was concentrated (Figure 11 (A)). At the outset, RDs were added to Dynabeads Streptavidin M-280 (Thermo Fisher Scientific) and Dynabeads Protein A (Thermo Fisher Scientific) each comprising human serum-derived IgG (Sigma Aldrich) biotinylated with EZ-Link NHS-PEG4-Bioin (Thermo Fisher Scientific) immobilized thereon and allowed to react with the human serum-derived IgG or the Protein A. RDs that did not bind were collected using a magnet stand (DynaMag-2, Thermo Fisher Scientific) and then allowed to react with the Dynabeads Protein A comprising hM23H1L1 bound thereto. RDs that did not bind to hM23H1L1 were removed by washing with the use of a magnet stand, and mRNAs were purified from RDs that had bound to hM23H1L1. Thereafter, RDs were prepared again by RT-PCR and *in vitro* translation. This process of panning was performed 3 times.

mRNAs after the third round of panning were subjected to *in vitro* translation to prepare RDs, and the prepared RDs were then allowed to react with Dynabeads Protein A comprising hM23H1L1 or human serum-derived IgG immobilized thereon (amount of input: $6 \times 10^{11}$). RDs that did not bind were removed by washing with the use of a magnet stand, mRNAs were collected from the RDs that had bound, and the amount collected (output) was quantified and evaluated by RT-qPCR. As shown in Figure 11 (B), the number of mRNAs collected under the HT1-11-immobilized conditions was approximately 200 times greater than that collected under the human serum IgG-immobilized conditions from both the peptide libraries Linear 15mer lib and ZPZP lib. The results indicate concentration of peptides binding specifically to hM23H1L1.

(Example 11) Preparation of anti-CD98 masked antibody and evaluation of binding intensity thereof

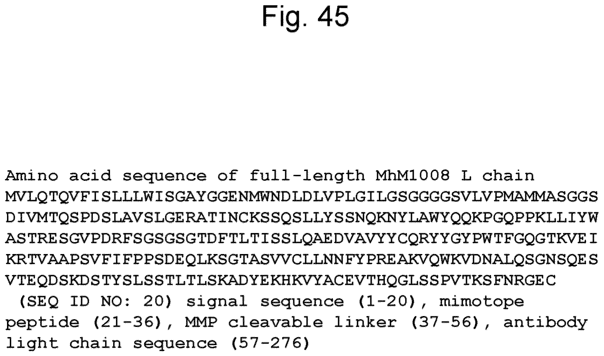

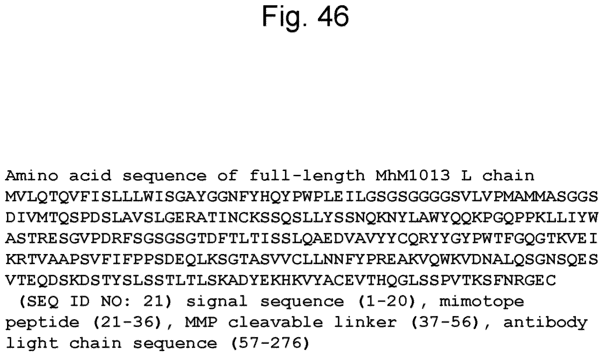

As with the case of Example 3, the random peptide obtained by panning was fused to anti-CD98 scFv, and a mimotope peptide capable of inhibiting binding of the anti-CD98 antibody was selected. The expression vectors for the anti-CD98 masked antibody MhM1008 (Table 3, Figure 45, SEQ ID NO: 20) and the anti-CD98 masked antibody MhM1013 (Table 3, Figure 46, SEQ ID NO: 21) comprising the mimotope peptide and the MMP cleavable linker (see Example 3)-1) selected from Linear 15mer lib and ZPZP lib, respectively, were constructed by a method known to a person skilled in the art. An expression vector of each antibody light chain was used in combination with the expression vector of the hM23H1L1 heavy chain, masked antibodies were purified from the culture supernatant of the Expi293F cells in the same manner as in Example 1)-1, and the protein concentration was then determined. The masked antibodies prepared were designated as MhM1008 and MhM1013 corresponding to the names of light chain expression vectors (Table 3).

Except for the ways described below, binding intensity to the human CD98 antigen was evaluated by ELISA under the condition with the addition of MMP1 and under the condition without the addition thereof in the same manner as in Example 4 and Example 9. Under the condition with the addition of a protease, 500 nM MMP1 was added to the 3 μM antibody, the reaction was allowed to proceed at 37°C, and the antibody with the concentration thereof being adjusted with ELISA buffer was added to wells comprising the human CD98 antigen immobilized thereon. Under the condition without the addition of a protease, the ELISA-buffered antibody was added to the wells.

As shown in Figure 12-1 (A), hM23H1L1 to which the mimotope peptide had not fused was found to have bound to the human CD98 antigen in a concentration-dependent manner under the condition with the addition of MMP1. MhM1008 and MhM1013 to which the mimotope peptide had fused were found to exhibit binding intensity equivalent to that of hM23H1L1 under the condition with the addition of MMP1. Under the condition without the addition of MMP1, in contrast, binding intensity of MhM1008 and that of MhM1013 were lower than the those under the condition with the addition of MMP1, and masking effects were observed (Figure 12-1(B), Figure 12-2(C)). The $EC_{50}$ ratio of binding of MhM1008 and that of MhM1013 (under the condition without the addition of MMP1/under the condition with the addition of MMP1) were 132 and 326, respectively.

In order to verify that the peptide obtained was the mimotope, the Fab region of MhM1013 comprising the masking peptide and the MMP cleavable linker (see Example 3)-1) was prepared by a method known to a person skilled in the art, and the Fab region was crystallized and subjected to X-ray crystallography. The results demonstrate that the peptide had bound to the CDR of hM23H1L1 (the data are not shown).

[Table 3]

| Anti-CD98 antibody and amino acid sequence information | | |
|---|---|---|
| Masked antibody | Heavy chain sequence | Light chain sequence |
| MhM1008 | hM23H1L1 (SEQ ID NO: 18) | MhM1008 (SEQ ID NO: 20) |
| MhM1013 | hM23H1L1 (SEQ ID NO: 18) | MhM1013 (SEQ ID NO: 21) |
| hM23-M1 | hM23-M1 (SEQ ID NO: 22) | hM23-M1 (SEQ ID NO: 23) |
| MhM1013-MI | hM23-M1 (SEQ ID NO: 22) | MhM1013 (SEQ ID NO: 21) |
| MhM1018-MI | hM23-M1 (SEQ ID NO: 22) | MhM1018 (SEQ ID NO: 24) |
| MhM1019-MI | hM23-M1 (SEQ ID NO: 22) | MhM1019 (SEQ ID NO: 25) |
| MhM1020-MI | hM23-M1 (SEQ ID NO: 22) | MhM1020 (SEQ ID NO: 26) |
| MhM1021-MI | hM23-M1 (SEQ ID NO: 22) | MhM1021 (SEQ ID NO: 27) |
| MhM1022-MI | hM23-M1 (SEQ ID NO: 22) | MhM 1022 (SEQ ID NO: 28) |

(continued)

| Anti-CD98 antibody and amino acid sequence information | | |
|---|---|---|
| Masked antibody | Heavy chain sequence | Light chain sequence |
| MhM1023-MI | hM23-M1 (SEQ ID NO: 22) | MhM 1023 (SEQ ID NO: 29) |

(Example 12) Masking effects of anti-CD98 antibody having different affinity 12)-1 Evaluation of binding affinity of anti-CD98 masked antibody

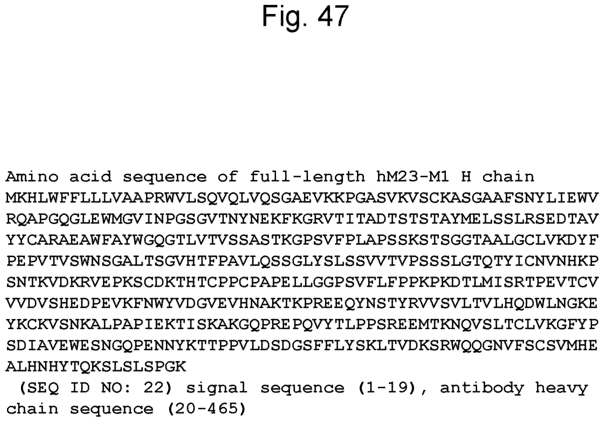

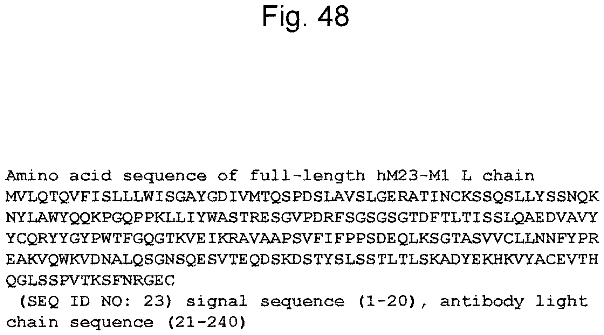

Biacore T200 was used to capture the anti-CD98 antibody (hM23H1L1 or hM23-M1 comprising a point mutation introduced into CDR1 of the H chain (Table 3, heavy chain sequence (Figure 47, SEQ ID NO: 22); light chain sequence (Figure 48, SEQ ID NO: 23)) as a ligand onto the immobilized anti-human IgG(Fc) antibody, and the CD98 antigen was analyzed as an analyte. The anti-human IgG (Fc) antibody (Human antibody Capture kit, Cytiva) was immobilized on Sensor Chip CM5 (Cytiva) in accordance with the instruction of the kit. The anti-CD98 antibodies diluted to 2 μg/ml with HBS-EP+ (Cytiva) to be evaluated were brought into contact at 5 μl/min for 60 seconds and immobilized. Thereafter, the CD98 antigen analytes diluted to various concentrations with HBS-EP+ were added as the analytes at a flow rate of 30 μl/min for 90 seconds and dissociation of the CD98 antigen was monitored for 250 seconds. $K_D$ was determined by multi-cycle kinetics analysis. As shown in Figure 13 (A), binding affinity ($K_D$) of hM23H1L1 and that of hM23-M1 were 0.58 nM and 10.3 nM, respectively.

12)-2 Evaluation of masking effects of anti-CD98 masked antibody

MhM1013 and MhM1013-M1 comprising the heavy chain of hM23-M1 and the light chain of MhM1013 (Table 3) were prepared, and binding intensity thereof to the human CD98 antigen under the condition with the addition of MMP 1 and under the condition without the addition thereof was evaluated by ELISA in the same manner as in Example 11, except for the ways described below. Under the condition with the addition of a protease, 200 nM MMP1 was added to the 2 μM antibody, the reaction was allowed to proceed at 37°C, and the antibody with the concentration thereof being adjusted with MMP buffer was added to the wells comprising the human CD98 antigen immobilized thereon. Under the condition without the addition of a protease, the MMP-buffered antibody was also added to the wells in the same manner.

As shown in Figure 13 (B), MhM1013 and MhM1013-M1 comprising the mimotope peptide fused thereto exhibited higher binding intensity under the condition with the addition of MMP 1 than under the condition without the addition of MMP1. The $EC_{50}$ ratio of binding intensity of MhM1013 and that of MhM1013-M1 (under the condition without the addition of M1VVIP1/under the condition with the addition of MMP1) were 239 and 552, respectively.

(Example 13) Preparation of anti-CD98 antibody to be subjected to ADC preparation and evaluation of binding intensity thereof

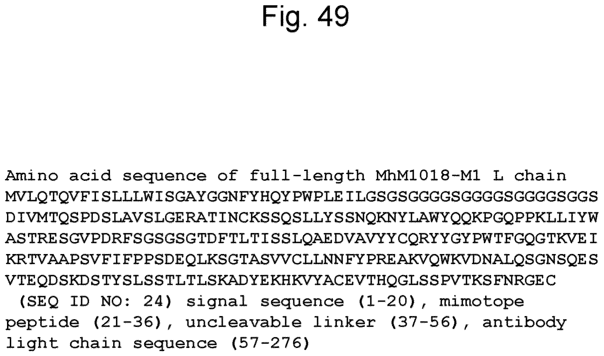

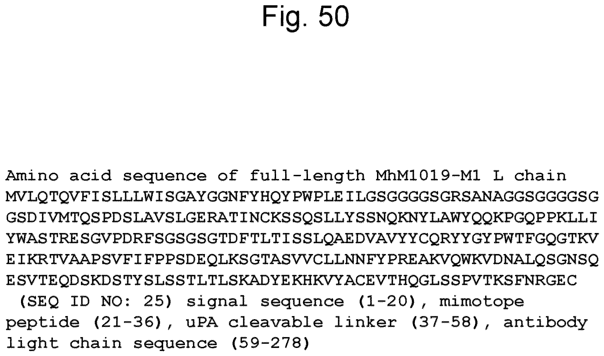

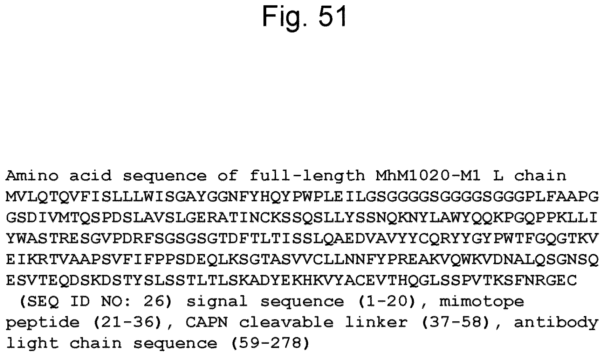

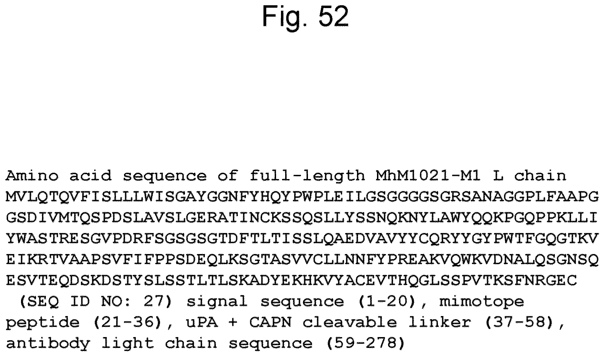

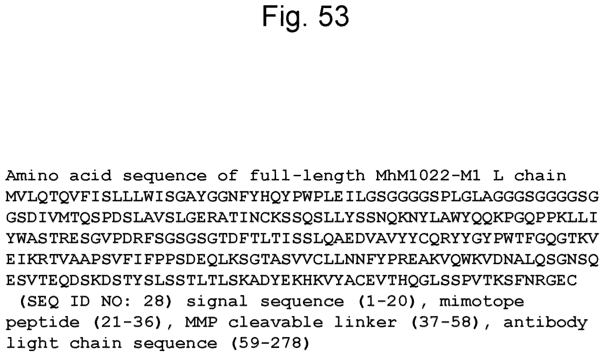

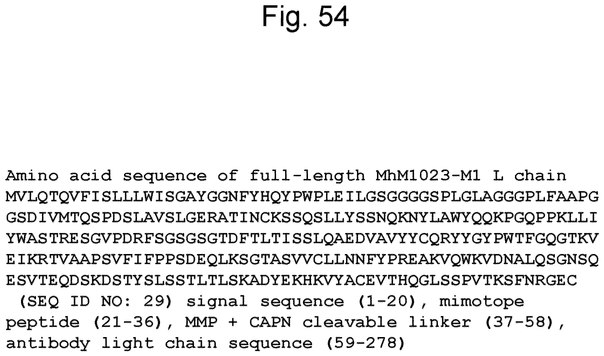

In order to examine as to whether or not binding intensity of a masked antibody would be improved with the use of an intracellular protease; i.e., the CAPN substrate sequence, in combination with an extracellular protease; i.e., the uPA substrate sequence, compared with a masked antibody selectively comprising the uPA substrate, various masked antibodies comprising the heavy chain of the anti-CD98 antibody hM23-M1 and the light chain with a different linker sequence were prepared (Table 3). MhM1018-M1 comprising no protease substrate (Table 3, Figure 49, SEQ ID NO: 24), M1019-M1 selectively comprising the uPA substrate (Table 3, Figure 50, SEQ ID NO: 25), M1020-M1 comprising the CAPN substrate (Table 3, Figure 51, SEQ ID NO: 26), M1021-M1 comprising both the uPA substrate and the CAPN substrate (Table 3, Figure 52, SEQ ID NO: 27), M1022-M1 selectively comprising the known MMP9 substrate composed of PLGLAG (Biopolymers; 40 (4): 399-416, 1996) (Table 3, Figure 53, SEQ ID NO: 28), and M1023-M1 comprising both MMP9 and the CAPN substrate (Table 3, Figure 54, SEQ ID NO: 29) were evaluated by ELISA in terms of the binding intensity to the human CD98 antigen under the condition with the addition of a protease and under the condition without the addition thereof in the same manner as in Example 6 and Example 11, except for the ways described below. After the antibodies were diluted to 2000 nM with MMP buffer, 200 nM uPA, CAPN1, or MMP9 was added, the reaction was allowed to proceed at 37°C, and the antibodies with the concentration thereof being adjusted with MMP buffer were added to the wells comprising the human CD98 antigen immobilized thereon. Under the condition without the addition of a protease, the antibodies prepared with MMP buffer were also added to the wells.

MhM1018-M1 exhibited equivalent binding intensity under the condition without the addition of an enzyme and under the condition with the addition of uPA or CAPN1 (Figure 14-1 (A)). Binding intensity of MhM1019-M1 was improved under the condition with the addition of uPA, compared with that under the condition without the addition of an enzyme,

and binding intensity thereof was not improved under the condition with the addition of CAPN1 (Figure 14-1 (B)). Also, binding intensity of MhM1020-M1 was improved selectively under the condition with the addition of CAPN1 (Figure 14-2 (C)). Binding intensity of MhM1021-M1 comprising both the uPA substrate and the CAPN substrate was improved both under the condition with the addition of uPA and under the condition with the addition of CAPN1 (Figure 14-2 (D). While MhM1018-M1 exhibited equivalent binding intensity under the condition without the addition of an enzyme and under the condition with the addition of MMP9 or CAPN1, binding intensity thereof was slightly improved under the condition with the addition of MMP9 (Figure 14-3 (E)). MhM1022-M1 exhibited improved binding intensity under the condition with the addition of MMP9, compared with that under the condition without the addition of an enzyme, and binding intensity thereof was slightly improved under the condition with the addition of CAPN1 (Figure 14-3 (F)). Binding intensity of MhM1023-M1 comprising both the MMP9 substrate and the CAPN substrate was improved both under the condition with the addition of MMP9 and under the condition with the addition of CAPN1 (Figure 14-4 (G)). The results demonstrate that the designed masked antibodies have protease sensitivity of interest.

(Example 14) *In vivo* pharmaceutical efficacy test of anti-CD98 masked antibody-drug conjugate

The antitumor effects of the antibody-drug conjugate were evaluated using animal models prepared by transplanting the CD98-positive human tumor cells into immunodeficient mice. Before the experiment, 4- to 5-week-old BALB/c nude mice (CAnN.Cg-Foxn1[nu]/CrlCrlj[Foxn1nu/Foxn1nu], Charles River Laboratories Japan, Inc.) were conditioned under SPF conditions for 3 or more days. The mice were fed with sterilized solid feeds (FR-2, Funabashi Farms Co., Ltd.) and sterilized tap water (prepared with the addition of 5 to 15 ppm sodium hypochlorite solutions). The major diameter and the minor diameter of the transplanted tumor were measured two times a week with the use of an electronic digital caliper (CD-15CX, Mitutoyo Corp.), and the tumor volume was determined in accordance with the equation indicated below.

$$\text{Tumor volume (mm}^3\text{)} = 1/2 \times \text{major diameter (mm)} \times [\text{minor diameter (mm)}]^2$$

All the antibody-drug conjugates were diluted with ABS buffer (10 mM acetate buffer, 5 (w/v/)% sorbitol, pH 5.5) (NACALAI), and the antibody solution was administered intravenously into the caudal vein at 10 ml/kg. ABS buffer was administered to a control group (a vehicle group) in the same manner. Groups each consisting of 6 mice were subjected to the experiment.

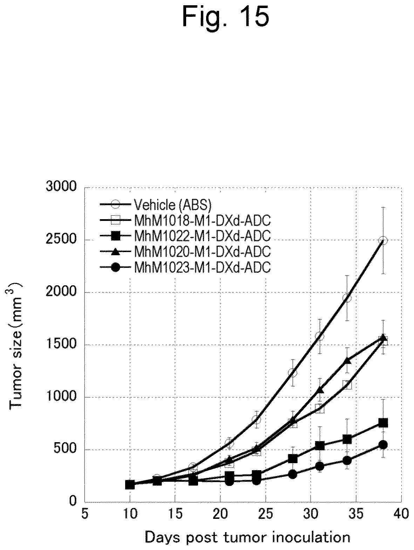

The cells of the CD98-positive human pharyngeal carcinoma cell line FaDu (ATCC) were suspended in saline, $3 \times 10^6$ cells were transplanted subcutaneously into the right abdominal region of female nude mice (Day 0), and the mice were assigned randomly into groups on Day 10. On the day of grouping, 4 types of the antibody-drug conjugates prepared in Example 7 (clone names: MhM1018-M1-DXd-ADC, MhM1020-M1-DXd-ADC, MhM1022-M1-DXd-ADC, and MhM1023-M1-DXd-ADC) were administered intravenously into the caudal vein at a dose of 1 mg/kg. The results are shown in Figure 15. The horizontal axis indicates the number of days, the vertical axis indicates the tumor volume, and the error range is the standard error (SE).

MhM1023-Ml-DXd-ADC comprising both the MMP9 substrate and the CAPN substrate exerted higher antitumor effects than MhM1022-M1-DXd-ADC selectively comprising the MMP9 substrate and MhM1020-M1-DXd-ADC selectively comprising the CAPN substrate

(Example 15) Preparation of anti-EGFR antibody and anti-GPRC5D antibody and evaluation of binding intensity thereof

15)-1 Preparation of anti-EGFR antibody and evaluation of binding intensity thereof

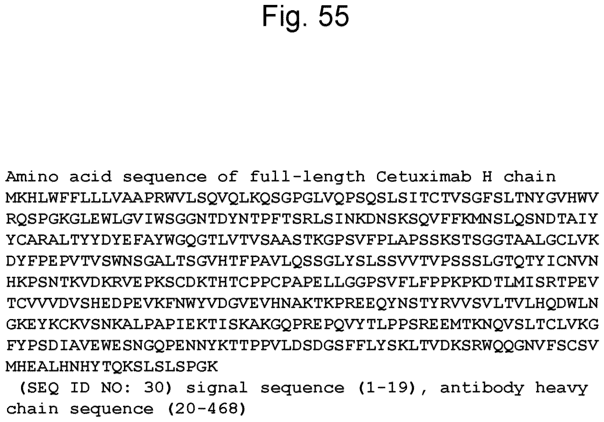

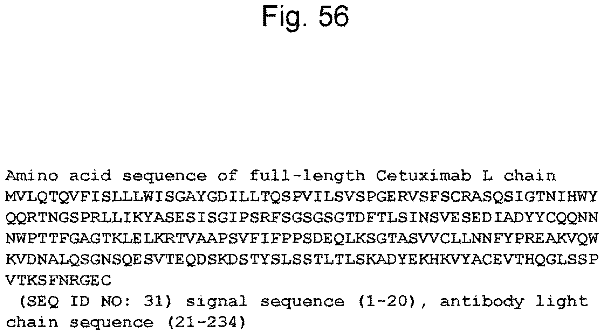

The conventional anti-EGFR antibody Cetuximab (Table 4, the heavy chain sequence (Figure 55, SEQ ID NO: 30); the light chain sequence (Figure 56, SEQ ID NO: 31)) was prepared in the same manner as in Example 1)-1, and binding intensity thereof to the human EGFR was evaluated by ELISA.

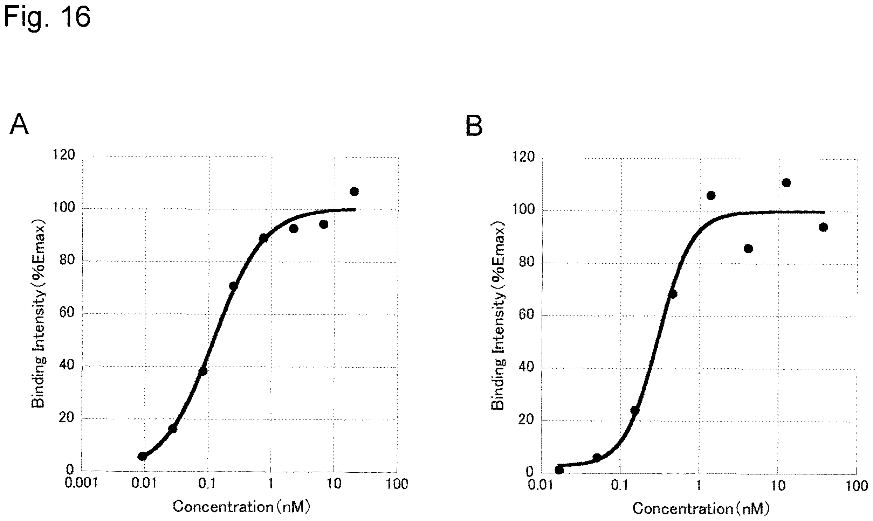

The human EGFR-Fc (a fusion protein of the extracellular domain of human EGFR (Accession Number: P00533) and the human IgG1 Fc region (Accession Number: P01857) purified by a method known to a person skilled in the art) diluted to 0.2 μg/ ml with PBS was added at 50 μl/well to a 96-well Maxi-sorp plate (Black, Nunc) and immobilized at 4°C overnight. The plate was washed with PBS (ELISA buffer) containing 0.05(w/v)% Tween-20 (BioRad) and then blocked with Blocker Casein (Thermo Fisher Scientific). The plate was washed with ELISA buffer, Cetuximab prepared with MMP buffer was added at 50 μl/well, and the resultant was agitated for 30 minutes at room temperature. The plate was washed with ELISA buffer, 50 μl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 16 (A), Cetuximab was found to have bound to the human EGFR antigen in a concentration-dependent manner.

15)-2 Preparation of anti-GPRC5D antibody and evaluation of binding intensity thereof

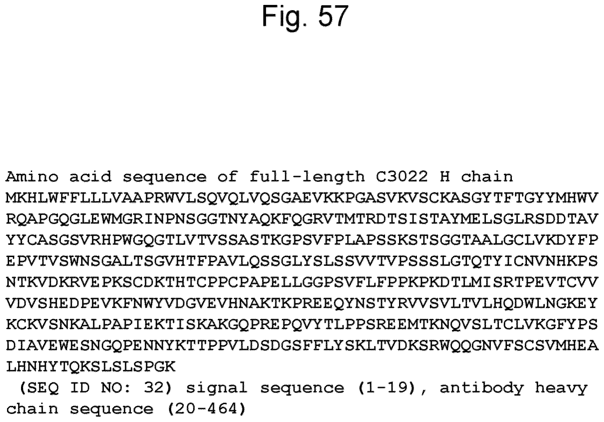

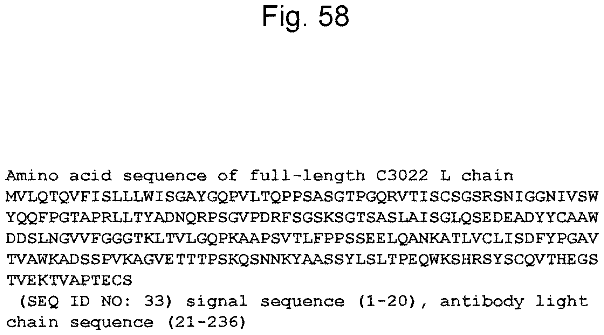

The conventional anti-GPRC5D antibody C3022 described in WO 2018/147245 (Table 4; the heavy chain sequence (Figure 57, SEQ ID NO: 32); the light chain sequence (Figure 58, SEQ ID NO: 33)) was prepared in the same manner as in Example 1)-1, and binding intensity thereof to the humanGPRC5D antigen was evaluated by ELISA.
NeutrAvidin (Thermo Fisher Scientific) diluted to 1 μg/ml with PBS was added at 50 μl/well to a 96-well Maxisorp plate (Black, Nunc) and immobilized at 4°C overnight. The plate was washed with PBS (ELISA buffer) containing 0.05(w/v)% Tween-20 (BioRad) and then blocked with Blocker Casein (Thermo Fisher Scientific). The plate was washed with ELISA buffer, the biotinylated human GPRC5D amino terminal peptide (MYKDCIESTGDYFLLCDAEGPWGIILE-K(Biotin)-$NH_2$ (Peptide Institute, Inc.)) diluted to 1 μg/ml with PBS was added at 50 μl/well, and the resultant was agitated for 30 minutes at room temperature. The plate was washed with ELISA buffer, the MMP-buffered antibody was added at 50 μl/well, and the resultant was then agitated for 30 minutes at room temperature. The plate was washed with ELISA buffer, 50 μl of the horseradish peroxidase (HRP)-labeled anti-human IgG antibody (Jackson Immuno Research Laboratories) diluted to 2,500-fold with ELISA buffer was added, and the resultant was then agitated for 30 minutes at room temperature. The plate was washed with ELISA buffer, the SuperSignal Pico ELISA chemiluminescent substrate (Thermo Fisher Scientific) was added, and chemiluminescence 10 minutes thereafter was then assayed using a plate reader. As shown in Figure 16 (B), C3022 was found to have bound to the human GPRC5D antigen in a concentration-dependent manner.

[Table 4]

| Anti-EGFR antibody and anti-GPRC5D antibody amino acid sequence information | | |
|---|---|---|
| Masked antibody | Heavy chain sequence | Light chain sequence |
| Cetuximab | Cetuximab(SEQ ID NO: 30) | Cetuximab(SEQ ID NO: 31) |
| C3022 | C3022 (SEQ ID NO: 32) | C3022 (SEQ ID NO: 33) |
| MCE-2105 | Cetuximab(SEQ ID NO: 30) | MCE-2105 (SEQ ID NO: 34) |
| MC3-9003 | MC3-9003(SEQ ID NO: 35) | C3022 (SEQ ID NO: 33) |

(Example 16) Concentration of mimotope peptide binding to anti-EGFR antibody Cetuximab and anti-GPRC5D antibody C3022

16)-1 Concentration of mimotope peptide binding to Cetuximab

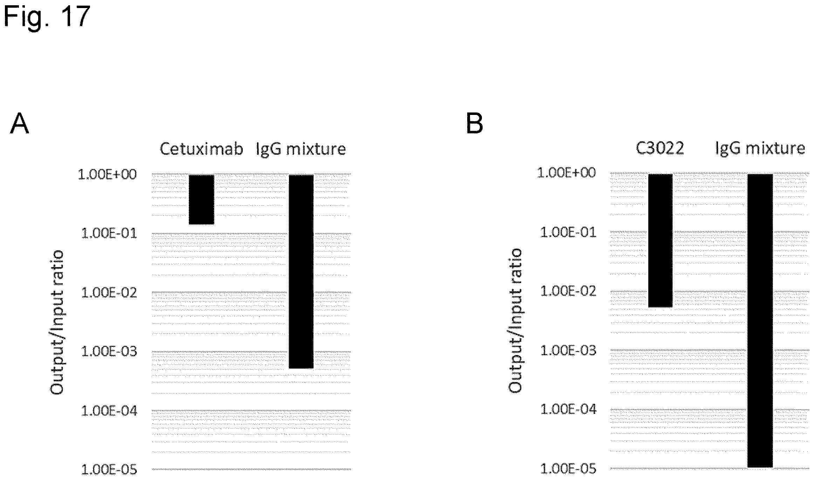

A peptide library by ribosome display composed of completely random 15 amino acids (Linear 15mer lib) was used to concentrate peptides capable of binding to Cetuximab. At the outset, RDs were added to Dynabeads Protein A (Thermo Fisher Scientific) and Dynabeads Protein A (Thermo Fisher Scientific) comprising human serum-derived IgG (Sigma Aldrich) immobilized thereon and allowed to react with Protein A or human serum-derived IgG. RDs that did not bind were collected using a magnet stand (DynaMag-2, Thermo Fisher Scientific) and then allowed to react with the Dynabeads Protein A comprising Cetuximab bound thereto. RDs that did not bind to Cetuximab were removed by washing with the use of a magnet stand, and mRNAs were purified from RDs that had bound to Cetuximab. Thereafter, RDs were prepared again by RT-PCR and *in vitro* translation. This process of panning was performed 4 times.
mRNAs after the fourth round of panning were subjected to *in vitro* translation to prepare RDs, and the prepared RDs were then allowed to react with Dynabeads Protein A comprising Cetuximab or human serum-derived IgG immobilized thereon (amount of input: $6 \times 10^{11}$). RDs that did not bind were removed by washing with the use of a magnet stand, mRNAs were collected from the RDs that had bound, and the amount collected (output) was quantified and evaluated by RT-qPCR. As shown in Figure 17 (A), the number of mRNAs collected under the Cetuximab-immobilized conditions was 276 times greater than that collected under the human serum IgG-immobilized conditions. The results indicate concentration of peptides binding specifically to Cetuximab.

16)-2 Concentration of mimotope peptide binding to anti-GPRC5D antibody C3022

A peptide library by ribosome display composed of completely random 15 amino acids (Linear 15mer lib) was used to concentrate peptides capable of binding to C3022. At the outset, RDs were added to Dynabeads Protein A (Thermo Fisher Scientific) and Dynabeads Protein A (Thermo Fisher Scientific) comprising human serum-derived IgG (Sigma-Aldrich) immobilized thereon and allowed to react with Protein A or human serum-derived IgG. RDs that did not bind were collected using a magnet stand (DynaMag-2, Thermo Fisher Scientific) and then allowed to react with the Dynabeads Protein A comprising C3022 bound thereto. RDs that did not bind to C3022 were removed by washing with the use of a magnet stand, and mRNAs were purified from RDs that had bound to C3022. Thereafter, RDs were prepared again by RT-PCR and *in vitro* translation. This process of panning was performed 3 times.

mRNAs after the third round of panning were subjected to *in vitro* translation to prepare RDs, and the prepared RDs were then allowed to react with Dynabeads Protein A comprising C3022 or human serum-derived IgG immobilized thereon (amount of input: $6 \times 10^{11}$). RDs that did not bind were removed by washing with the use of a magnet stand, mRNAs were collected from the RDs that had bound, and the amount collected (output) was quantified and evaluated by RT-qPCR. As shown in Figure 17 (B), the number of mRNAs collected under the C3022-immobilized conditions was 503 times greater than that collected under the human serum IgG-immobilized conditions. The results indicate concentration of peptides binding specifically to C3022.

(Example 17) General applicability of cleavable linker comprising uPA substrate and CAPN substrate

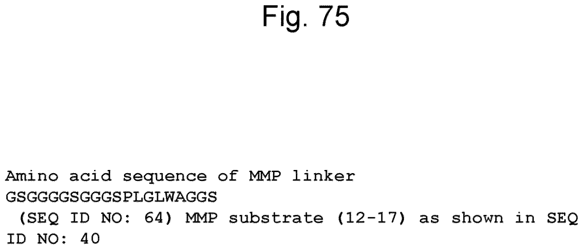

In the same manner as in Example 3, mimotope peptides capable of inhibiting binding of the anti-GPRC5D antibody were selected. Concerning the anti-EGFR antibody Cetuximab, mimotopes were selected in the IgG form instead of the scFv form. In the same manner as in Example 3, a DNA fragment encoding a random peptide portion was digested with a restriction enzyme, purified, and then inserted into a mammalian cell expression vector, so that the secretory signal sequence, a DNA fragment, an MMP cleavable linker (SEQ ID NO: 64; Figure 75), and Cetuximab (the heavy chain or the light chain) would be translated in that order. Cetuximab comprising a peptide and an MMP cleavable linker fused to the N terminus of the heavy chain or the light chain was expressed in the Expi293F cell, and mimotope peptides capable of inhibiting binding of Cetuximab were selected.

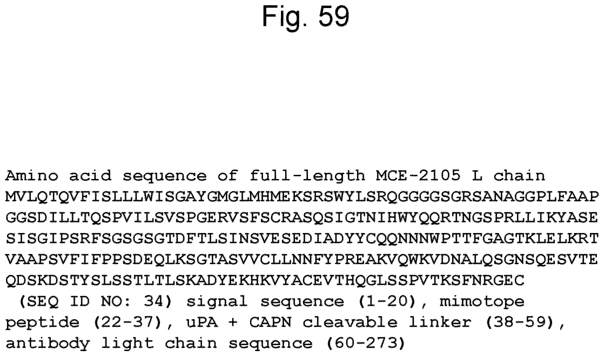

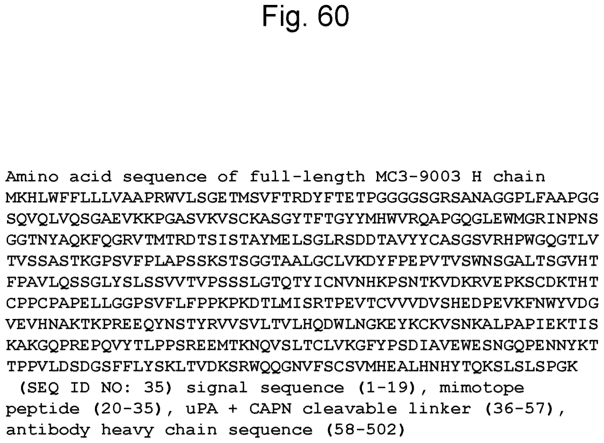

In order to verify that a cleavable linker comprising the uPA substrate and the CAPN substrate is generally applicable to masked antibodies, the anti-EGFR masked antibody MCE-2105 (Table 4, Figure 59, SEQ ID NO: 34) and the anti-GPRC5D masked antibody MC3-9003 (Table 4, Figure 60, SEQ ID NO: 35) each comprising the mimotopes identified by screening were designed. The antibodies were prepared in the same manner as in Example 1)-1 except for the ways described below, and binding intensity thereof to the antigens was evaluated by ELISA in the same manner as in Example 15. After the antibodies were diluted to 2000 nM with MMP buffer, 200 nM uPA or CAPN1 was added, the reaction was allowed to proceed at 37°C, the concentration was adjusted with MMP buffer, and the resultants were added to wells comprising the human EGFR antigen or the humanGPRC5D antigen immobilized thereon.

MCE-2105 exhibited improved binding intensity under the condition with the addition of uPA and CAPN1, compared with that under the condition without the addition of an enzyme (Figure 18 (A)). MC3-9003 also exhibited improved binding intensity under the condition with the addition of uPA and CAPN1, compared with that under the condition without the addition of an enzyme (Figure 18 (B)). The results demonstrate that a cleavable linker comprising the uPA substrate and the CAPN substrate is generally applicable to masked antibodies.

(Example 18) *In vivo* pharmaceutical efficacy test of anti-CD98 masked antibody-drug conjugate

The antitumor effects of the antibody-drug conjugate were evaluated using animal models prepared by transplanting the CD98-positive human tumor cells into immunodeficient mice. Before the experiment, 4- to 5-week-old BALB/c nude mice (CAnN.Cg-Foxn1[nu]/CrlCrlj[Foxn1nu/Foxn1nu], Charles River Laboratories Japan, Inc.) were conditioned under SPF conditions for 3 or more days. The mice were fed with sterilized solid feeds (FR-2, Funabashi Farms Co., Ltd.) and sterilized tap water (prepared with the addition of 5 to 15 ppm sodium hypochlorite solutions). The major diameter and the minor diameter of the transplanted tumor were measured two times a week with the use of an electronic digital caliper (CD-15CX, Mitutoyo Corp.), and the tumor volume was determined in accordance with the equation indicated below.

$$\text{Tumor volume (mm}^3\text{)} = 1/2 \times \text{major diameter (mm)} \times [\text{minor diameter (mm)}]^2$$

All the antibody-drug conjugates were diluted with ABS buffer (10 mM acetate buffer, 5 (w/v/)% sorbitol, pH 5.5) (NACALAI), and the antibody solution was administered intravenously into the caudal vein at 10 ml/kg. ABS buffer

was administered to a control group (a vehicle group) in the same manner. Groups each consisting of 6 mice were subjected to the experiment.

18)-1 Antitumor effects (1)

The cells of the CD98-positive lung squamous cell cancer cell line EBC-1 (JCRB) were suspended in saline/50% Matrigel, 1 × $10^6$ cells were transplanted subcutaneously into the right abdominal region of female nude mice (Day 0), and the mice were assigned randomly into groups on Day 10. On the day of grouping, 4 types of the antibody-drug conjugates prepared in Example 7 (clone names: MhM1018-M1-DXd-ADC, MhM1019-M1-DXd-ADC, MhM1020-M1-DXd-ADC, and MhM1021-M1-DXd-ADC) were administered intravenously into the caudal vein at a dose of 3 mg/kg. The results are shown in Figure 19 (A). The horizontal axis indicates the number of days, the vertical axis indicates the tumor volume, and the error range is the standard error (SE).

MhM1021-M1-DXd-ADC comprising both the uPA substrate and the CAPN substrate exerted higher antitumor effects than MhM1019-M1-DXd-ADC selectively comprising the uPA substrate and MhM1020-M1-DXd-ADC selectively comprising the CAPN substrate.

18)-2 Antitumor effects (2)

In the same manner, the EBC-1 cells were suspended in saline/50% Matrigel, 1 × $10^6$ cells were transplanted subcutaneously into the right abdominal region of female nude mice (Day 0), and the mice were assigned randomly into groups on Day 9. On the day of grouping, 4 types of the antibody-drug conjugates prepared in Example 7 (clone names: MhM1018-M1-DXd-ADC, MhM1020-M1-DXd-ADC, MhM1022-M1-DXd-ADC, and MhM1023-M1-DXd-ADC) were administered intravenously into the caudal vein at a dose of 1 mg/kg. The results are shown in Figure 19 (B).

MhM1023-M1-DXd-ADC comprising both the MMP9 substrate and the CAPN substrate exerted higher antitumor effects than MhM1022-M1-DXd-ADC selectively comprising the MMP9 substrate and MhM1020-M1-DXd-ADC selectively comprising the CAPN substrate.

(Example 19) Activation of anti-TROP2 masked antibody by intracellular CAPN 19)-1 Preparation of cell lysate

The cells of the human head and neck cancer cell line FaDu (ATCC) were cultured in E-MEM medium (Wako) including 10% fetal bovine serum (Hyclone). The cultured FaDu cells were detached by trypsin and washed with PBS, and 1.0 × $10^7$ cells were suspended in 1 ml of lysis buffer (25 mM Tris-HCl (pH 7.5), 1 mM EDTA). After sonication, the cell suspension was centrifuged at 13,000 rpm and 4°C for 15 minutes. Thereafter, the supernatant was collected, protein concentration was measured, and DTT was added to the final concentration of 1 mM in the supernatant. The supernatant supplemented with DTT was stored at -80°C before use and it was used as a cell lysate including intracellular CAPN.

19)-2 Binding intensity of anti-TROP2 masked antibody incubatedwith cell lysate

The anti-TROP2 masked antibody comprising the CAPN substrate was incubated with cell lysate under the condition with the addition of calcium chloride necessary for CAPN activation or without the addition of calcium chloride, and the binding activity to the human TROP2 antigen was evaluated by ELISA. In order to examine activation of the masked antibody by CAPN in the cell lysate, inhibition experiment was performed with CAPN-specific inhibitor PD150606 (Proc. Natl. Acad. Sci., U.S.A.; 93 (13): 6687-6692, 1996). The cell lysate was adjusted to be the final protein concentration to 0.01 μg/ml. Under the condition with the addition of calcium chloride, calcium chloride was added to the final concentration of 2 mM. The antibody was diluted with MilliQ to the final concentration of 20 nM and added to the cell lysate. In the inhibition experiment, PD150606 was added to the cell lysate supplemented with calcium chloride to the final concentration of 100 μM. The reaction was conducted in 75 μl on a 96-well V-bottom plate (Greiner) at 37°C for 1 hour. As a negative control, the reaction was conducted with the lysis buffer instead of the cell lysate.

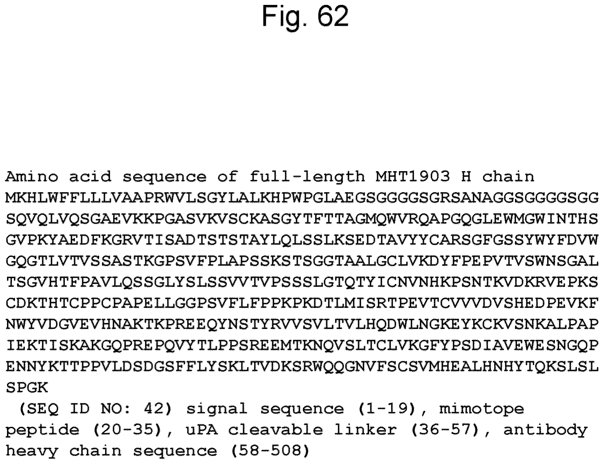

Binding activity of the antibody was evaluated by ELISA in the same manner as in Example 1)-1. As shown in Figure 20 (A), the binding activity of the anti-TROP2 masked antibody MHT3203 comprising both the uPA substrate and the CAPN substrate to the human TROP2 antigen was increased when it was incubated with the cell lysate supplemented with calcium chloride. In contrast, the binding activity of MHT1903 comprising the uPA substrate (Table 5, Figure 62, SEQ ID NO: 42) was not increased. As shown in Figure 20 (B), the CAPN inhibitor PD150606 inhibited an elevation in the binding activity of MHT3203 to the human TROP2 antigen caused by the cell lysate supplemented with calcium chloride. The results demonstrate that the anti-TROP2 masked antibody MHT3203 comprising the CAPN substrate is activated by intracellular CAPN.

(Example 20) Activation of anti-TROP2 masked antibody by two types of enzymes

With the use of the anti-TROP2 masked antibody MHT3202 comprising both the uPA substrate and the CAPN substrate, the correlation between a type of an enzyme to be reacted with a masked antibody and binding intensity of the activated masked antibody was analyzed. The antibody was adjusted to 1000 nM with MMP buffer, and either of 20 nM uPA or 100 nM CAPN1 or both thereof was added thereto by itself or simultaneously. After the reaction was allowed to proceed at 37°C for 30 minutes, binding intensity to the human TROP2 antigen was evaluated by ELISA in the same manner as in Example 1)-1. Binding intensity was evaluated 3 times, the average binding intensity and SD were determined, and a significant difference was then evaluated by the t test (significance level 1%).

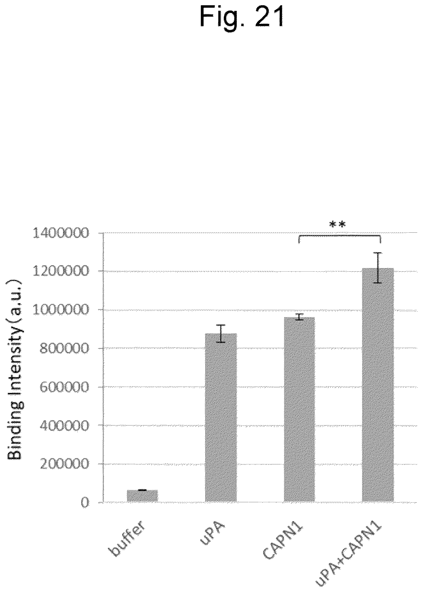

As shown in Figure 21, binding intensity of MHT3202 was further improved under the condition with the addition of uPA and CAPN1, compared with binding intensity attained under the condition with the addition of uPA or CAPN1 ($p < 0.01$). The results demonstrate that binding intensity of a masked antibody can be enhanced when the masked antibody is allowed to react with two types of enzymes; i.e., an extracellular protease and an intracellular protease, compared with the case in which the masked antibody is allowed to react with either of the two types of enzymes. The results also indicate that a masked antibody that can be activated by two types of enzymes; i.e., an extracellular protease and an intracellular protease, may have higher binding intensity in a tumor environment than a masked antibody that can be activated by a single type of protease.

(Example 21) Examination of position of uPA substrate and CAPN substrate using anti-TROP2 masked antibody

21)-1 Activation of masked antibody by uPA and CAPN1

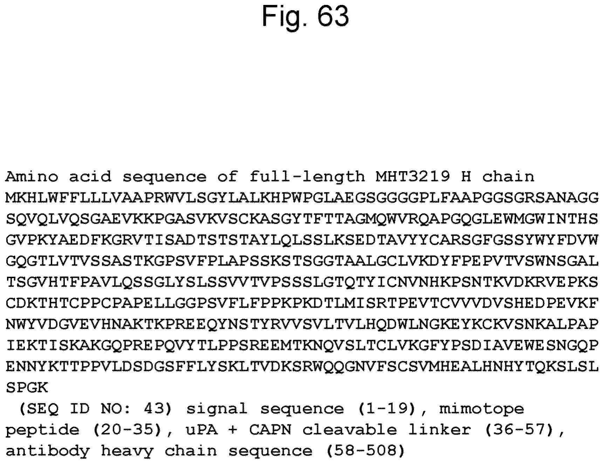

The anti-TROP2 masked antibody MHT3203 comprising both the uPA substrate and the CAPN substrate and MHT3219 with the position of the uPA substrate being switched with the position of the CAPN substrate in a cleavable linker (Table 5, Figure 63, SEQ ID NO: 43) were prepared in the same manner as in Example 1-1), and activation of the masked antibodies by uPA and CAPN1 was evaluated by ELISA in the same manner as in Example 6.

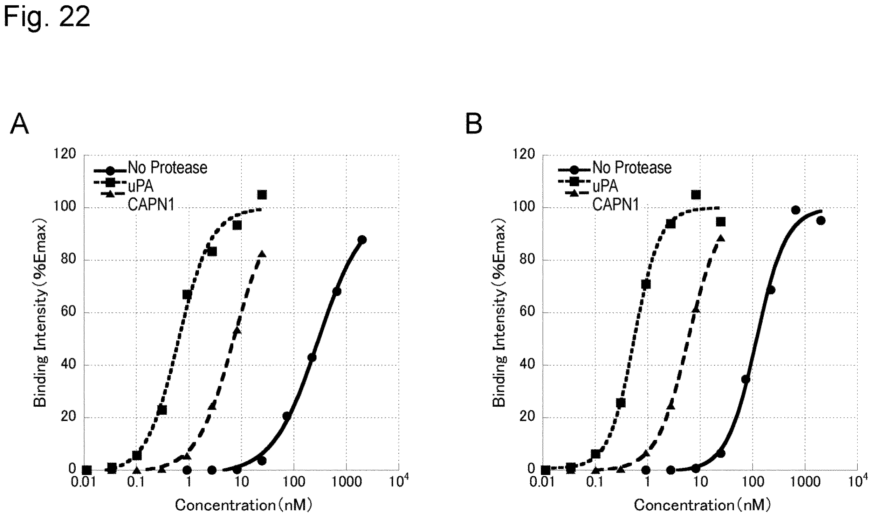

MHT3203 was activated by uPA and CAPN1 (Figure 22 (A)), and MHT3219 was also activated by uPA and CAPN1 as with the case of MHT3203 (Figure 22 (B)).

[Table 5]

Anti-TROP2 masked antibody and amino acid sequence information

| Masked antibody | Heavy chain sequence | Light chain sequence |
|---|---|---|
| MHT1903 | MHT1903 (SEQ ID NO: 42) | HT1-11 (SEQ ID NO: 2) |
| MHT3219 | MHT3219 (SEQ ID NO: 43) | HT1-11 (SEQ ID NO: 2) |
| MHT5082 | MHT5082 (SEQ ID NO: 54) | HT1-11 (SEQ ID NO: 2) |
| MHT5084 | MHT5084 (SEQ ID NO: 55) | HT1-11 (SEQ ID NO: 2) |
| MHT5085 | MHT5085 (SEQ ID NO: 56) | HT1-11 (SEQ ID NO: 2) |
| MHT5086 | MHT5086 (SEQ ID NO: 57) | HT1-11 (SEQ ID NO: 2) |
| MHT5090 | MHT5090 (SEQ ID NO: 58) | HT1-11 (SEQ ID NO: 2) |
| MHT5091 | MHT5091 (SEQ ID NO: 59) | HT1-11 (SEQ ID NO: 2) |
| MHT5092 | MHT5092 (SEQ ID NO: 60) | HT1-11 (SEQ ID NO: 2) |
| MHT5093 | MHT5093 (SEQ ID NO: 61) | HT1-11 (SEQ ID NO: 2) |
| MHT5094 | MHT5094 (SEQ ID NO: 62) | HT1-11 (SEQ ID NO: 2) |
| MHT5095 | MHT5095 (SEQ ID NO: 63) | HT1-11 (SEQ ID NO: 2) |

21)-2 Anti-tumor effects of anti-TROP2 masked antibody-drug conjugate (1)

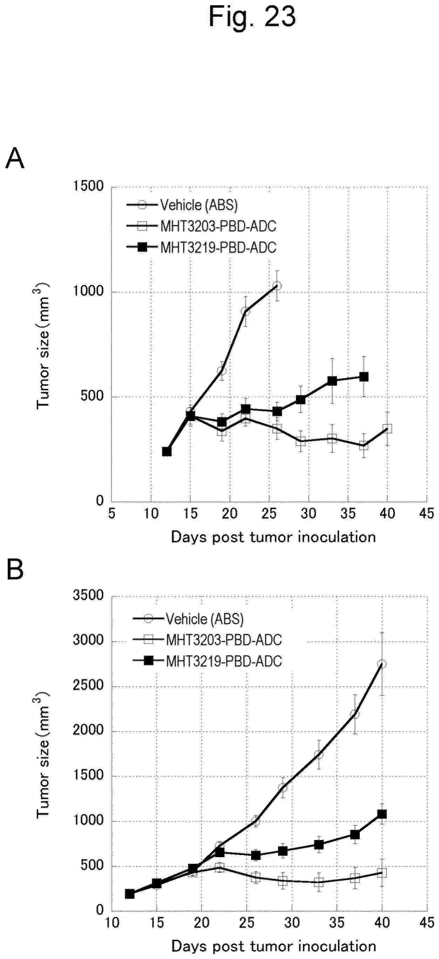

The antitumor effects of the antibody-drug conjugate was evaluated in the same manner as in Example 8. The cells of the TROP2-positive human lung mucoepidermoid carcinoma cell line NCI-H292 (ATCC) were suspended in saline, $5 \times 10^6$ cells were transplanted subcutaneously into the right abdominal region of female nude mice (Day 0), and the mice were assigned randomly into groups on Day 12. On the day of grouping, 2 types of the antibody-drug conjugates prepared in Example 7 (clone names: MHT3219-PBD-ADC and MHT3203-PBD-ADC) were administered intravenously into the caudal vein at a dose of 0.4 mg/kg. The results are shown in Figure 23 (A). The horizontal axis indicates the number of days, the vertical axis indicates the tumor volume, and the error range is the standard error (SE).

MHT3203-PBD-ADC comprising both the uPA substrate and the CAPN substrate exerted higher antitumor effects than MHT3219-PBD-ADC with the substrate positions being switched. Accordingly, a cleavable linker comprising

the uPA substrate in a position closer to the amino terminus than the CAPN substrate was found to be more preferable to a cleavable linker comprising the substrates in opposite positions.

21)-3 Anti-tumor effects of anti-TROP2 antibody-drug conjugate (2)

The cells of the TROP2-positive human pharyngeal carcinoma cell line FaDu cell line (ATCC) were suspended in saline, $3 \times 10^6$ cells were transplanted subcutaneously into the right abdominal region of female nude mice (Day 0), and the mice were assigned randomly into groups on Day 12. On the day of grouping, 2 types of the antibody-drug conjugates prepared in Example 7 (clone names: MHT3219-PBD-ADC and MHT3203-PBD-ADC) were administered intravenously into the caudal vein at a dose of 0.4 mg/kg. The results are shown in Figure 23 (B). The horizontal axis indicates the number of days, the vertical axis indicates the tumor volume, and the error range is the standard error (SE).
MHT3203-PBD-ADC comprising both the uPA substrate and the CAPN substrate exerted higher antitumor effects than MHT3219-PBD-ADC with the substrate positions being switched.

(Example 22) Examination of CAPN substrate sequence

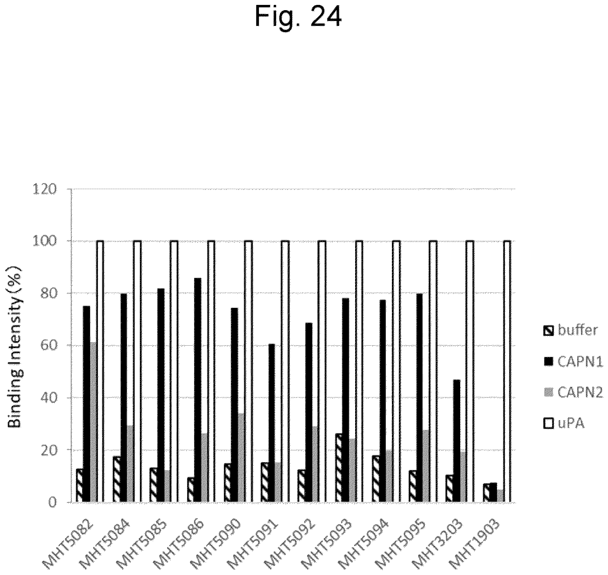

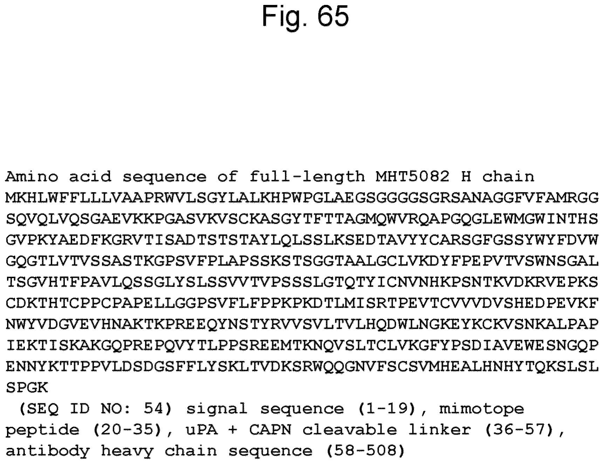

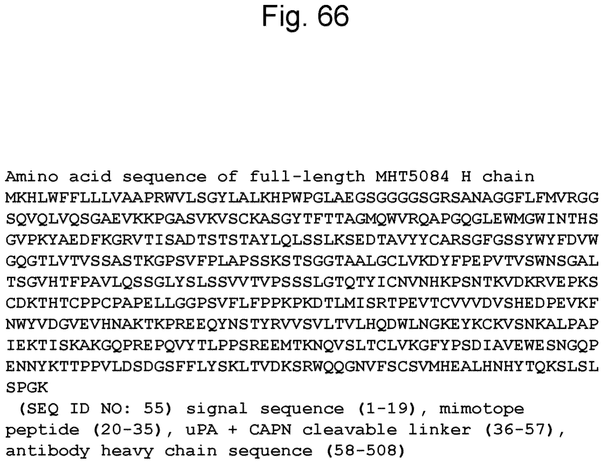

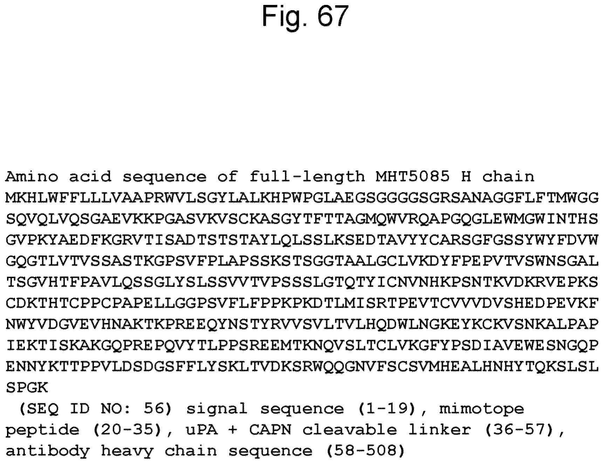

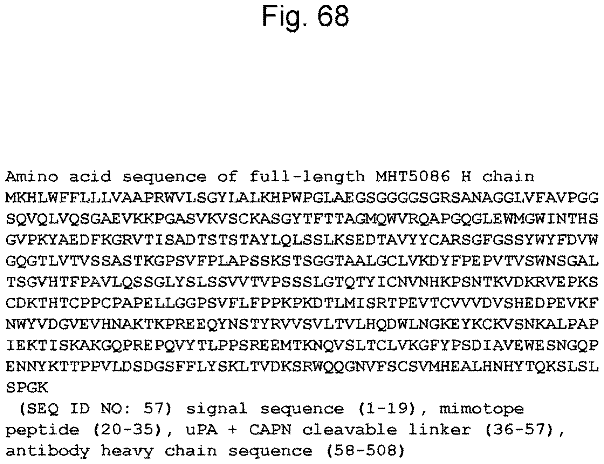

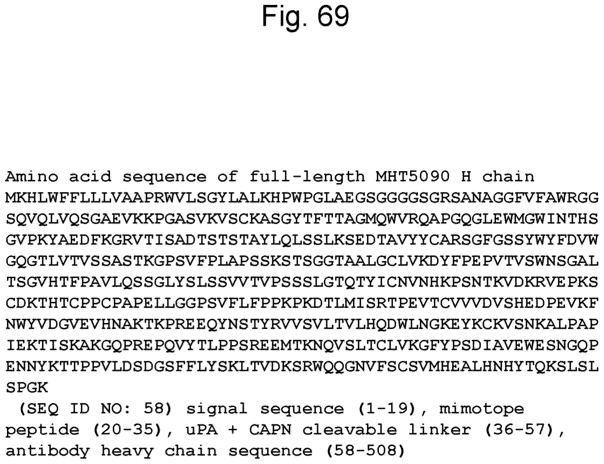

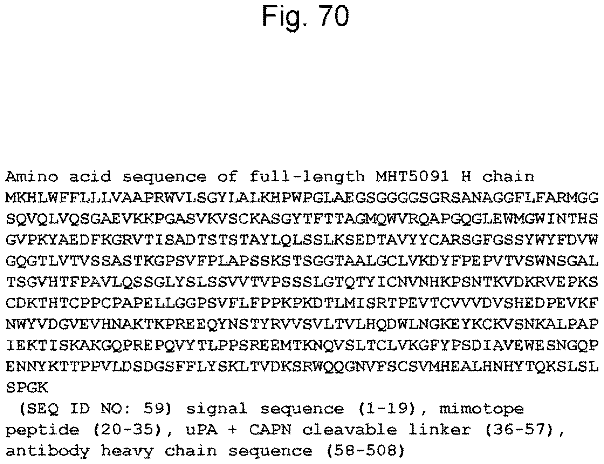

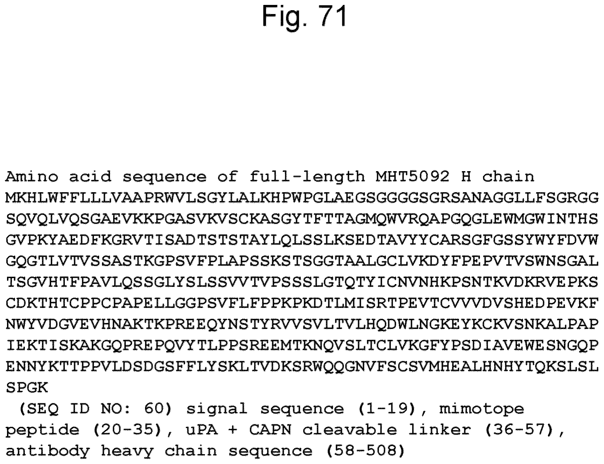

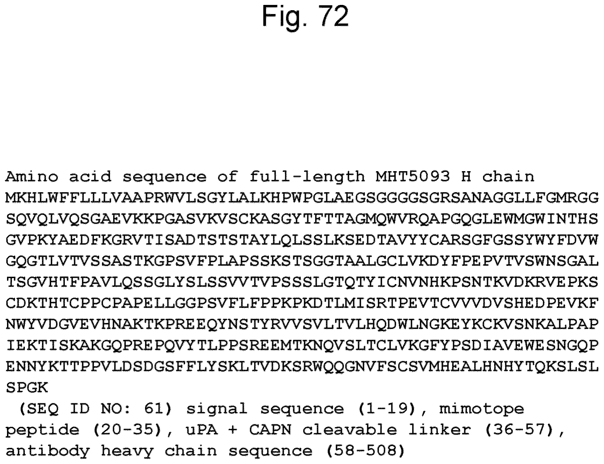

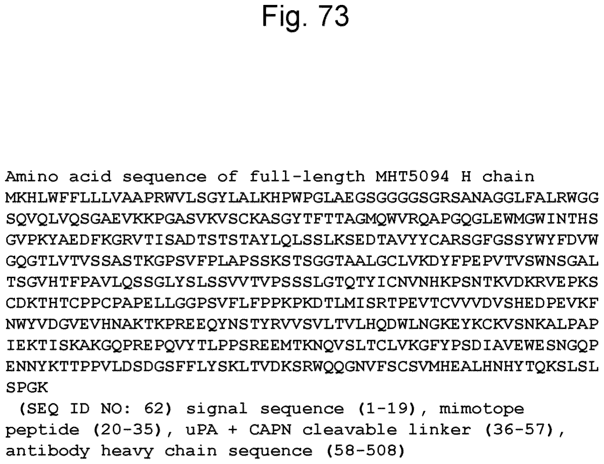

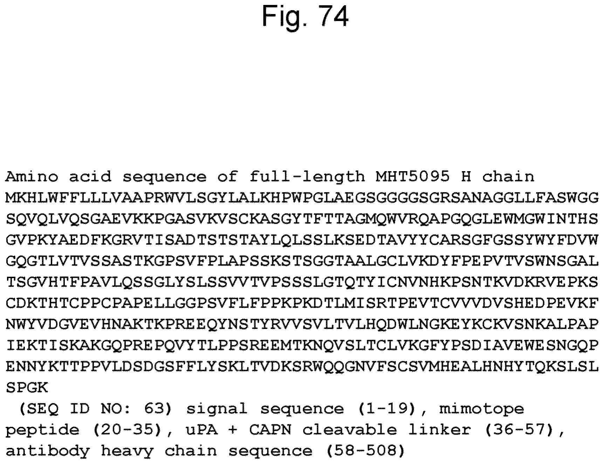

The anti-TROP2 masked antibodies each comprising both the uPA substrate and the CAPN substrate : MHT5082 (Table 5, Figure 65, SEQ ID NO: 54), MHT5084 (Table 5, Figure 66, SEQ ID NO: 55), MHT5085 (Table 5, Figure 67, SEQ ID NO: 56), MHT5086 (Table 5, Figure 68, SEQ ID NO: 57), MHT5090 (Table 5, Figure 69, SEQ ID NO: 58), MHT5091 (Table 5, Figure 70, SEQ ID NO: 59), MHT5092 (Table 5, Figure 71, SEQ ID NO: 60), MHT5093 (Table 5, Figure 72, SEQ ID NO: 61), MHT5094 (Table 5, Figure 73, SEQ ID NO: 62), MHT5095 (Table 5, Figure 74, SEQ ID NO: 63), and MHT3203, and MHT1903 selectively comprising the uPA substrate were prepared in the same manner as in Example 1-1), and activation by uPA, CAPN1, and CAPN2 (Accession Number: P17655) was evaluated by ELISA in the same manner as in Example 5)-1, except for the ways described below. The antibodies were diluted to 20 nM with MMP buffer, 50 nM uPA, 100 nM CAPN1, or 100 nM CAPN2 was added thereto, the reaction was allowed to proceed at 37°C, and the resultants were added to wells comprising the human TROP2 antigen immobilized thereon. Binding intensity attained upon the reaction with uPA was designated as 100%, and protease sensitivity of clones was compared therewith.
As shown in Figure 24, binding intensity of MHT1903 was improved under the condition with the addition of uPA, but it was not improved under the condition with the addition of CAPN1 or CAPN2. In contrast, binding intensity of the anti-TROP2 masked antibody comprising both the uPA substrate and the CAPN substrate was improved both under the condition with the addition of uPA and under the condition with the addition of CAPN (CAPN1 or CAPN2).

(Example 23) *In vivo* pharmaceutical efficacy of PBD-ADC comprising different CAPN substrate sequence

23)-1 Antitumor effects of anti-TROP2 antibody-drug conjugate (1)

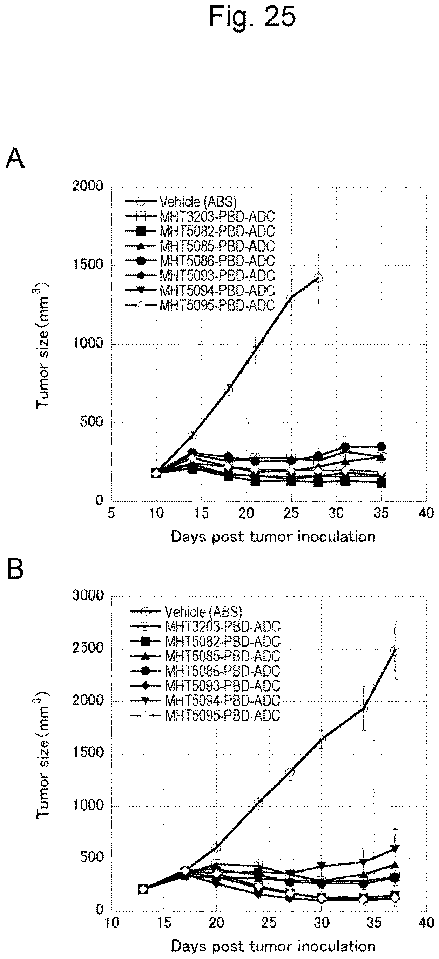

The antitumor effects of the antibody-drug conjugate was evaluated in the same manner as in Example 8. The cells of the TROP2-positive human lung mucoepidermoid carcinoma cell line NCI-H292 (ATCC) were suspended in saline, $5 \times 10^6$ cells were transplanted subcutaneously into the right abdominal region of female nude mice (Day 0), and the mice were assigned randomly into groups on Day 10. On the day of grouping, 7 types of the antibody-drug conjugates prepared in Example 7 (clone names: MHT3203-PBD-ADC, MHT5082-PBD-ADC, MHT5085-PBD-ADC, MHT5086-PBD-ADC, MHT5093-PBD-ADC, MHT5094-PBD-ADC, and MHT5095-PBD-ADC) were administered intravenously into the caudal vein at a dose of 0.4 mg/kg. The results are shown in Figure 25 (A). The horizontal axis indicates the number of days, the vertical axis indicates the tumor volume, and the error range is the standard error (SE).
As with the case of MHT3203-PBD-ADC comprising both the uPA substrate and the CAPN substrate, MHT5082-PBD-ADC, MHT5085-PBD-ADC, MHT5086-PBD-ADC, MHT5093-PBD-ADC, MHT5094-PBD-ADC, and MHT5095-PBD-ADC exerted high antitumor effects.

23)-2 Antitumor effects of anti-TROP2 antibody-drug conjugate (2)

The cells of the TROP2-positive human pharyngeal carcinoma cell line FaDu (ATCC) were suspended in saline, $3 \times 10^6$ cells were transplanted subcutaneously into the right abdominal region of female nude mice (Day 0), and the mice were assigned randomly into groups on Day 13. On the day of grouping, 7 types of the antibody-drug conjugates prepared in Example 7 (clone names: MHT3203-PBD-ADC, MHT5082-PBD-ADC, MHT5085-PBD-ADC, MHT5086-PBD-ADC, MHT5093-PBD-ADC, MHT5094-PBD-ADC, and MHT5095-PBD-ADC) were administered intravenously into the caudal vein at a dose of 0.4 mg/kg. The results are shown in Figure 25 (B). The horizontal axis indicates the number of days, the vertical axis indicates the tumor volume, and the error range is the standard error (SE).

As with the case of MHT3203-PBD-ADC comprising both the uPA substrate and the CAPN substrate, MHT5082-PBD-ADC, MHT5085-PBD-ADC, MHT5086-PBD-ADC, MHT5093-PBD-ADC, MHT5094-PBD-ADC, and MHT5095-PBD-ADC exerted high antitumor effects.

Industrial Applicability

The improved masked antibodies according to the present invention can be used for treatment of various cancer species.

[Sequence Listing Free Text]

SEQ ID NO: 1: the amino acid sequence of the heavy chain of the anti-TROP2 antibody (Figure 26)
SEQ ID NO: 2: the amino acid sequence of the light chain of the anti-TROP2 antibody (Figure 27)
SEQ ID NO: 3: the amino acid sequence of MHT1001 (Figure 28)
SEQ ID NO: 4: the amino acid sequence of MHT1002 (Figure 29)
SEQ ID NO: 5: the amino acid sequence of HT1-11-scFv-HL (Figure 30)
SEQ ID NO: 6: the amino acid sequence of HT1-11-scFv-LH (Figure 31)
SEQ ID NO: 7: the amino acid sequence of the heavy chain of MHT1007 (Figure 32)
SEQ ID NO: 8: the amino acid sequence of the heavy chain of MHT1008 (Figure 33)
SEQ ID NO: 9: the amino acid sequence of the heavy chain of MHT1009 (Figure 34)
SEQ ID NO: 10: the amino acid sequence of the heavy chain of MHT3002 (Figure 35)
SEQ ID NO: 11: the amino acid sequence of CAPN1 (Figure 36)
SEQ ID NO: 12: the amino acid sequence of a novel CAPN substrate (Figure 37)
SEQ ID NO: 13: the amino acid sequence of the heavy chain of MHT3201 (Figure 38)
SEQ ID NO: 14: the amino acid sequence of the heavy chain of MHT3202 (Figure 39)
SEQ ID NO: 15: the amino acid sequence of the heavy chain of MHT1713 (Figure 40)
SEQ ID NO: 16: the amino acid sequence of the heavy chain of HMT342 (Figure 41)
SEQ ID NO: 17: the amino acid sequence of the heavy chain of MHT3203 (Figure 42)
SEQ ID NO: 18: the amino acid sequence of the heavy chain of the anti-CD98 antibody hM23H1L1 (Figure 43)
SEQ ID NO: 19: the amino acid sequence of the light chain of the anti-CD98 antibody hM23H1L1 (Figure 44)
SEQ ID NO: 20: the amino acid sequence of the light chain of MhM1008 (Figure 45)
SEQ ID NO: 21: the amino acid sequence of the light chain of MhM1013 (Figure 46)
SEQ ID NO: 22: the amino acid sequence of the heavy chain of hM23-M1 (Figure 47)
SEQ ID NO: 23: the amino acid sequence of the light chain of hM23-M1 (Figure 48)
SEQ ID NO: 24: the amino acid sequence of the light chain of MhM1018 (Figure 49)
SEQ ID NO: 25: the amino acid sequence of the light chain of MhM1019 (Figure 50)
SEQ ID NO: 26: the amino acid sequence of the light chain of MhM1020 (Figure 51)
SEQ ID NO: 27: the amino acid sequence of the light chain of MhM1021 (Figure 52)
SEQ ID NO: 28: the amino acid sequence of the light chain of MhM1022 (Figure 53)
SEQ ID NO: 29: the amino acid sequence of the light chain of MhM1023 (Figure 54)
SEQ ID NO: 30: the amino acid sequence of the heavy chain of the anti-EGFR antibody cetuximab (Figure 55)
SEQ ID NO: 31: the amino acid sequence of the light chain of the anti-EGFR antibody cetuximab (Figure 56)
SEQ ID NO: 32: the amino acid sequence of the heavy chain of the anti-GPRC5D antibody C3022 (Figure 57)
SEQ ID NO: 33: the amino acid sequence of the light chain of the anti-GPRC5D antibody C3022 (Figure 58)
SEQ ID NO: 34: the amino acid sequence of the light chain of MCE-2105 (Figure 59)
SEQ ID NO: 35: the amino acid sequence of the heavy chain of MC3-9003 (Figure 60)
SEQ ID NO: 36: the amino acid sequence recognized by human uPA and serving as a substrate for human uPA (Figure 61)
SEQ ID NO: 37: the amino acid sequence recognized by human uPA and serving as a substrate for human uPA (Figure 61)
SEQ ID NO: 38: the amino acid sequence recognized by human uPA and serving as a substrate for human uPA (Figure 61)
SEQ ID NO: 39: the amino acid sequence recognized by human MMP1 and serving as a substrate for human MMP1 (Figure 61)
SEQ ID NO: 40: the amino acid sequence recognized by human MMP1 and serving as a substrate for human MMP1 (Figure 61)

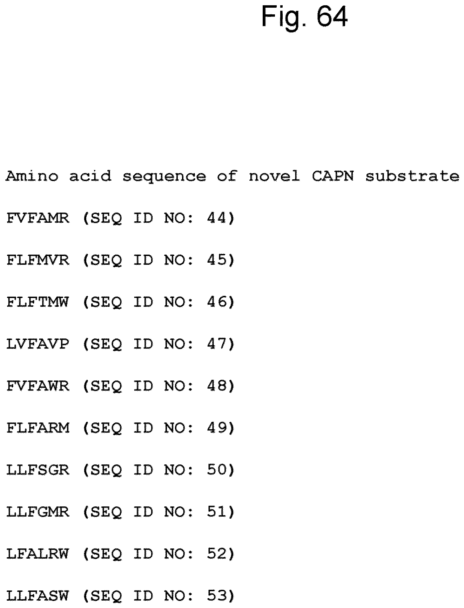

SEQ ID NO: 41: the amino acid sequence recognized by human MMP9 and serving as a substrate for human MMP9 (Figure 61)
SEQ ID NO: 42: the amino acid sequence of the heavy chain of MHT1903 (Figure 62)
SEQ ID NO: 43: the amino acid sequence of the heavy chain of MHT3219 (Figure 63)
SEQ ID NO: 44: the amino acid sequence of a novel CAPN substrate (Figure 64)
SEQ ID NO: 45: the amino acid sequence of a novel CAPN substrate (Figure 64)
SEQ ID NO: 46: the amino acid sequence of a novel CAPN substrate (Figure 64)
SEQ ID NO: 47: the amino acid sequence of a novel CAPN substrate (Figure 64)
SEQ ID NO: 48: the amino acid sequence of a novel CAPN substrate (Figure 64)
SEQ ID NO: 49: the amino acid sequence of a novel CAPN substrate (Figure 64)
SEQ ID NO: 50: the amino acid sequence of a novel CAPN substrate (Figure 64)
SEQ ID NO: 51: the amino acid sequence of a novel CAPN substrate (Figure 64)
SEQ ID NO: 52: the amino acid sequence of a novel CAPN substrate (Figure 64)
SEQ ID NO: 53: the amino acid sequence of a novel CAPN substrate (Figure 64)
SEQ ID NO: 54: the amino acid sequence of the heavy chain of MHT5082 (Figure 65)
SEQ ID NO: 55: the amino acid sequence of the heavy chain of MHT5084 (Figure 66)
SEQ ID NO: 56: the amino acid sequence of the heavy chain of MHT5085 (Figure 67)
SEQ ID NO: 57: the amino acid sequence of the heavy chain of MHT5086 (Figure 68)
SEQ ID NO: 58: the amino acid sequence of the heavy chain of MHT5090 (Figure 69)
SEQ ID NO: 59: the amino acid sequence of the heavy chain of MHT5091 (Figure 70)
SEQ ID NO: 60: the amino acid sequence of the heavy chain of MHT5092 (Figure 71)
SEQ ID NO: 61: the amino acid sequence of the heavy chain of MHT5093 (Figure 72)
SEQ ID NO: 62: the amino acid sequence of the heavy chain of MHT5094 (Figure 73)
SEQ ID NO: 63: the amino acid sequence of the heavy chain of MHT5095 (Figure 74)
SEQ ID NO: 64: the amino acid sequence of the MMP linker (Figure 75)

All publications, patents, and patent applications cited herein are incorporated herein by reference in their entirety.

## Claims

**1.** A molecule comprising a moiety [a], a moiety [b], and a moiety [c] indicated below and binding to a target antigen:

a moiety [a]: a moiety binding to the target antigen;
a moiety [b]: a first peptide recognizing a target antigen-binding site included in the moiety [a]; and
a moiety [c]: a second peptide comprising an amino acid sequence cleaved by a protease localized in the cytoplasm.

**2.** The molecule according to claim 1, wherein the second peptide has higher binding affinity to the target antigen after it is cleaved by a protease localized in the cytoplasm than that before it is cleaved.

**3.** The molecule according to claim 1 or 2, wherein the second peptide in the moiety [c] further comprises an amino acid sequence cleaved by an extracellular protease.

**4.** The molecule according to any one of claims 1 to 3, wherein the first peptide, the second peptide, and the moiety binding to the target antigen are connected in that order.

**5.** The molecule according to any one of claims 1 to 4, wherein the moiety binding to the target antigen is a polypeptide consisting of an amino acid sequence that is not comprised in the first peptide or the second peptide.

**6.** The molecule according to any one of claims 1 to 5, which consists of a polypeptide.

**7.** The molecule according to claim 6, which comprises the first peptide, the second peptide, and the moiety binding to the target antigen or the moiety binding to the target antigen, the second peptide, and the first peptide are connected in that order from the amino terminus toward the carboxyl terminus.

**8.** The molecule according to any one of claims 1 to 7, wherein a moiety selected from the group consisting of the first

peptide, the second peptide, and the moiety binding to the target antigen is connected to either or both of the other two moieties via linker (or linkers).

**9.** The molecule according to any one of claims 1 to 8, wherein the protease localized in the cytoplasm leaks extracellularly upon cell death or a damage imposed on cell membrane.

**10.** The molecule according to any one of claims 1 to 9, wherein the protease localized in the cytoplasm is selected from among calpain, caspase, and tripeptidyl peptidase and it is preferable that calpain be calpain 1 or calpain 2, caspase be caspase 1, caspase 8, caspase 3, or caspase 7, and tripeptidyl peptidase be tripeptidyl peptidase 1 or tripeptidyl peptidase 2.

**11.** The molecule according to any one of claims 1 to 10, wherein the protease localized in the cytoplasm is a calcium-dependent or calcium-requiring protease.

**12.** The molecule according to any one of claims 1 to 11, wherein the protease localized in the cytoplasm is calpain 1 or calpain 2.

**13.** The molecule according to any one of claims 3 to 12, wherein the extracellular protease is expressed at a higher level, exists in a larger amount, or has higher catalytic activity in diseased tissue than in healthy tissue.

**14.** The molecule according to claim 13, wherein the diseased tissue is tumor tissue and/or stromal tissue.

**15.** The molecule according to any one of claims 3 to 14, wherein the extracellular protease is selected from the group consisting of matrix metalloproteinase, urokinase type plasminogen activator, matriptase, legumain, and cathepsin and it is preferable that the matrix metalloproteinase be at least one substance selected from among MMP1, MMP2, MMP3, MMP7, MMP9, MMP12, and MMP14 and that the cathepsin be at least one substance selected from among cathepsin B, cathepsin D, cathepsin S, and cathepsin L.

**16.** The molecule according to any one of claims 1 to 15, wherein the second peptide comprises an amino acid sequence cleaved by the extracellular protease and the amino acid sequence cleaved by the protease localized in the cytoplasm or an amino acid sequence cleaved by a protease localized in the cytoplasm and an amino acid sequence cleaved by the extracellular protease located in that order from the amino terminus toward the carboxyl terminus, preferably, an amino acid sequence cleaved by the extracellular protease and an amino acid sequence cleaved by the protease localized in the cytoplasm located in that order from the amino terminus toward the carboxyl terminus.

**17.** The molecule according to any one of claims 1 to 16, wherein the amino acid sequence cleaved by the protease localized in the cytoplasm comprises at least one sequence selected from among SEQ ID NOs: 12 and 37 to 46.

**18.** The molecule according to any one of claims 1 to 17, wherein the target antigen is a tumor antigen.

**19.** The molecule according to any one of claims 1 to 18, which comprises another moiety [d] that binds to a moiety binding to the target antigen and the moiety [d] does not comprise the first peptide or the second peptide.

**20.** The molecule according to claim 19, wherein the moiety [d] is at least one substance selected from the group consisting of an antibody that is not a moiety binding to the target antigen or an antigen-binding fragment thereof, a peptide comprising an amino acid sequence that is not comprised in the first peptide or the second peptide, a cytokine, a toxin, a radioactive isotope, a label molecule, a photosensitive substance, an immunostimulant, an antitumor compound, a drug, a payload, and a polymer.

**21.** The molecule according to claim 20, wherein the antitumor compound is a camptothecin derivative or a pyrrolobenzodiazepine derivative, preferably, the camptothecin derivative be N-[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]-2-hydroxyacetamide.

**22.** The molecule according to claim 20, wherein the immunostimulant is a cyclic dinucleotide derivative.

**23.** The molecule according to any one of claims 19 to 22, which consists of a polypeptide, or the moiety [d] and a polypeptide.

**24.** A method for identifying a peptide binding to a complementarity determining region (CDR) comprised in an antibody or an antigen-binding fragment of an antibody comprising a step (i) and a step (ii):

(i) a step of bringing a peptide library comprising a repeat sequence of aromatic amino acid and Pro into contact with the CDR; and
(ii) a step of collecting a peptide which binds to the CDR.

**25.** The method according to claim 24, wherein the repeat sequence of aromatic amino acid and Pro is a ZPZP motif (wherein Z represents any aromatic amino acid selected from among histidine (His), phenylalanine (Phe), tyrosine (Tyr), and tryptophan (Trp); and P represents proline).

**26.** The method according to claim 24 or 25, which further comprises a step of preparing the peptide by recombination, *in vitro* translation, chemical synthesis, or peptide synthesis.

**27.** A molecule binding to a target antigen, wherein the CDR is comprised in the moiety that binds to the target antigen as defined in claim 1 and the first peptide is obtained by the method according to claim 26.

**28.** A method for producing the molecule according to any one of claims 1 to 23, which comprises a step of preparing a peptide comprising an amino acid sequence comprised in the first peptide and/or an amino acid sequence comprised in the second peptide by recombination, *in vitro* translation, chemical synthesis, or peptide synthesis.

**29.** A molecule binding to a target antigen, which is obtained by the method according to claim 28.

**30.** A method for producing the molecule according to claim 6, 7, or 23, which comprises a step (iii):
(iii) a step of introducing a polynucleotide comprising a nucleotide sequence encoding an amino acid sequence comprised in a moiety that binds to the target antigen and optionally an amino acid sequence comprised in the first peptide, and/or an amino acid sequence comprised in the second peptide into a cell, culturing the cell, and collecting a polypeptide binding to the target antigen from the culture product.

**31.** A molecule binding to the target antigen, which is obtained by the method according to claim 30.

**32.** A pharmaceutical composition comprising the molecule according to any one of claims 1 to 23, 27, 29, and 31, a salt thereof, or a hydrate of the molecule or salt.

**33.** The pharmaceutical composition according to claim 32, which is an anti-cancer agent.

**34.** A peptide library comprising a repeat sequence of aromatic amino acid and Pro.

**35.** The peptide library according to claim 34, wherein the repeat sequence of aromatic amino acid and Pro is a ZPZP motif (wherein Z represents any aromatic amino acid selected from among histidine (His), phenylalanine (Phe), tyrosine (Tyr), and tryptophan (Trp); and P represents proline).

Fig. 1

A

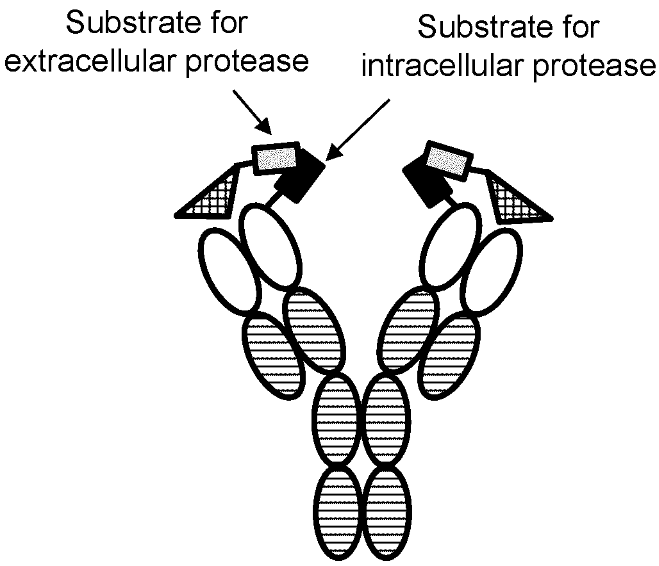
Substrate for
extracellular protease
Substrate for
intracellular protease

B

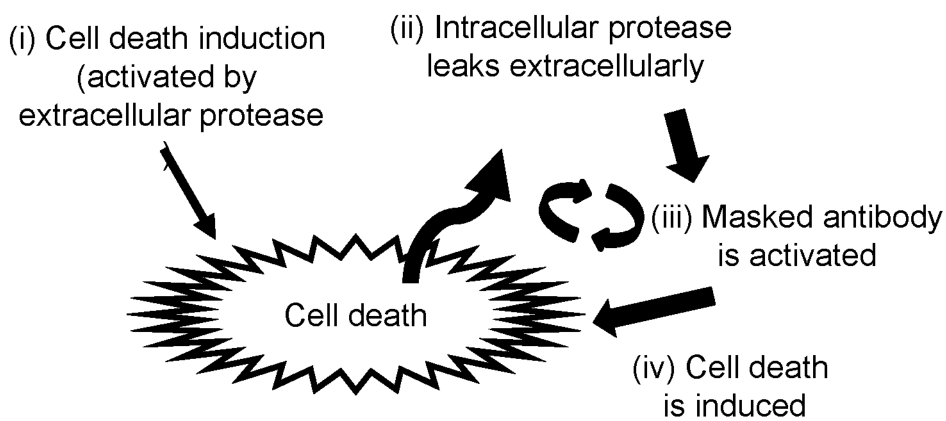
(i) Cell death induction
(activated by
extracellular protease
(ii) Intracellular protease
leaks extracellularly
(iii) Masked antibody
is activated
Cell death
(iv) Cell death
is induced

Fig. 2

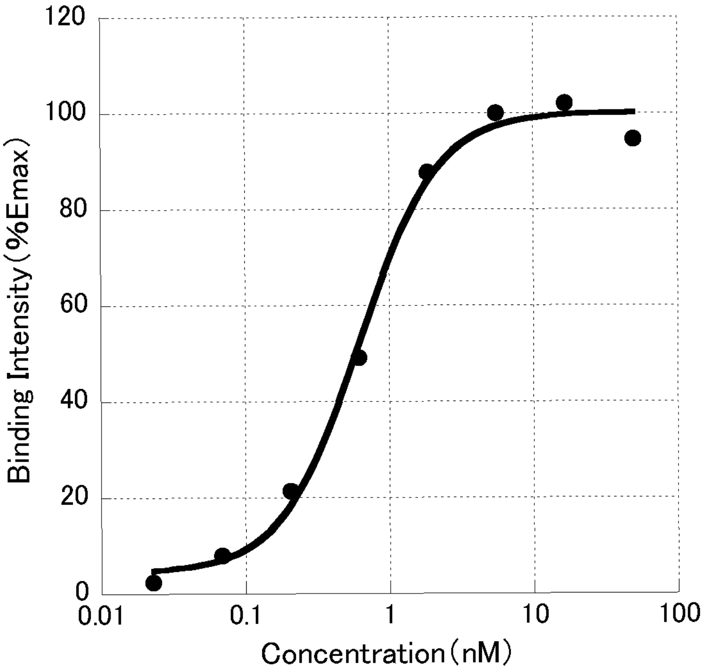
120
100
80
60
40
20
0
Binding Intensity (%Emax)
0.01
0.1
1
10
100
Concentration (nM)

Fig. 3

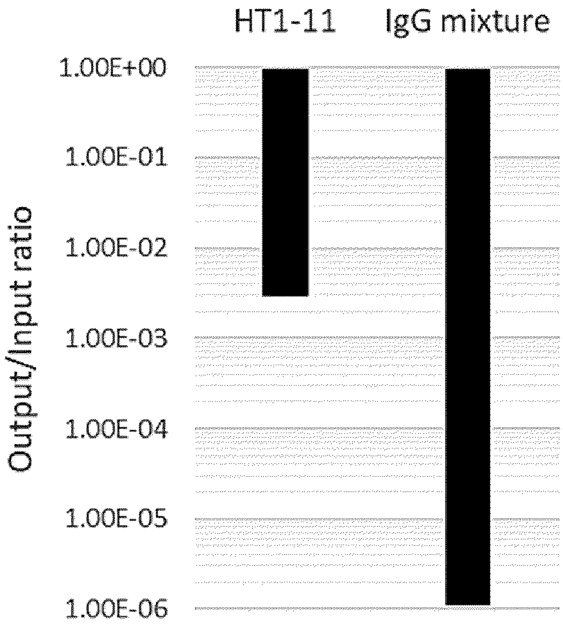
HT1-11
IgG mixture
Output/Input ratio
1.00E+00
1.00E-01
1.00E-02
1.00E-03
1.00E-04
1.00E-05
1.00E-06

Fig. 4-1

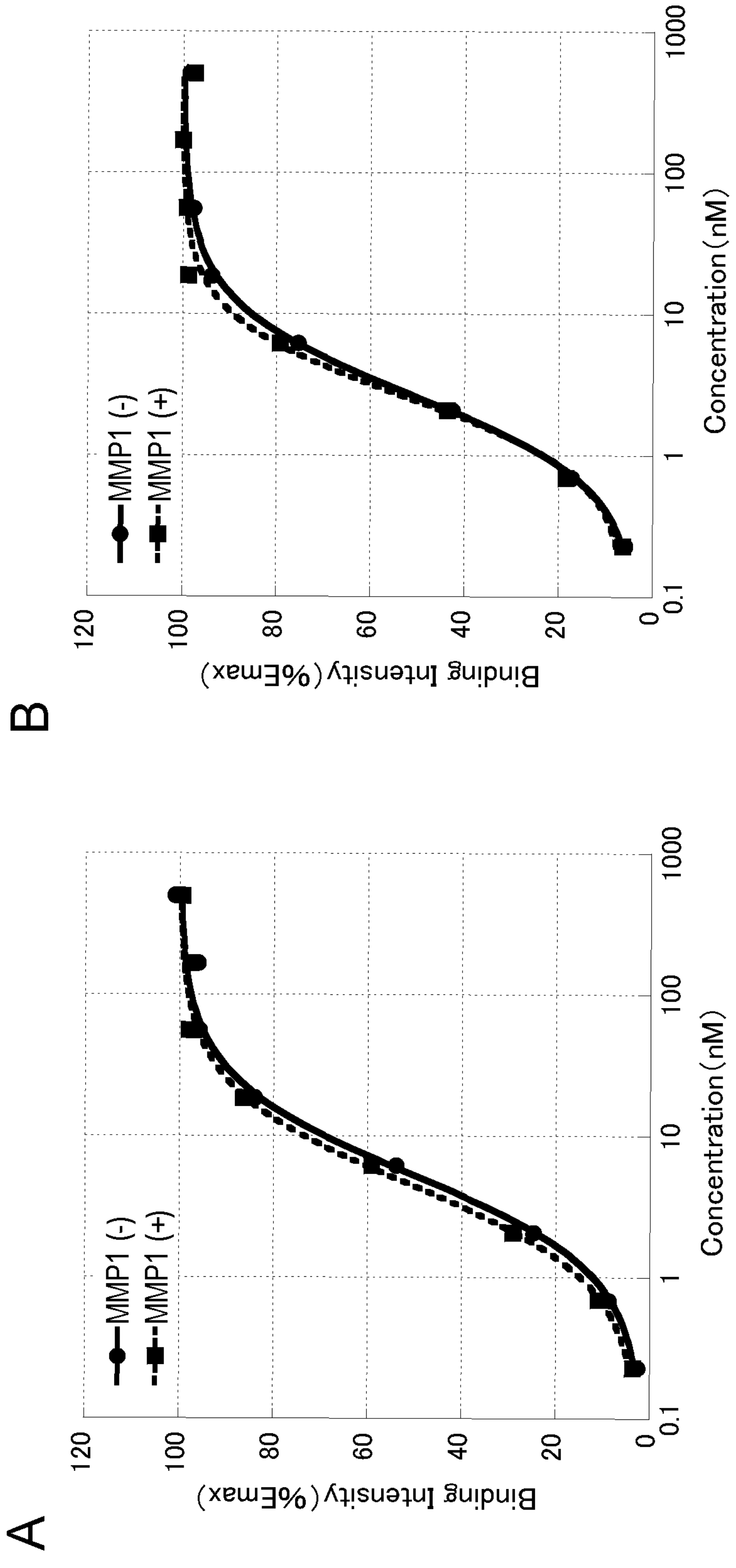
A
B
MMP1 (-)
MMP1 (+)
Binding Intensity (%Emax)
Concentration (nM)
0
20
40
60
80
100
120
0.1
1
10
100
1000

Fig. 4-2

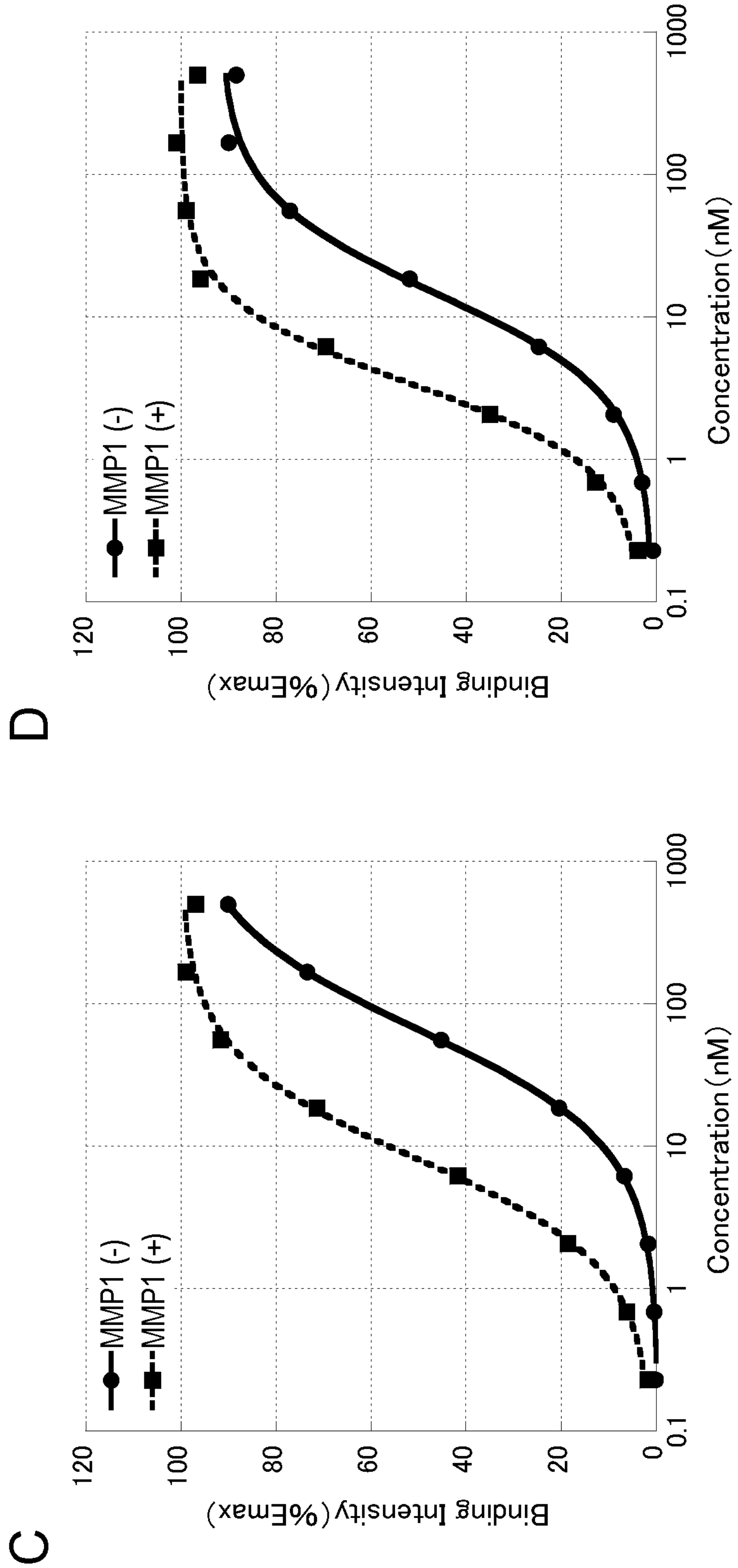
C
D
MMP1 (-)
MMP1 (+)
Binding Intensity (%Emax)
Concentration (nM)
0
20
40
60
80
100
120
0.1
1
10
100
1000

Fig. 5-1

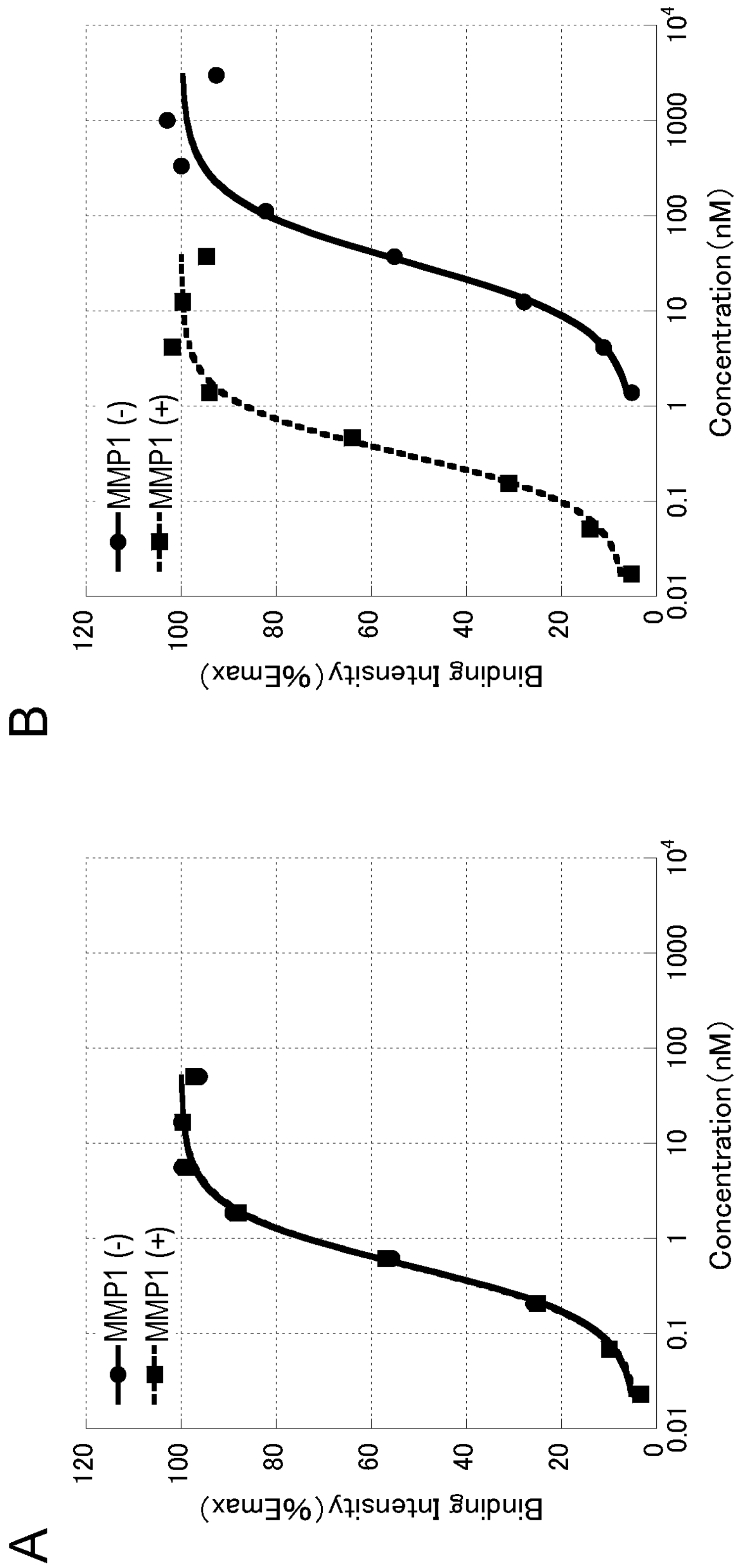
A
B
MMP1 (-)
MMP1 (+)
Binding Intensity (%Emax)
Concentration (nM)
0
20
40
60
80
100
120
0.01
0.1
1
10
100
1000
$10^4$

Fig. 5-2

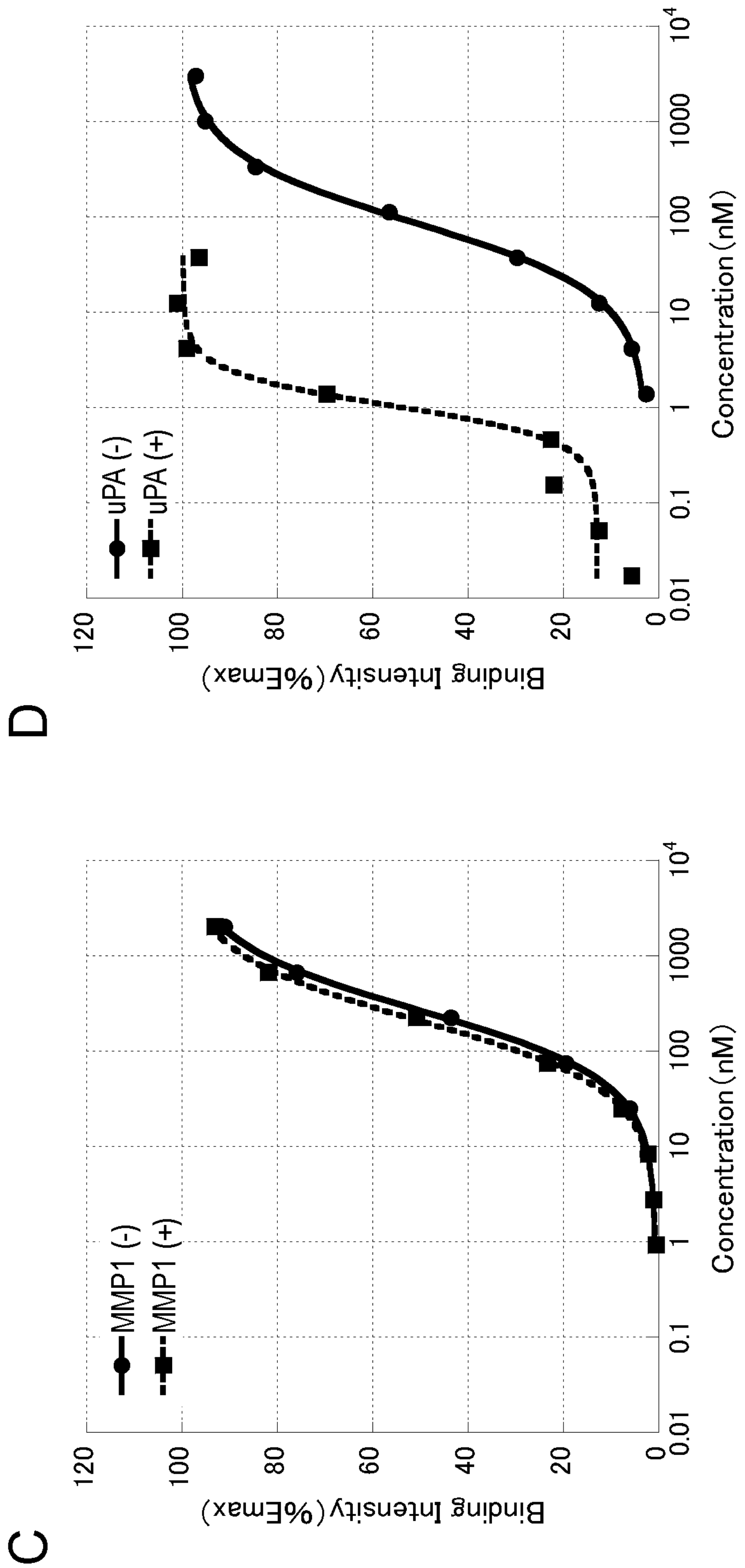
C
D
MMP1 (-)
MMP1 (+)
uPA (-)
uPA (+)
Binding Intensity (%Emax)
Concentration (nM)
0
20
40
60
80
100
120
0.01
0.1
1
10
100
1000
$10^4$

Fig. 6

A

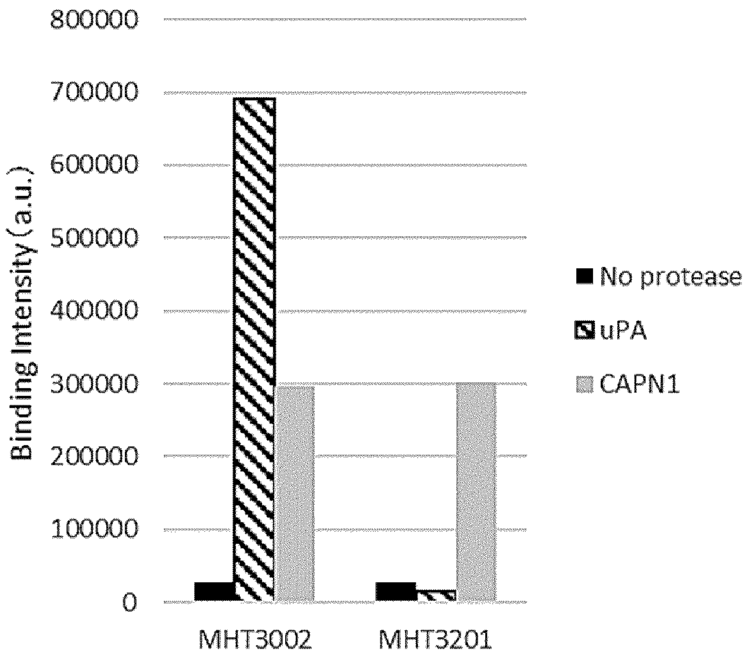
800000
700000
600000
500000
400000
300000
200000
100000
0
Binding Intensity (a.u.)
MHT3002
MHT3201
No protease
uPA
CAPN1

B

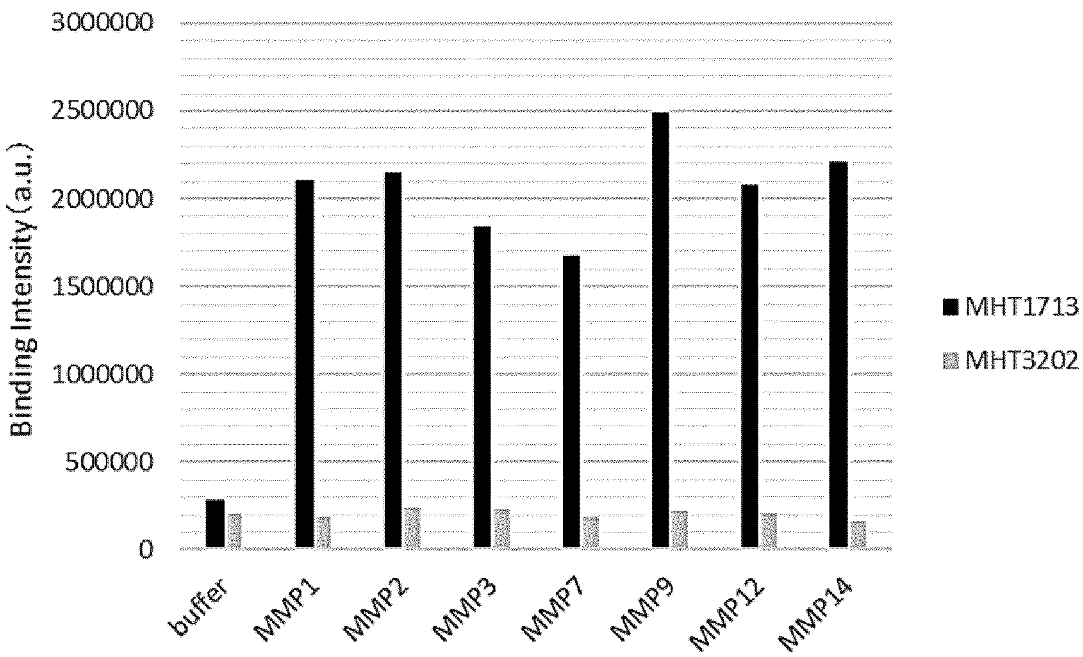
3000000
2500000
2000000
1500000
1000000
500000
0
Binding Intensity (a.u.)
buffer
MMP1
MMP2
MMP3
MMP7
MMP9
MMP12
MMP14
MHT1713
MHT3202

Fig. 7-1

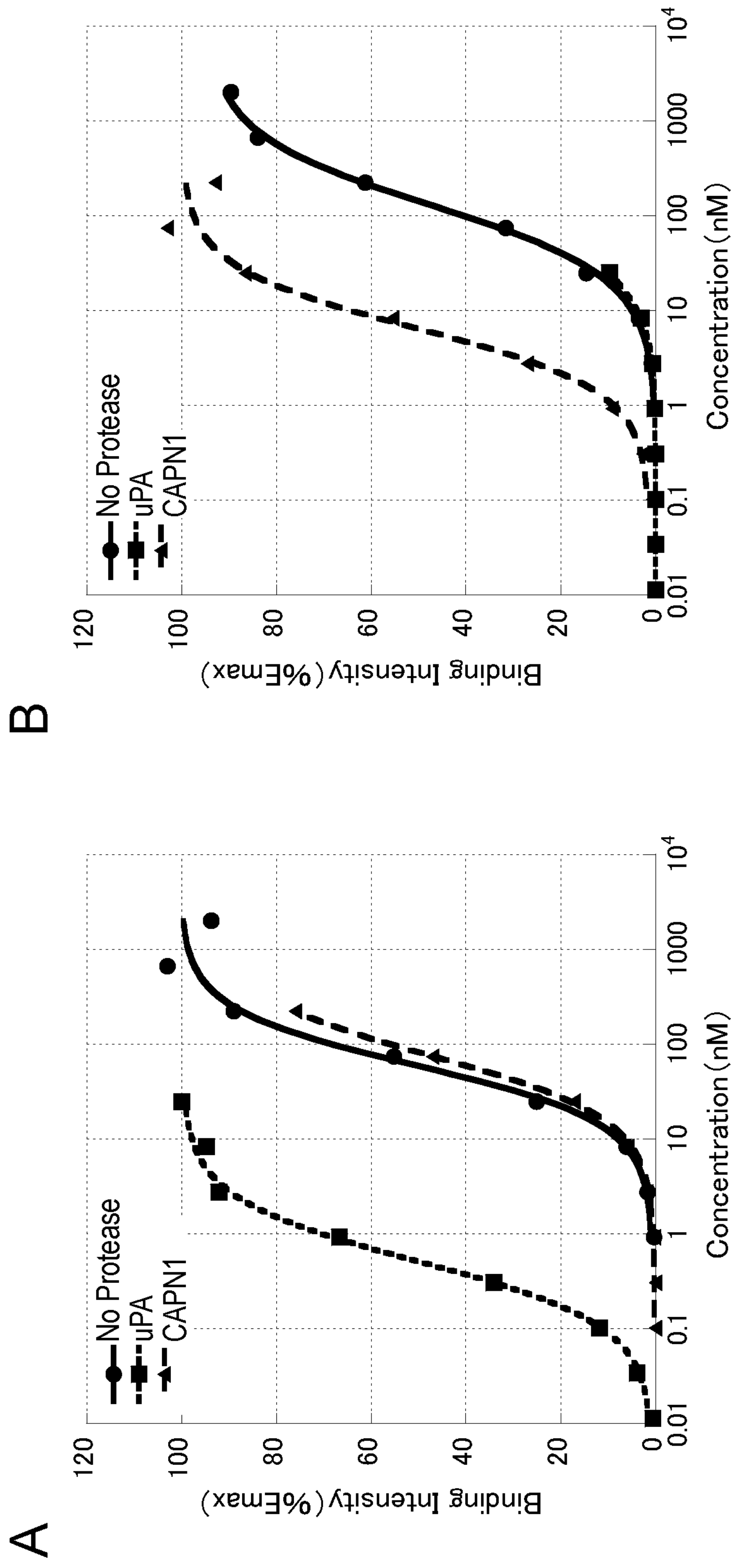
A
B
No Protease
uPA
CAPN1
Binding Intensity (%Emax)
Concentration (nM)
0
20
40
60
80
100
120
0.01
0.1
1
10
100
1000
10^4

Fig. 7-2

C

D

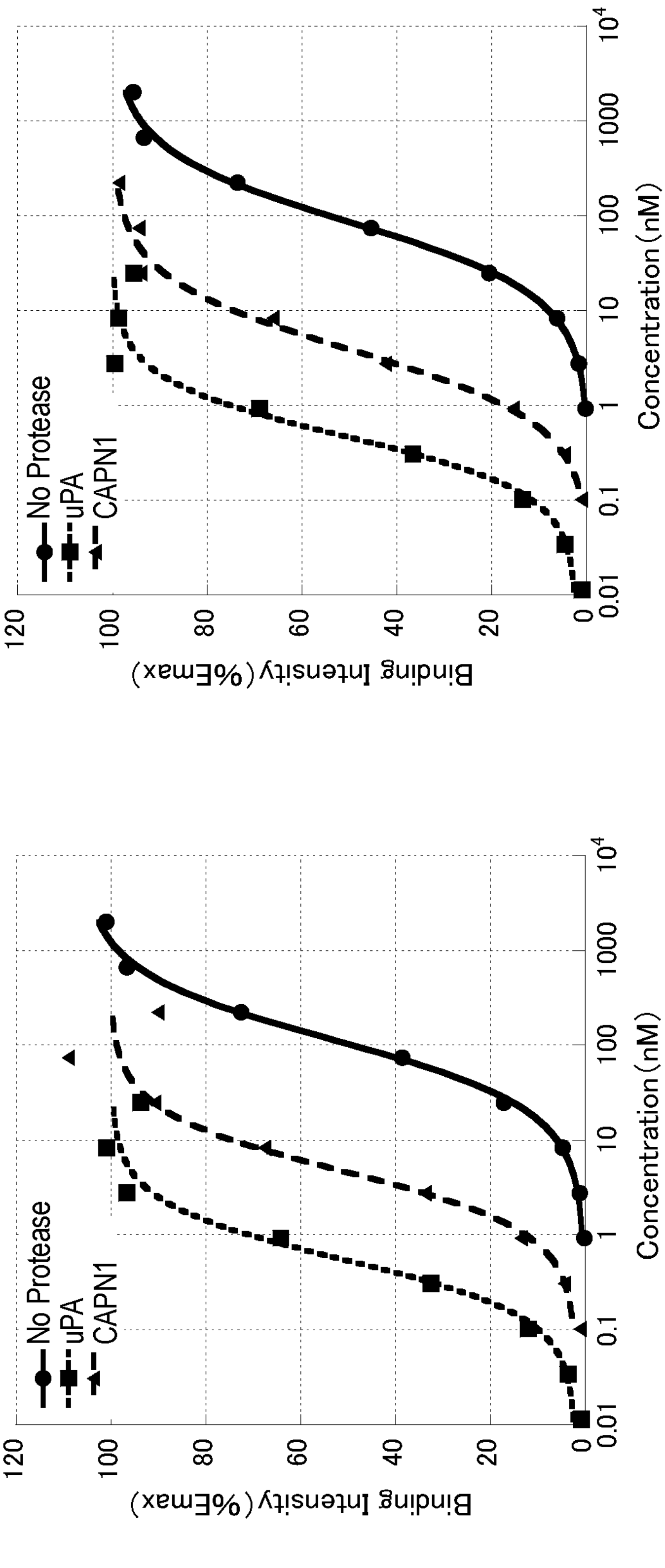
No Protease
uPA
CAPN1
Binding Intensity (%Emax)
120
100
80
60
40
20
0
0.01
0.1
1
10
100
1000
$10^4$
Concentration (nM)

Fig. 8

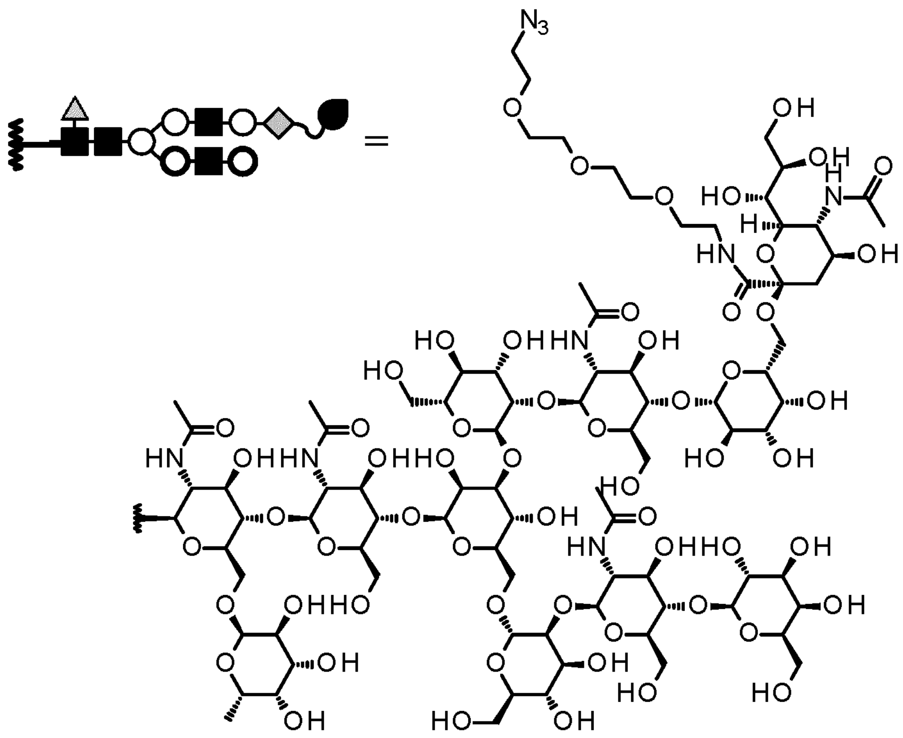

Structural formula of
N297 glycan

# Fig. 9

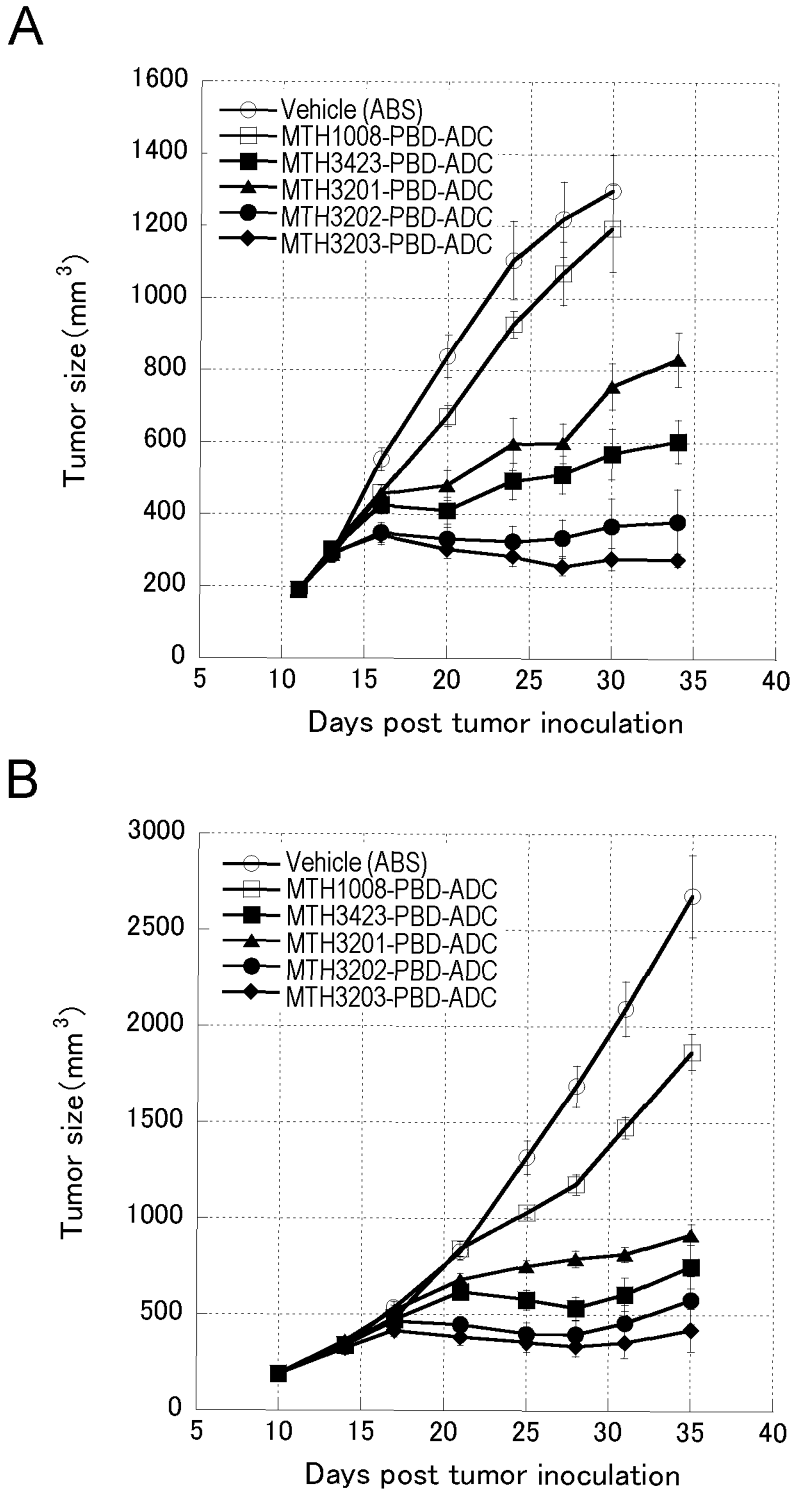
A
Vehicle (ABS)
MTH1008-PBD-ADC
MTH3423-PBD-ADC
MTH3201-PBD-ADC
MTH3202-PBD-ADC
MTH3203-PBD-ADC
Tumor size (mm$^3$)
0
200
400
600
800
1000
1200
1400
1600
5
10
15
20
25
30
35
40
Days post tumor inoculation
B
Vehicle (ABS)
MTH1008-PBD-ADC
MTH3423-PBD-ADC
MTH3201-PBD-ADC
MTH3202-PBD-ADC
MTH3203-PBD-ADC
Tumor size (mm$^3$)
0
500
1000
1500
2000
2500
3000
5
10
15
20
25
30
35
40
Days post tumor inoculation

Fig. 10

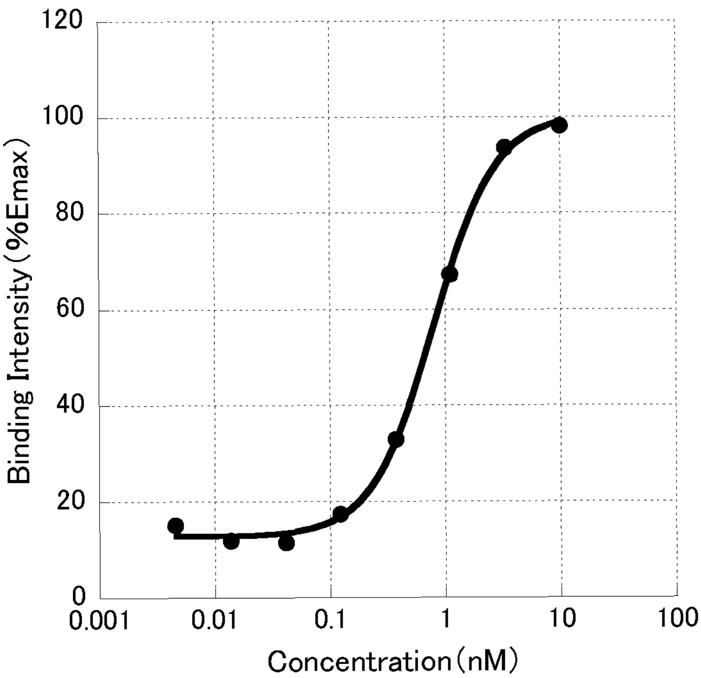
120
100
80
60
40
20
0
0.001
0.01
0.1
1
10
100
Binding Intensity (%Emax)
Concentration (nM)

Fig. 11

A

| Linear 15 mer lib | ZPZP lib |
|---|---|
| N-XXXXXXXXXXXXXXX-C | N-XXXXXZPZPXXXXGS-C |

X : 20 amino acids based on NNS codon
Z : 6 amino acids (H, Y, W, R, Q, C) based on YRK codon

B

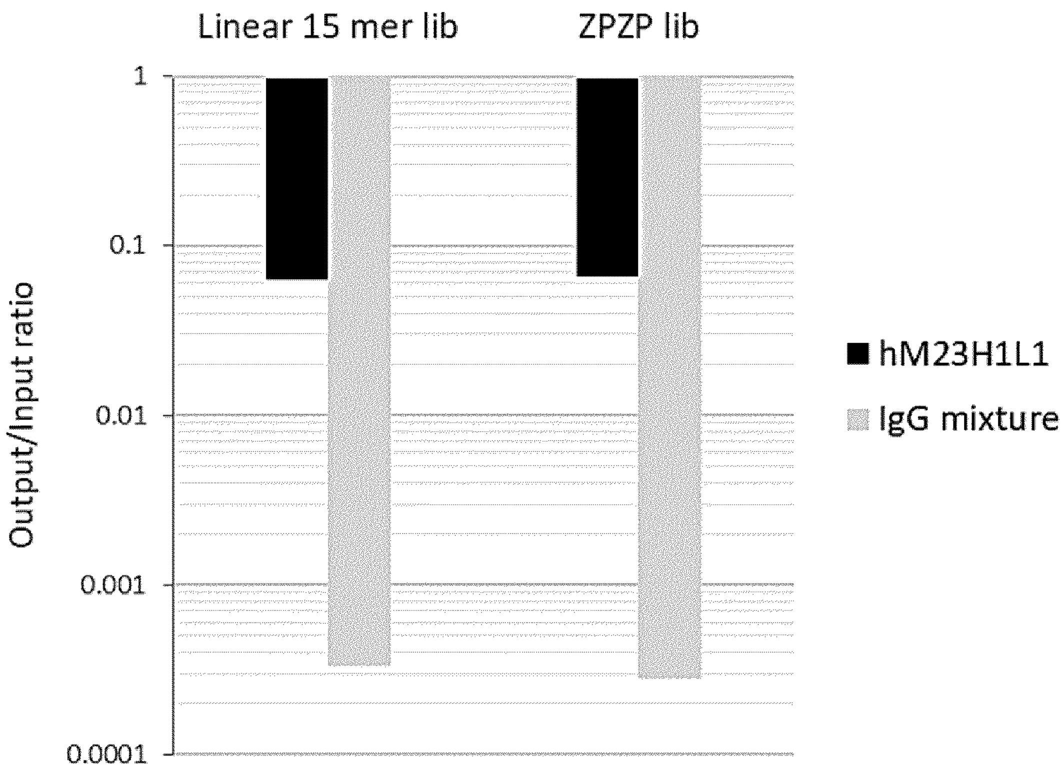

Fig. 12-1

A

B

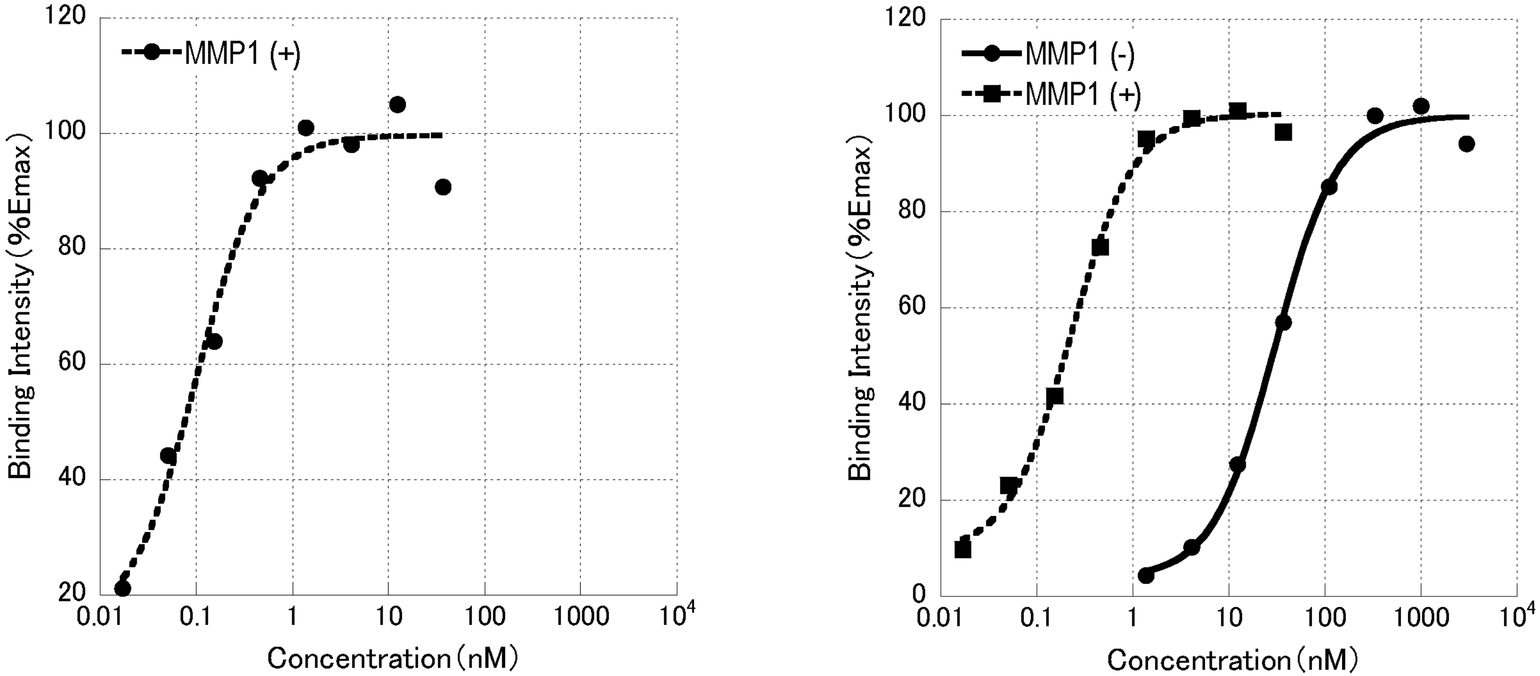
MMP1 (+)
Binding Intensity (%Emax)
Concentration (nM)
120
100
80
60
40
20
0.01
0.1
1
10
100
1000
$10^4$
MMP1 (-)
MMP1 (+)
Binding Intensity (%Emax)
Concentration (nM)
120
100
80
60
40
20
0
0.01
0.1
1
10
100
1000
$10^4$

Fig. 12-2

C

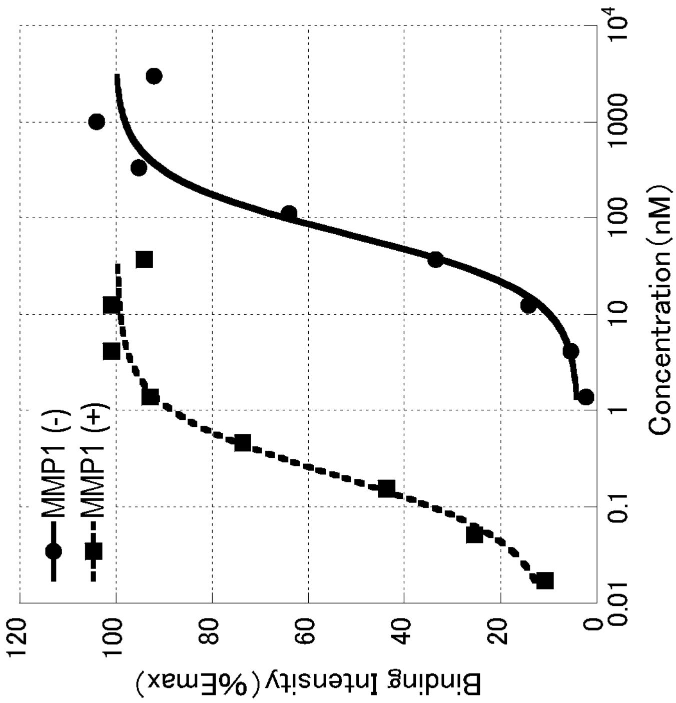

MMP1 (-)
MMP1 (+)
Binding Intensity (%Emax)
Concentration (nM)
0
20
40
60
80
100
120
0.01
0.1
1
10
100
1000
$10^4$

Fig. 13

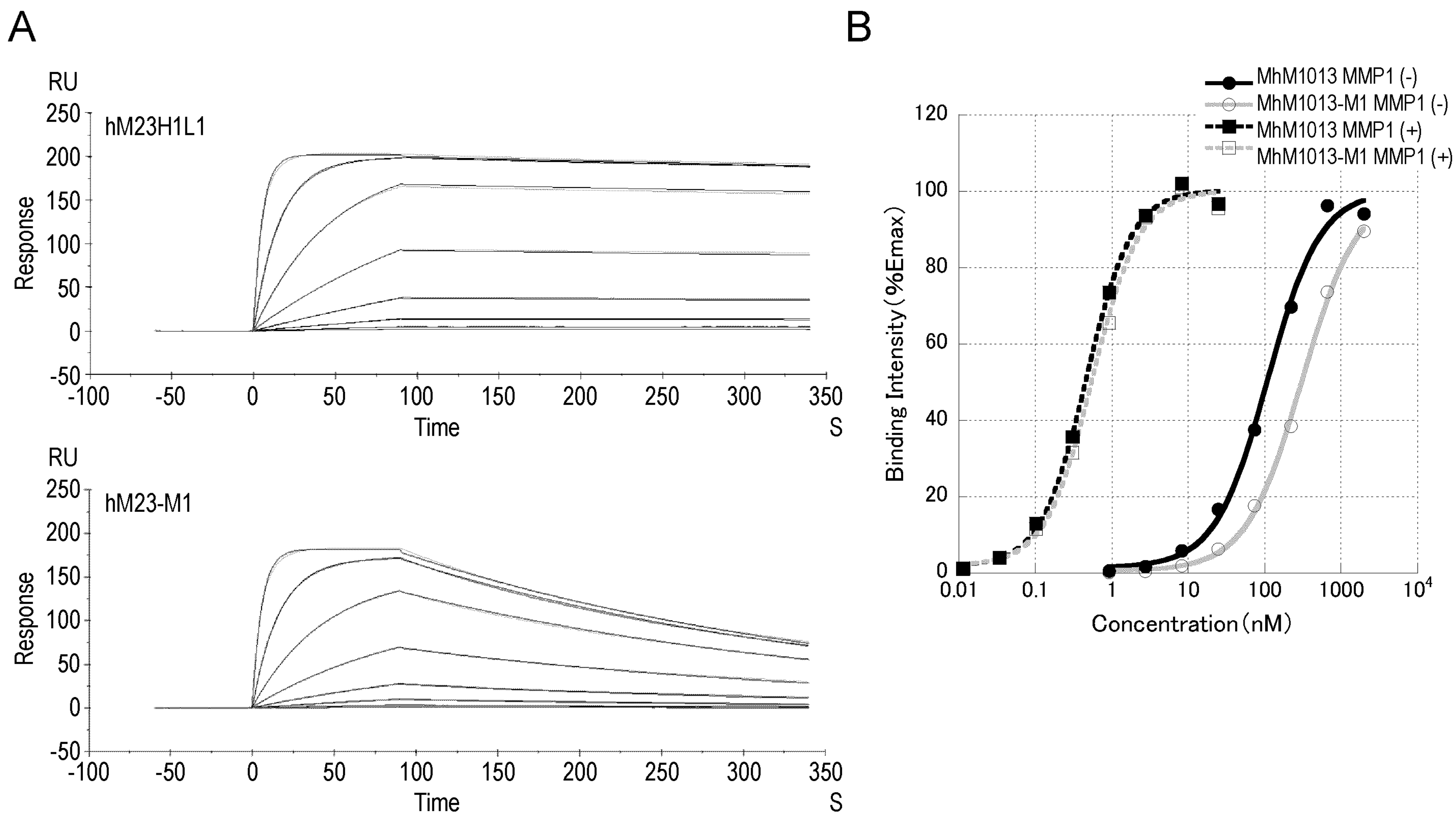
A
RU
Response
hM23H1L1
Time
S
hM23-M1
B
MhM1013 MMP1 (-)
MhM1013-M1 MMP1 (-)
MhM1013 MMP1 (+)
MhM1013-M1 MMP1 (+)
Binding Intensity (%Emax)
Concentration (nM)

Fig. 14-1

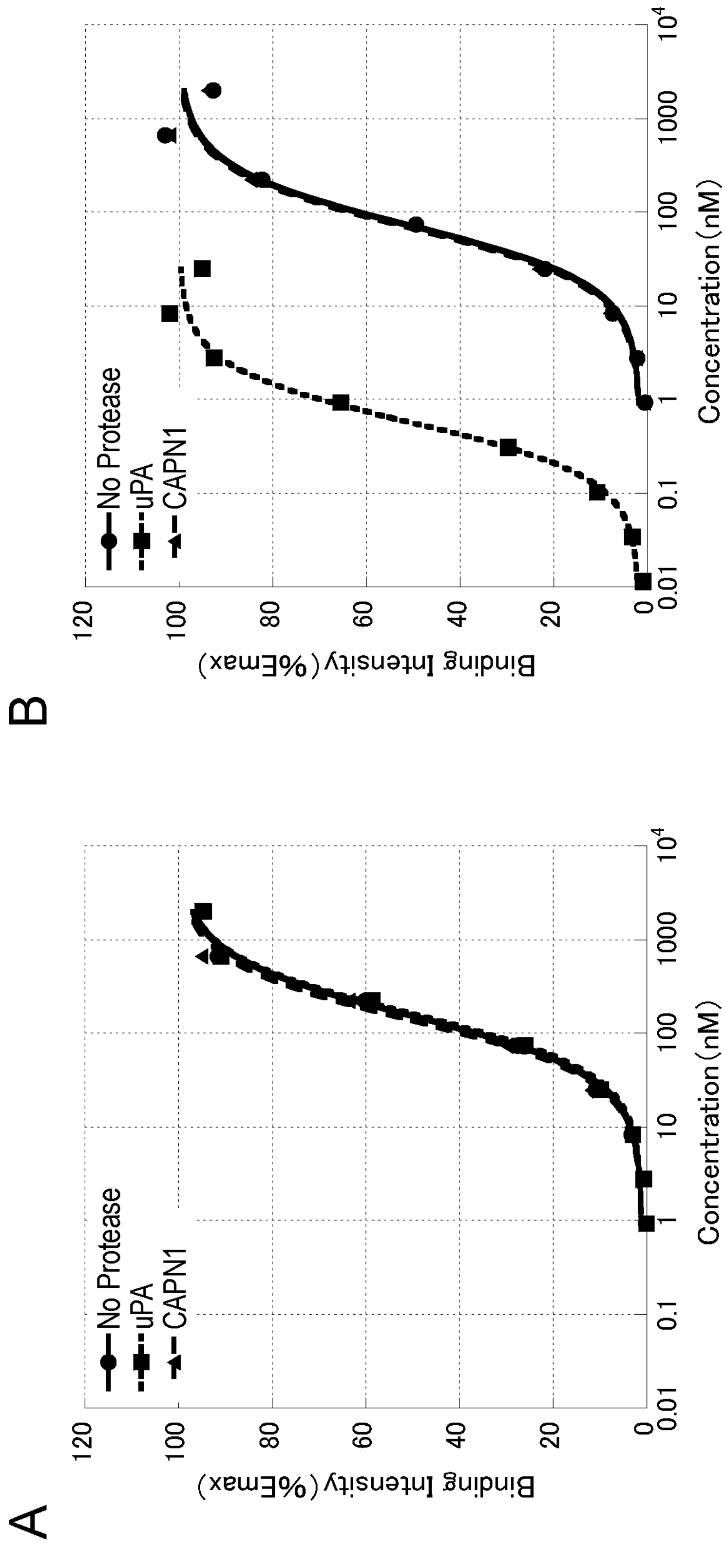
A
B
No Protease
uPA
CAPN1
Binding Intensity (%Emax)
Concentration (nM)
0
20
40
60
80
100
120
0.01
0.1
1
10
100
1000
$10^4$

Fig. 14-2

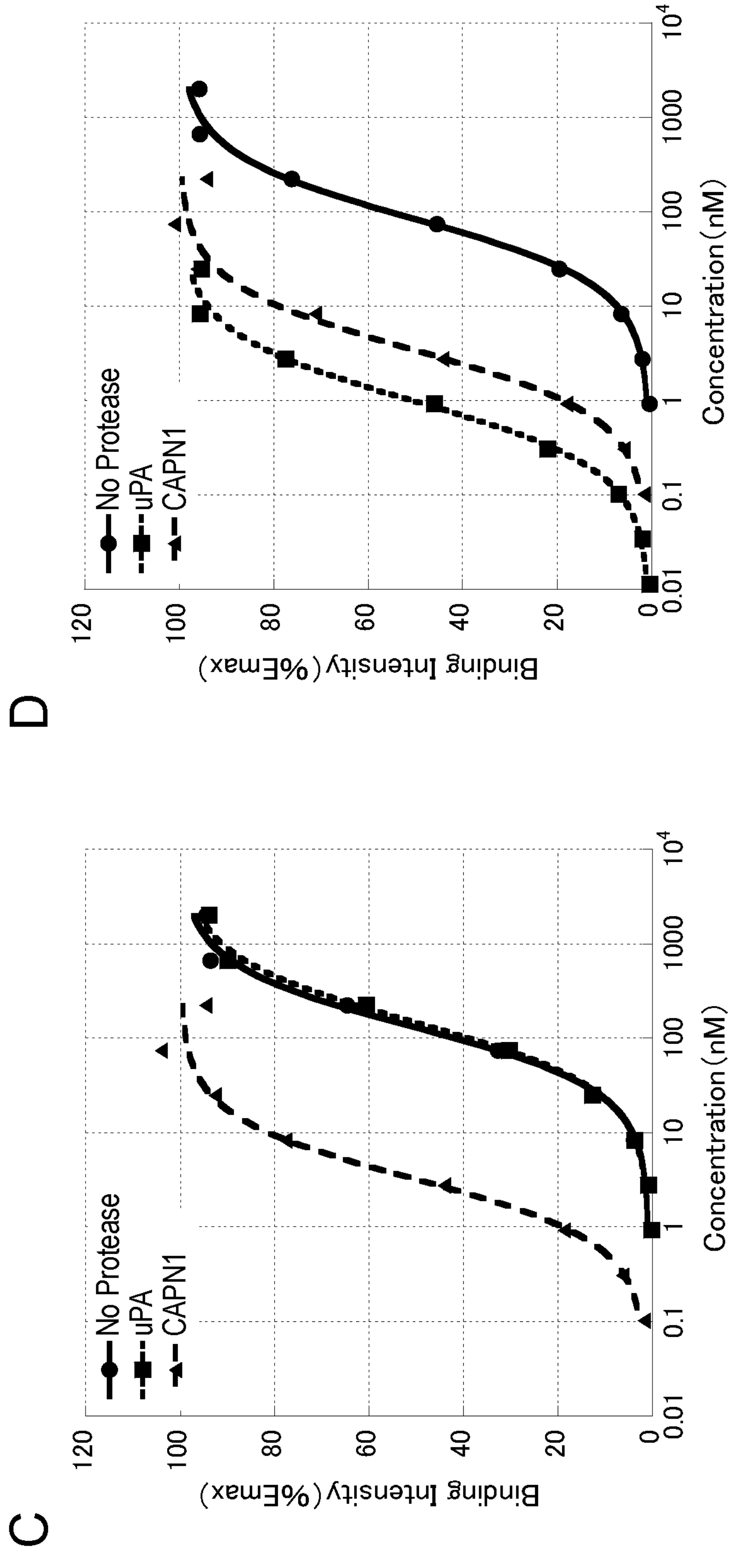

C
D
No Protease
uPA
CAPN1
Binding Intensity (%Emax)
Concentration (nM)
0
20
40
60
80
100
120
0.01
0.1
1
10
100
1000
$10^4$

Fig. 14-3

E

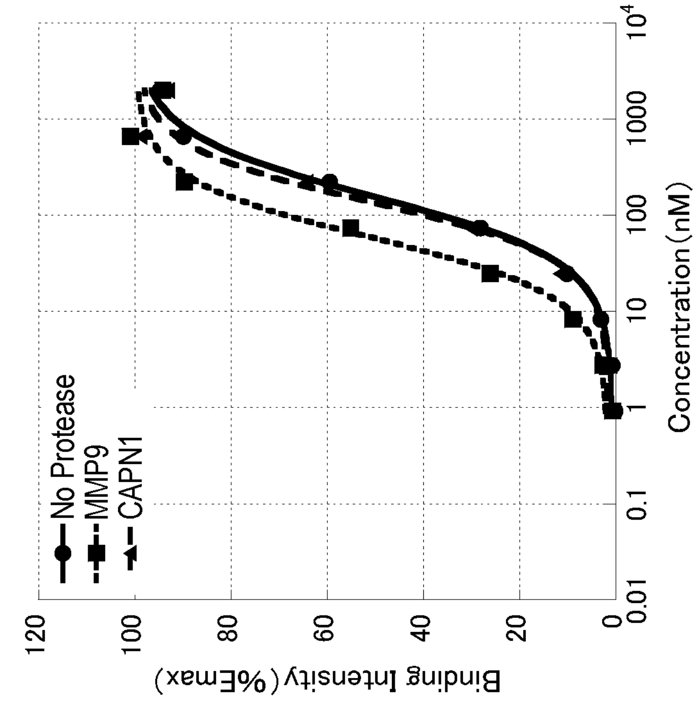
No Protease
MMP9
CAPN1
Binding Intensity (%Emax)
120
100
80
60
40
20
0
0.01
0.1
1
10
100
1000
$10^4$
Concentration (nM)

F

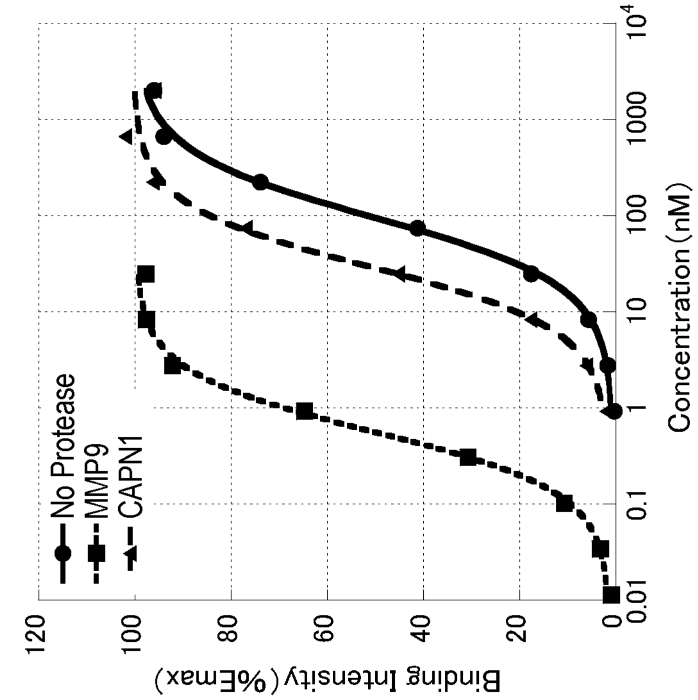
No Protease
MMP9
CAPN1
Binding Intensity (%Emax)
120
100
80
60
40
20
0
0.01
0.1
1
10
100
1000
$10^4$
Concentration (nM)

Fig. 14-4

G

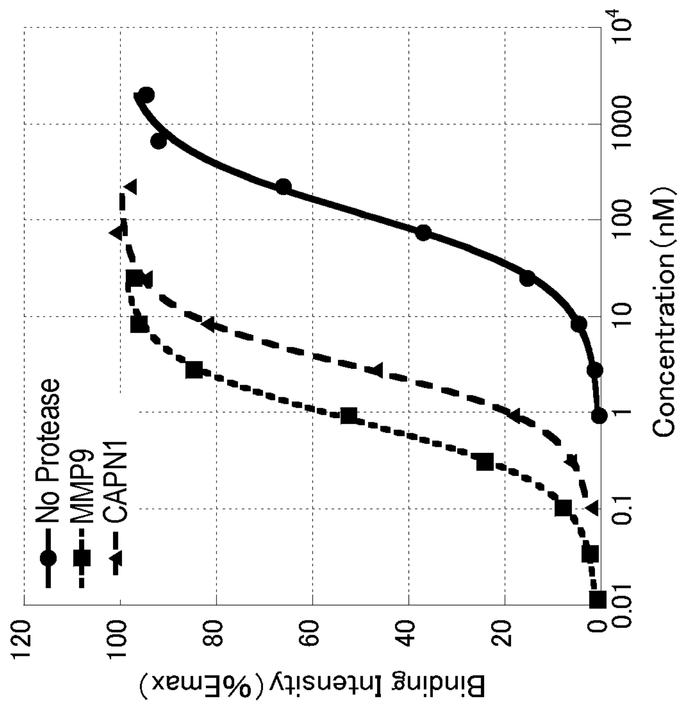
No Protease
MMP9
CAPN1
Binding Intensity (%Emax)
120
100
80
60
40
20
0
0.01
0.1
1
10
100
1000
10⁴
Concentration (nM)

Fig. 15

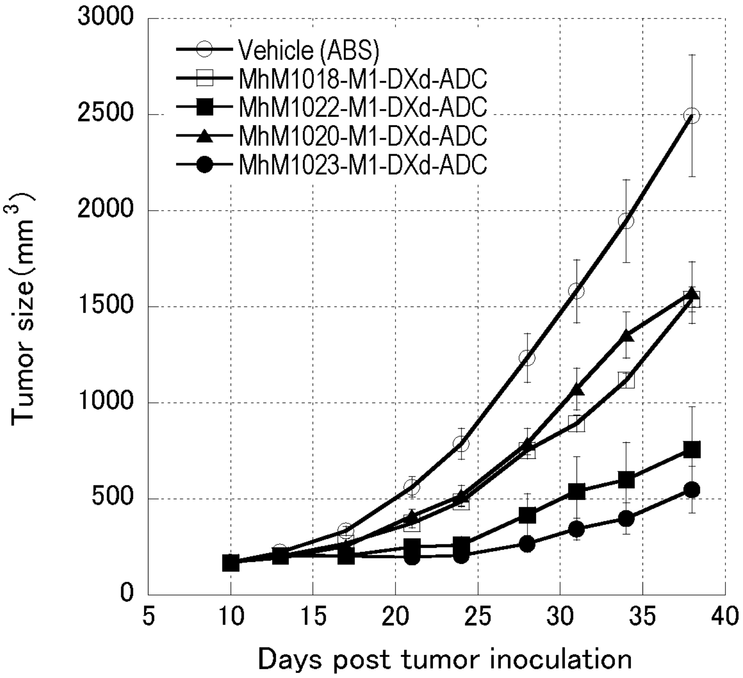
Vehicle (ABS)
MhM1018-M1-DXd-ADC
MhM1022-M1-DXd-ADC
MhM1020-M1-DXd-ADC
MhM1023-M1-DXd-ADC
Tumor size (mm3)
Days post tumor inoculation
0
500
1000
1500
2000
2500
3000
5
10
15
20
25
30
35
40

Fig. 16

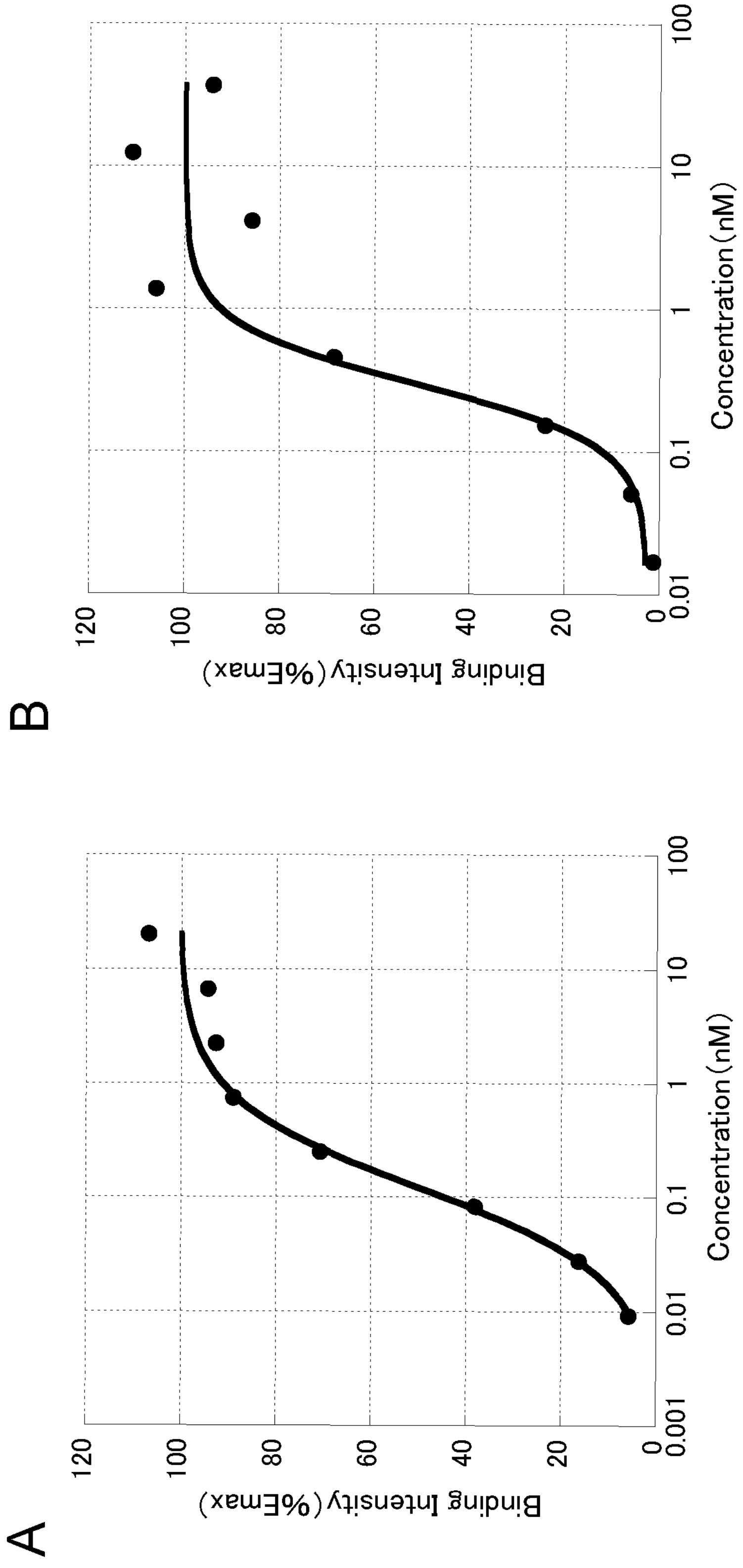
A
Binding Intensity (%Emax)
0
20
40
60
80
100
120
Concentration (nM)
0.001
0.01
0.1
1
10
100
B
Binding Intensity (%Emax)
0
20
40
60
80
100
120
Concentration (nM)
0.01
0.1
1
10
100

Fig. 17

A

B

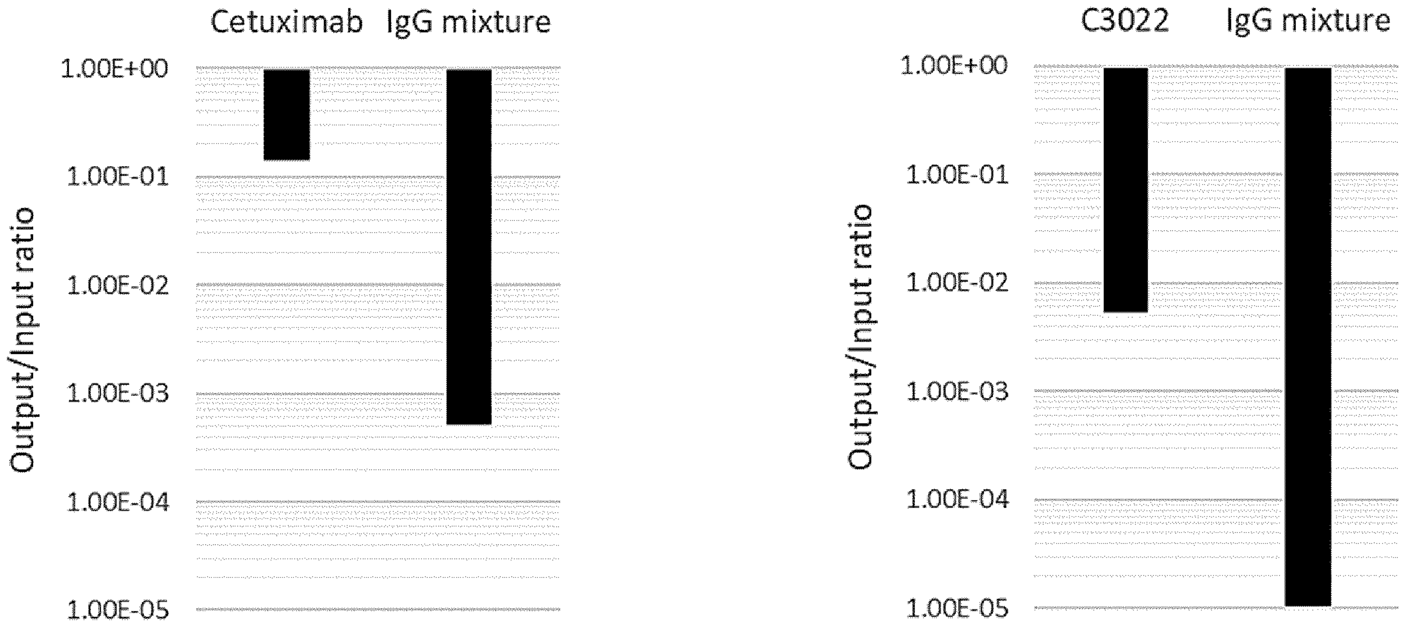
Cetuximab
IgG mixture
Output/Input ratio
1.00E+00
1.00E-01
1.00E-02
1.00E-03
1.00E-04
1.00E-05
C3022
IgG mixture
Output/Input ratio
1.00E+00
1.00E-01
1.00E-02
1.00E-03
1.00E-04
1.00E-05

Fig. 18

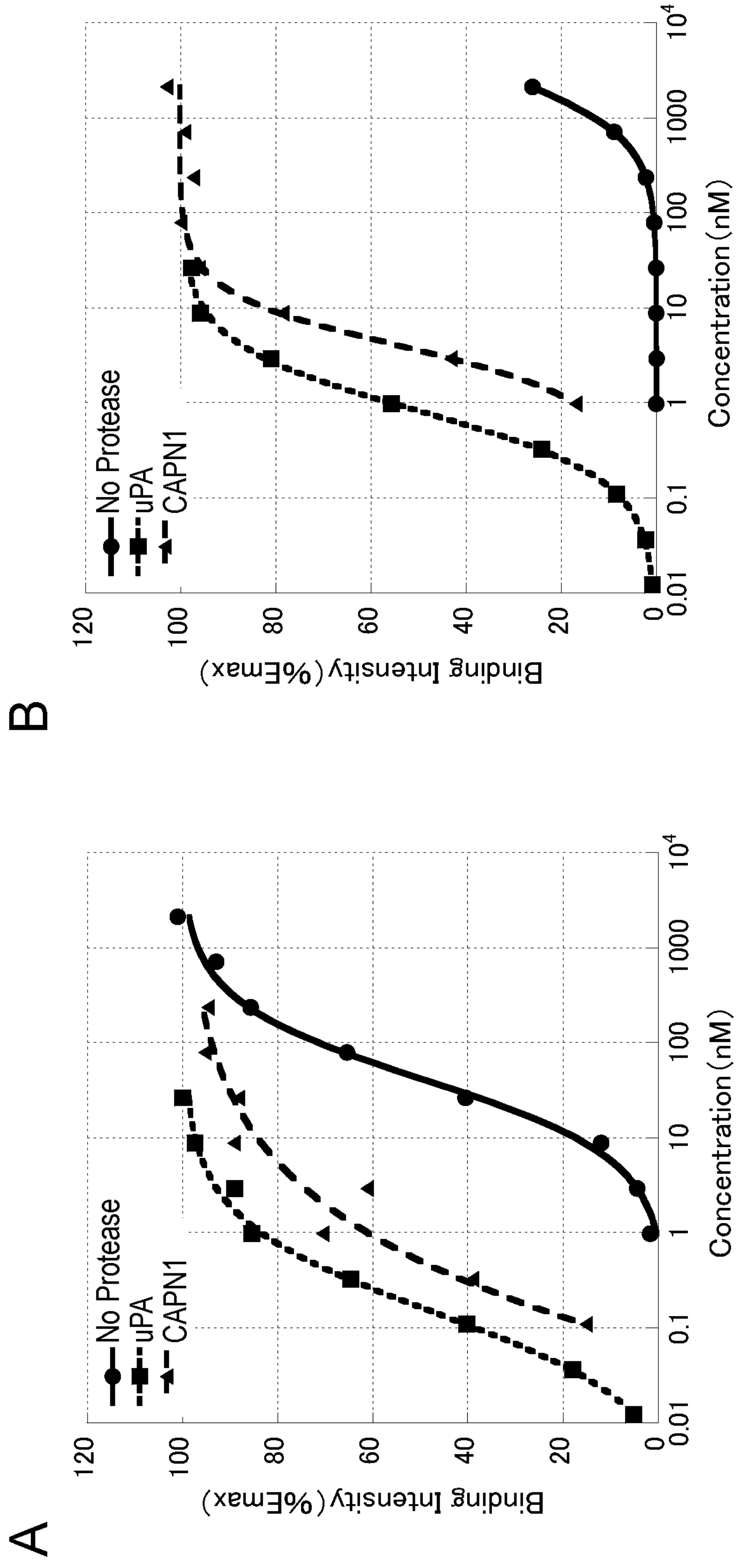
A
B
No Protease
uPA
CAPN1
Binding Intensity (%Emax)
Concentration (nM)
0
20
40
60
80
100
120
0.01
0.1
1
10
100
1000
$10^4$

Fig. 19

A

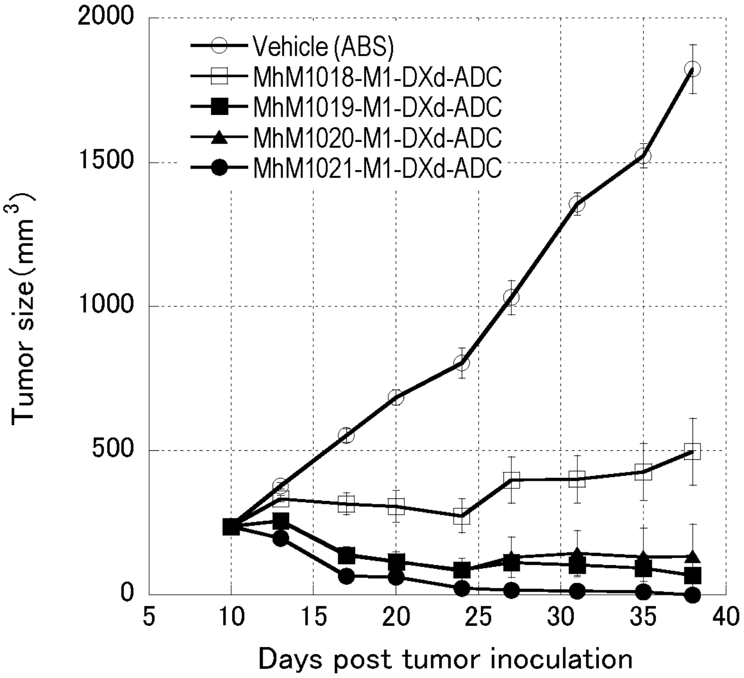
Vehicle (ABS)
MhM1018-M1-DXd-ADC
MhM1019-M1-DXd-ADC
MhM1020-M1-DXd-ADC
MhM1021-M1-DXd-ADC
Tumor size (mm³)
2000
1500
1000
500
0
5
10
15
20
25
30
35
40
Days post tumor inoculation

B

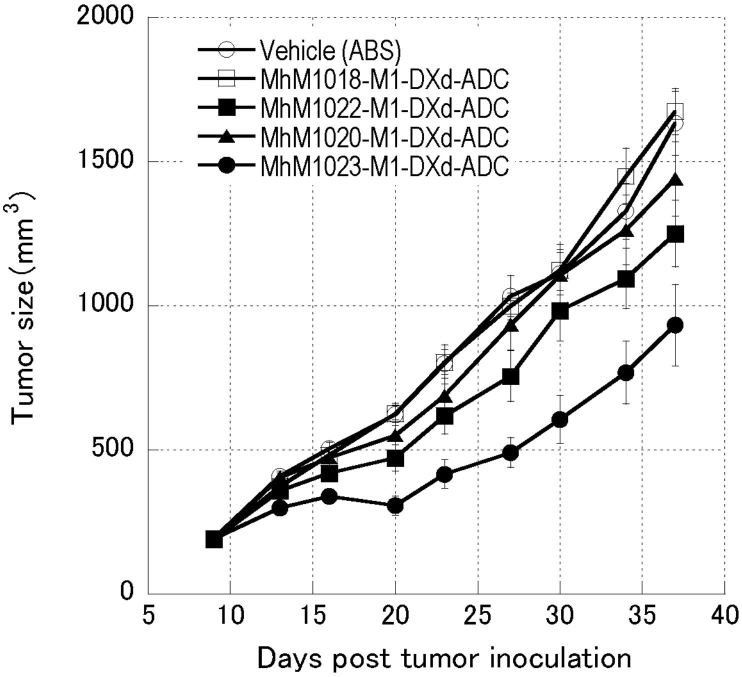
Vehicle (ABS)
MhM1018-M1-DXd-ADC
MhM1022-M1-DXd-ADC
MhM1020-M1-DXd-ADC
MhM1023-M1-DXd-ADC
Tumor size (mm³)
2000
1500
1000
500
0
5
10
15
20
25
30
35
40
Days post tumor inoculation

Fig. 20

A

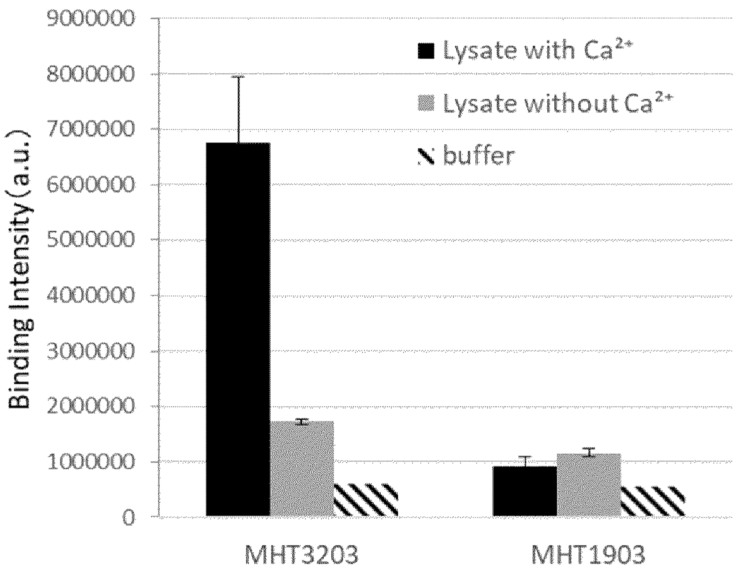
9000000
8000000
7000000
6000000
5000000
4000000
3000000
2000000
1000000
0
Binding Intensity (a.u.)
Lysate with Ca2+
Lysate without Ca2+
buffer
MHT3203
MHT1903

B

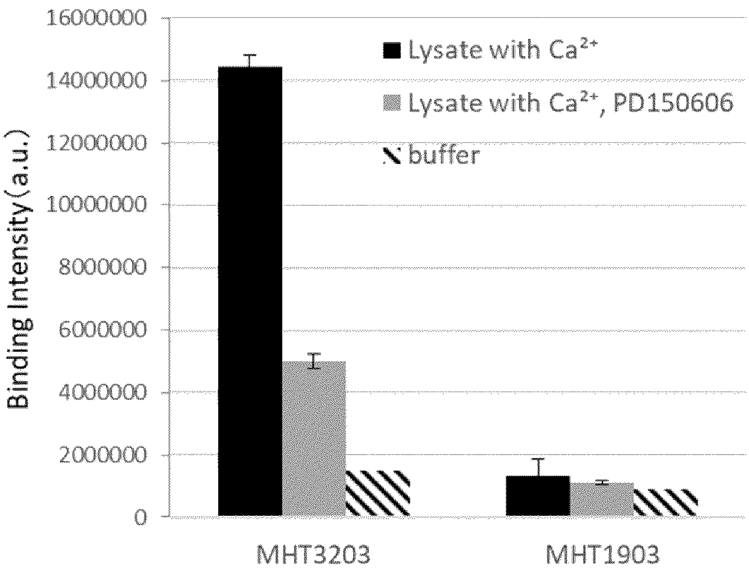
16000000
14000000
12000000
10000000
8000000
6000000
4000000
2000000
0
Binding Intensity (a.u.)
Lysate with Ca2+
Lysate with Ca2+, PD150606
buffer
MHT3203
MHT1903

Fig. 21

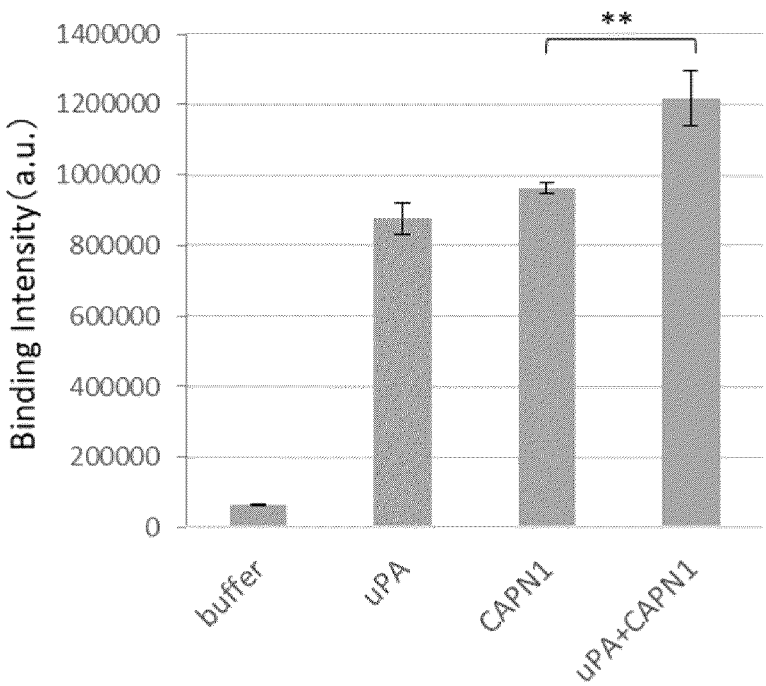

**
1400000
1200000
1000000
800000
600000
400000
200000
0
Binding Intensity（a.u.）
buffer
uPA
CAPN1
uPA+CAPN1

Fig. 22

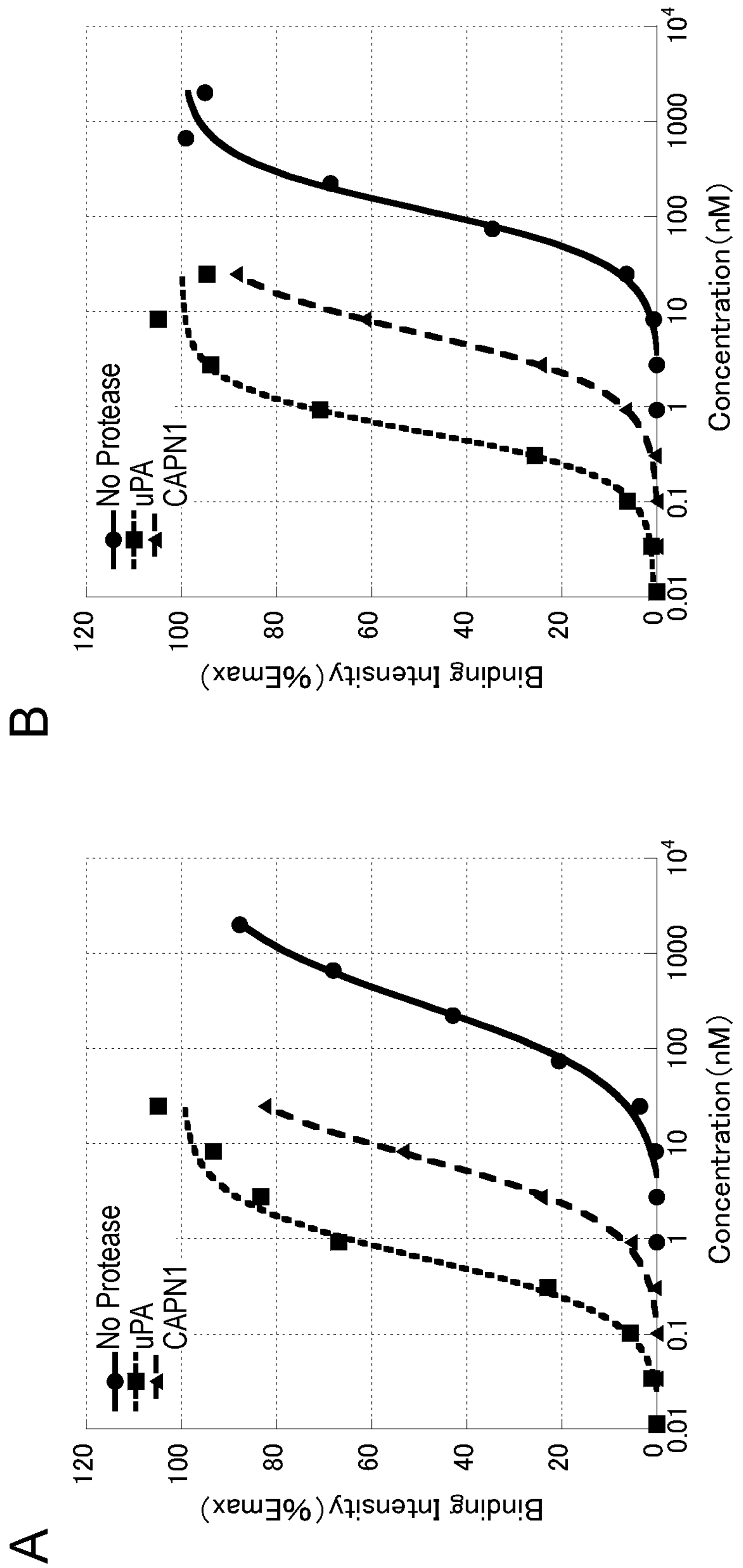
A
B
No Protease
uPA
CAPN1
Binding Intensity (%Emax)
Concentration (nM)
0
20
40
60
80
100
120
0.01
0.1
1
10
100
1000
$10^4$

Fig. 23

A

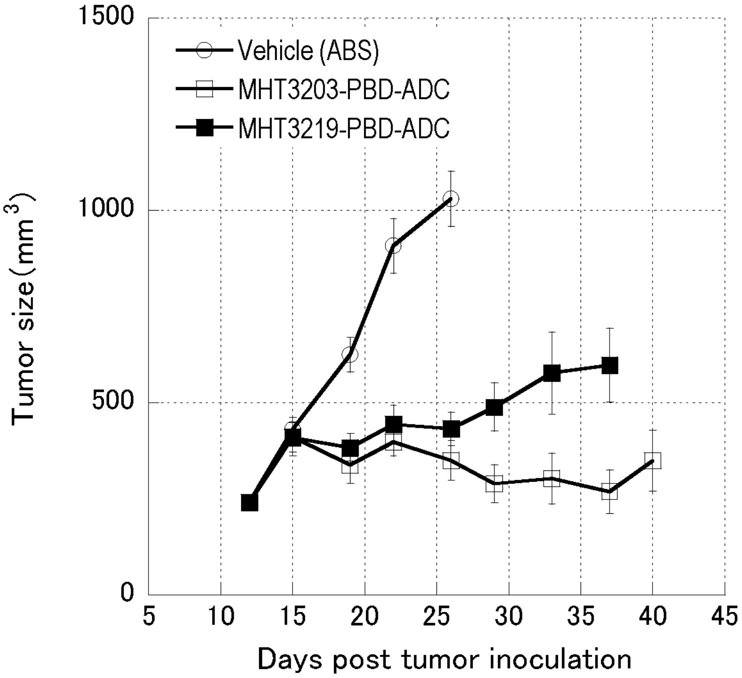
Vehicle (ABS)
MHT3203-PBD-ADC
MHT3219-PBD-ADC
Tumor size (mm$^3$)
1500
1000
500
0
5
10
15
20
25
30
35
40
45
Days post tumor inoculation

B

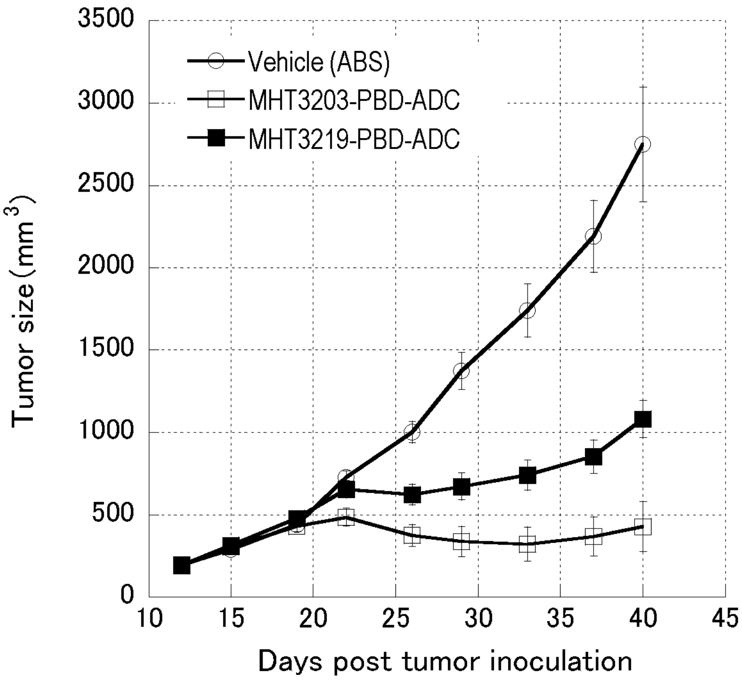
Vehicle (ABS)
MHT3203-PBD-ADC
MHT3219-PBD-ADC
Tumor size (mm$^3$)
3500
3000
2500
2000
1500
1000
500
0
10
15
20
25
30
35
40
45
Days post tumor inoculation

Fig. 24

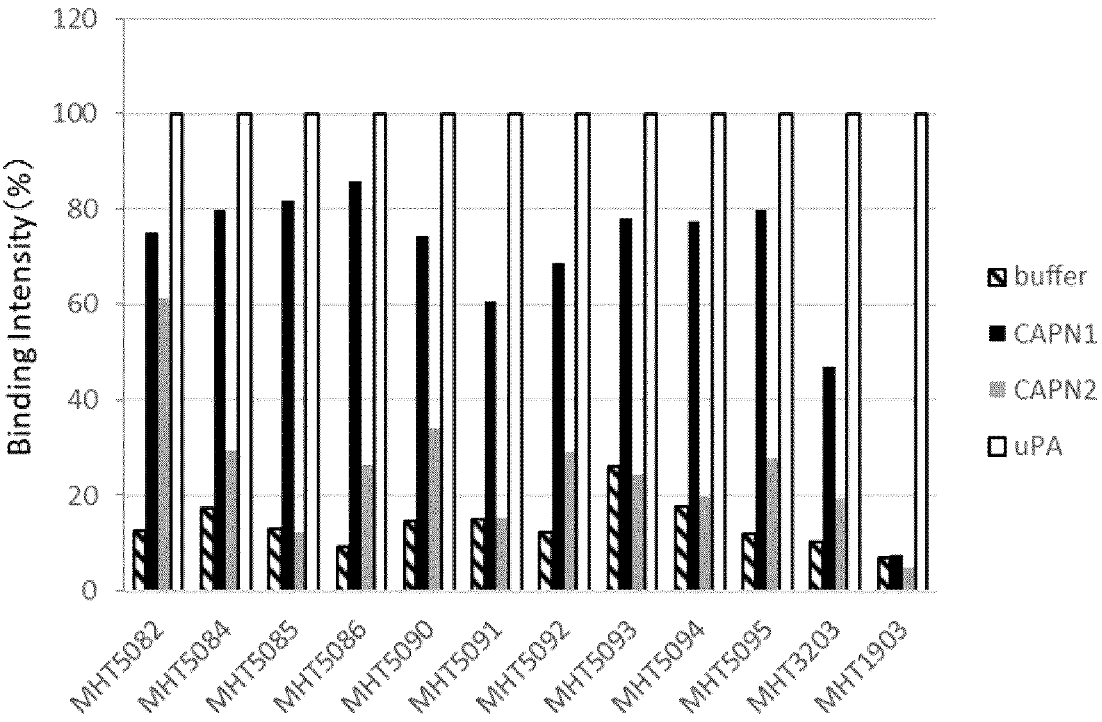
120
100
80
60
40
20
0
Binding Intensity (%)
MHT5082
MHT5084
MHT5085
MHT5086
MHT5090
MHT5091
MHT5092
MHT5093
MHT5094
MHT5095
MHT3203
MHT1903
buffer
CAPN1
CAPN2
uPA

# Fig. 25

A

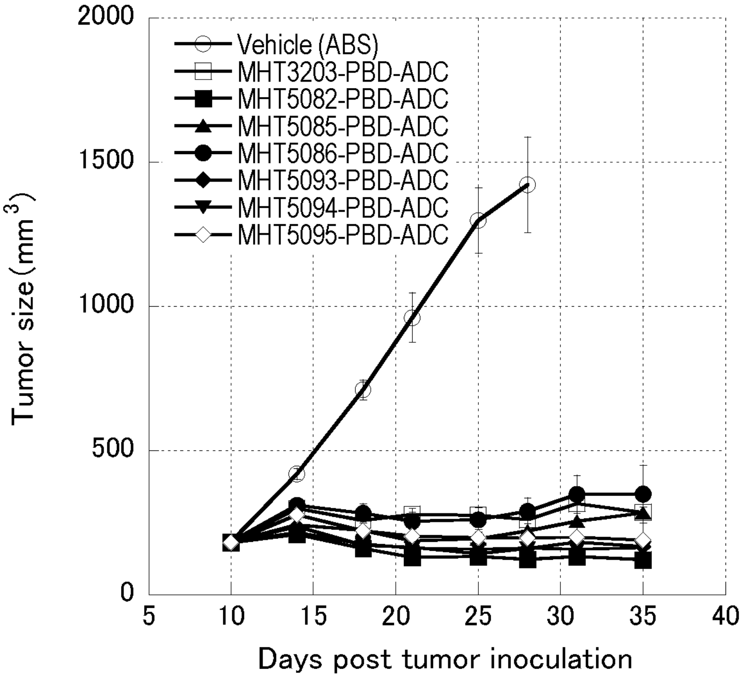
Vehicle (ABS)
MHT3203-PBD-ADC
MHT5082-PBD-ADC
MHT5085-PBD-ADC
MHT5086-PBD-ADC
MHT5093-PBD-ADC
MHT5094-PBD-ADC
MHT5095-PBD-ADC
Tumor size (mm3)
Days post tumor inoculation
0
500
1000
1500
2000
5
10
15
20
25
30
35
40

B

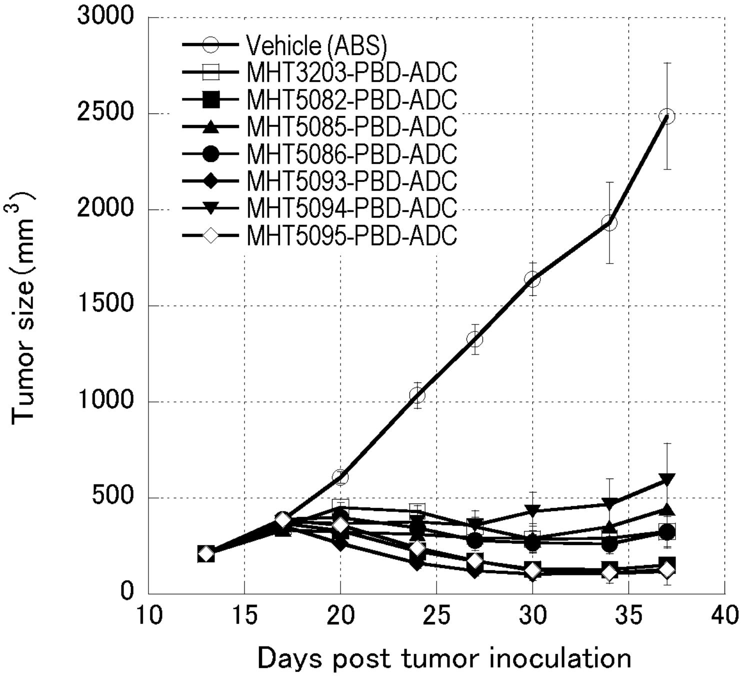
Vehicle (ABS)
MHT3203-PBD-ADC
MHT5082-PBD-ADC
MHT5085-PBD-ADC
MHT5086-PBD-ADC
MHT5093-PBD-ADC
MHT5094-PBD-ADC
MHT5095-PBD-ADC
Tumor size (mm3)
Days post tumor inoculation
0
500
1000
1500
2000
2500
3000
10
15
20
25
30
35
40

# Fig. 26

Amino acid sequence of full-length HT1-11 H chain
MKHLWFFLLLVAAPRWVLSQVQLVQSGAEVKKPGASVKVSCKASGYTFTTAGMQWV
RQAPGQGLEWMGWINTHSGVPKYAEDFKGRVTISADTSTSTAYLQLSSLKSEDTAV
YYCARSGFGSSYWYFDVWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICN
VNHKPSNTKVDKRVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSC
SVMHEALHNHYTQKSLSLSPGK
(SEQ ID NO: 1) signal sequence (1-19), antibody heavy chain sequence (20-470)

Fig. 27

Amino acid sequence of full-length HT1-11 L chain
MVLQTQVFISLLLWISGAYGDIQMTQSPSSLSASVGDRVTITCKASQDVSTAVAWY
QQKPGKAPKLLIYSASYRYTGVPSRFSGSGSGTDFTLTISSLQPEDFAVYYCQQHY
ITPLTFGQGTKLEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSP
VTKSFNRGEC
(SEQ ID NO: 2) signal sequence (1-20), antibody light chain sequence (21-234)

Fig. 28

Amino acid sequence of full-length MHT1001
MKYLLPTAAAGLLLLAAQPAMAAMGYLALKHPWPGLAENSGSGGGGSVLVPMAMMA
SGGSDVNSQVQLVQSGAEVKKPGASVKVSCKASGYTFTTAGMQWVRQAPGQGLEWM
GWINTHSGVPKYAEDFKGRVTISADTSTSTAYLQLSSLKSEDTAVYYCARSGFGSS
YWYFDVWGQGTLVTVSSGGGGSGGGGSGGGGSDIQMTQSPSSLSASVGDRVTITCK
ASQDVSTAVAWYQQKPGKAPKLLIYSASYRYTGVPSRFSGSGSGTDFTLTISSLQP
EDFAVYYCQQHYITPLTFGQGTKLEIKRGAPSGDYKDDDDKGAAAHHHHHH
(SEQ ID NO: 3) signal sequence (1-23), mimotope peptide (24-40), MMP cleavable linker (41-60), HT1-11-scFv-HL (65–308), FLAG-His tag (309-331)

# Fig. 29

Amino acid sequence of full-length MHT1002
MKYLLPTAAAGLLLLAAQPAMAAMGYLALKHPWPGLAENSGSGGGGSVLVPMAMMA
SGGSDVNSDIQMTQSPSSLSASVGDRVTITCKASQDVSTAVAWYQQKPGKAPKLLI
YSASYRYTGVPSRFSGSGSGTDFTLTISSLQPEDFAVYYCQQHYITPLTFGQGTKL
EIKRGGGGSGGGGSGGGGSQVQLVQSGAEVKKPGASVKVSCKASGYTFTTAGMQWV
RQAPGQGLEWMGWINTHSGVPKYAEDFKGRVTISADTSTSTAYLQLSSLKSEDTAV
YYCARSGFGSSYWYFDVWGQGTLVTVSSGAPSGDYKDDDDKGAAAHHHHHH
(SEQ ID NO: 4) signal sequence (1-23), mimotope peptide (24-40), MMP cleavable linker (41-60), HT1-11-scFv-LH (65–308), FLAG-His tag (309-331)

Fig. 30

Amino acid sequence of full-length HT1-11-scFv-HL
MKYLLPTAAAGLLLLAAQPAMAADVNSQVQLVQSGAEVKKPGASVKVSCKASGYTF
TTAGMQWVRQAPGQGLEWMGWINTHSGVPKYAEDFKGRVTISADTSTSTAYLQLSS
LKSEDTAVYYCARSGFGSSYWYFDVWGQGTLVTVSSGGGGSGGGGSGGGGSDIQMT
QSPSSLSASVGDRVTITCKASQDVSTAVAWYQQKPGKAPKLLIYSASYRYTGVPSR
FSGSGSGTDFTLTISSLQPEDFAVYYCQQHYITPLTFGQGTKLEIKRGAPSGDYKD
DDDKGAAAHHHHHH
(SEQ ID NO: 5) signal sequence (1-23), HT1-11-scFv-HL (28-271), FLAG-His tag (272-294)

# Fig. 31

Amino acid sequence of full-length HT1-11-scFv-LH
MKYLLPTAAAGLLLLAAQPAMAADVNSDIQMTQSPSSLSASVGDRVTITCKASQDV
STAVAWYQQKPGKAPKLLIYSASYRYTGVPSRFSGSGSGTDFTLTISSLQPEDFAV
YYCQQHYITPLTFGQGTKLEIKRGGGGSGGGGSGGGGSQVQLVQSGAEVKKPGASV
KVSCKASGYTFTTAGMQWVRQAPGQGLEWMGWINTHSGVPKYAEDFKGRVTISADT
STSTAYLQLSSLKSEDTAVYYCARSGFGSSYWYFDVWGQGTLVTVSSGAPSGDYKD
DDDKGAAAHHHHHH
(SEQ ID NO: 6) signal sequence (1-23), HT1-11-scFv-LH(28-271), FLAG-His tag (272-294)

# Fig. 32

Amino acid sequence of full-length MHT1007 H chain
MKHLWFFLLLVAAPRWVLSGYLALKHPWPGLAENSGSGGGGSVLVPMAMMASGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK

(SEQ ID NO: 7) signal sequence (1-19), mimotope peptide (20-35), MMP cleavable linker (36-55), antibody heavy chain sequence (56-506)

# Fig. 33

Amino acid sequence of full-length MHT1008 H chain
MKHLWFFLLLVAAPRWVLSGYLALKHPWPGLAENSGSGGGGSGGGGSGGGGSGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
(SEQ ID NO: 8) signal sequence (1-19), mimotope peptide (20-35), uncleavable linker (36-55), antibody heavy chain sequence (56-506)

# Fig. 34

Amino acid sequence of full-length MHT1009 H chain
MKHLWFFLLLVAAPRWVLSGYLALKHPWPGLAENSGSGGSGGSGRSANAILEGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK

(SEQ ID NO: 9) signal sequence (1-19), mimotope peptide (20-35), uPA cleavable linker (36-55), antibody heavy chain sequence (56-506)

# Fig. 35

Amino acid sequence of full-length MHT3002 H chain
MKHLWFFLLLVAAPRWVLSGYLALKHPWPGLAEGSGGGGSGGGGSGPLFAARGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
(SEQ ID NO: 10) signal sequence (1-19), mimotope peptide (20-35), CAPN substrate (47-52) in CAPN cleavable linker (36-55), antibody heavy chain sequence (56-506)

Fig. 36

Amino acid sequence of full-length human CAPN1
SEEIITPVYCTGVSAQVQKQRARELGLGRHENAIKYLGQDYEQLRVRCLQSGTLFR
DEAFPPVPQSLGYKDLGPNSSKTYGIKWKRPTELLSNPQFIVDGATRTDICQGALG
DCWLLAAIASLTLNDTLLHRVVPHGQSFQNGYAGIFHFQLWQFGEWVDVVVDDLLP
IKDGKLVFVHSAEGNEFWSALLEKAYAKVNGSYEALSGGSTSEGFEDFTGGVTEWY
ELRKAPSDLYQIILKALERGSLLGCSIDISSVLDMEAITFKKLVKGHAYSVTGAKQ
VNYRGQVVSLIRMRNPWGEVEWTGAWSDSSSEWNNVDPYERDQLRVKMEDGEFWMS
FRDFMREFTRLEICNLTPDALKSRTIRKWNTTLYEGTWRRGSTAGGCRNYPATFWV
NPQFKIRLDETDDPDDYGDRESGCSFVLALMQKHRRRERRFGRDMETIGFAVYEVP
PELVGQPAVHLKRDFFLANASRARSEQFINLREVSTRFRLPPGEYVVVPSTFEPNK
EGDFVLRFFSEKSAGTVELDDQIQANLPDEQVLSEEEIDENFKALFRQLAGEDMEI
SVKELRTILNRIISKHKDLRTKGFSLESCRSMVNLMDRDGNGKLGLVEFNILWNRI
RNYLSIFRKFDLDKSGSMSAYEMRMAIESAGFKLNKKLYELIITRYSEPDLAVDFD
NFVCCLVRLETMFRFFKTLDTDLDGVVTFDLFKWLQLTMFA

(SEQ ID NO: 11) CAPN sequence (1-713) with the start methionine being removed

Fig. 37

Amino acid sequence of novel CAPN substrate
PLFAAP
(SEQ ID NO: 12) CAPN substrate that is not cleaved by uPA

# Fig. 38

Amino acid sequence of full-length MHT3201 H chain
MKHLWFFLLLVAAPRWVLSGYLALKHPWPGLAEGSGGGGSGGGGSGPLFAAPGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
(SEQ ID NO: 13) signal sequence (1-19), mimotope peptide (20-35), CAPN cleavable linker (36-55), antibody heavy chain sequence (56-506)

# Fig. 39

Amino acid sequence of full-length MHT3202 H chain
MKHLWFFLLLVAAPRWVLSGYLALKHPWPGLAEGSGGGGSGRSAGGPLFAAPGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
(SEQ ID NO: 14) signal sequence (1-19), mimotope peptide (20-35), uPA + CAPN cleavable linker (36-55), antibody heavy chain sequence (56-506)

# Fig. 40

Amino acid sequence of full-length MHT1713 H chain
MKHLWFFLLLVAAPRWVLSGYLALKHPWPGLAEGSGGGGSGRSAGGPLGLWAGGSQ
VQLVQSGAEVKKPGASVKVSCKASGYTFTTAGMQWVRQAPGQGLEWMGWINTHSGV
PKYAEDFKGRVTISADTSTSTAYLQLSSLKSEDTAVYYCARSGFGSSYWYFDVWGQ
GTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCD
KTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSP
GK
(SEQ ID NO: 15) signal sequence (1-19), mimotope peptide (20-35), uPA + MMP cleavable linker (36-55), antibody heavy chain sequence (56-506)

# Fig. 41

Amino acid sequence of full-length MHT3423 H chain
MKHLWFFLLLVAAPRWVLSGYLALKHPWPGLAEGSGGGGSGRSAGGGGGSGGGGSG
GGGSGGGGSQVQLVQSGAEVKKPGASVKVSCKASGYTFTTAGMQWVRQAPGQGLEW
MGWINTHSGVPKYAEDFKGRVTISADTSTSTAYLQLSSLKSEDTAVYYCARSGFGS
SYWYFDVWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKV
DKRVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNH
YTQKSLSLSPGK
(SEQ ID NO: 16) signal sequence (1-19), mimotope peptide (20-35), uPA cleavable linker (36-65), antibody heavy chain sequence (66-516)

Fig. 42

Amino acid sequence of full-length MHT3203 H chain
MKHLWFFLLLVAAPRWVLSGYLALKHPWPGLAEGSGGGGSGRSANAGGPLFAAPGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 17) signal sequence (1-19), mimotope peptide (20-35), uPA + CAPN cleavable linker (36-57), antibody heavy chain sequence (58-508)

# Fig. 43

Amino acid sequence of full-length hM23H1L1 H chain
MKHLWFFLLLVAAPRWVLSQVQLVQSGAEVKKPGASVKVSCKASGYAFSNYLIEWV
RQAPGQGLEWMGVINPGSGVTNYNEKFKGRVTITADTSTSTAYMELSSLRSEDTAV
YYCARAEAWFAYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHKP
SNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHE
ALHNHYTQKSLSLSPGK
(SEQ ID NO: 18) signal sequence (1-19), antibody heavy chain sequence (20-465)

Fig. 44

Amino acid sequence of full-length hM23H1L1 L chain
MVLQTQVFISLLLWISGAYGDIVMTQSPDSLAVSLGERATINCKSSQSLLYSSNQK
NYLAWYQQKPGQPPKLLIYWASTRESGVPDRFSGSGSGTDFTLTISSLQAEDVAVY
YCQRYYGYPWTFGQGTKVEIKRTVAAPSVFIFPPSDEQLKSGTASVVCLLNNFYPR
EAKVQWKVDNALQSGNSQESVTEQDSKDSTYSLSSTLTLSKADYEKHKVYACEVTH
QGLSSPVTKSFNRGEC
(SEQ ID NO: 19) signal sequence (1-20), antibody light chain sequence (21-240)

# Fig. 45

Amino acid sequence of full-length MhM1008 L chain
MVLQTQVFISLLLWISGAYGGENMWNDLDLVPLGILGSGGGGSVLVPMAMMASGGS
DIVMTQSPDSLAVSLGERATINCKSSQSLLYSSNQKNYLAWYQQKPGQPPKLLIYW
ASTRESGVPDRFSGSGSGTDFTLTISSLQAEDVAVYYCQRYYGYPWTFGQGTKVEI
KRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 20) signal sequence (1-20), mimotope peptide (21-36), MMP cleavable linker (37-56), antibody light chain sequence (57-276)

Fig. 46

Amino acid sequence of full-length MhM1013 L chain
MVLQTQVFISLLLWISGAYGGNFYHQYPWPLEILGSGSGGGGSVLVPMAMMASGGS
DIVMTQSPDSLAVSLGERATINCKSSQSLLYSSNQKNYLAWYQQKPGQPPKLLIYW
ASTRESGVPDRFSGSGSGTDFTLTISSLQAEDVAVYYCQRYYGYPWTFGQGTKVEI
KRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 21) signal sequence (1-20), mimotope peptide (21-36), MMP cleavable linker (37-56), antibody light chain sequence (57-276)

# Fig. 47

Amino acid sequence of full-length hM23-M1 H chain
MKHLWFFLLLVAAPRWVLSQVQLVQSGAEVKKPGASVKVSCKASGAAFSNYLIEWV
RQAPGQGLEWMGVINPGSGVTNYNEKFKGRVTITADTSTSTAYMELSSLRSEDTAV
YYCARAEAWFAYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHKP
SNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKE
YKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHE
ALHNHYTQKSLSLSPGK
(SEQ ID NO: 22) signal sequence (1-19), antibody heavy chain sequence (20-465)

Fig. 48

Amino acid sequence of full-length hM23-M1 L chain
MVLQTQVFISLLLWISGAYGDIVMTQSPDSLAVSLGERATINCKSSQSLLYSSNQK
NYLAWYQQKPGQPPKLLIYWASTRESGVPDRFSGSGSGTDFTLTISSLQAEDVAVY
YCQRYYGYPWTFGQGTKVEIKRAVAAPSVFIFPPSDEQLKSGTASVVCLLNNFYPR
EAKVQWKVDNALQSGNSQESVTEQDSKDSTYSLSSTLTLSKADYEKHKVYACEVTH
QGLSSPVTKSFNRGEC
(SEQ ID NO: 23) signal sequence (1-20), antibody light chain sequence (21-240)

Fig. 49

Amino acid sequence of full-length MhM1018-M1 L chain
MVLQTQVFISLLLWISGAYGGNFYHQYPWPLEILGSGSGGGGSGGGGSGGGGSGGS
DIVMTQSPDSLAVSLGERATINCKSSQSLLYSSNQKNYLAWYQQKPGQPPKLLIYW
ASTRESGVPDRFSGSGSGTDFTLTISSLQAEDVAVYYCQRYYGYPWTFGQGTKVEI
KRTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 24) signal sequence (1-20), mimotope peptide (21-36), uncleavable linker (37-56), antibody light chain sequence (57-276)

Fig. 50

Amino acid sequence of full-length MhM1019-M1 L chain
MVLQTQVFISLLLWISGAYGGNFYHQYPWPLEILGSGGGGSGRSANAGGSGGGGSG
GSDIVMTQSPDSLAVSLGERATINCKSSQSLLYSSNQKNYLAWYQQKPGQPPKLLI
YWASTRESGVPDRFSGSGSGTDFTLTISSLQAEDVAVYYCQRYYGYPWTFGQGTKV
EIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO: 25) signal sequence (1-20), mimotope peptide (21-36), uPA cleavable linker (37-58), antibody light chain sequence (59-278)

Fig. 51

Amino acid sequence of full-length MhM1020-M1 L chain
MVLQTQVFISLLLWISGAYGGNFYHQYPWPLEILGSGGGGSGGGGSGGGPLFAAPG
GSDIVMTQSPDSLAVSLGERATINCKSSQSLLYSSNQKNYLAWYQQKPGQPPKLLI
YWASTRESGVPDRFSGSGSGTDFTLTISSLQAEDVAVYYCQRYYGYPWTFGQGTKV
EIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO: 26) signal sequence (1-20), mimotope peptide (21-36), CAPN cleavable linker (37-58), antibody light chain sequence (59-278)

# Fig. 52

Amino acid sequence of full-length MhM1021-M1 L chain
MVLQTQVFISLLLWISGAYGGNFYHQYPWPLEILGSGGGGSGRSANAGGPLFAAPG
GSDIVMTQSPDSLAVSLGERATINCKSSQSLLYSSNQKNYLAWYQQKPGQPPKLLI
YWASTRESGVPDRFSGSGSGTDFTLTISSLQAEDVAVYYCQRYYGYPWTFGQGTKV
EIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO: 27) signal sequence (1-20), mimotope peptide (21-36), uPA + CAPN cleavable linker (37-58), antibody light chain sequence (59-278)

Fig. 53

Amino acid sequence of full-length MhM1022-M1 L chain
MVLQTQVFISLLLWISGAYGGNFYHQYPWPLEILGSGGGGSPLGLAGGGSGGGGSG
GSDIVMTQSPDSLAVSLGERATINCKSSQSLLYSSNQKNYLAWYQQKPGQPPKLLI
YWASTRESGVPDRFSGSGSGTDFTLTISSLQAEDVAVYYCQRYYGYPWTFGQGTKV
EIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO: 28) signal sequence (1-20), mimotope peptide (21-36), MMP cleavable linker (37-58), antibody light chain sequence (59-278)

Fig. 54

Amino acid sequence of full-length MhM1023-M1 L chain
MVLQTQVFISLLLWISGAYGGNFYHQYPWPLEILGSGGGGSPLGLAGGGPLFAAPG
GSDIVMTQSPDSLAVSLGERATINCKSSQSLLYSSNQKNYLAWYQQKPGQPPKLLI
YWASTRESGVPDRFSGSGSGTDFTLTISSLQAEDVAVYYCQRYYGYPWTFGQGTKV
EIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
(SEQ ID NO: 29) signal sequence (1-20), mimotope peptide (21-36), MMP + CAPN cleavable linker (37-58), antibody light chain sequence (59-278)

Fig. 55

Amino acid sequence of full-length Cetuximab H chain
MKHLWFFLLLVAAPRWVLSQVQLKQSGPGLVQPSQSLSITCTVSGFSLTNYGVHWV
RQSPGKGLEWLGVIWSGGNTDYNTPFTSRLSINKDNSKSQVFFKMNSLQSNDTAIY
YCARALTYYDYEFAYWGQGTLVTVSAASTKGPSVFPLAPSSKSTSGGTAALGCLVK
DYFPEPVTVSWNSGALTSGVHTFPAVLQSSGLYSLSSVVTVPSSSLGTQTYICNVN
HKPSNTKVDKRVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFSCSV
MHEALHNHYTQKSLSLSPGK
(SEQ ID NO: 30) signal sequence (1-19), antibody heavy chain sequence (20-468)

Fig. 56

Amino acid sequence of full-length Cetuximab L chain
MVLQTQVFISLLLWISGAYGDILLTQSPVILSVSPGERVSFSCRASQSIGTNIHWY
QQRTNGSPRLLIKYASESISGIPSRFSGSGSGTDFTLSINSVESEDIADYYCQQNN
NWPTTFGAGTKLELKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSP
VTKSFNRGEC
(SEQ ID NO: 31) signal sequence (1-20), antibody light chain sequence (21-234)

# Fig. 57

Amino acid sequence of full-length C3022 H chain
MKHLWFFLLLVAAPRWVLSQVQLVQSGAEVKKPGASVKVSCKASGYTFTGYYMHWV
RQAPGQGLEWMGRINPNSGGTNYAQKFQGRVTMTRDTSISTAYMELSGLRSDDTAV
YYCASGSVRHPWGQGTLVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKPS
NTKVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEA
LHNHYTQKSLSLSPGK
(SEQ ID NO: 32) signal sequence (1-19), antibody heavy chain sequence (20-464)

Fig. 58

Amino acid sequence of full-length C3022 L chain
MVLQTQVFISLLLWISGAYGQPVLTQPPSASGTPGQRVTISCSGSRSNIGGNIVSW
YQQFPGTAPRLLTYADNQRPSGVPDRFSGSKSGTSASLAISGLQSEDEADYYCAAW
DDSLNGVVFGGGTKLTVLGQPKAAPSVTLFPPSSEELQANKATLVCLISDFYPGAV
TVAWKADSSPVKAGVETTTPSKQSNNKYAASSYLSLTPEQWKSHRSYSCQVTHEGS
TVEKTVAPTECS
(SEQ ID NO: 33) signal sequence (1-20), antibody light chain sequence (21-236)

Fig. 59

Amino acid sequence of full-length MCE-2105 L chain
MVLQTQVFISLLLWISGAYGMGLMHMEKSRSWYLSRQGGGGSGRSANAGGPLFAAP
GGSDILLTQSPVILSVSPGERVSFSCRASQSIGTNIHWYQQRTNGSPRLLIKYASE
SISGIPSRFSGSGSGTDFTLSINSVESEDIADYYCQQNNNWPTTFGAGTKLELKRT
VAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
(SEQ ID NO: 34) signal sequence (1-20), mimotope peptide (22-37), uPA + CAPN cleavable linker (38-59), antibody light chain sequence (60-273)

Fig. 60

Amino acid sequence of full-length MC3-9003 H chain
MKHLWFFLLLVAAPRWVLSGETMSVFTRDYFTETPGGGGSGRSANAGGPLFAAPGG
SQVQLVQSGAEVKKPGASVKVSCKASGYTFTGYYMHWVRQAPGQGLEWMGRINPNS
GGTNYAQKFQGRVTMTRDTSISTAYMELSGLRSDDTAVYYCASGSVRHPWGQGTLV
TVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK
(SEQ ID NO: 35) signal sequence (1-19), mimotope peptide (20-35), uPA + CAPN cleavable linker (36-57), antibody heavy chain sequence (58-502)

# Fig. 61

Amino acid sequence recognized by human uPA and serving as a substrate therefor

SGRSANAILE (SEQ ID NO: 36)

SGRSANA (SEQ ID NO: 37)

SGRSA (SEQ ID NO: 38)

Amino acid sequence recognized by human MMP1 and serving as a substrate therefor

VLVPMAMMAS (SEQ ID NO: 39)

PLGLWA (SEQ ID NO: 40)

Amino acid sequence recognized by human MMP9 and serving as a substrate therefor

PLGLAG (SEQ ID NO: 41)

# Fig. 62

Amino acid sequence of full-length MHT1903 H chain
MKHLWFFLLLVAAPRWVLSGYLALKHPWPGLAEGSGGGGSGRSANAGGSGGGGSGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK

(SEQ ID NO: 42) signal sequence (1-19), mimotope peptide (20-35), uPA cleavable linker (36-57), antibody heavy chain sequence (58-508)

Fig. 63

Amino acid sequence of full-length MHT3219 H chain
MKHLWFFLLLVAAPRWVLSGYLALKHPWPGLAEGSGGGGPLFAAPGGSGRSANAGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 43) signal sequence (1-19), mimotope peptide (20-35), uPA + CAPN cleavable linker (36-57), antibody heavy chain sequence (58-508)

Fig. 64

Amino acid sequence of novel CAPN substrate

FVFAMR (SEQ ID NO: 44)

FLFMVR (SEQ ID NO: 45)

FLFTMW (SEQ ID NO: 46)

LVFAVP (SEQ ID NO: 47)

FVFAWR (SEQ ID NO: 48)

FLFARM (SEQ ID NO: 49)

LLFSGR (SEQ ID NO: 50)

LLFGMR (SEQ ID NO: 51)

LFALRW (SEQ ID NO: 52)

LLFASW (SEQ ID NO: 53)

# Fig. 65

Amino acid sequence of full-length MHT5082 H chain
MKHLWFFLLLVAAPRWVLSGYLALKHPWPGLAEGSGGGGSGRSANAGGFVFAMRGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 54) signal sequence (1-19), mimotope peptide (20-35), uPA + CAPN cleavable linker (36-57), antibody heavy chain sequence (58-508)

# Fig. 66

Amino acid sequence of full-length MHT5084 H chain
MKHLWFFLLLVAAPRWVLSGYLALKHPWPGLAEGSGGGGSGRSANAGGFLFMVRGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 55) signal sequence (1-19), mimotope peptide (20-35), uPA + CAPN cleavable linker (36-57), antibody heavy chain sequence (58-508)

# Fig. 67

Amino acid sequence of full-length MHT5085 H chain
MKHLWFFLLLVAAPRWVLSGYLALKHPWPGLAEGSGGGGSGRSANAGGFLFTMWGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK

(SEQ ID NO: 56) signal sequence (1-19), mimotope peptide (20-35), uPA + CAPN cleavable linker (36-57), antibody heavy chain sequence (58-508)

# Fig. 68

Amino acid sequence of full-length MHT5086 H chain
MKHLWFFLLLVAAPRWVLSGYLALKHPWPGLAEGSGGGGSGRSANAGGLVFAVPGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 57) signal sequence (1-19), mimotope peptide (20-35), uPA + CAPN cleavable linker (36-57), antibody heavy chain sequence (58-508)

# Fig. 69

Amino acid sequence of full-length MHT5090 H chain
MKHLWFFLLLVAAPRWVLSGYLALKHPWPGLAEGSGGGGSGRSANAGGFVFAWRGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 58) signal sequence (1-19), mimotope peptide (20-35), uPA + CAPN cleavable linker (36-57), antibody heavy chain sequence (58-508)

# Fig. 70

Amino acid sequence of full-length MHT5091 H chain
MKHLWFFLLLVAAPRWVLSGYLALKHPWPGLAEGSGGGGSGRSANAGGFLFARMGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK

(SEQ ID NO: 59) signal sequence (1-19), mimotope peptide (20-35), uPA + CAPN cleavable linker (36-57), antibody heavy chain sequence (58-508)

Fig. 71

Amino acid sequence of full-length MHT5092 H chain
MKHLWFFLLLVAAPRWVLSGYLALKHPWPGLAEGSGGGGSGRSANAGGLLFSGRGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 60) signal sequence (1-19), mimotope peptide (20-35), uPA + CAPN cleavable linker (36-57), antibody heavy chain sequence (58-508)

# Fig. 72

Amino acid sequence of full-length MHT5093 H chain
MKHLWFFLLLVAAPRWVLSGYLALKHPWPGLAEGSGGGGSGRSANAGGLLFGMRGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK

(SEQ ID NO: 61) signal sequence (1-19), mimotope peptide (20-35), uPA + CAPN cleavable linker (36-57), antibody heavy chain sequence (58-508)

Fig. 73

Amino acid sequence of full-length MHT5094 H chain
MKHLWFFLLLVAAPRWVLSGYLALKHPWPGLAEGSGGGGSGRSANAGGLFALRWGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 62) signal sequence (1-19), mimotope peptide (20-35), uPA + CAPN cleavable linker (36-57), antibody heavy chain sequence (58-508)

# Fig. 74

Amino acid sequence of full-length MHT5095 H chain
MKHLWFFLLLVAAPRWVLSGYLALKHPWPGLAEGSGGGGSGRSANAGGLLFASWGG
SQVQLVQSGAEVKKPGASVKVSCKASGYTFTTAGMQWVRQAPGQGLEWMGWINTHS
GVPKYAEDFKGRVTISADTSTSTAYLQLSSLKSEDTAVYYCARSGFGSSYWYFDVW
GQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLSL
SPGK
(SEQ ID NO: 63) signal sequence (1-19), mimotope peptide (20-35), uPA + CAPN cleavable linker (36-57), antibody heavy chain sequence (58-508)

Fig. 75

Amino acid sequence of MMP linker
GSGGGGSGGGSPLGLWAGGS
(SEQ ID NO: 64) MMP substrate (12-17) as shown in SEQ ID NO: 40

## INTERNATIONAL SEARCH REPORT

International application No.
**PCT/JP2022/043846**

**A. CLASSIFICATION OF SUBJECT MATTER**

***C12N 15/62***(2006.01)i; ***A61K 31/4745***(2006.01)i; ***A61K 39/395***(2006.01)i; ***A61K 45/00***(2006.01)i; ***A61K 47/68***(2017.01)i; ***A61P 35/00***(2006.01)i; ***A61P 43/00***(2006.01)i; ***C07K 7/08***(2006.01)i; ***C07K 16/18***(2006.01)i; ***C07K 19/00***(2006.01)i; ***C12N 9/64***(2006.01)i; ***C12N 15/13***(2006.01)i; ***C12P 21/02***(2006.01)i; ***C40B 40/10***(2006.01)i

FI: C12N15/62 Z ZNA; A61K31/4745; A61K39/395 L; A61K39/395 N; A61K45/00; A61K47/68; A61P35/00; A61P43/00 123; C07K7/08; C07K16/18; C07K19/00; C12N9/64 Z; C12N15/13; C12P21/02 C; C40B40/10

According to International Patent Classification (IPC) or to both national classification and IPC

**B. FIELDS SEARCHED**

Minimum documentation searched (classification system followed by classification symbols)

C12N15/62; A61K31/4745; A61K39/395; A61K45/00; A61K47/68; A61P35/00; A61P43/00; C07K7/08; C07K16/18; C07K19/00; C12N9/64; C12N15/13; C12P21/02; C40B40/10

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN), JSTPlus/JMEDPlus/JST7580 (JDreamIII), Japio-GPG/FX , PubMed

**C. DOCUMENTS CONSIDERED TO BE RELEVANT**

| Category* | Citation of document, with indication, where appropriate, of the relevant passages | Relevant to claim No. |
|---|---|---|
| X | JP 2010-536370 A (THE REGENTS OF THE UNIVERSITY OF CALIFORNIA) 02 December 2010 (2010-12-02) claims 1, 31, 73, 74, 79, paragraph [0232], fig. 1 | 1-10, 13-20, 23, 27-33 |
| Y | claims 1, 31, 73, 74, 79, paragraph [0232], fig. 1 | 1-35 |
| Y | JP 2018-520642 A (GENENTECH, INC) 02 August 2018 (2018-08-02) claims 1-18, 93-97, paragraph [0109], fig. 4D | 1-23, 27-33 |
| A | claims 1-18, 93-97, paragraph [0109], fig. 4D | 24-26, 34-35 |
| Y | WO 2015/146132 A1 (DAIICHI SANKYO CO LTD) 01 October 2015 (2015-10-01) claims 1-37 | 1-23, 27-33 |
| A | claims 1-37 | 24-26, 34-35 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/043846**

**C. DOCUMENTS CONSIDERED TO BE RELEVANT**

| Category* | Citation of document, with indication, where appropriate, of the relevant passages | Relevant to claim No. |
|---|---|---|
| Y | WO 2020/050406 A1 (DAIICHI SANKYO CO LTD) 12 March 2020 (2020-03-12)<br>claims 1-106, paragraph [0059] | 1-23, 27-33 |
| A | claims 1-106, paragraph [0059] | 24-26, 34-35 |
| Y | ITOH, K. et al. Identification of cell proliferation-associated epitope on CD98 oncoprotein using phage display random peptide library. Cancer Science. 2007, vol. 98, no. 11, pp. 1696-1700<br>abstract, p. 1697, right column, 4th paragraph to p. 1698, left column, 1st paragraph, fig. 1 | 24-27, 32-35 |
| A | abstract, p. 1697, right column, 4th paragraph to p. 1698, left column, 1st paragraph, fig. 1 | 1-23, 28-31 |
| A | WO 2016/182064 A1 (CHUGAI SEIYAKU KABUSHIKI KAISHA) 17 November 2016 (2016-11-17)<br>entire text, all drawings | 1-35 |

Form PCT/ISA/210 (second sheet) (January 2015)

**INTERNATIONAL SEARCH REPORT**

International application No.
**PCT/JP2022/043846**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   The "form of an Annex C/ST.25 text file" is replaced with the "form of ST.26."

Form PCT/ISA/210 (continuation of first sheet) (January 2015)

**INTERNATIONAL SEARCH REPORT**
**Information on patent family members**

International application No.
**PCT/JP2022/043846**

| Patent document cited in search report | Publication date (day/month/year) | Patent family member(s) | Publication date (day/month/year) |
|---|---|---|---|
| JP 2010-536370 A | 02 December 2010 | WO 2009/025846 A2<br>claims 1, 31, 73, 74, 79, paragraph [0271], fig. 1<br>US 2009/0304719 A1<br>EP 2190861 A1 | |
| JP 2018-520642 A | 02 August 2018 | WO 2016/179003 A1<br>claims 1-18, 93-97, p. 30, 2nd paragraph, fig. 4D<br>US 2018/0057593 A1<br>EP 3288981 A1 | |
| WO 2015/146132 A1 | 01 October 2015 | (Family: none) | |
| WO 2020/050406 A1 | 12 March 2020 | US 2022/0008549 A1<br>claims 1-114, paragraph [0185]<br>EP 3848054 A1 | |
| WO 2016/182064 A1 | 17 November 2016 | US 2018/0125972 A1<br>entire text, all drawings<br>EP 3296395 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009025846 A
- WO 2010081173 A
- WO 2016014974 A
- WO 2020069398 A
- JP 2021194701 A
- WO 2015098099 A
- WO 2019065964 A
- WO 2015146132 A
- WO 2018147245 A
- WO 2013063702 A
- WO 199627011 A
- WO 2009089004 A
- WO 2014110601 A
- WO 2010151792 A
- WO 199201047 A
- WO 199220791 A
- WO 199306213 A
- WO 199311236 A
- WO 199319172 A
- WO 199501438 A
- WO 199515388 A
- WO 2013147153 A
- WO 199954342 A
- WO 200061739 A
- WO 200231140 A
- JP 2017114763 A
- WO 2007114496 A
- WO 2008017828 A
- WO 2009043922 A
- WO 2009090553 A
- JP 2012092068 A
- WO 2011118804 A
- WO 2013078377 A
- WO 2008144891 A
- WO 2011145744 A
- WO 2011155579 A
- WO 2013077458 A
- WO 2003074566 A
- WO 2011068845 A
- WO 2013068946 A
- US 7999083 B
- WO 2016090329 A
- WO 2001000244 A
- WO 2001000245 A
- EP 137145 A1
- US 4604463 A
- EP 495432 A1
- US 5637770 A
- WO 2014057687 A
- WO 2014061277 A
- WO 2020100954 A
- WO 2013173496 A
- WO 2014130879 A
- WO 2017004330 A
- WO 2017004025 A
- WO 2017020972 A
- WO 2016036804 A
- WO 2015095124 A
- WO 2015052322 A
- WO 2015052534 A
- WO 2016115191 A
- WO 2015052321 A
- WO 2015031693 A
- WO 2011130613 A
- WO 2021202984 A
- WO 2020229982 A
- WO 2020050406 A
- WO 2021177438 A
- WO 2018009916 A
- WO 2019084060 A
- WO 2022050300 A
- WO 2018003983 A
- US 5633275 A
- US RE37180 E
- WO 2013009475 A
- WO 2004038378 A
- WO 2015187677 A
- WO 2017031363 A
- WO 2017031367 A
- WO 2018156815 A
- WO 2019232478 A
- WO 2020205623 A
- WO 2016151458 A
- WO 2020196474 A
- WO 2017010559 A

### Non-patent literature cited in the description

- *Nature Review Drug Discovery,* 2018, vol. 17, 197-223

- *Expert Opin. Biol. Ther.,* 2014, vol. 14 (8), 1049-53
- *ACS Cent. Sci.,* 2021, vol. 7, 724-38
- *Sci. Transl. Med.,* 2013, vol. 5 (1-10), 207
- *Annual Review of Cancer Biology,* 2018, vol. 2, 353-76
- *Scientific Reports,* 2015, vol. 5 (16599), 1-9
- *Anal. Biochem.,* 2007, vol. 366 (2), 197-206
- *Molecular Immunology,* 1986, vol. 23 (7), 709-715
- *Developmental and Comparative Immunology,* 2003, vol. 27, 55-77
- *Annu. Rev. Immunol.,* 1994, vol. 12, 433-455
- **TOMIZUKA, K. et al.** *Nature Genetics,* 1997, vol. 16, 133-143
- **KUROIWA, Y.** *Nuc. Acids Res.,* 1998, vol. 26, 3447-3448
- **YOSHIDA, H.** Animal Cell Technology: Basic and Applied Aspects. Kluwer Academic Publishers, vol. 10, 69-73
- **TOMIZUKA, K.** *Proc. Natl. Acad. Sci., U.S.A.,* 2000, vol. 97, 722-727
- *Journal of Chromatography A,* 1995, vol. 705, 129-134
- *Analytical Biochemistry,* 2007, vol. 360, 75-83
- **LINNENBACH A. J. et al.** *Proc. Natl. Acad. Sci.,* 1989, vol. 86 (1), 27-31
- *Methods Mol. Biol.,* 2013, vol. 1045, 1-27
- *Nature Biotechnology,* 2005, vol. 23, 1137-1146
- **LIANG, X. et al.** *Eur. J. Med. Chem.,* 2019, vol. 171, 129-168
- *Antibodies,* 2013, vol. 2, 270-305
- **SAMBROOK, J. ; FRITSCH, E. F. ; MANIATIS, T.** Molecular Cloning. Cold Spring Harbor Laboratory Press, 1989
- *Protein Science,* 1995, vol. 4 (11), 2411-2423
- *Journal of Controlled Release,* 2012, vol. 161, 804-812
- *Journal of Biological Chemistry,* 1997, vol. 272 (33), 20456-20462
- *Biochimica et Biophysica Acta,* 2009, vol. 1794 (10), 1505-1509
- *Biopolymers,* 1996, vol. 40 (4), 399-416
- *Protein Science,* 1995, vol. 4, 2411-2423
- *Proc. Natl. Acad. Sci., U.S.A.,* 1996, vol. 93 (13), 6687-6692